# EUROPEAN PATENT SPECIFICATION

(11) **EP 1 949 916 B1**
(45) Date of publication and mention of the grant of the patent: **10.08.2011**
(21) Application number: 06254532.2
(22) Date of filing: 31.08.2006
(51) Int. Cl.: A61K 48/00

(54) **Chromatin remodeling agents for increasing expression of PDX-1 and promoting cell differentiation**
Chromatin-remodellierende Substanzen zur Erhöhung der Expression von PDX-1 und Förderung der Zelldifferenzierung
Agents de remodelage de la chromatine pour l'augmentation de l'expression de PDX-1 et pour la stimulation de la différenciation cellulaire

(30) Priority: 31.08.2005 US 713072 P
(43) Date of publication of application: 30.07.2008
(73) Proprietor: LifeScan, Inc., Milpitas, CA 95035-6312 (US)
(72) Inventor: Davis, Janet E., Branchburg, NJ 08876 (US); Fung, Ramie, Princeton, NJ 08540 (US)
(74) Representative: Marshall, Cameron John

(56) References cited:
- WO-A-00/72885
- WO-A-03/103613

## Description

### FIELD OF THE INVENTION

This invention relates to an vitro method for inducing the differentiation of cells. In particular, this invention relates to in vitro methods that induce cells to differentiate into a pancreatic lineage, or into a pancreatic hormone-producing cell, or into a β-cell lineage. This invention also provides methods and compositions for utilizing such cells in the therapeutic treatment of diabetes.

### BACKGROUND

Loss of organ function can result from congenital defects, injury or disease. One example of a disease causing loss of organ function is diabetes mellitus, or diabetes. The majority of diabetes cases fall into two clinical types: Type 1, also known as juvenile-onset diabetes or insulin dependent diabetes mellitus (IDDM); and Type 2, also known as adult-onset diabetes. A common method of treatment of Type 1 diabetes involves the exogenous administration of insulin, typically by injection with either a syringe or a pump. This method does not completely normalize blood glucose levels and is often associated with an increased risk of hypoglycemia. More effective glycemic control can be achieved if the function of the pancreas can be restored or rejuvenated via transplantation or cell-based therapies.

There are many transplantation therapies currently used to treat diabetes: One such treatment involves transplanting isolated islets of Langerhans into the diabetic patient. One challenge to human islet transplantation has been the lack of sufficient number of islets to treat the large number of diabetic patients.

Alternative sources of cellular material for transplantation may include, for example, cells derived from other tissues such as, for example, chorionic villus, amniotic fluid, or bone marrow. These other tissues may be fetal or embryonic tissues. In addition, the endocrine cells of the islets of Langerhans, including β-cells, are constantly turning over by processes of apoptosis and proliferation of new islet cells (neogenesis). As such, the pancreas is thought to be a source of undifferentiated cells that are capable of differentiating into pancreatic hormone producing cells.

However, one challenge of these cellular approaches has been the ability of these cells to differentiate into a β- cell lineage or a pancreatic hormone-secreting cell. Such differentiation involves changes in gene expression.

*Mechanism for cellular differentiation:* Gene expression is the combined process of the transcription of a gene into mRNA, the processing of that mRNA, and its translation into protein (for protein-encoding genes). A comparison of the gene-expression patterns of cells from the pancreas, which secrete digestive enzymes and hormones, and the liver, the site of lipid transport and energy transduction, reveals marked differences in genes that arc highly expressed. This difference is consistent with the physiological roles of these tissues. For example, insulin gene expression in a mammal is restricted to β-cells of the pancreas through control mechanisms mediated in part by specific transcription factors, including MafA, and NeuroD. In other cells of the body, pancreatic hormones, such as, for example, insulin, as well as the other specific peptidase genes are trancriptionally silent.

DNA is never found as a naked molecule in animal or plant cell nuclei. DNA is always packaged into chromatin and found in association with proteins and other molecules. The molecules include, for example, histone proteins (soluble in acid solutions), HMG proteins (soluble in neutral saline), residual proteins (soluble in concentrated urea solutions), phosphoproteins (soluble in basic solutions), RNA species (soluble in aqueous phenol solutions), and lipid species (soluble in chloroform-methanol solutions). Chromatin is that portion of the cell nucleus that contains the entire DNA of the nucleus in animal or plant cells.

Covalent modification of histones has been implicated in regulation of gene expression. Reversible acetylation of histone proteins combines with DNA methylation and other modifications to generate an epigenetic code of altered chromatin structure and function. The covalent modifications to histones may include acetylation, methylation, phosphorylation, ubiquitinylation and sumolylation.

The acetylation state of histones and other proteins is dynamically regulated by the competing actions of acetyltransferases and deacetylases. Hypoacetylated histones promote chromatin condensation and are associated with transcriptionally silent loci, wherein access of the DNA to transcription factors or the transcriptional apparatus is limited. Such alterations to chromatin may play a seminal role in tissue differentiation by determining the complement of genes expressed within individual cell types.

The methylation of DNA at cytosine residues is associated with gene silencing. Treatment with agents that alter this secondary DNA modification to create demethylated sites is correlated with active gene transcription and expression. The drug, 5-azacytidine, may be incorporated into DNA in place of cytidine. As a consequence, the 5' position is blocked, leading to creation and perpetuation of demethylated DNA sequences. Changes in the state of cellular differentiation have been reported after 5'-azacytidine treatment; for example, muscle cells have been induced to develop from non-muscle cell precursors. The drug also has been shown to activate genes on a silent X chromosome.

*Factors that control pancreatic development:* The homeodomain protein PDX-1 (Pancreatic and Duodenal Homeobox gene-1, also known as IDX- 1, IPF-1, STF-1 or IUF-1) plays a central role in regulating pancreatic islet development and function. PDX-1 regulates transcription of these genes associated with β-cell identity, including insulin, glucokinase, islet amyloid polypeptide, and glucose transporter type 2 (GLUT2).

US20050090465 states the ectopic expression of PDX-1 in liver and skin induces a pancreatic islet cell phenotype in liver and skin cells and results in the expression, production, and processing of pancreatic hormones.

US20040002447 provides methods for inducing insulin gene expression in cells. In some embodiments, the methods comprise the steps of: (i) providing a cell that expresses a

PDX-1 polynucleotide; and (ii) contacting the cell with a histone deacetylase inhibitor, thereby inducing insulin gene expression in the cells.

The methods disclosed in US20050090465 and US20040002447 require the ectopic expression of PDX-1 in order to induce insulin gene expression in cells. Thus, there remains a significant need to develop methods to generate pancreatic hormone-secreting cells from an abundant cell source that does not require the ectopic expression of PDX-1.

### SUMMARY

The present invention includes in-vitro methods that promote the differentiation of cells by altering the expression of genes within the cells. In one embodiment, the genes may be required for the differentiation of a desired cell type. Alternatively, the genes may be associated with the function of a desired cell type. In one embodiment, the expression of genes required for the differentiation and the function of a desired cell type may be altered.

The cells to be differentiated may themselves by fully differentiated cells of another cell lineage or type, or they may be partially differentiated progenitor cells, or they may be undifferentiated progenitor cells.

In one embodiment, the present invention is directed to in-vitro methods that cause a cell that does not normally express PDX-1 to differentiate into a cell of the pancreatic lineage, or into a pancreatic hormone-producing cell, or into a cell of the β-cell lineage.

In one embodiment, the present invention is directed to in-vitro methods that cause a cell that has lost the expression of PDX-1 to differentiate into a cell of the pancreatic lineage, or into a pancreatic hormone-producing cell, or into a cell of the β-cell lineage.

In one embodiment, the differentiation of cells may be promoted by contacting the cells with at least one chromatin-remodeling agent. Cells may be contacted with a single treatment of at least one chromatin-remodeling agent. In an alternate embodiment, the cells may be contacted with multiple treatments of the at least one chromatin-remodeling agent. The multiple treatments may be with the same agent, or a different agent.

In one embodiment the at least one chromatin-remodeling agent may be an inhibitor of histone deacetylase activity. The inhibitor of histone deacetylase activity may be selected from the group consisting of butyrates, hydroxamic acids, cyclic peptides and benzamides. In some embodiments, the inhibitor of histone deacetylase activity may be selected from the group consisting of 4-phenylbutyrate, sodium butyrate, trichostatin A, suberoyl anilide hydroxamic acid (SAHA), oxamflatin, trapoxin B, FR901228, apicidin, chlamydocin, depuecin, scriptaid, depsipeptide, and N- acetyldinaline.

In one embodiment, the present invention may be directed to in-vitro methods that promote the differentiation of cells into a pancreatic lineage, or into a pancreatic hormone-producing cell, or into a β-cell lineage.

In one embodiment, differentiation may be promoted by increasing the expression of at least one differentiation-related gene. In one embodiment, the at least one differentiation-related gene may be PDX-1. In an alternate embodiment, the at least one differentiation-related gene may be Sox-17. In an alternate embodiment, the at least one differentiation-related gene may be HNF-3 beta.

In one embodiment, the cells do not normally express the homeodomain protein PDX-1, and treatment with the at least one chromatin-remodeling agent stimulates the expression of PDX-1. In another embodiment, expression of PDX-1 may be restored in cells that have lost the expression of PDX-1 during culture *in vitro*.

In one embodiment, cells may be contacted with multiple treatments of at least one chromatin-remodeling agent. The multiple treatments may stimulate or increase the expression of PDX-1. In an alternate embodiment, the initial treatment with the at least one chromatin-remodeling agent may stimulate or increases the expression of PDX-1, and the subsequent treatments may stimulate the expression of pancreatic hormones. The multiple treatments may be with the same agent or a different agent.

In an alternate embodiment, the initial treatment with the at least one chromatin-remodeling agent may stimulate or increases the expression of PDX-1, and the subsequent treatments with the at least one chromatin-remodeling agent may stimulate the expression of at least one other differentiation-related gene.

### BRIEF DESCRIPTION OF THE DRAWINGS

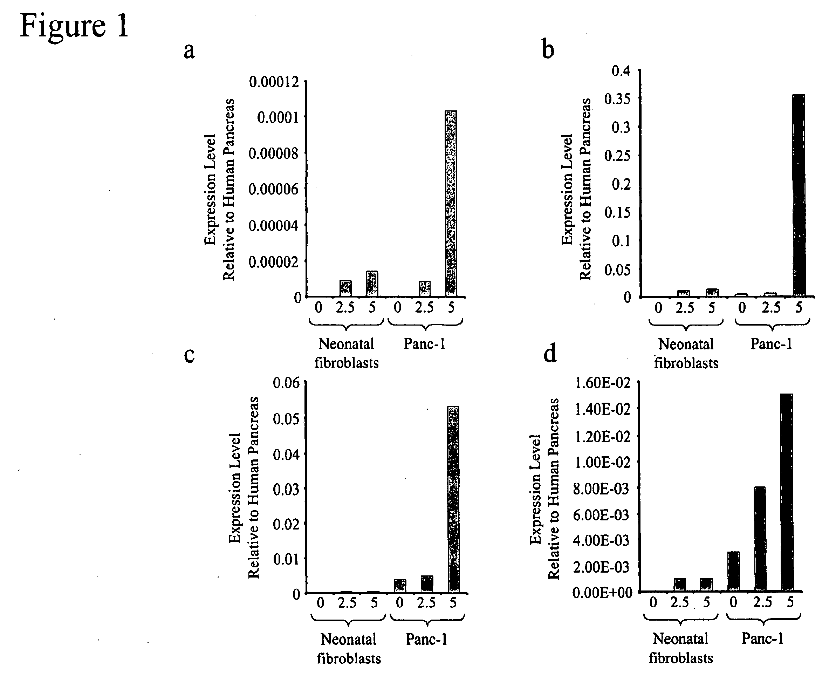

**Figure 1** shows the effects of Histone Deacetylase Inhibitor Treatment on Gene Expression in Panc-1 Cells and Neonatal Fibroblasts. The data shown is the effect of 2.5 µM and 5 µM trichostatin A treatment on the expression of glucagon (panel a), Sox-17 (panel b), Pdx-1 (panel c) and HNF-3 beta (panel d). Untreated cells are shown as a negative control for comparison. The experimental procedure is outlined in **Example 6**.

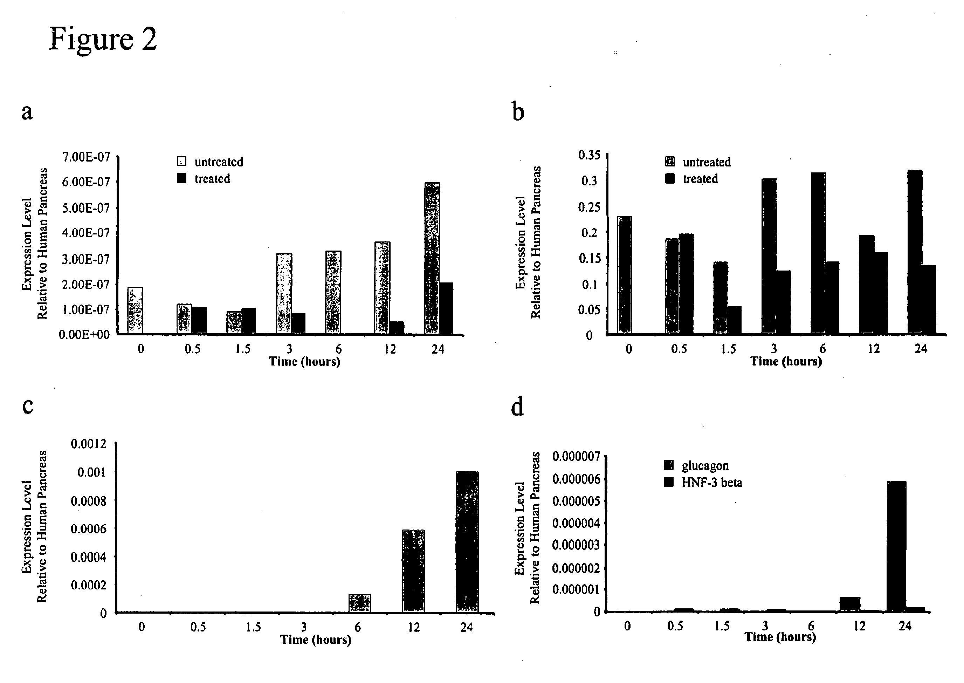

**Figure 2** shows changes in gene expression in amniotic fluid-derived cells over time, following addition of 1.25 µM trichostatin A. Data shown is relative expression of insulin (panel a), Sox-17 (panel b), Pdx-1 (panel c) and HNF-3 beta (panel d) compared to an untreated control. The experimental procedure is outlined in **Example 7**.

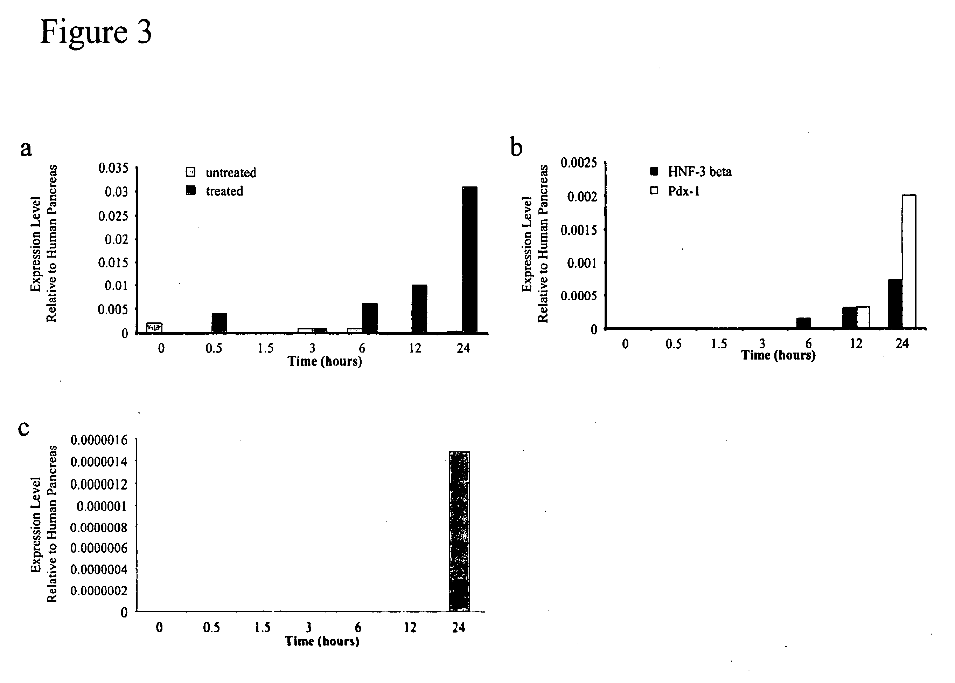

**Figure 3** shows changes in gene expression in late passage pancreatic-derived stromal cells over time, following addition of 2.5 µM trichostatin A. Data shown is relative expression of Sox-17 (panel a), HNF-3 beta and Pdx-1 (panel b), and glucagon (panel c) compared to an untreated control. The experimental procedure is outlined in **Example 8**.

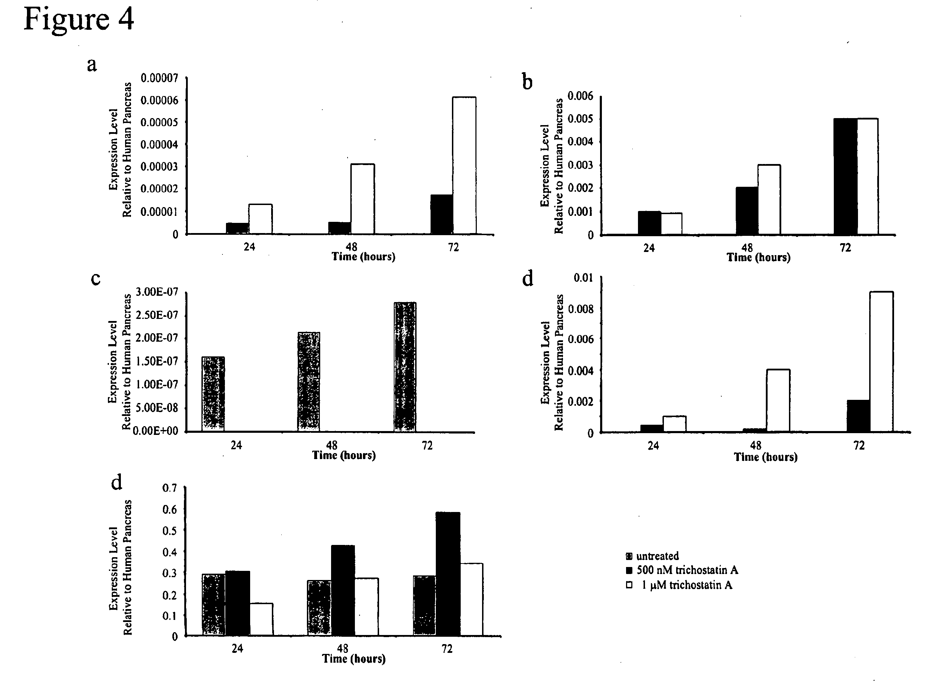

**Figure 4** shows the changes in gene expression in amniotic fluid-derived cells with time, following continuous, chronic treatment with trichostatin A. Data shown is relative expression of glucagon (panel a), HNF-3 beta (panel b), insulin (panel c), Pdx-1 (panel d) and sox-17 (panel e) compared to an untreated control. The experimental procedure is outlined in **Example 9**.

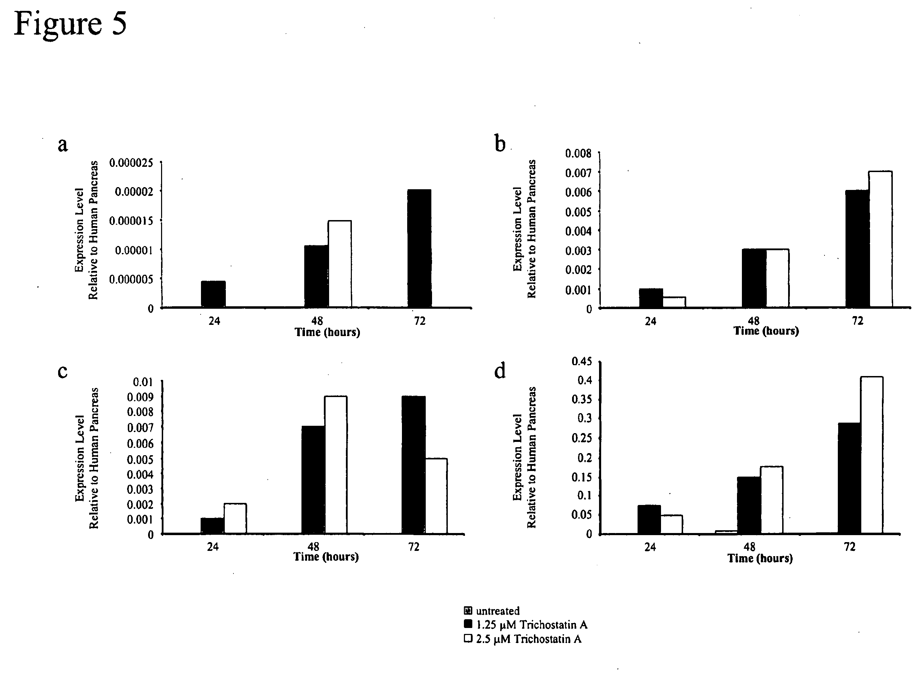

**Figure 5** shows the changes in gene expression in late passage pancreatic-derived stromal cells with time, following continuous, chronic treatment with trichostatin A. Data shown are relative expression of glucagon (panel a), HNF-3 beta (panel b), Pdx-1 (panel c) and sox-17 (panel d) compared to an untreated control. The experimental procedure is outlined in **Example 10**.

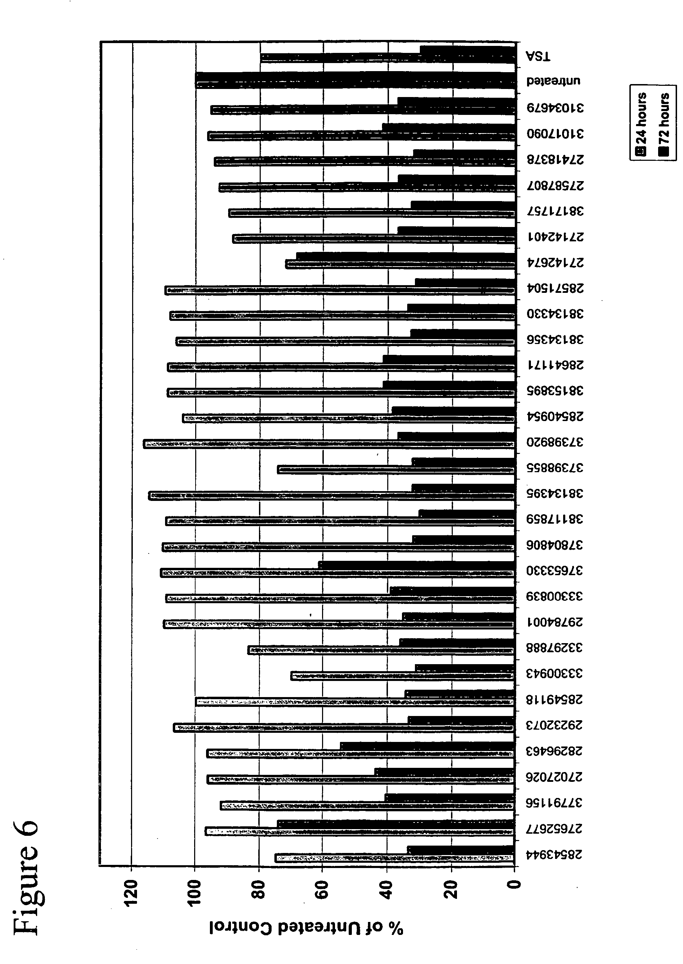

**Figure 6** shows the effects of a selection of HDAC inhibitors on the viability of an amniotic fluid-derived cell line at 24 h and 72 h post addition. The experimental procedure is outlined in **Example 16**. Compounds, identified by the numbers on the x-axis were added to the cells at a concentration of 5µM.

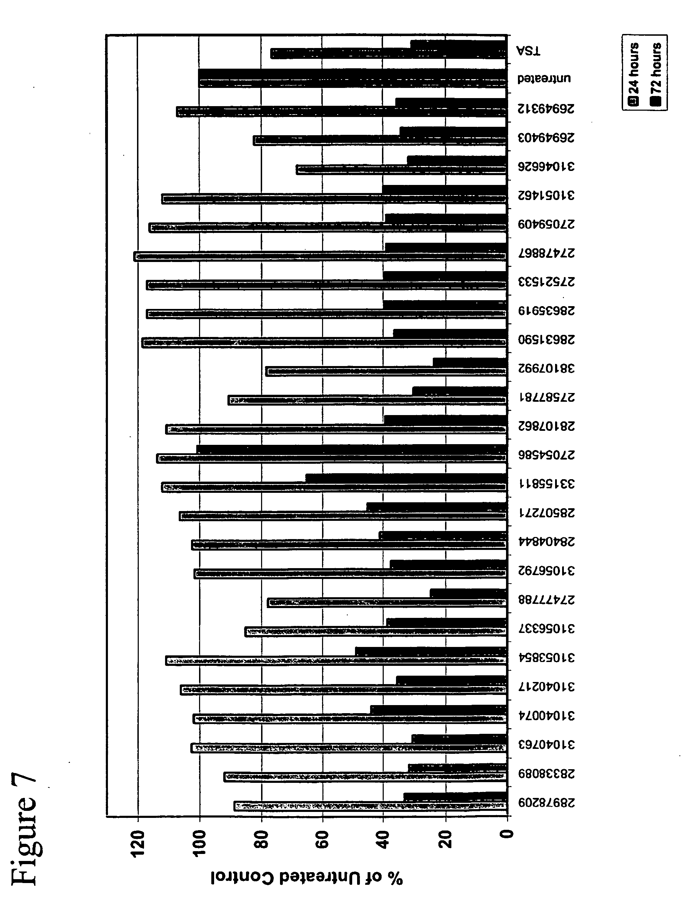

**Figure 7** shows the effects of a selection of HDAC inhibitors on the viability of an amniotic fluid-derived cell line at 24 h and 72 h post addition. The experimental procedure is outlined in **Example 16**. Compounds, identified by the numbers on the x-axis were added to the cells at a concentration of 5µM.

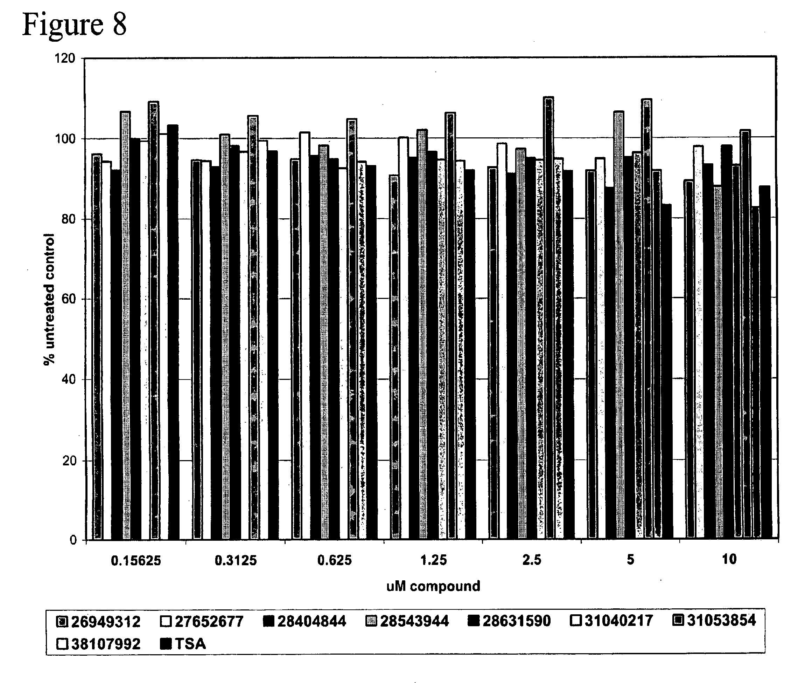

**Figure 8** shows the effects of a selection of HDAC inhibitors on the viability of an amniotic fluid-derived cell line. The experimental procedure is outlined in **Example 16**. Compounds, identified in the legend were added to the cells at the concentrations shown on the x axis. Viability was determined 24h post compound treatment.

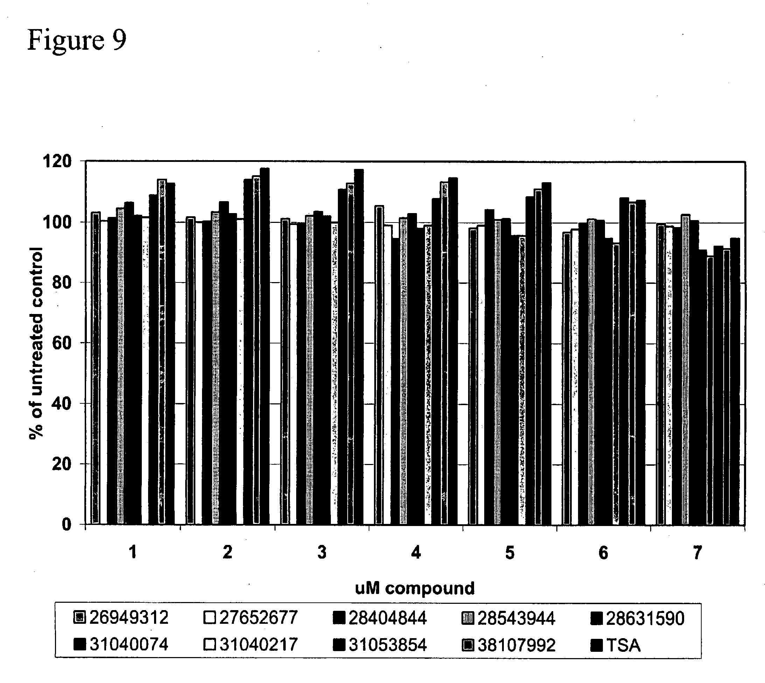

**Figure 9** shows the effects of a selection of HDAC inhibitors on the viability of an adult pancreatic-derived cell line. The experimental procedure is outlined in **Example 16**. Compounds, identified in the legend were added to the cells at the concentrations shown on the x axis. Viability was determined 24h post compound treatment.

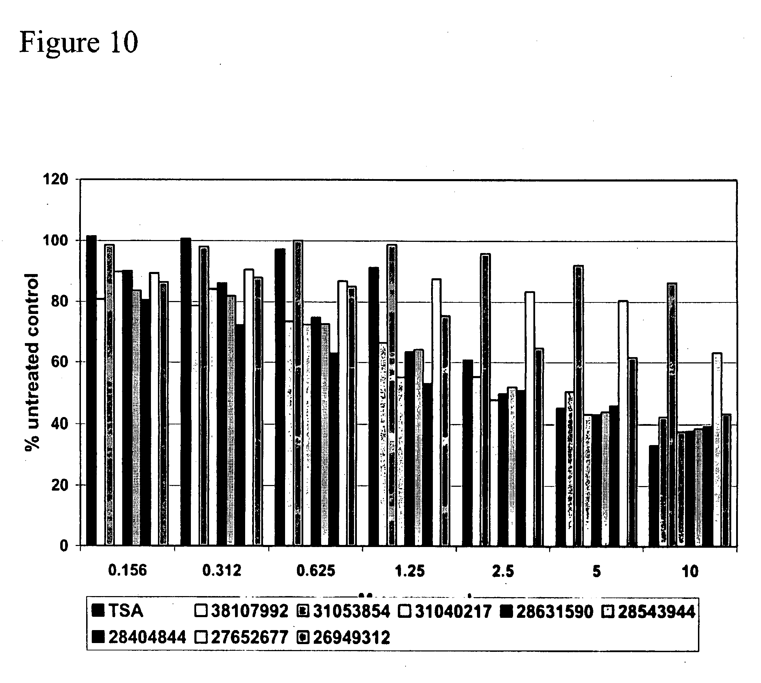

**Figure 10** shows the effects of a selection of HDAC inhibitors on the viability of an amniotic fluid-derived cell line. The experimental procedure is outlined in **Example 16**. Compounds, identified in the legend were added to the cells at the concentrations shown on the x axis. Viability was determined 72h post compound treatment.

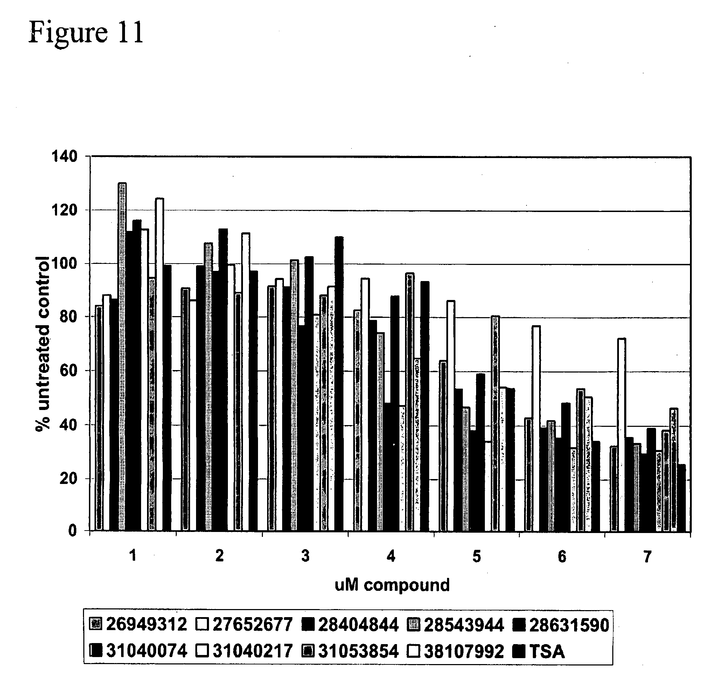

**Figure 11** shows the effects of a selection of HDAC inhibitors on the viability of an adult pancreatic-derived cell line. The experimental procedure is outlined in **Example 16**. Compounds, identified in the legend were added to the cells at the concentrations shown on the x axis. Viability was determined 72h post treatment.

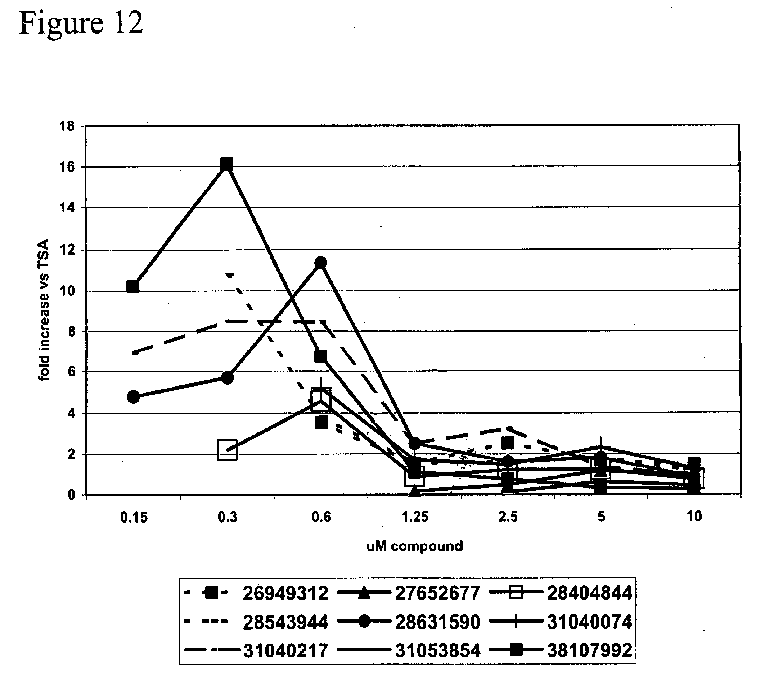

**Figure 12** shows the effects of a selection of putative HDAC inhibitor compounds on HDAC enzyme activity. Compounds, identified in the legend were incubated with

HDAC enzyme at the concentrations shown. Results were normalized to results observed with TSA.

### DETAILED DESCRIPTION OF THE INVENTION

For clarity of disclosure, and not by way of limitation, the detailed description of the invention is divided into the following subsections that describe or illustrate certain features, embodiments or applications of the present invention.

### Cells useful in the present invention.

Cells suitable for use in the methods of the present invention may be obtained from tissues such as, for example, bone marrow, umbilical cord blood, amniotic sac, amniotic fluid, placenta, skin, fat, muscle, vasculature, liver, pancreas, or peripheral blood, using methods that are well known in the art. The cells may be fully differentiated, or they may be partially differentiated or undifferentiated cells. The cells may be stem cells of progenitor cells.

Differentiation is the process by which an unspecialized ("uncommitted") or less specialized cell acquires the features of a specialized cell, such as a nerve cell or a muscle cell, for example. A differentiated cell is one that has taken on a more specialized ("committed") position within the lineage of a cell. The term committed, when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. De-differentiation refers to the process by which a cell reverts to a less specialized (or committed) position within the lineage of a cell. As used herein, the lineage of a cell defines the heredity of the cell, i.e. which cells it came from and what cells it can give rise to. The lineage of a cell places the cell within a hereditary scheme of development and differentiation.

A progenitor cell is a cell that has the capacity to create progeny that are more differentiated than itself, yet retains the capacity to replenish pool of progenitors. By that definition, stem cells themselves are also progenitor cells, as are the more immediate precursors to terminally differentiated cells.

Isolation of a population of cells may be achieved using monoclonal antibodies specific proteins expressed on the surface of the cells. The monoclonal antibodies may be adhered to substrate to facilitate the separation of the bound cells. The methods that may be used to isolate cells suitable for use in the present invention may be chosen by one of ordinary skill in the art. Examples of such methods are taught in US6087113, US6261549, US5914262, US5908782, and US20040058412.

Cells may be characterized, for example, by growth characteristics (e.g., population doubling capability, doubling time, passages to senescence), karyotype analysis (e.g., normal karyotype; maternal or neonatal lineage), flow cytometry (e.g., FACS analysis), immunohistochemistry and/or immunocytochemistry (e.g., for detection of epitopes), gene expression profiling (e.g., gene chip arrays; polymerase chain reaction (for example, reverse transcriptase PCR, real time PCR, and conventional PCR)), protein arrays, protein secretion (e.g., by plasma clotting assay or analysis of conditioned medium, for example, by Enzyme Linked Immuno-Sorbent Assay (ELISA)), mixed lymphocyte reaction (e.g., as measure of stimulation of PBMCs), and/or other methods known in the art.

Cells suitable for use in the methods of the present invention may also include cells obtained from commercial sources, such as, for example human mesenchymal stem cells sold under the trade name POIETICS™ (Cat. No PT-2501, Cambrex). These mesenchymal stem cells are positive for the expression of the following markers: CD29, CD44, CD 105 and CD 166. The cells are negative for the expression of the markers CD14, CD34 and CD45.

In one aspect of the present invention, the cells may be pancreatic-derived stromal cells. These cells may be isolated by a multi-stage method, which is described in **Example 1**. Alternatively, they may be isolated by any suitable method known to those of skill in the art. Examples of suitable isolation methods are taught in US2003/0082155, US5,834,308, US6,001,647, US6,703,017, US6,815,203, WO2004/011621.

In one aspect of the present invention, the cells may be amniotic fluid-derived cells. These cells may be isolated by a multi-stage method that is described in detail in **Example 2.** Alternatively, they may be isolated by any suitable method known to those of skill in the art. Examples of suitable isolation methods are taught in W02003/042405, US2005/0054093, in't Anker et al, Blood 102, 1548-1549, 2003, Tsai et al, Human Reproduction 19, 1450-1456, 2004.

Isolated cells, or tissue from which cells are obtained may be used to initiate, or seed, cell cultures. Isolated cells may be transferred to sterile tissue culture vessels, either uncoated or coated with extracellular matrix or ligands such as laminin, collagen (native, denatured or crosslinked), gelatin, fibronectin, and other extracellular matrix proteins. Cells may be cultured in any culture medium capable of sustaining growth of the cells such as, for example, DMEM (high or low glucose), advanced DMEM, DMEM/MCDB 201, Eagle's basal medium, Ham's F 10 medium (F10), Ham's F-12 medium (F12), Iscove's modified Dulbecco's - 17 medium, Mesenchymal Stem Cell Growth Medium (MSCGM), DMEM/F12, RPMI 1640, and CELL-GRO-FREE. The culture medium may be supplemented with one or more components including, for example, fetal bovine serum (FBS); equine serum (ES); human serum (HS); beta- mercaptoethanol (BME or 2-ME); one or more growth factors, for example, platelet-derived growth factor (PDGF), epidermal growth factor (EGF), fibroblast growth factor (FGF), vascular endothelial growth factor (VEGF), insulin-like growth factor-1 (IGF-1), leukocyte inhibitory factor (LIF) and erythropoietin; amino acids, including L-valine; and one or more antibiotic and/or antimycotic agents to control microbial contamination, such as, for example, penicillin G. streptomycin sulfate, amphotericin B. gentamicin, and nystatin, either alone or in combination. The cells may be seeded in culture vessels at a density to allow cell growth.

Methods for the selection of the most appropriate culture medium, medium preparation, and cell culture techniques are well known in the art and are described in a variety of sources, including Doyle et al., (eds.), 1995, CELL &TISSUE CULTURE: LABORATORY PROCEDURES, John Wiley &Sons, Chichester; and Ho and Wang (eds.), 1991, ANIMAL CELL BIOREACTORS, Butterworth-Heinemann, Boston.

Cells suitable for use in the present invention may be expanded by culturing in a defined growth media containing at least one factor that stimulates the proliferation of the cells. The at least one factor may include, for example, nicotinamide, members of TGF-β family, including TGF-β 1, 2, and 3, bone morphogenic proteins (BMP-2, -4, 6, -7, -11, - 12, and -13), serum albumin, fibroblast growth factor family, platelet-derived growth factor-AA, and -BB, platelet rich plasma, insulin growth factor (IGF-I, II) growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as triethylene pentamine, TGF-β, forskolin, sodium butyrate, activin, betacellulin, noggin, neuron growth factor, nodal, insulin/transferring/selenium (ITS), hepatocyte growth factor (HGF), keratinocyte growth factor (KGF), bovine pituitary extract, islet neogenesis-associated protein (INGAP), proteasome inhibitors, notch pathway inhibitors, sonic hedgehog inhibitors, or combinations thereof. Alternatively, cells suitable for use in the present invention may be expanded by culturing in conditioned media. By "conditioned media" is meant that a population of cells is grown in a basic defined cell culture medium and contributes soluble factors to the medium. In one such use, the cells are removed from the medium, while the soluble factors the cells produce remain. This medium is then used to nourish a different population of cells.

### Characterization of The Cells of the Present Invention

Methods for assessing expression of genes, via protein or nucleic acid levels in cultured or isolated cells are standard in the art. These include real-time polymerase chain reaction (RT-PCR), see, for example, the methods described in **Example 3**, Northern blots, *in situ* hybridization (see, e.g., Current Protocols in Molecular Biology (Ausubel et al., eds. 2001 supplement)), Western blotting, and for markers that are accessible in intact cells, flow cytometry analysis (FACS) (see, e.g., Harlow and Lane, Using Antibodies: A Laboratory Manual, New York: Cold Spring Harbor Laboratory Press (1998), and the methods described in **Example 4**) and immunoassays, such as immunohistochemical analysis of sectioned material (See, for example, the methods described in **Example 5**).

Examples of antibodies useful for detecting certain protein markers are listed in **Table I A&B.** It should be noted that other antibodies directed to the same markers that are recognized by the antibodies listed in **Table I A&B** are available, or can be readily developed. Such other antibodies can also be employed for assessing expression of markers in the cells isolated in accordance with the present invention.

Characteristics of cells of the β-cell lineage are well known to those skilled in the art, and additional characteristics of the β-cell lineage continue to be identified. These characteristics can be used to confirm that the amniotic fluid-derived cells isolated in accordance with the present invention have differentiated to acquire the properties characteristic of the β-cell lineage. β-cell lineage specific characteristics include the expression of one or more transcription factors such as, for example, PDX-1 (pancreatic and duodenal homeobox gene-1), NGN-3 (neurogenin-3), Hlxb9, Nkx6, Isl1, Pax6, NeuroD, Hnfla, Hnf6, Hnf3 Beta, and Mafa, among others. These transcription factors are well established in the art for identification of endocrine cells. See, for example, Edlund (Nature Reviews Genetics 3: 524-632 (2002)).

### Differentiation Of Cells by Chromatin-Remodeling.

Differentiation of the cells useful in the present invention may be achieved by altering the expression of genes within the cells. This may be achieved by treating the cells with at least one agent that remodels the chromatin structure within the cells, such that a region of DNA containing active or potentially active genes is more loosely packaged, less condensed, and can be accessed for transcription. Ideally, treatment of the cells with the at least one agent that remodels the chromatin structure within the cells should result in the alteration of gene expression without being toxic to the cell, or triggering an adverse reaction, such as, for example, cell death. One of skill in the art can readily determine such toxic or adverse events and select an agent that remodels the chromatin structure within the cells where such events are minimized.

In one embodiment, the cells are part of an *in vitro* cell culture. Generally, a cell, such as a stem cell, or a progenitor cell in culture, may be contacted with an amount of at least one chromatin-remodeling agent effective to alter the expression of genes within the cell.

Cells treated with a chromatin-remodeling agent may exhibit global changes in gene expression not restricted to any single gene or family of genes. The outcome may down-regulate some genes, up-regulate others, and may leave still other genes unchanged depending on the cell type, its differentiation stage, and responses over time to both the treatment protocol and environmental or other stimulatory signals, such as, for example, cell division.

Further complexity may arise where chromatin-remodeling agents alter expression of genes that themselves regulate other downstream genes, for example transcription factor genes. Finally, chromatin-remodeling agents may not affect all gene regulatory domains in an equivalent manner and therefore may not yield full gene expression commiserate with a fully differentiated cell. For example, a chromatin-remodeling agent may not alter to the same degree gene enhancer regions, which operate bi-directionally at variable distances from promoter regions, and as a consequence, a gene may be turned on without achieving full expression.

The genes whose expression levels are altered by treatment with chromatin-remodeling agents may be required for the differentiation of a desired cell type, herein referred to as "differentiation-related" genes. Alternatively, the genes may be associated with the function of a desired cell type. The function may include, for example, secretion of insulin in the case of a β-cell. The chromatin-remodeling agents may simultaneously affect the expression of differentiation-related genes and genes associated with the function of a desired cell type.

Chromatin remodeling may be achieved by direct covalent modification of histones. The covalent modification may be by acetylation, methylation, phosphorylation, ubiquitinylation, and sumolylation. The covalent modification may be achieved by adding at least one chromatin-remodeling agent that stimulates one or all of these modifications. Alternatively, the at least one chromatin-remodeling agent may inhibit one or more of these modifications.

In one embodiment, method of the present invention essentially involves:
- Isolating a population of cells,
- Contacting the population of cells with at least one chromatin-remodeling agent,
- Determining the changes in gene expression of the population of cells,
- Culturing the treated population of cells *in vitro.*

In one embodiment, the method of the present invention essentially involves:
- Isolating a population of cells that does not express PDX-1 or has lost the expression of PDX-1 during culture *in vitro*,
- Contacting the population of cells with at least one chromatin-remodeling agent,
- Determining subsequent the changes in gene expression of the population of cells,
- Culturing the treated population of cells *in vitro*.

Changes in gene expression may by up-regulation, or by down-regulation, or a combination. The at least one chromatin-remodeling agent may affect the expression level of one gene, or multiple genes, to the same, or a different extent. The at least one chromatin-remodeling agent may affect the expression level of one gene, or multiple genes and may not affect the expression levels of other genes.

The cells may require one or more than one treatment of the at least one chromatin-remodeling agent. The more than one treatment may be with the same chromatin-remodeling agent or a different chromatin-remodeling agent.

The concentration of the at least one chromatin-remodeling agent may vary depending on the cell used, the choice of chromatin-remodeling agent or agents, the gene or genes whose expression levels are to be altered, the culture conditions, and the like. The at least one chromatin-remodeling agent may be contacted with the cells for up to about 72 hours, or up to about 48 hours, or up to about 24 hours, or up to about 12 hours, or up to about 6 hours, or up to about 4 hours, or up to about 2 hours, or up to about 1 hour.

Cells treated with at the at least one chromatin-remodeling agent may be treated with at least one other factor to promote the differentiation of the cells into a specific cell type. Factors may include, for example, nicotinamide, members of the TGF-β family, including TGF-β1, 2, and 3, bone morphogenic proteins (BMP-2, -4, 6, -7, -11, -12, and-13), serum albumin, fibroblast growth factor family members, platelet-derived growth factor-AA, and -BB, platelet rich plasma, insulin growth factor (IGF-I, -II) growth differentiation factor (GDF-5, -6, -8, -10, -11), glucagon like peptide-I and -II (GLP-I and -II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as triethylene pentamine, forskolin, sodium-Butyrate, activin, betacellulin, ITS, noggin, neurite growth factor, nodal, hepatocyte growth factor (HGF), keratinocyte growth factor (KGF), bovine pituitary extract, islet neogenesis-associated protein (INGAP), proteasome inhibitors, notch pathway inhibitors, sonic hedgehog inhibitors, or combinations thereof.

The combination and concentrations of growth factors, the length of culture, and other culture conditions can be optimized by those skilled in the art to achieve effective differentiation by, for example, monitoring the percentage of cells that have differentiated into cells characteristic of the desired lineage. The one or more growth factors may be added in an amount sufficient to induce the differentiation of the cells of the present invention into cells bearing markers of a β-cell lineage over a time period of about one to four weeks.

### Chromatin-Remodeling Agents.

An aspect of the present invention, the chromatin-remodeling agent is a modulator of histone deacetylase activity. "Histone deacetylase" refers to enzymes that remove acetyl groups from histones. The modulator of histone deacetylase activity may enhance the activity of histone deacetylase enzymes, or it may inhibit the activity of histone deacetylase enzymes.

In one aspect, the inhibitor of histone deacetylase activity may be a delta dicarbonyl compound, such as, for example, compounds disclosed in European Patent Application EP 1216986, having the general formula:

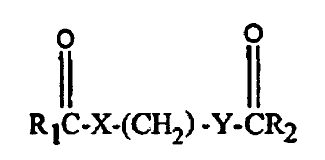

Wherein X is selected from the group consisting of oxygen, sulfur and N(R); wherein Y is selected from the group consisting of sulfur, N(R), and CH₂; wherein R is either H or CH₃; wherein R₁ and R₂ are the same or different and have the general formula:

**-(CH₂)ₒ-(R₃)ₚ-(CH₂)_{q}-(R₄)ᵣ-(CH₂)ₛ-Z**

Wherein R₃ and R₄ are the same or different and are selected from the group (CH=CH), (C≡C), sulfur and oxygen; wherein Z is selected from the group consisting of hydrogen and substituted or unsubstituted aryl, heteroaryl, cycloalkyl having the general formula CₙH₂ₙ-1 and alkoxy; wherein n is 3 or greater; and wherein o, p, q, r and s are the same or different and are each between 0 and 10.

In one aspect, the inhibitor of histone deacetylase activity may be a hydroxamate compound, such as, for example, compounds disclosed in WO0222577, having the general formula:

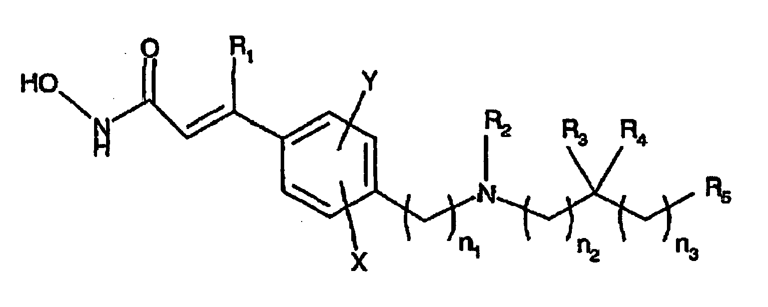

Wherein R₁ is H, halo, or a straight chain C₁-C₆ alkyl; R₂ is selected from H, C₁-C₁₀ alkyl, C₄-C₉ cycloalkyl, C₄-C₉ heterocycloalkyl, C₄-C₉ heterocycloalkylalkyl, cycloalkylalkyl, aryl, heteroaryl, arylalkyl, heteroarylalkyl, -(CH₂)ₙC(O)R₆, - (CH₂)ₙOC(O)R₆, amino acyl, HON-C(O)- CH=C(R₁)-aryl-alkyl- and -(CH₂)ₙR₇; R₃ and R₄ are the same or different and independently H, C₁-C₆ alkyl, acyl or acylamino, or R₃ and R₄ together with the carbon to which they are bound represent C=Q C=S, or C=NR₈, or R₂ together with the nitrogen to which it is bound and R₃ together with the carbon to which it is bound can form a C₄-C₉ heterocycloalkyl, a heteroaryl, a polyheteroaryl, a non-aromatic polyheterocycle, or a mixed aryl and non-aryl polyheterocycle ring; R₅ is selected from H, C₁-C₆ alkyl, C₄-C₉ cycloalkyl, C₄-C₉ heterocycloalkyl, acyl, aryl, heteroaryl, arylalkyl, heteroarylalkyl, aromatic polycycle, non-aromatic polycycle, mixed aryl and non-aryl polycycle, polyheteroaryl, non-aromatic polyheterocycle, and mixed aryl and non-aryl polyheterocycle; n, n₁, n₂ and n₃ are the same or different and independently selected from 0 - 6, when n₁ is 1-6, each carbon atom can be optionally and independently substituted with R₃ and/or R₄; X and Y are the same or different and independently selected from H, halo, C₁-C₄ alkyl, NO₂, C(O)R₁, OR₉, SR₉, CN, and N1R₁₀R₁₁; R₆ is selected from H, C₁-C₆ alkyl, C₄-C₉ cycloalkyl, C₄-C₉ heterocycloalkyl, cycloalkylalkyl, aryl, heteroaryl, arylalkyl, heteroarylalkyl, OR₁₂, and NR₁₃R₁₄; R₇ is selected from OR₁₅, SR₁₅, S(O)R₁₆, SO₂R₁₇, NR₁₃R₁₄, and NR₁₂SO₂R₆; R₈ is selected from H, OR₁₅, NR₁₃R₁₄, C₁-C₆ alkyl, C₄-C₉ cycloalkyl, C₄-C₉ heterocycloalkyl, aryl, heteroaryl, arylalkyl, and heteroarylalkyl; R₉ is selected from C₁-C₄ alkyl and C(O)-alkyl; R₁₀ and R₁₁ are the same or different and independently selected from H, C₁-C₄ alkyl, and -C(O)-alkyl; R₁₂ is selected from H, C₁-C₆ alkyl, C₄-C₉ cycloalkyl, C₄-C₉ heterocycloalkyl, C₄-C₉ heterocycloalkylalkyl, aryl, mixed aryl and non-aryl polycycle, heteroaryl, arylalkyl, and heteroarylalkyl; R₁₃ and R₁₄ are the same or different and independently selected from H, C₁-C₆, alkyl, C₄-C₉ cycloalkyl, C₄-C₉ heterocycloalkyl, aryl, heteroaryl, arylalkyl, heteroarylalkyl, amino acyl, or R₁₃ and R₁₄ together with the nitrogen to which they are bound are C₄-C₉ heterocycloalkyl, heteroaryl, polyheteroaryl, non-aromatic polyheterocycle or mixed aryl and non-aryl polyheterocycle; R₁₅ is selected from H, C₁-C₆ alkyl, C₄-C₉ cycloalkyl, C₄-C₉ heterocycloalkyl, aryl, heteroaryl, arylalkyl, heteroarylalkyl and (CH₂)ₘZ1R₁₂; R₁₆ is selected from C₁-C₆ alkyl, C₄-C₉ cycloalkyl, C₄-C₉ heterocycloalkyl, aryl, heteroaryl, polyheteroaryl, arylalkyl, heteroarylalkyl and (CH₂)ₘZ1R₁₂; R₁₇ is selected from C₁-C₆ alkyl, C₄-C₉ cycloalkyl, C₄-C₉ heterocycloalkyl, aryl, aromatic polycycle, heteroaryl, arylalkyl, heteroarylalkyl, polyheteroaryl and NR₁₃R₁₄; m is an integer selected from 0 to 6; and Z is selected from O, NR₁₃, S and S(O); or a pharmaceutically acceptable salt thereof.

In one aspect, the inhibitor of histone deacetylase activity may be a cyclic tetrapeptide compound, such as, for example, compounds disclosed in WO0021979, having the general formula:

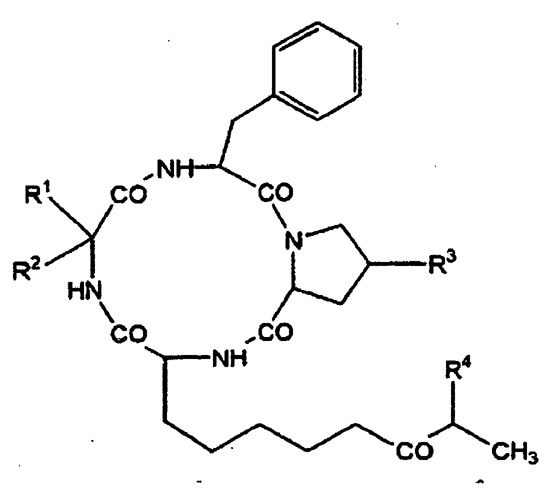

Wherein R1 is methyl, W is methyl or ethyl, W is hydrogen or methyl and W is hydroxy optionally having a hydroxy-protective group, providing that when W is hydrogen, W is ethyl.

In one aspect, the inhibitor of histone deacetylase activity may be a depsipeptide compound, such as, for example, compounds disclosed in WO0142282, having the general formula:

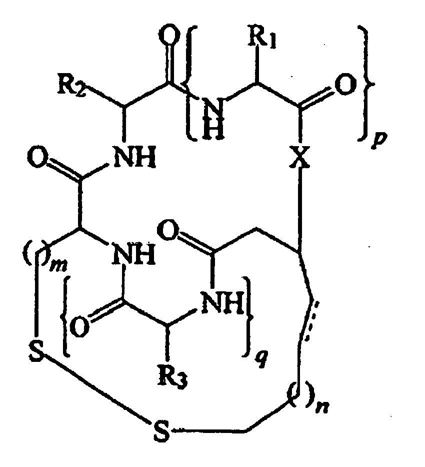

Wherein m is 1, 2, 3 or 4; n is 0, 1, 2 or 3; p and q are independently 1 or 2; X is O, NH or NR; R₁, R₂, and R₃ are the same or different and independently an amino acid side-chain moiety or an amino acid side-chain derivative; and R is a lower chain alkyl, aryl or arylalkyl moiety, with the proviso that the compound is not FR901228.

In one aspect, the inhibitor of histone deacetylase activity may be 6-(1,3-Dioxo-1H, 3H-benzo[de]isoquinolin-2-yl)-hexanoic acid hydroxyamide, termed "scriptaid", as disclosed in WO0149290.

In one aspect, the inhibitor of histone deacetylase activity may be compounds having the general formula:

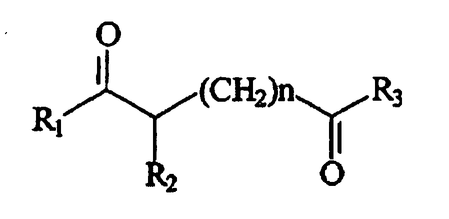

Wherein R₁, and R₂ are the same or different and are each a hydrophobic moiety; wherein R₃ is a hydroxamic acid, hydroxylamino, hydroxyl, amino, alkylamino, or alkyloxy group; and n is an integer from 3 to 10, or a pharmaceutically acceptable salt thereof, such as, for example, compounds disclosed in WO0118171.

In one aspect, the inhibitor of histone deacetylase activity may be a tricyclic alkylhydroxamate compound, such as, for example, compounds disclosed in WO2002085883, having the general formula:

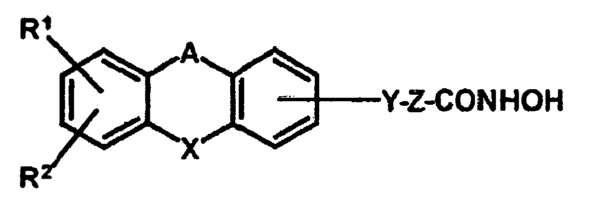

Wherein A denotes a bond, the groups -CH₂-O-, -CH₂-S-, -CH₂-CH₂-, or - NH-CO-; X denotes the group -NR³-, =CO, or -CH(OH,)-; Y denotes an oxygen atom, a sulfur atom, or the group -NR⁴-; Z denotes a straight chain alkylene group comprising 4, 5, 6, 7, or 8 carbon atoms, wherein one CH₂ group may be replaced by an oxygen or a sulfur atom, or wherein 2 carbon atoms form a C=C double bond, and which is either unsubstituted or substituted by one or two substituents selected from (₁-₄C)alkyl and halogen atoms; R¹ and R² denote substituents independently selected from a hydrogen atom, halogen atoms, (₁-₄C)alkyl, trifluoromethyl, hydroxy, (₁-₄C)alkoxy, benzyloxy, (₁-₃C)alkylenedioxy, nitro, amino, (₁-₄C) alkylamino, di[(₁-₄C)alkyl]-amino, or (₁-₄C)alkanoylamino groups; R³ and R⁴ independently denote hydrogen atoms or (₁-₄C)alkyl groups; their enantiomers, diastereoisomers, racemates and mixtures thereof.

In one aspect, the inhibitor of histone deacetylase activity may be a tricyclic lactam or sultam derivative, such as, for example, compounds disclosed in W02002062773, having the general formula:

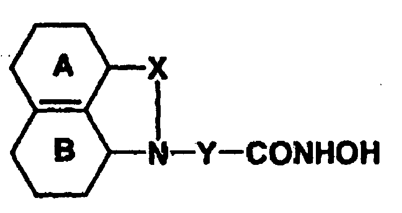

Wherein 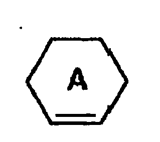 denotes a cyclohexenyl group or a phenyl group, 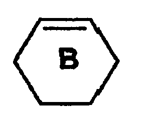 denotes a cyclohexenyl or a phenyl group which may be unsubstituted or substituted by one or more substituents independently selected from a halogen atom, a nitro group, an amino group, an (₁-₄C)alkylamino group, a di[(₁-₄C) alkyl]-amino group, or an (₁-₄C)alkanoylamino group, X is a carbonyl group or a sulfonyl group, Y is a straight chain alkylene group comprising 5, 6, or 7 carbon atoms, wherein one CH₂ group may be replaced by an oxygen or a sulfur atom, or wherein 2 carbon atoms form a C=C double bond, and which is either unsubstituted or substituted by one or two substituents selected from (₁-₄C)alkyl and halogen atoms, their enantiomers, diastereoisomers, racemates and mixtures thereof and pharmaceutically acceptable salts.

In one aspect, the inhibitor of histone deacetylase activity may be tetrahydropyridine derivative, such as, for example, compounds disclosed in WO2002051842, having the general formula:

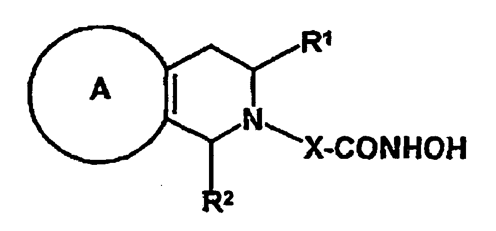

Wherein

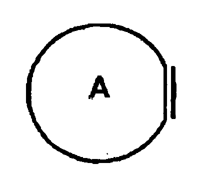

Denotes (a) a phenyl group which may be unsubstituted or substituted with 1, 2 or 3 substituents independently selected from a halogen atom, an (1-4C)alkyl-, trifluoromethyl-, hydroxy-, (1-4C)alkoxy-, benzyloxy-, (1-3C)alkylenedioxy-, nitro-, amino-, (1-4C)alkylamino-, di[(1-4C)alkyl]-amino-, (1-4C)alkanoyl-amino-, or a phenyl group, which may be unsubstituted or substituted by 1, 2, or 3 substituents independently selected from a chlorine atom, an (1-4C)alkyl-, trifluoromethyl-, hydroxy-, (1-4C)alkoxy-, (1-3C)alkylenedioxy-, nitro-, amino-, (1-4C)alkylamino-, di[(1-4C)alkyl]amino-, and a (1-4C) alkanoylamino group, or (b) denotes an indolyl group which may be unsubstituted or substituted with 1, 2 or 3 substituents independently selected from a halogen atom, an (1-4C)alkyl-, trifluoromethyl-, hydroxy-, (1-4C)alkoxy-, benzyloxy-, (1-3C)alkylenedioxy-, nitro-, amino-, (1-4C)alkylamino-, di[(1-4C)alkyl]amino-, or a (1-4C)alkanoylamino-group, R¹ and R² are the same as or different from each other and are a hydrogen atom, an (1 -4C) alkyl-, a trifluoromethyl group, or an aryl group, X is a straight chain alkylene group comprising 5, 6, or 7 carbon atoms, wherein one CH₂ group may be replaced by an oxygen or a sulfur atom, or wherein 2 carbon atoms form a C=C double bond, and which is either unsubstituted or substituted by one or two substituents selected from (1-4C)alkyl and halogen atoms, their enantiomers, diastereoisomers, racemates and mixtures thereof and pharmaceutically acceptable salts.

In one aspect, the inhibitor of histone deacetylase activity may be a carbamic acid compound, such as, for example, compounds disclosed in W02002026696, having the general formula:

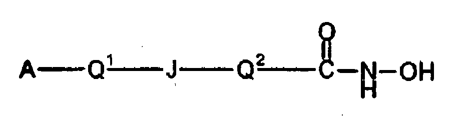

Wherein A is an aryl group; Q¹ is an aryl leader group having a backbone of at least 2 carbon atoms; J is an amide linkage selected from:

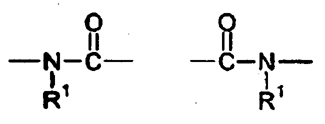

R₁ is an amido substituent; and, Q² is an acid leader group; and wherein: A, is a C₅₋₂₀aryl group, and is optionally substituted; the aryl leader group is a C₁₋₇alkylene group and is optionally substituted; the amido substituent, R¹, is hydrogen, C₁₋₇alkyl, C₃₋₂₀heterocyclyl, or C₅₋₂₀aryl; the acid leader group, Q², is C₁₋₇alkylene; C₅₋₂₀arylene; C₅₋₂₀arylene-C₁₋₇alkylene; C₁₋₇alkylene-C₅₋₂₀arylene; and is optionally substituted; and, the acid leader group, Q², has a backbone of at least 3 carbon atoms; and pharmaceutically acceptable salts, solvates, amides, esters, ethers, chemically protected forms, and prodrugs thereof.

In one aspect, the inhibitor of histone deacetylase activity may be a dioxane compound, such as, for example, compounds disclosed in WO2002089782, having the general formula:

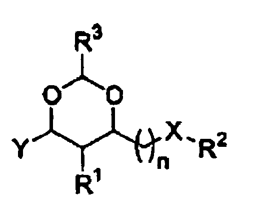

Wherein R¹ is hydrogen, or an aliphatic, heteroaliphatic, aryl, heteroaryl, -(aliphatic)aryl, -(aliphatic)heteroaryl, -(heteroaliphatic)aryl, or -(heteroaliphatic) heteroaryl moiety; n is 1-5; R² is hydrogen, a protecting group, or an aliphatic, heteroaliphatic, aryl, heteroaryl, - (aliphatic)aryl, -(aliphatic)heteroaryl, -(heteroaliphatic)aryl, or-(heteroaliphatic)heteroaryl moiety; X is -O-, -C(R^{2A})₂-, -S-, or -NR^{2A}-, wherein R^{2A} is hydrogen, a protecting group, or an aliphatic, heteroaliphatic, aryl, heteroaryl, - (aliphatic)aryl, -(aliphatic) heteroaryl, (heteroaliphatic)aryl, or-(heteroaliphatic)heteroaryl moiety; or wherein two or more occurrences of R² and R^{2A}, taken together, form a cyclic aliphatic or heteroaliphatic moiety, or an aryl or heteroaryl moiety; R³ is an aliphatic, heteroaliphatic, aryl, heteroaryl, -(aliphatic)aryl, -(aliphatic)heteroaryl, - (heteroaliphatic)aryl, or -(heteroaliphatic)heteroaryl moiety; and Y is hydrogen or an aliphatic, heteroaliphatic, aryl, heteroaryl, -(aliphatic)aryl, (aliphatic) heteroaryl, - (heteroaliphatic)aryl, or-(heteroaliphatic)heteroaryl moiety, whereby each of the foregoing aliphatic and heteroaliphatic groups is independently substituted or unsubstituted, cyclic or acyclic, linear or branched, and each of the foregoing aryl and heteroaryl groups is substituted or unsubstituted.

In one aspect, the inhibitor of histone deacetylase activity may be a compound having the general formula:

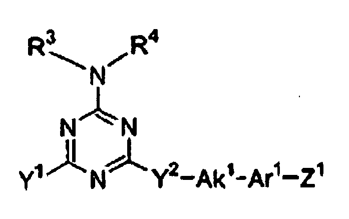

Wherein R³ and R⁴ are independently selected from the group consisting of hydrogen, L¹, Cy¹, and -L¹-Cy¹, wherein L¹ is C₁-C₆ alkyl, C₂-C₆ heteroalkyl, or C₃-C₆ alkenyl; and Cy¹ is cycloalkyl, aryl, heteroaryl, or heterocyclyl, each of which is optionally substituted, and each of which is optionally fused to one or two aryl or heteroaryl rings, or to one or two saturated or partially unsaturated cycloalkyl or heterocyclic rings, each of which rings is optionally substituted; or R³ and R⁴ are taken together with the adjacent nitrogen atom to form a 5-, 6-, or 7- membered ring, wherein the ring atoms are independently selected from the group consisting of C, O. S. and N. and wherein the ring is optionally substituted, and optionally forms part of a bicyclic ring system, or is optionally fused to one or two aryl or heteroaryl rings, or to one or two saturated or partially unsaturated cycloalkyl or heterocyclic rings, each of which rings and ring systems is optionally substituted; Y¹ is selected from the group consisting of -N(R¹)(R²), -CH2-C(O)-N(R¹)(R²), halogen, and hydrogen, wherein R¹ and R² are independently selected from the group consisting of hydrogen, L¹, Cy¹, and -L¹-Cy¹, wherein L¹ is C₁-C₆ alkyl, C₂-C₆ heteroalkyl, or C₃-C₆ alkenyl; and Cy¹ is cycloalkyl, aryl, heteroaryl, or heterocyclyl, each of which is optionally substituted, and each of which is optionally fused to one or two aryl or heteroaryl rings, or to one or two saturated or partially unsaturated cycloalkyl or heterocyclic rings, each of which rings is optionally substituted; or R¹ and R² are taken together with the adjacent nitrogen atom to form a 5-, 6- , or 7-membered ring, wherein the ring atoms are independently selected from the group consisting of C, O. S. and N. and wherein the ring is optionally substituted, and optionally forms part of a bicyclic ring system, or is optionally fused to one or two aryl or heteroaryl rings, or to one or two saturated or partially unsaturated cycloalkyl or heterocyclic rings, each of which rings and ring systems is optionally substituted; Y² is a chemical bond or N(R⁰), where R⁰ is selected from the group consisting of hydrogen, alkyl, aryl, aralkyl, and acyl; Ak¹ is C₁-C₆ alkylene, C₁-C₆- heteroalkylene (preferably, in which one (H₂- is replaced with -NH-, and more preferably -NH-CH₂-), C₂-C₆ alkenylene or C₂-C₆ alkynylene; Ar¹ is arylene or heteroarylene, either of which is optionally substituted; and Z¹ is selected from the group consisting of 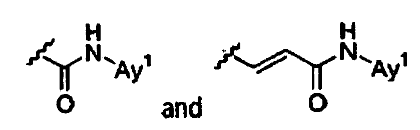 wherein Ay¹ is aryl or heteroaryl, each of which is optionally substituted. Such compounds are disclosed in WO2003024448.

In one aspect, the inhibitor of histone deacetylase activity may be a carbamic acid compound, such as, for example, compounds disclosed in W02002030879, having the general formula:

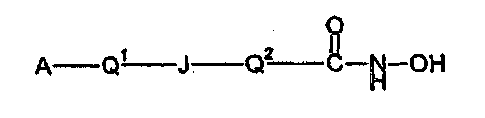

Wherein A is an aryl group; Q¹ is a covalent bond or an aryl leader group; J is a sulfonamide linkage selected from:

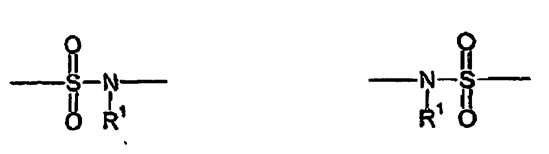

R¹ is a sulfonamido substituent; and, Q² is an acid leader group; with the proviso that if J is: 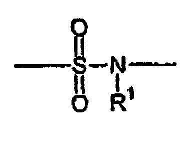 then Q¹ is an aryl leader group; and wherein: A, is aC₅-₂₀aryl group, and is optionally substituted; the aryl leader group, if present, is a C₁-₇alkylene group and is optionally substituted; the sulfonamido substituent, R¹, is hydrogen, C₁-₇alkyl, C₃-₂₀heterocyclyl, or C₅-₂₀aryl; the acid leader group, Q², is C₁-₇alkylene; C₅-₂₀arylene; C₅-₂₀arylene-C₁-₇alkylene; C₁-₇alkylene-C₅-₂₀arylene; or an ether linkage; and is optionally substituted; and pharmaceutically acceptable salts, solvates, amides, esters, ethers, chemically protected forms, and prodrugs thereof.

In one aspect, the inhibitor of histone deacetylase activity may be a carbamic acid compound, such as, for example, compounds disclosed in WO2003082288, having the general formula:

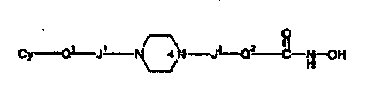

Wherein Cy is independently a cyclyl group; Q¹ is independently a covalent bond or cyclyl leader group; the piperazin-1,4-diyl group is optionally substituted; J¹ is independently a covalent bond or-C(=O)-; J² is independently-C(=O)- or-S(=O)₂-; Q² is independently an acid leader group; wherein: Cy is independently: C₃-₂₀carbocyclyl, C₃-₂₀heterocyclyl, or C₅-₂₀aryl; and is optionally substituted; Q¹ is independently: a covalent bond; C₁-₇alkylene; or C₁-₇alkylene-X-C₁-₇alkylene, -X-C₁-₇alkylene, or C₁-₇alkylene-X-, wherein X is -O- or -S-; and is optionally substituted; Q² is independently: C₄-₈ alkylene; and is optionally substituted; and has a backbone length of at least 4 atoms; or: Q² is independently: C₅-₂₀arylene; C₅-₂₀arylene-C₁-₇alkylene; C₁-₇alkylene-C₅-₂₀arylene; or, C₁-₇alkylene-C₅-₂₀arylene-C₁-₇alkylene; and is optionally substituted; and has a backbone length of at least 4 atoms; or a pharmaceutically acceptable salt, solvate, amide; ester, ether, chemically protected form, or prodrug thereof.

In one aspect, the inhibitor of histone deacetylase activity may be piperazinyl-, piperidinyl- and morpholinyl- derivatives, such as, for example, compounds disclosed in W02003076438, having the general formula: 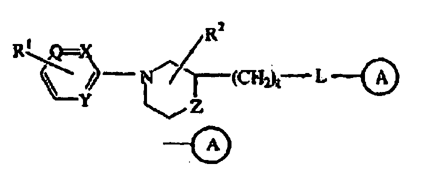
the N-oxide forms, the pharmaceutically acceptable addition salts and the stereo chemically isomeric forms thereof, wherein t is 0, 1, 2, 3 or 4 and when t is 0 then a direct bond is intended;
each Q is nitrogen or 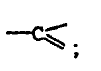
each X is nitrogen or 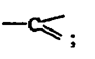
each Y is nitrogen or 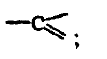
each Z is -NH-, -O- or 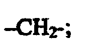
R¹ is - C(O)NR³R⁴, -NHC(O)R⁷, -C(O)-C₁₋₆alkanediylSR⁷, -NR⁸C(O)N(OH)R⁷, - NR⁸C(O)C₁-₆alkanediylSR⁷, -NR⁸C(O)C=N(OH)R⁷ or another Zn-chelating-group wherein R³ and R⁴ are each independently selected from hydrogen, hydroxy, C₁-₆alkyl, hydroxyC₁-₆alkyl, aminoC₁-₆alkyl or aminoaryl; R⁷ is hydrogen, C₁₋₆alkyl, C₁-₆alkylcarbonyl, arylC₁₋₆alkyl, C₁₋₆alkylpyrazinyl, pyridinone, pyrrolidinone or methylimidazolyl; R⁸ is hydrogen or C₁-₆alkyl; R² is hydrogen, hydroxy, amino, hydroxyC₁-₆alkyl, C₁₋₆alkyl, C₁₋₆alkyloxy, arylC₁₋₆alkyl, aminocarbonyl, hydroxycarbonyl, aminoC₁₋₆alkyl, : aminocarbonylC₁₋₆alkyl, hydroxycarbonylC₁₋₆alkyl, hydroxyaminocarbonyl, C₁₋₆alkyloxycarbonyl, C₁₋₆alkylaminoC₁₋₆alkyl or di(C₁₋₆alkyl)aminoC₁₋₆ alkyl; -L- is a bivalent radical selected from -NR⁹C(O)-, -NR⁹SO₂- or - NR⁹CH₂- wherein R⁹ is hydrogen, C₁₋₆alkyl, C₃₋₁₀cycloalkyl, hydroxyC₁₋₆alkyl, C₁₋₆alkyloxyC₁₋₆alkyl or di(C₁₋₆alkyl)aminoC₁₋₆ alkyl; 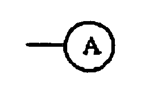 is a radical selected from 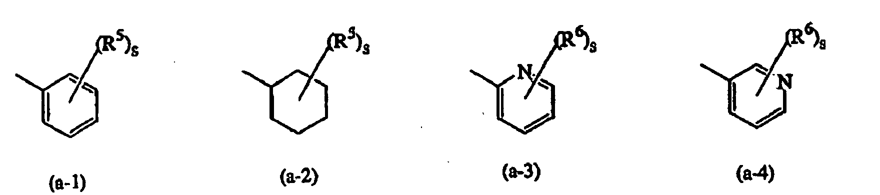 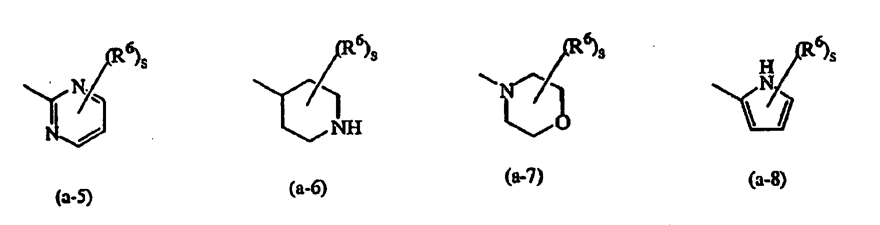 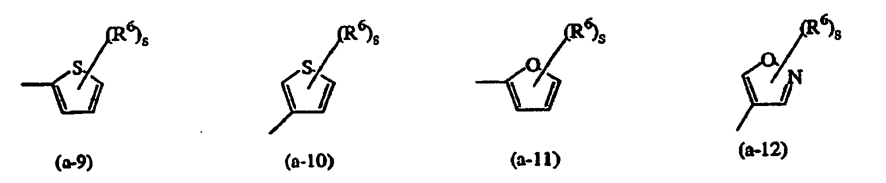 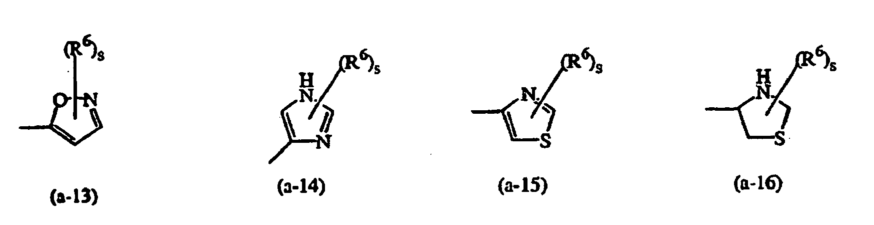 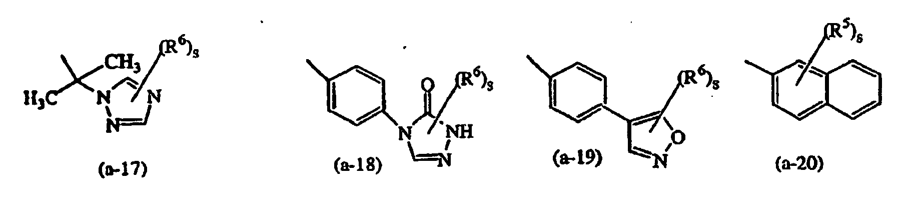 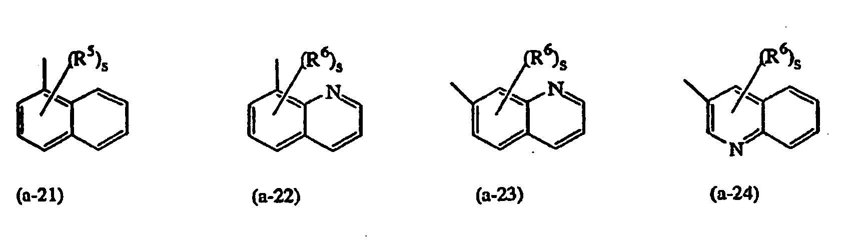 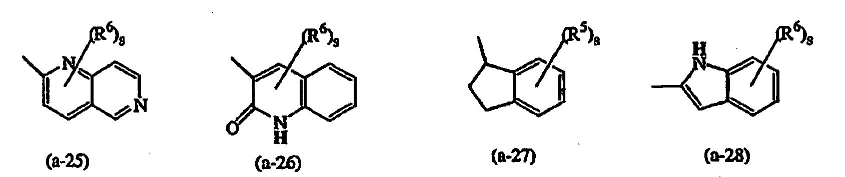 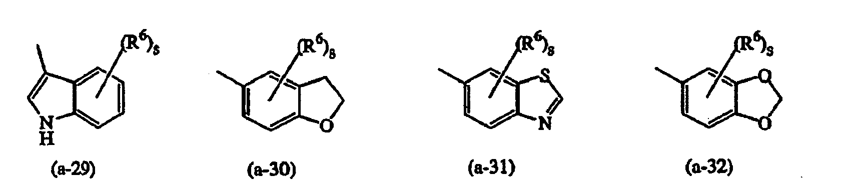 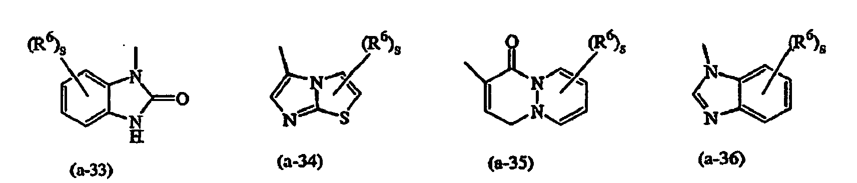 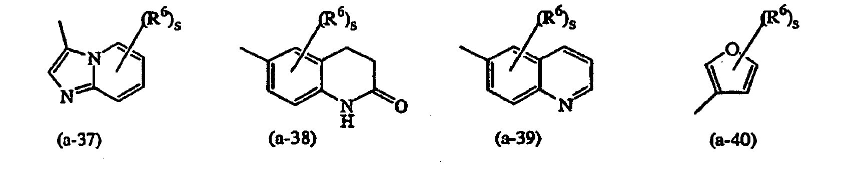 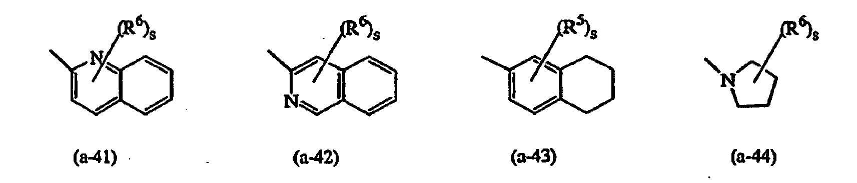 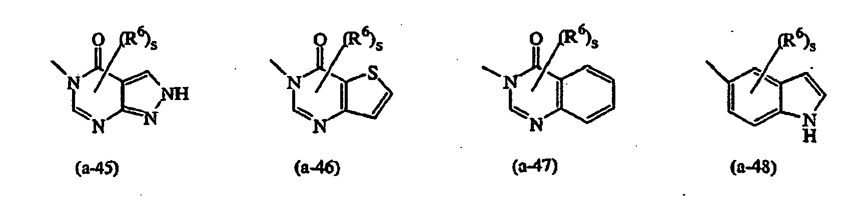 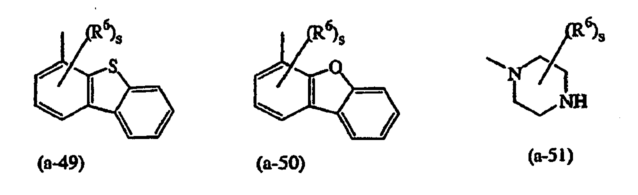
wherein each s is independently 0, 1, 2, 3, 4 or 5; each R⁵ and R⁶ are independently selected from hydrogen; halo; hydroxy; amino; nitro; trihaloC₁₋₆alkyl; trihaloC₁₋₆alkyloxy; C₁₋₆alkyl; C₁₋₆alkyl substituted with aryl and C₃₋₁₀cycloalkyl; C₁₋₆alkyloxy; C₁₋₆alkyloxyC₁₋₆alkyloxy; C₁₋₆alkylcarbonyl; C₁₋₆alkyloxycarbonyl; C₁₋₆alkylsulfonyl; cyanoC₁₋₆alkyl; hydroxyC₁₋₆alkyl; hydroxyC₁₋₆alkyloxy; hydroxyC₁₋₆alkylamino; aminoC₁₋₆alkyloxy; di(C₁₋₆alkyl)aminocarbonyl; di(hydroxyC₁₋₆alkyl)amino; (aryl)(C₁₋₆alkyl)amino; di(C₁₋₆alkyl)amino C₁₋₆alkyloxy; di(C₁₋₆alkyl)amino C₁₋₆alkylamino; di(C₁₋₆alkyl)amino C₁₋₆alkylamino C₁₋₆alkyl; arylsulfonyl; arylsulfonylamino; aryloxy; aryloxy C₁₋₆alkyl; arylC_{2 6}alkenediyl; di(C₁₋₆alkyl)amino; di(C₁₋₆alkyl)amino C₁₋₆alkyl; di(C₁₋₆alkyl)amino(C₁₋₆alkyl) amino; di(C₁₋₆alkyl)amino(C₁₋₆alkyl)amino C₁₋₆alkyl; di(C₁₋₆alkyl)amino C₁₋₆alkyl(C₁₋₆alkyl)amino; di(C₁₋₆alkyl)aminoC₁₋₆alkyl(C₁₋₆alkyl)amino C₁₋₆alkyl; aminosulfonylamino (C₁₋₆alkyl)amino; aminosulfonylamino(C₁₋₆alkyl)amino C₁₋₆alkyl; di(C₁₋₆alkyl) aminosulfonylamino(C₁₋₆alkyl)amino; di(C₁₋₆alkyl)aminosulfonylamino(C₁₋₆alkyl)amino C₁₋₆alkyl; cyano; thiophenyl; thiophenyl substituted with di(C₁₋₆alkyl)amino C₁₋₆alkyl(C₁₋₆alkyl)amino C₁₋₆alkyl, di(C₁₋₆alkyl)amino C₁₋₆alkyl, C₁₋₆alkylpiperazinyl C₁₋₆alkyl, hydroxy C₁₋₆alkylpiperazinyl C₁₋₆alkyl, hydroxy C₁₋₆alkyloxy C₁₋₆alkylpiperazinyl C₁₋₆alkyl, di(C₁₋₆alkyl) aminosulfonylpiperazinyl C₁₋₆alkyl, C₁₋₆alkyloxypiperidinyl, C₁₋₆alkyloxypiperidinyl C₁₋₆alkyl), morpholinyl C₁₋₆alkyl, hydroxy C₁₋₆alkyl(C₁₋₆alkyl)amino C₁₋₆alkyl, or di(hydroxy C₁₋₆alkyl)amino C₁₋₆alkyl; furanyl; furanyl substituted with hydroxy C₁₋₆alkyl; benzofuranyl; imidazolyl; oxazolyl; oxazolyl substituted with aryl and C₁₋₆alkyl; C₁₋₆alkyltriazolyl; tetrazolyl; pyrrolidinyl; pyrrolyl; piperidinyl C₁₋₆alkyloxy; morpholinyl; C₁₋₆alkylmorpholinyl; morpholinyl C₁₋₆alkyloxy; morpholinyl C₁₋₆alkyl; morpholinyl C₁₋₆alkylamino; morpholinylC₁₋₆alkylamino C₁₋₆alkyl; piperazinyl; C₁₋₆alkylpiperazinyl; C₁₋₆alkylpiperazinyl C₁₋₆alkyloxy, piperazinyl C₁₋₆alkyl; naphtalenylsulfonylpiperazinyl; naphtalenylsulfonylpiperidinyl; naphtalenylsulfonyl; C₁₋₆alkylpiperazinyl C₁₋₆alkyl; C₁₋₆alkylpiperazinyl C₁₋₆alkylamino; C₁₋₆alkylpiperazinyl C₁₋₆alkylamino C₁₋₆alkyl; C₁₋₆alkylpiperazinylsulfonyl; aminosulfonylpiperazinyl C₁₋₆alkyloxy; aminosulfonylpiperazinyl; aminosulfonylpiperazinyl C₁₋₆alkyl; di(C₁₋₆alkyl) aminosulfonylpiperazinyl; di(C₁₋₆alkyl)aminosulfonylpiperazinyl C₁₋₆alkyl; hydroxy C₁₋₆alkylpiperazinyl; hydroxy C₁₋₆alkylpiperazinyl C₁₋₆alkyl; C₁₋₆alkyloxypiperidinyl; C_{1- 6}allcyloxypiperidinyl C₁₋₆alkyl; piperidinylamino C₁₋₆alkylamino; piperidinylamino C₁₋₆alkylaminoC₁₋₆alkyl; (C₁₋₆alkylpiperidillyl)(hydroxyC₁₋₆alkyl)amino C₁₋₆alkylamino; (C₁₋₆alkylpiperidinyl)(hydroxy C₁₋₆alkyl)amino C₁₋₆alkylamino C₁₋₆alkyl; hydroxy C₁₋₆alkyloxy C₁₋₆alkylpiperazinyl; hydroxy C₁₋₆alkyloxy C₁₋₆alkylpiperazinyl C₁₋₆alkyl; (hydroxy C₁₋₆alkyl)(C₁₋₆alkyl)amino; (hydroxy C₁₋₆alkyl)(C₁₋₆alkyl)aminoC₁₋₆alkyl; hydroxy C₁₋₆alkylamino C₁₋₆alkyl; di(hydroxy C₁₋₆alkyl)amino C₁₋₆alkyl; pyrrolidinyl C₁₋₆alkyl; pyrrolidinylC₁₋₆alkyloxy; pyrazolyl; thiopyrazolyl; pyrazolyl substituted with two substituents selected from C₁₋₆alkyl or trihaloC₁₋₆alkyl; pyridinyl; pyridinyl substituted with C₁₋₆alkyloxy, aryloxy or aryl; pyrimidinyl; tetrahydropyrimidinylpiperazinyl; tetrahydropyrimidinylpiperazinylC₁₋₆alkyl; quinolinyl; indole; phenyl; phenyl substituted with one, two or three substituents independently selected from halo, amino, nitro, C₁₋₆alkyl, C₁₋₆alkyloxy, hydroxyC₁₋₄alkyl, trifluoromethyl, trifluoromethyloxy, hydroxyC₁₋₄alkyloxy, C₁₋₄alkylsulfonyl, C₁₋₄alkyloxyC₁₋₄alkyloxy, C₁₋₄alkyloxycarbonyl, amino C₁₋₄alkyloxy, di(C₁₋₄alkyl)amino C₁₋₄alkyloxy, di(C₁₋₄alkyl)amino, di(C₁₋₄alkyl)aminocarbonyl, di(C₁₋₄alkyl)amino C₁₋₄alkyl, di(C₁₋₄alkyl)aminoC₁₋₄alkylamino C₁₋₄alkyl, di(C₁₋₄alkyl)amino(C₁₋₄alkyl)amino, di(C₁₋₄alkyl) amino(C₁₋₄alkyl)aminoC₁₋₄alkyl, di(C₁₋₄alkyl)amino C₁₋₄alkyl(C₁₋₄alkyl)amino, di(C₁₋₄alkyl)amino C₁₋₄alkyl(C₁₋₄alkyl)amino C₁₋₄alkyl, aminosulfonylamino(C₁₋₄alkyl)amino, aminosulfonylamino(C₁₋₄ alkyl) amino C₁₋₄alkyl, di(C₁₋₄ alkyl)aminosulfonylamino(C₁₋₄alkyl)amino, di(C₁₋₄alkyl)aminosulfonylamino(C₁₋₄alkyl)amino C₁₋₆alkyl, cyano, piperidinyl C₁₋₄alkyloxy, pyrrolidinyl C₁₋₄alkyloxy, aminosulfonylpiperazinyl, aminosulfonylpiperazinyl C₁₋₄alkyl, di(C₁₋₄alkyl) aminosulfonylpiperazinyl, di(C₁₋₄alkyl)aminosulfonylpiperazinyl C₁₋₄alkyl, hydroxyC₁₋₄alkylpiperazinyl, hydroxy C₁₋₄alkylpiperazinyl C₁₋₄alkyl, C₁₋₄alkyloxypiperidinyl, C₁₋₄alkyloxypiperidinylC₁₋₄alkyl, hydroxy C₁₋₄alkyloxyC₁₋₄alkylpiperazinyl, hydroxyC₁₋₄alkyloxyC₁₋₄alkylpiperazinylC₁₋₄alkyl, (hydroxyC₁₋₄alkyl)( C₁₋₄alkyl)amino, (hydroxyC₁₋₄alkyl)(C₁₋₄alkyl)aminoC₁₋₄alkyl, hydroxyC₁₋₄alkylaminoC₁₋₄alkyl, di(hydroxyC₁₋₄alkyl) aminoC₁₋₄alkyl, furanyl, furanyl substituted with -CH=CH-CH=CH-, pyrrolidinylC₁₋₄alkyl, pyrrolidinylC₁₋₄alkyloxy, morpholinyl, morpholinylC₁₋₄alkyloxy, morpholinylC₁₋₄alkyl, morpholinylC₁₋₄alkylamino, morpholinylC₁₋₄alkylaminoC₁₋₄alkyl, piperazinyl, C₁₋₄alkylpiperazinyl, C₁₋₄alkylpiperazinylC₁₋₄alkyloxy, piperazinylC₁₋₄alkyl, C₁₋₄alkylpiperazinylC₁₋₄alkyl, C₁₋₄alkylpiperazinylC₁₋₄alkylamino, C₁₋₄alkylpiperazinylC₁₋₄alkylamino C₁₋₆alkyl, pyrimidinylpiperazinyl, pyrimidinylpiperazinylC₁₋₄alkyl, piperidinylaminoC₁₋₄alkylamino, piperidinylaminoC₁₋₄alkylaminoC₁₋₄alkyl, (C₁₋₄alkylpiperidinyl)(hydroxyC₁₋₄alkyl)aminoC₁₋₄alkylamino, (C₁₋₄alkylpiperidinyl)(hydroxyC₁₋₄alkyl) aminoC₁₋₄alkylaminoC₁₋₄alkyl, pyridinylC₁₋₄alkyloxy, hydroxyC₁₋₄alkylamino, di(hydroxyC₁₋₄alkyl)amino, di(C₁₋₄alkyl)aminoC₁₋₄alkylamino, aminothiadiazolyl, aminosulfonylpiperazinylC₁₋₄alkyloxy, or thiophenylC₁₋₄alkylamino; each R⁵ and R⁶ can be placed on the nitrogen in replacement of the hydrogen; aryl in the above is phenyl, or phenyl substituted with one or more substituents each independently selected from halo, C₁₋₆alkyl, C₁₋₆alkyloxy, trifluoromethyl, cyano or hydroxycarbonyl.

In one aspect, the inhibitor of histone deacetylase activity may be a compound of the general formula: 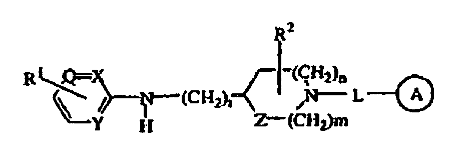
the *N*-oxide forms, the pharmaceutically acceptable addition salts and the stereo-chemically isomeric forms thereof, wherein n is 0, 1, 2 or 3 and when n is 0 then a direct bond is intended; m is 0, 1, 2 or 3 and when m is 0 then a direct bond is intended; t is O or 1 and when t is 0 then a direct bond is intended;
each Q is nitrogen or 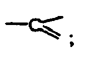
each X is nitrogen or 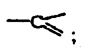
each Y is nitrogen or 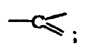
each Z is -CH₂- or -O-;
R¹ is -C(O)NR³R⁴, -N(H)C(O)R⁷, -C(O)-C₁₋₆alkanediylSR⁷, -NR⁸C(O)N(OH)R⁷, - NR⁸C(O) C₁₋₆alkanediylSR⁷, -NR³C(O)C=N(OH)R⁷ or another Zn-chelating-group wherein R³ and R⁴ are each independently selected from hydrogen, hydroxy, C₁₋₆alkyl, hydroxy C₁₋₆alkyl, amino C₁₋₆alkyl or aminoaryl; R⁷ is independently selected from hydrogen, C₁₋₆alkyl, C₁₋₆alkylcarbonyl, arylC₁₋₆alkyl, C₁₋₆alkylpyrazinyl, pyridinone, pyrrolidinone or methylimidazolyl; R⁸ is independently selected from hydrogen or C₁₋₆alkyl; R² is hydrogen, hydroxy, amino, hydroxyC₁₋₆alkyl, C₁₋₆alkyl, C₁₋₆alkyloxy, arylC₁₋₆alkyl), aminocarbonyl, hydroxycarbonyl, aminoC₁₋₆alkyl, aminocarbonylC₁₋₆alkyl, hydroxycarbonylC₁₋₆alkyl, hydroxyaminocarbonyl, C₁₋₆alkyloxycarbonyl, C₁₋₆alkylaminoC₁₋₆alkyl or di(C₁₋₆alkyl)aminoC₁₋₆alkyl; -L- is a bivalent radical selected from C₁₋₆alkanediyl, carbonyl, sulfonyl, or C₁₋₆alkanediyl substituted with phenyl 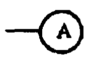 is a radical selected from 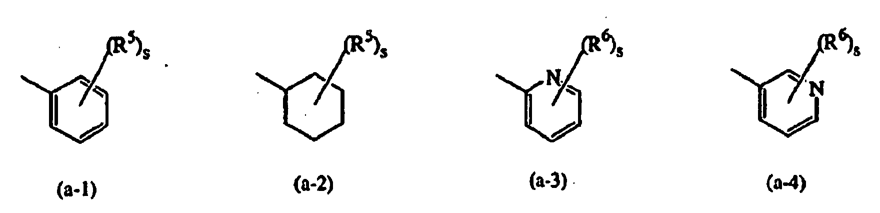 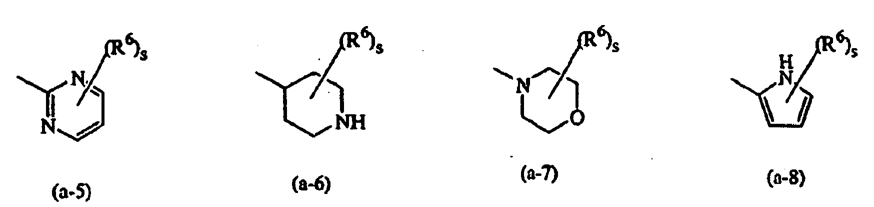 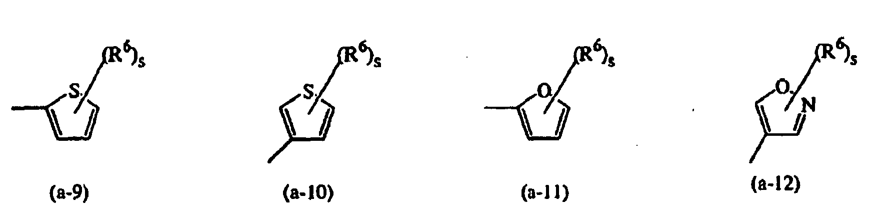 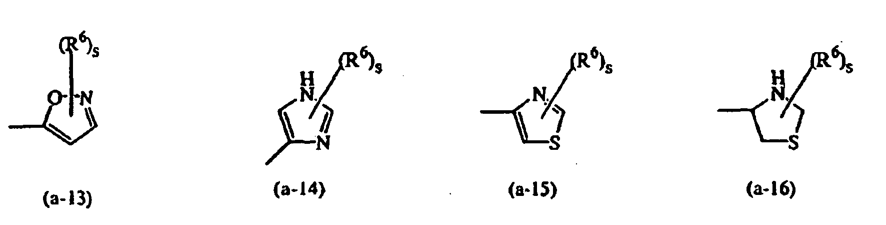 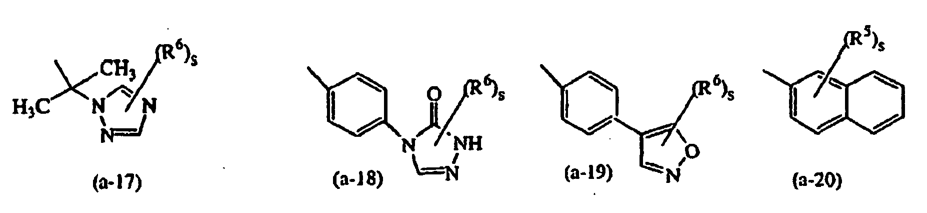 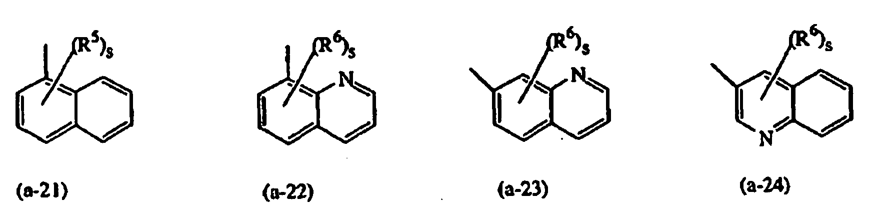 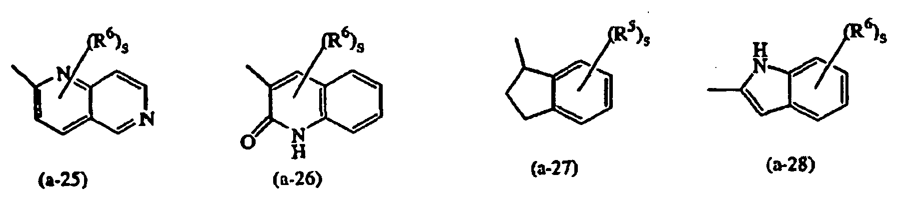 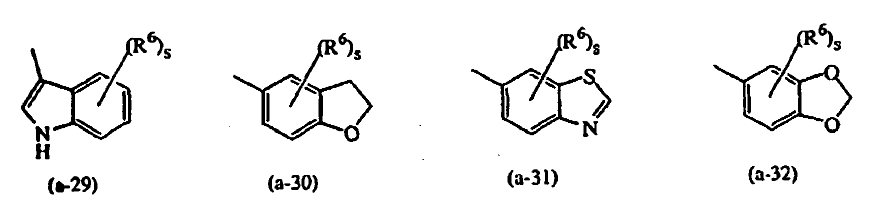 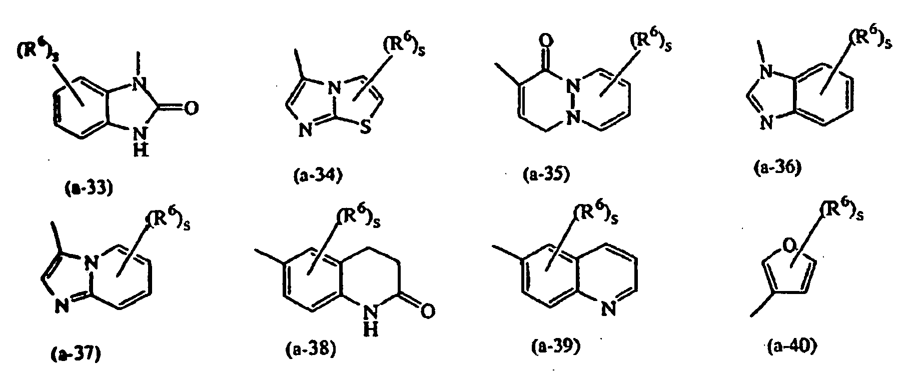 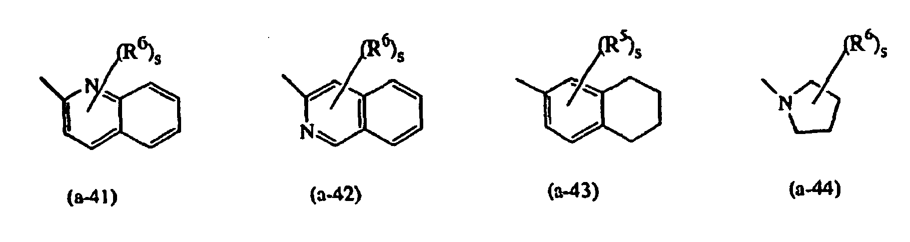 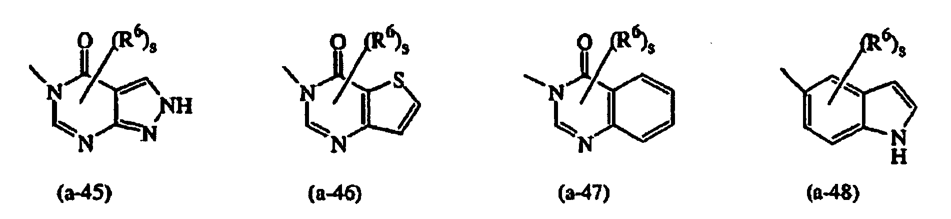 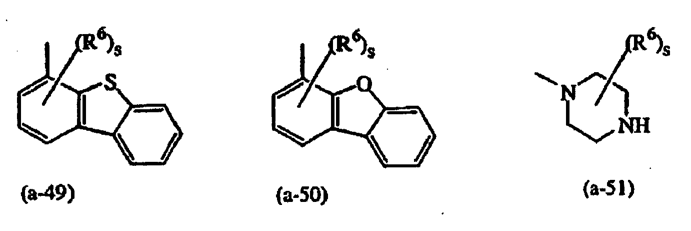
wherein each s is independently 0, 1, 2, 3, 4 or 5; each R⁵ and R⁶ are independently selected from hydrogen; halo; hydroxy; amino; nitro; trihaloC₁₋₆alkyl; trihaloC₁₋₆alkyloxy; C₁₋₆alkyl; C₁₋₆alkyl substituted with aryl and C₃₋₁₀cycloalkyl; C₁₋₆alkyloxy; C₁₋₆alkyloxyC₁₋₆alkyloxy; C₁₋₆alkylcarbonyl; C₁₋₆alkyloxycarbonyl; C₁₋₆alkylsulfonyl; cyanoC₁₋₆alkyl; hydroxyC₁₋₆alkyl; hydroxyC₁₋₆alkyloxy; hydroxyC₁₋₆alkylamino; aminoC₁₋₆alkyloxy; di(C₁₋₆alkyl)aminocarbonyl; di(hydroxyC₁₋₆alkyl)amino; (aryl)( C₁₋₆alkyl)amino; di(C₁₋₆alkyl)aminoC₁₋₆alkyloxy; di(C₁₋₆alkyl)aminoC₁₋₆alkylamino; di(C₁₋₆alkyl)aminoC₁₋₆alkylaminoC₁₋₆alkyl; arylsulfonyl; arylsulfonylamino; aryloxy; aryloxyC₁₋₆alkyl; arylC₂₋₆alkenediyl; di(C₁₋₆alkyl)amino; di(C₁₋₆alkyl)aminoC₁₋₆alkyl; di(C₁₋₆alkyl)amino(C₁₋₆alkyl)amino; di(C₁₋₆alkyl)amino(C₁₋₆alkyl)amino C₁₋₆alkyl; di(C₁₋₆alkyl)amino C₁₋₆alkyl (C₁₋₆alkyl)amino; di(C₁₋₆alkyl)amino C₁₋₆alkyl (C₁₋₆alkyl)amino C₁₋₆alkyl; aminosulfonylamino(C₁₋₆alkyl)amino; aminosulfonylamino(C₁₋₆alkyl)amino C₁₋₆alkyl; di(C₁₋₆alkyl)annnosulfonylamino(C₁₋₆alkyl)amino; di(C₁₋₆alkyl) aminosulfonylamino(C₁₋₆alkyl)amino C₁₋₆alkyl; cyano; thiophenyl; thiophenyl substituted with di(C₁₋₆alkyl)amino C₁₋₆alkyl(C₁₋₆alkyl)amino C₁₋₆alkyl, di(C₁₋₆alkyl)amino C₁₋₆alkyl, C₁₋₆alkylpiperazinylC₁₋₆alkyl, hydroxyC₁₋₆alkylpiperazinylC₁₋₆alkyl, hydroxy C₁₋₆alkyloxyC₁₋₆alkylpiperazinylC₁₋₆alkyl, di(C₁₋₆alkyl)aminosulfonylpiperazinylC₁₋₆alkyl, C₁₋₆alkyloxypiperidinyl, C₁₋₆alkyloxypiperidinylC₁₋₆alkyl, morpholinylC₁₋₆alkyl, hydroxyC₁₋₆alkyl(C₁₋₆alkyl)aminoC₁₋₆alkyl, or di(hydroxyC₁₋₆alkyl) aminoC₁₋₆alkyl; furanyl; furanyl substituted with hydroxyC₁₋₆alkyl; benzofuranyl; imidazolyl; oxazolyl; oxazolyl substituted with aryl and C₁₋₆alkyl; C₁₋₆alkyltriazolyl; tetrazolyl; pyrrolidinyl; pyrrolyl; piperidinylC₁₋₆alkyloxy; morpholinyl; C₁₋₆alkylmorpholinyl; morpholinylC₁₋₆alkyloxy; morpholinylC₁₋₆alkyl; morpholinylC₁₋₆alkylamino; morpholinylC₁₋₆alkylarninoC₁₋₆alkyl; piperazinyl; C₁₋₆alkylpiperazinyl; C₁₋₆alkypiperazinylC₁₋₆alkyloxy; piperazinylC₁₋₆alkyl; naphtalenylsulfonylpiperazinyl; naphtalenylsulfonylpiperidinyl; naphtalenylsulfonyl: C₁₋₆alkylpiperazinylC ₁₋₆alkyl; C₁₋₆alkylpiperazinyl C₁₋₆alkylamino; C₁₋₆alkylpiperazinylC₁₋₆alkylaminoC₁₋₆alkyl; C₁₋₆alkylpiperazinylsulfonyl; aminosulfonylpiperazinylC₁₋₆alkyloxy; aminosulfonylpiperazinyl; aminosulfonylpiperazinylC₁₋₆alkyl; di(C₁₋₆alkyl) aminosulfonylpiperazinyl; di(C₁₋₆alkyl)aminosulfonylpiperazinylC₁₋₆alkyl; hydroxyC₁₋₆alkylpiperazinyl; hydroxyC₁₋₆alkylpiperazinylC₁₋₆alkyl; C₁₋₆alky loxypiperidinyl; C₁₋₆alkyloxypiperidinylC ₁₋₆alkyl,; piperidinylaminoC₁₋₆alkylamino; piperidinylammoC₁₋₆alkylaminoC₁₋₆alkyl; (C₁₋₆alkylpiperidinyl)(hydroxyC₁₋₆alkyl)aminoC₁₋₆alkylamino; (C₁₋₆alkylpiperidinyl)(hydroxyC₁₋₆alkyl)aminoC₁-₆alkylaminoC₁₋₆alkyl; hydroxyC₁₋₆alkyloxyC₁₋₆alkylpiperazinyl; hydroxyC₁₋₆alkyloxyC₁₋₆alkylpiperazinyl C₁₋₆alkyl; (hydroxyC₁₋₆alkyl)( C₁₋₆alkyl)amino; (hydroxyC₁₋₆alkyl)(C₁₋₆alkyl)aminoC₁₋₆alkyl; hydroxyC₁₋₆alkylamino C₁₋₆alkyl; di(hydroxyC₁₋₆alkyl)aminoC₁₋₆alkyl; pyrrolidinylC₁₋₆alkyl; pyrrolidinylC₁₋₆alkyloxy; pyrazolyl; thiopyrazolyl; pyrazolyl substituted with two substituents selected from C₁₋₆allcyl or trihaloC₁₋₆alkyl; pyridinyl; pyridinyl substituted with C₁₋₆alkyloxy, aryloxy or aryl; pyrimidinyl; tetrahydropyrimidinylpiperazinyl; tetrahydropyrimidinylpiperazinylC₁₋₆alkyl; quinolinyl; indolyl; phenyl; phenyl substituted with one, two or three substituents independently selected from halo, amino, nitro, C₁₋₆alkyl, C₁₋₆alkyloxy, hydroxyC₁₋₄alkyl, trifluoromethyl, trifluoromethyloxy, hydroxyC₁₋₄alkyloxy, C₁₋₄alkylsulfonyl, C₁₋₄alkyloxyC₁₋₄alkyloxy, C₁₋₄alkyloxycarbonyl, aminoC₁₋₄alkyloxy, di(C₁₋₄alkyl)aminoC₁₋₄alkyloxy, di(C₁₋₄alkyl)amino, di(C₁₋₄alkyl)aminocarbonyl, di(C₁₋₄alkyl)aminoC₁₋₄alkyl, di(C₁₋₄alkyl)aminoC,-₄alkylaminoC₁₋₄alkyl, di(C₁₋₄alkyl)amino(C₁₋₄alkyl)amino, di( C₁₋₄alkyl)amino(C₁₋₄alkyl)aminoC₁₋₄alkyl, di(C₁₋₄alkyl) aminoC₁₋₄alkyl (C₁₋₄alkyl)amino, di(C₁₋₄alkyl)aminoC₁₋₄alkyl(C₁₋₄alkyl) aminoC₁₋₄alkyl, aminosulfonylamino(C₁₋₄alkyl)amino, aminosulfonylamino(C₁₋₄alkyl)aminoC₁₋₄alkyl, di(C₁₋₄alkyl) aminosulfonylamino(C₁₋₄alkyl)amino, di(C₁₋₄alkyl)aminosulfonylamino(C₁₋₄alkyl)amino C₁₋₆alkyl, cyano, piperidinylC₁₋₄alkyloxy, pyrrolidinylC₁₋₄alkyloxy, aminosulfonylpiperazinyl, aminosulfonylpiperazinylC₁₋₄alkyl di(C₁₋₄alkyl) aminosulfonylpiperazinyl, di(C₁₋₄alkyl)aminosulfonylpiperazinylC₁₋₄alkyl, hydroxyC₁₋₄alkylpiperazinyl, hydroxyC₁₋₄alkylpiperazinyl C₁₋₄alkyl, C₁₋₄alkyloxypiperidinyl, C₁₋₄alkyloxypiperidinylC₁₋₄alkyl, hydroxyC₁₋₄alkyloxyC₁₋₄alkylpiperazinyl, hydroxyC₁₋₄alkyloxyC₁₋₄alkylpiperazinylC₁₋₄alkyl, (hydroxyC₁₋₄alkyl)( C₁₋₄alkyl)amino, (hydroxyC₁₋₄alkyl)(C₁₋₄alkyl)aminoC₁₋₄alkyl, di(hydroxyC₁₋₄alkyl)amino, di(hydroxyC₁₋₄alkyl)aminoC₁₋₄alkyl, furanyl, furanyl substituted with - CH=CH-CH=CH-, pyrrolidinylC₁₋₄alkyl, pyrrolidinylC₁₋₄alkyloxy, morpholinyl, morpholinylC₁₋₄alkyloxy, morpholinylC₁₋₄alkyl, morpholinylC₁₋₄alkylamino, morpholinylC₁₋₄alkylaminoC₁₋₄alkyl, piperazinyl, C₁₋₄alkylpiperazinyl, C₁₋₄alkylpiperazinylC₁₋₄alkyloxy, piperazinylC₁₋₄alkyl, C₁₋₄alkylpiperazinylC₁₋₄alkyl, C₁₋₄alkylpiperazinylC₁₋₄alkylamino, C₁₋₄alkylpiperazinylC₁₋₄alkylaminoC₁₋₆alkyl, tetrahydropyrimidinylpiperazinyl, tetrahydropyrimidinyipiperazinylC₁₋₄alky, piperidinylaminoC₁₋₄alkylamino, piperidinylaminC₁₋₄alkylamino₁₋₄alkyl, (C₁₋₄alkylpiperidinyl)(hydroxyC₁₋₄alkyl)aminoC₁₋₄alkylamino, (C₁₋₄alkylpiperidinyl)(hydroxyC₁₋₄alkyl)aminoC₁₋₄alkylaminoC₁₋₄alkyl, pyridinylC₁₋₄alkyloxy, hydroxyC₁₋₄alkylamino, hydroxyC₁₋₄alkylaminoC₁₋₄alkyl, di(C₁₋₄alkyl)aminoC₁₋₄alkylamino, aminothiadiazolyl, aminosulfonylpiperazinylC₁₋₄alkyloxy, or thiophenylC₁₋₄alkylamino; each R⁵ and R⁶ can be placed on the nitrogen in replacement of the hydrogen; aryl in the above is phenyl, or phenyl substituted with one or more substituents each independently selected from halo, C₁₋₆alkyl, C₁₋₆alkyloxy, trifluoromethyl, cyano or hydroxycarbonyl. See, for example, compounds disclosed in W02003076430.

In one aspect, the inhibitor of histone deacetylase activity may be a compound of the general formula: 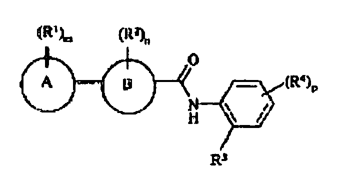 wherein: Ring A is a heterocyclyl, wherein if said heterocyclyl contains an -NH- moiety that nitrogen may be optionally substituted by a group selected from G; R¹ is a substituent on carbon and is selected from halo, nitro, cyano, hydroxy, oxo, trifluoromethyl, trifluoromethoxy, amino, carboxy, carbamoyl, mercapto, sulphamoyl, C₁₋₆alkyl), C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆Alkyl)2amino, C₁₋₆alkanoylamino, N-(C₁₋₆alkyl)carbamoyl, N,N-(C₁₋₆alkyl)₂carbamoyl, C₁₋₆alkylS(O)a wherein a is 0 to 2, C₁₋₆alkoxycarbonyl, N-(C₁₋₆alkyl)sulphamoyl, N,N-(C₁₋₆alkyl)₂sulphamoyl, aryl, aryloxy, arylC₁₋₆alkyl, heterocyclic group, (heterocyclic group)C₁₋₆alkyl or a group (D-E-); wherein R¹, including group (D-E-), may be optionally substituted on carbon by one or more V; and wherein, if said heterocyclic group contains an -NH- moiety that nitrogen may be optionally substituted by a group selected from J; V is halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alKyl)carbamoyl, N,N-(C₁₋₆alKyl)₂carbamoyl, C₁₋₆alkylS(O)ₐ wherein a is 0 to 2, C₁₋₆alkoxycarbonyl, N-(C₁₋₆alkyl)sulphamoyl, N,N-(C₁₋₆alkyl)₂sulphamoyl or a group (D'-E'-); wherein V, including group (D'-E'-), may be optionally substituted on carbon by one or more W; W and Z are independently selected from halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂-₆alkynyl, C₁₋₆alkoxy, C₁₋₆alkanoyl, C₁₋₆alkanoyloxy, N-(C₁₋₆alkyl)amino, N,N-(C)₁₋₆alkyl)₂amino, C₁₋₆alkanoylamino, N-(C₁₋₆alkyl)carbamoyl, N,N-(C₁₋₆alkyl)₂carbamoyl, C₁₋₆a1ky1S(O)ₐ wherein a is 0 to 2, C₁₋₆alkoxycarbonyl, N-(C₁₋₆alkyl)sulphamoyl or N,N-(C₁₋₆alkyl)₂suphamoyl; G. J and K are independently selected from C₁₋₈alkyl, C₂₋₈alkenyl, C₂₋₈allcynyl, C₁₋₈alkanoyl, C₁₋₆alkylsulphonyl, C₁₋₈alkoxycarbonyl, carbamoyl, N-(C₁₋₆alkyl)carbamoyl, N,N-(C₁₋₈alkyl) carbamoyl, benzyloxycarbonyl, benzoyl and phenylsulphonyl, aryl, arylC₁₋₆alkyl or (heterocyclic group)C₁₋₆alkyl; wherein G, J and K may be optionally substituted on carbon by one or more Q; and wherein if said heterocyclic group contains an - NH- moiety that nitrogen may be optionally substituted by a group selected from hydrogen or C₁₋₆alkyl; Q is halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, C₁₋₆alkoxycarbonylamino, N-(C₁₋₆alkyl)sulphamoyl, N,N-(C₁₋₆alkyl)₂sulphamoyl, aryl, aryloxy, arylC₁₋₆alkyl, arylC₁₋₆alkoxy, heterocyclic group, (heterocyclic group)C₁₋₆alkyl, (heterocyclic group)C₁₋₆alkoxy, or a group (D"-E"-); wherein Q including group (D"-E"-), may be optionally substituted on carbon by one or more Z; D, D' and D" are independently selected from C₁₋₆alkyl, C₂₋₆alkenyl, C₂₋₆alkynyl, C₃₋₈cycloalkyl, C₃₋₈cycloalkylC₁₋₆alkyl, aryl, arylC₁₋₆alkyl, heterocyclic group, (heterocyclic group)C₁₋₆alkyl; wherein D, D' and D" may be optionally substituted on carbon by one or more F'; and wherein if said heterocyclic group contains an -NH- moiety that nitrogen may be optionally substituted by a group selected from K; E, E' and E" are independently selected from - N(R^{a})-, -O-, -C(O)O-,-OC(O)-, -C(O)-, -N(R^{a})C(O)-, -N(R^{a})C(O)N(R^{b})-, - N(R^{a})C(O)O-, -OC(O)N(R^{a})-,-C(O)N(R^{a})-, -S (O)ᵣ, -SO₂N(R^{a})-, -N(R^{a})SO₂-; wherein R^{a} and R^{b} are independently selected from hydrogen or C₁₋₆alkyl optionally substituted by one or more F and r is 0-2; F and F' are independently selected from halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkyls(O)ₐ wherein a is 0 to 2, C₁₋₆alkoxycarbonyl, N-(C₁₋₆alkyl) sulphamoyl and N,N-(C₁₋₆alkyl)₂sulphamoyl; m is 0, 1, 2, 3 or 4; wherein the values of R¹ may be the same or different; Ring B is a ring selected from 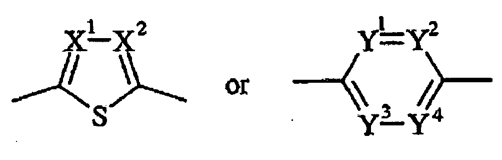 wherein, X¹ and X² are selected from CH or N. and Y¹, Y², Y³ and Y⁴ are selected from CH or N provided that at least one of Y¹, Y², Y³ and Y⁴ is N; R² is halo; n is 0, 1 or 2; wherein the values of R² may be the same or different; R³ is amino or hydroxy; R⁴ is halo, nitro, cyano, hydroxy, trifluoromethyl, trifluoromethoxy, amino, carboxy, carbamoyl, mercapto, sulphamoyl, C₁₋₃alkyl, C₂₋₃alkenyl, C₁₋₃alkynyl, C₁₋₃alkoxy, C₁₋₃alkanoyl, C₁₋₃alkanoyloxy, N-(C₁₋₃alkyl)amino, N,N-(C₁₋₃alkyl)₂amino, C₁₋₃alkanoylamino, N-(C₁₋₃alkyl)carbamoyl, N,N-(C₁₋₃alkyl)₂carbamoyl, C₁₋₃alkylS(O)ₐ wherein a is 0 to 2, C₁₋₃alkoxycarbonyl, N-(C₁₋₃alkyl)sulphamoyl, N,N-(C₁₋₃alkyl)₂sulphamoyl; and p is 0, 1 or 2; wherein the values of R⁴ may be the same or different; or a pharmaceutically acceptable salt or *in vivo* hydrolysable ester or amide thereof. See for example, compounds disclosed in WO2003092686.

In one aspect, the inhibitor of histone deacetylase activity may be an alpha-ketoepoxide compound, such as, for example, compounds disclosed in W02003099272, having the general formula:

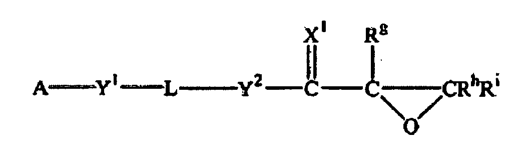

Wherein A is a cyclic moiety selected from the group consisting of C₃₋₁₄cycloalkyl, 3-14 membered heterocycloalkyl, C₄₋₁₄cycloalkenyl, 3-8 membered heterocycloalkenyl, aryl, and heteroaryl; the cyclic moiety being optionally substituted with alkyl, alkenyl, alkynyl, alkoxy, hydroxyl, hydroxylalkyl, halo, haloalkyl, amino, thio, alkylthio, arylthio, aralkylthio, acylthio, alkylcarbonyloxy, alkyloxycarbonyl, alkylcarbonyl, alkylsulfonylamino, aminosulfonyl, or alkylsulfonyl; or A is a saturated branched C₃₋₁₂hydrocarbon chain or an unsaturated branched C₃₋₁₂hydrocarbon chain optionally interrupted by -O-, -S-, -N(R^{a})-, -C(O)- , -N(R^{a})-SO₂-, -SO2-N(R^{a})-, -N(R^{a})-C(O)-O-, -O-C(O)-N(R^{a})-, -N(R^{a})-C(O)- N(R^{b})-, -O- C(O) -, - C (O)-O-, -O- S O₂ -, - SO₂-O-, or - O-C(O) -O-, where each of R^{a} and R^{b}, independently, is hydrogen, alkyl, alkenyl, alkynyl, alkoxy, hydroxylalkyl, hydroxyl, or haloalkyl; each of the saturated and the unsaturated branched hydrocarbon chain being optionally substituted with alkyl, alkenyl, alkynyl, alkoxy, hydroxyl, hydroxylalkyl, halo, haloalkyl, amino, thio, alkylthio, arylthio, aralkylthio, acylthio, alkylcarbonyloxy, alkyloxycarbonyl, alkylcarbonyl, alkylsulfonylamino, aminosulfonyl, or alkylsulfonyl; each of Y¹ and Y² independently, is -CH₂-, -O-, -S-, -N(R^{c})-, -N(R^{c})-C(O)-O-, -N(R^{c})-C(O) -, -C(O)-N(R^{c}) -, -O-C(O)-N(R^{c})-, -N(R^{c})-C(O)-N(R^{d})-, -C(O)-, -C(NR^{c})-, -O-C(O)-O-, or a bond; each of R^{c} and R^{d}, independently, being hydrogen, alkyl, alkenyl, alkynyl, alkoxy, hydroxylalkyl, hydroxyl, or haloalkyl,; L is a straight C₄₋₁₂hydrocarbon chain optionally containing at least one double bond, at least one triple bond, or at least one double bond and one triple bond; the hydrocarbon chain being optionally substituted with C₁₋₄alkyl, C₂₋₄alkenyl, C₂₋₄alkynyl, C₁₋₄alkoxy, hydroxyl, halo, amino, thio, alkylthio, arylthio, aralkylthio, acylthio, nitro, cyano, C₃₋₅cycloalkyl, 3-5 membered heterocycloalkyl, monocyclic aryl, 5-6 membered heteroaryl, C₁₋₄alkylcarbonyloxy, C₁₋₄alkyloxycarbonyl, C₁₋₄alkylcarbonyl, or formyl; and further being optionally interrupted by -O-, -N(R^{e})-, -N(R^{e})-C(O)-O-, -O-C(O)-N(R^{e})-, -N(R^{e})-C(O)-N(R^{f})-, or -O-C(O)-O-; each of R^{e} and R^{f}, independently, being hydrogen, alkyl, alkenyl, alkynyl, alkoxy, hydroxylalkyl, hydroxyl, or haloalkyl; X¹ is O or S; and each of R^{g}, R^{h}, and Rⁱ, independently, is hydrogen or C₁₋₆alkyl; provided that when each of Y¹ and Y² independently, is a bond or CH₂, A is unsubstituted phenyl or heterocyclyl, and L is C₄₋₇, L has at least one double bond or at least one triple bond, and when each of Y¹ and Y² is a bond, A is unsubstituted phenyl, and L is C₄, L is not a diene; or a salt thereof.

In one aspect, the inhibitor of histone deacetylase activity may be a benzamide derivative, such as, for example, compounds disclosed in W02003087057, having the general formula:

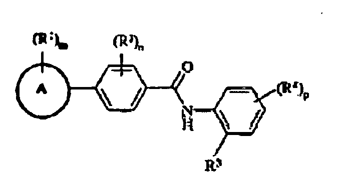

Wherein Ring A is a heterocyclyl, wherein if said heterocyclyl contains an -NH- moiety that nitrogen may be optionally substituted by a group selected from K; R¹ is a substituent on carbon and is selected from halo, nitro, cyano, hydroxy, trifluoromethyl, trifluoromethoxy, amino, carboxy, carbamoyl, mercapto, sulphamoyl, C₁₋₆alkyl, C_{2- 6}alkenyl, C₂₋₆alkynyl, C₁₋₆alkoxy, C₁₋₆alkanoyl, C₁₋₆alkanoyloxy, N-(C₁₋₆alkyl)amino, N,N-(C₆alkyl)₂amino, C₁₋₆alkanoylamino, N-(C₁₋₆alkyl)carbamoyl, N,N-(C₁₋₆alkyl)₂carbamoyl, C₁₋₆alkylS(O)a wherein a is 0 to 2, C₁₋₆alkoxycarbonyl, N-(C₁₋₆alkyl) sulphamoyl, N,N-(C₁₋₆alkyl)₂sulphamoyl, aryl, aryloxy, arylC₁₋₆alkyl, heterocyclic group, (heterocyclic group)C₁₋₆alkyl, or a group (B-E-); wherein R¹, including group (B-E-), may be optionally substituted on carbon by one or more W; and wherein if said heterocyclic group contains an -NH- moiety that nitrogen may be optionally substituted by J; W is halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)2amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkbxycarbonyl, N-(C₁₋₆alkyl)sulphamoyl, N. N-(C₁₋₆alkyl)₂sulphamoyl, or a group (B'-E'-); wherein W including group (B'-E'-), may be optionally substituted on carbon by one or more Y; Y and Z are independently selected from halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, N-(C₁₋₆alkyl)sulphamoyl or N,N-(C₁₋₆alkyl)₂sulphamoyl; G. J and K are independently selected from C₁₋₈alkyl, C₂₋₈alkenyl, C₁₋₈alkanoyl, C₁₋₈alkylsulphonyl, C₁₋₈alkoxycarbonyl, carbamoyl, N-(C₁₋₈alkyl) carbamoyl, N,N-(C₁₋₈alkyl)carbamoyl, benzyloxycarbonyl, benzoyl, phenylsulphonyl, aryl, arylC₁₋₆alkyl or (heterocyclic group)C₁₋₆alkyl; wherein G, J and K may be optionally substituted on carbon by one or more Q; and wherein if said heterocyclic group contains an -NH- moiety that nitrogen may be optionally substituted by hydrogen or C₁₋₆alkyl; Q is halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylSO)ₐ wherein a is 0 to 2, C₁₋₆alkoxycarbonyl, C₁₋₆alkoxycarbonylamino, N-(C₁₋₆alkyl)sulphamoyl, N,N-(C₁₋₆alkyl)₂sulphamoyl, aryl, aryloxy, arylC₁₋₆alkyl, arylC_{1- 6}alkoxy, heterocyclic group, (heterocyclic goup)C₁₋₆alkyl, (heterocyclic group)C₁₋₆alkoxy, or a group (B"-E"-); wherein Q. including group (B"-E"-), may be optionally substituted on carbon by one or more Z; B, B' and B" are independently selected from C₁₋₆alkyl, C₂₋₆alkenyl, C₂₋₆alkynyl, C₃₋₈cycloalkyl, C₃₋₈cycloalkylC₁₋₆alkyl, aryl, arylC₁₋₆alkyl, heterocyclic group, (heterocyclic group)C₁₋₆alkyl, phenyl or phenylC₁₋₆alkyl; wherein B, B' and B" may be optionally substituted on carbon by one or more D; and wherein if said heterocyclic group contains an -NH- moiety that nitrogen may be optionally substituted by a group selected from G; E, E' and E" are independently selected from -N(R^{a})-, -O-, -C(O)O-, -OC(O)-, -C(O)-, - N(R^{a})C(O)-, -N(R^{a})C(O)N(R^{b})-, -N(R^{a})C(O)O-, -OC(O)N(R^{a})-, -C(O)N(R^{a})-, -S(O)ᵣ-, -SO₂N(R^{a})-, -N(R^{a})SO₂-; wherein R^{a} and R^{b} are independently selected from hydrogen or C₁₋₆alkyl optionally substituted by one or more F and r is 0-2; D and F are independently selected from halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, N-(C₁₋₆alkyl)sulphamoyl or N,N-(C₁₋₆alkyl)₂sulphamoyl; m is 0, 1, 2, 3 or 4; wherein the values of R¹ may be the same or different; R² is halo; n is 0, 1 or 2; wherein the values of ^{R}2 may be the same or different; R³ is amino or hydroxy; R⁴ is halo, nitro, cyano, hydroxy, trifluoromethyl, trifluoromethoxy, amino, carboxy, carbamoyl, mercapto, sulphamoyl, C₁₋₃alkyl, C₂₋₃alkenyl, C₂₋₃alkynyl, C₁₋₃alkoxy, C₁₋₃alkanoyl, C₁₋₃alkanoyloxy, N-(C₁₋₃alkyl)amino, N,N-(C₁₋₃alkyl)₂amino, C₁₋₃alkanoylamino, N-(C₁₋₃alkyl)carbamoyl, N,N-(C₁₋₃alkyl)₂carbamoyl, C₁₋₃alkylS(O)ₐ wherein a is 0 to 2, C₁₋₃alkoxycarbonyl, N-(C₁₋₃alkyl)sulphamoyl, N,N-(C₁₋₃alkyl)₂sulphamoyl; p is 0, 1 or 2; wherein the values of R⁴ may be the same or different; or a pharmaceutically acceptable salt or *in vivo* hydrolysable ester or amide thereof; with the proviso that said compound is not N-(2-amino-6-hydroxyphenyl)-4-1-methylhomopiperazin- 4-yl)benzamide; N-(2-amino-6-methylphenyl)-4-(1-methylhomopiperazin-4-yl) benzamide; N-(2-aminophenyl)-4-(1-t-butoxycarbonylhomopiperazin-4-yl) benzamide; or N-(2-aminophenyl)-4-(1-methylh6mopiperazin-4-yl)benzamide.

In one aspect, the inhibitor of histone deacetylase activity may be a hydroxamic acid derivative, such as, for example, compounds disclosed in W02003087066, having the general formula:

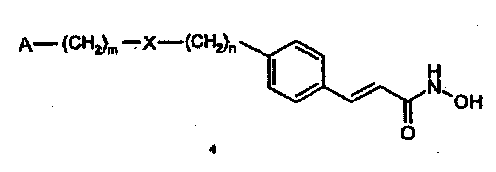

Wherein A is an optionally substituted phenyl or aromatic heterocyclic group which has 1 to 4 substituents selected from the group consisting of a halogen atom, a hydroxyl group, an amino group, a nitro group, a cyano group, an alkyl group having 1 to 4 carbons, an alkoxy group having 1 to 4 carbons, an aminoalkyl group having 1 to 4 carbons, an alkylamino group having 1 to 4 carbons, an acyl group lo having 1 to 4 carbons, an acylamino group having 1 to 4 carbons, an alkylthio group having 1 to 4 carbons, a perfluoroalkyl group having 1 to 4 carbons, a perfluoroalkoxy group having 1 to 4 carbons, a carboxyl group, an alkoxycarbonyl group having 1 to 4 carbons, a phenyl group, an aromatic heterocyclic group and a heterocyclic group, said heterocyclic group being optionally substituted with an alkyl group having 1 to 4 carbons, a benzyl group, or a pyridylmethyl group; m is an integer of 0 to 4; n is an integer of 1 to 4; X is a moiety having a structure selected from those illustrated in formula

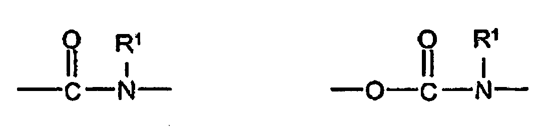

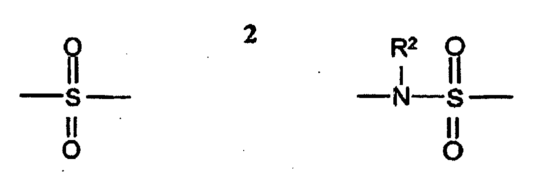

R¹ and R² are independently H or an optionally substituted alkyl group having 1 to 4 carbons; or a pharmaceutically acceptable salt thereof.

In one aspect, the inhibitor of histone deacetylase activity may be a sulfonyl derivative, such as, for example, compounds disclosed in WO2003076422, having the general formula: 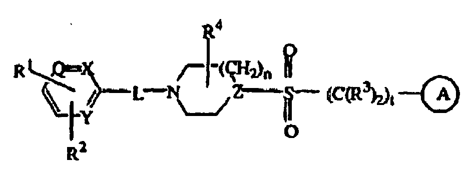
the N-oxide forms, the pharmaceutically acceptable addition salts and the stereo-chemically isomeric forms thereof, wherein n is 0, 1, 2, or 3 and when n is 0 then a direct bond is intended; t is 0, 1, 2, 3, or 4 and when t is 0 then a direct bond is intended; each Q is nitrogen or 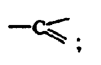
each X is nitrogen or 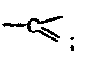
each Y is nitrogen or 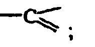
each Z is nitrogen or 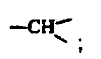
R¹ is-C(O)NR⁷R⁸,-N(H)C(O)R⁹, -C(O)-C₁₋₆alkanediylSR⁹, -NR¹⁰C(O)N(OH)R⁹, - NR¹⁰C(O)C₁₋₆alkanediylSR⁹, -NR¹⁰C(O)C=N(OH)R⁹ or another Zn chelating group wherein k⁷ and R⁸ are each independently selected from hydrogen, hydroxy, C₁₋₆alkyl, hydroxyC₁₋₆alkyl, aminoC₁₋₆alkyl or aminoaryl; R⁹ is independently selected hydrogen, C₁₋₆alkyl, C₁₋₆alkylcarbonyl, arylC₁₋₆alkyl, C₁₋₆alkylpyrazinyl, pyridinone, pyrrolidinone or methylimidazolyl; R¹⁰ is independently selected hydrogen or C₁₋₆alkyl;
R² is hydrogen, halo, hydroxy, amino, nitro, C₁₋₆alkyl, C₁₋₆alkyloxy, trifluoromethyl, di(C₁₋₆alkyl)amino, hydroxyamino or naphtalenylsulfonylpyrazinyl;
-L- is a direct bond or a bivalent radical selected from C₁₋₆alkanediyl, amino, carbonyl or aminocarbonyl;
   each R³ represents a hydrogen atom and one hydrogen atom can be replaced by aryl;
R⁴ is hydrogen, hydroxy, amino, hydroxyC₁₋₆alkyl, C₁₋₆alkyl, C₁₋₆alkyloxy, arylC₁₋₆alkyl, aminocarbonyl, hydroxycarbonyl, aminoC₁₋₆alkyl, aminocarbonylC₁₋₆alkyl, hydroxycarbonylC₁₋₆alkyl, hydroxyaminocarbonyl, C₁₋₆alkyloxycarbonyl, C₁₋₆alkylaminoC₁₋₆alkyl or di(C₁₋₆alkyl)aminoC₁₋₆alkyl; 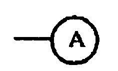 is a radical selected from 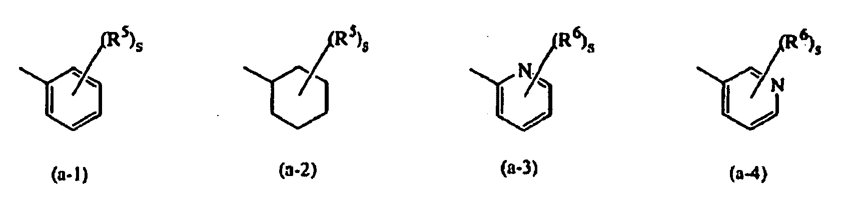 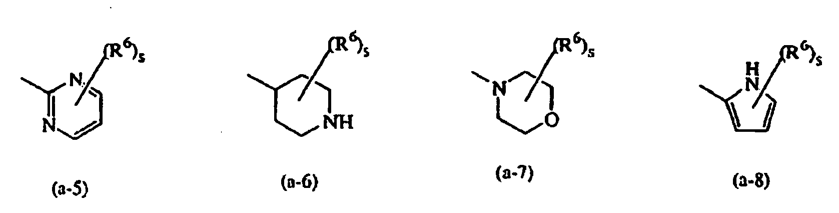 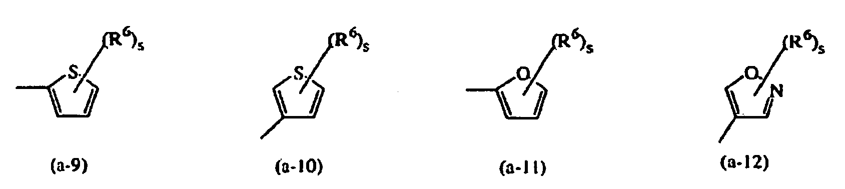 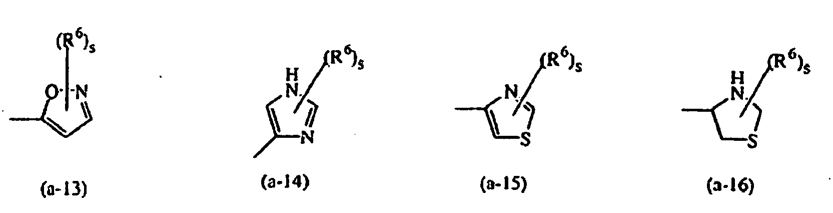 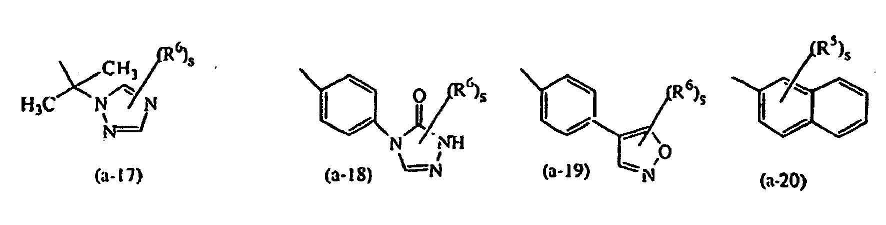 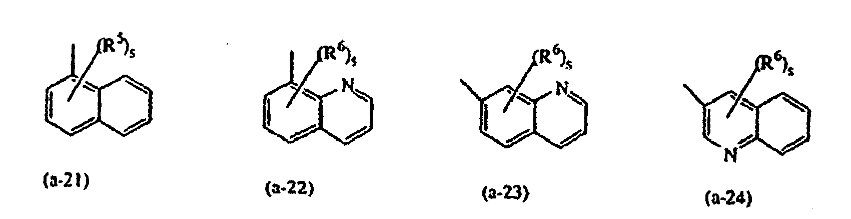 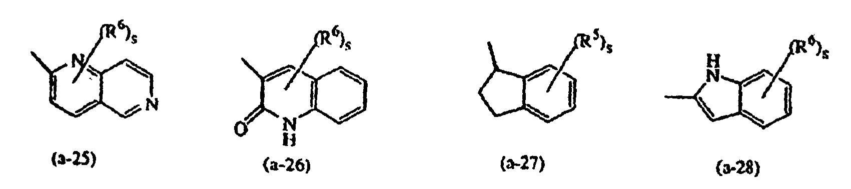 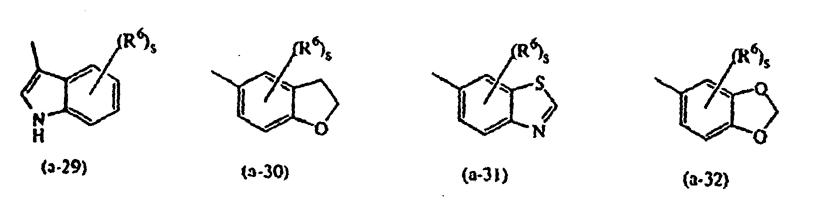 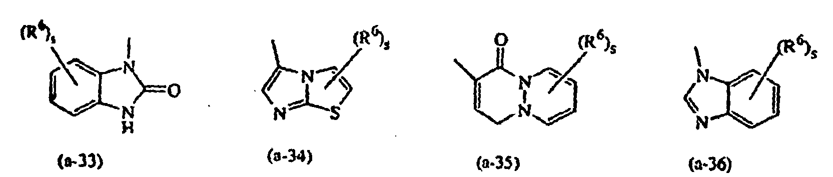 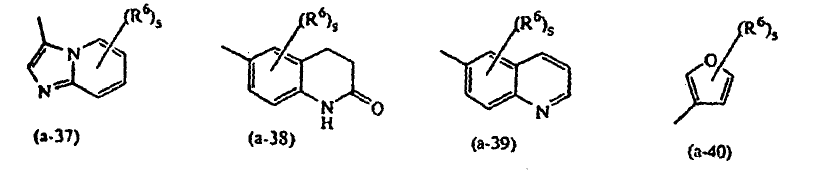 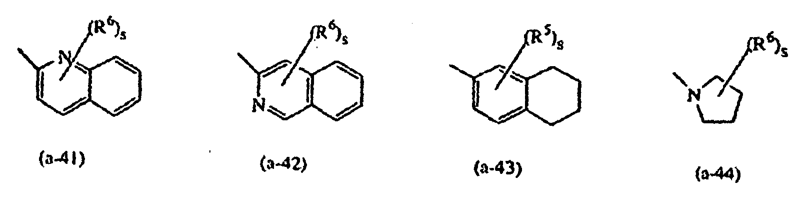 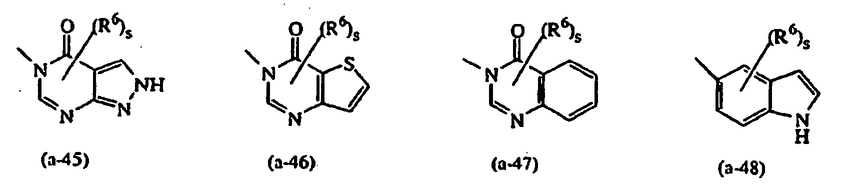 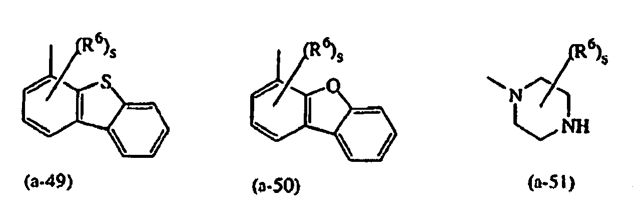
wherein each s is independently 0, 1, 2, 3, 4 or 5;
each R⁵ and R⁶ are independently selected from hydrogen; halo; hydroxy; amino; nitro; trihaloC₁₋₆alkyl; trihaloC₁₋₆alkyloxy; C₁₋₆alkyl; C₁₋₆alkyl substituted with aryl and C₃₋₁₀cycloalkyl; C₁₋₆alkyloxy; C₁₋₆alkyloxyC₁₋₆alkyloxy; C₁₋₆alkylcarbonyl; C₁₋₆alkyloxycarbonyl; C₁₋₆alkylsulfonyl; cyanoC₁₋₆alkyl; hydroxyC₁₋₆alkyl; hydroxyC₁₋₆alkyloxy; hydroxyC₁₋₆alkylamino; aminoC₁₋₆alkyloxy; di(C₁₋₆alkyl)aminocarbonyl; di(hydroxyC₁₋₆alkyl)amino; (aryl)(C₁₋₆alkyl)amino; di(C₁₋₆alkyl)aminoC₁₋₆alkyloxy; di(C₁₋₆alkyl)aminoC₁₋₆alkylamino; di(C₁₋₆alkyl)aminoC₁₋₆alkylaminoC₁₋₆alkyl; arylsulfonyl; arylsulfonylamino; aryloxy; aryloxyC₁₋₆alkyl; arylC₂₋₆alkenediyl; di(C₁₋₆alkyl)amino; di(C₁₋₆alkyl)aminoC₁₋₆alkyl; di(C₁₋₆alkyl)amino(C₁₋₆alkyl)amino; di(C₁₋₆alkyl)amino(C₁₋₆alkyl)aminoC₁₋₆alkyl; di(C₁₋₆alkyl)aminoC₁₋₆alkyl(C₁₋₆alkyl)amino; di(C₁₋₆alkyl)aminoC₁₋₆alkyl(C₁₋₆alkyl)aminoC₁₋₆alkyl; aminosulfonylamino(C₁₋₆alkyl)amino; aminosulfonylamino(C₁₋₆alkyl)aminoC₁₋₆alkyl; di(C₁₋₆alkyl)aminosulfonylamino(C₁₋₆alkyl)amino; di(C₁₋₆alkyl) aminosulfonylamino(C₁₋₆alkyl)aminoC₁₋₆alkyl; cyano; thiophenyl; thiophenyl substituted with di(C₁₋₆alkyl)aminoC₁₋₆alkyl(C₁₋₆alkyl)aminoC₁₋₆alkyl, di(C₁₋₆alkyl)aminoC₁₋₆alkyl, C₁₋₆alkylpiperazinylC₁₋₆alkyl, hydroxyC₁₋₆alkylpiperazinylC₁₋₆alkyl, hydroxyC₁₋₆alkyloxyC₁₋₆alkylpiperazinylC₁₋₆alkyl, di(C₁₋₆alkyl)aminosulfonylpiperazinylC₁₋₆alkyl, C₁₋₆alkyloxypiperidinyl, C₁₋₆alkyloxypiperidinylC₁₋₆alkyl, morpholinylC₁₋₆alkyl, hydroxyC₁₋₆alkyl(C₁₋₆alkyl)aminoC₁₋₆alkyl, or di(hydroxyC₁₋₆alkyl)aminoC₁₋₆alkyl; furanyl; furanyl substituted with hydroxyC₁₋₆alkyl; benzofuranyl; imidazolyl; oxazolyl; oxazolyl substituted with aryl and C₁₋₆alkyl; C₁₋₆alkyltriazolyl; tetrazolyl; pyrrolidinyl; pyrrolyl; piperidinylC₁₋₆alkyloxy; morpholinyl; C₁₋₆alkylmorpholinyl; morpholinylC₁₋₆alkyloxy; morpholinylC₁₋₆alkyl; morpholinylC₁₋₆alkylamino; morpholinylC₁₋₆,alkylaminoC₁₋₆alkyl; piperazinyl; C₁₋₆alkylpiperazinyl; C₁₋₆alkylpiperazinylC₁₋₆alkyloxy; piperazinylC₁₋₆alkyl; naphtalenylsulfonylpiperazinyl; naphtalenylsulfonylpiperidinyl; naphtalenylsulfonyl: C₁₋₆alkylpiperazinylC₁₋₆alkyl; C₁₋₆alkylpiperazinylC₁₋₆alkylamino; C₁₋₆alkylpiperazinylC₁₋₆alkylaminoC₁₋₆alkyl; C₁₋₆alkylpiperazinylsulfonyl; aminosulfonylpiperazinylC₁₋₆alkyloxy; aminosulfonylpiperazinyl; aminosulfonylpiperazinylC₁₋₆alkyl; di(C₁₋₆alkyl) aminosulfonylpiperazinyl; di(C₁₋₆alkyl)aminosulfonylpiperazinylC₁₋₆alkyl; hydroxyC₁₋₆alkylpiperazinyl; hydroxyC₁₋₆alkylpiperazinylC₁₋₆alkyl; C₁₋₆alkyloxypiperidinyl; C₁₋₆alkyloxypiperidinylC₁₋₆alkyl; piperidinylaminoC₁₋₆alkylamino; piperidinylaminoC₁₋₆alkylaminoC₁₋₆alkyl; (C₁₋₆alkylpiperidinyl)(hydroxyC₁₋₆alkyl)aminoC₁₋₆alkylamino; (C₁₋₆alkylpiperidinyl)(hydroxyC₁₋₆alkyl)aminoC₁₋₆alkylaminoC₁₋₆alkyl; hydroxyC₁₋₆alkyloxyC₁₋₆alkylpiperazinyl; hydroxyC₁₋₆alkyloxyC₁₋₆alkylpiperazinylC₁₋₆alkyl; (hydroxyC₁₋₆alkyl)(C₁₋₆alkyl)amino; (hydroxyC₁₋₆alkyl)(C₁₋₆alkyl) aminoC₁₋₆alkyl; hydroxyC₁₋₆alkylaminoC₁₋₆alkyl; di(hydroxyC₁₋₆alkyl) aminoC₁₋₆alkyl; pyrrolidinylC₁₋₆alkyl; pyrrolidinylC₁₋₆alkyloxy; pyrazolyl; thiopyrazolyl; pyrazolyl substituted with two substituents selected from C₁₋₆alkyl or trihaloC₁₋₆alkyl; pyridinyl; pyridinyl substituted with C₁₋₆alkyloxy, aryloxy or aryl; pyrimidinyl; tetrahydropyrimidinylpiperazinyl; tetrahydropyrimidinylpiperazinylC₁₋₆alkyl; quinolinyl; indolyl; phenyl; phenyl substituted with one, two or three substituents independently selected from halo, amino, nitro, C₁₋₆alkyl, C₁₋₆alkyloxy, hydroxyC₁₋₄alkyl, trifluoromethyl, trifluoromethyloxy, hydroxyC₁₋₄alkyloxy, C₁₋₄alkylsulfonyl, C₁₋₄alkyloxyC₁₋₄alkyloxy, C₁₋₄alkyloxycarbonyl, aminoC₁₋₄alkyloxy, di(C₁₋₄alkyl)aminoC₁₋₄alkyloxy, di(C₁₋₄alkyl)amino, di(C₁₋₄alkyl)aminocarbonyl, di(C₁₋₄alkyl)aminoC₁₋₄alkyl, di(C₁₋₄alkyl)aminoC₁₋₄alkylaminoC₁₋₄alkyl, di(C₁₋₄alkyl)amino(C₁₋₄alkyl) amino, di(C₁₋₄alkyl)amino(C₁₋₄alkyl)aminoC₁₋₄alkyl, di(C₁₋₄alkyl)aminoC₁₋₄alkyl(C₁₋₄alkyl)amino, di(C₁₋₄alkyl)aminoC₁₋₄alkyl(C₁₋₄alkyl)aminoC₁₋₄alkyl, aminosulfonylamino(C₁₋₄alkyl)amino, aminosulfonylamino(C₁₋₄alkyl)aminoC₁₋₄alkyl, di(C₁₋₄alkyl)aminosulfonylamino(C₁₋₄alkyl)amino, di(C₁₋₄alkyl)aminosulfonylamino(C₁₋₄alkyl)aminoC₁₋₆alkyl, cyano, piperidinylC₁₋₄alkyloxy, pyrrolidinylC₁₋₄alkyloxy, aminosulfonylpiperazinyl, aminosulfonylpiperazinylC₁₋₄alkyl, di(C₁₋₄alkyl) aminosulfonylpiperazinyl, di(C₁₋₄alkyl)aminosulfonylpiperazinylC₁₋₄alkyl, hydroxyC₁₋₄alkylpiperazinyl, hydroxyC₁₋₄alkylpiperazinylC₁₋₄alkyl, C₁₋₄alkyloxypiperidinyl, C₁₋₄alkyloxypiperidinylC₁₋₄alkyl, hydroxyC₁₋₄alkyloxyC₁₋₄alkylpiperazinyl, hydroxyC₁₋₄alkyloxyC₁₋₄alkylpiperazinylC₁₋₄alkyl, (hydroxyC₁₋₄alkyl)(C₁₋₄alkyl)amino, (hydroxyC₁₋₄alkyl)(C₁₋₄alkyl)aminoC₁₋₄alkyl, di(hydroxyC₁₋₄alkyl)amino, di(hydroxyC₁₋₄alkyl)aminoC₁₋₄alkyl, furanyl, furanyl substituted with-CH=CH-CH=CH-, pyrrolidinylC₁₋₄alkyl, pyrrolidinylC₁₋₄alkyloxy, morpholinyl, morpholinylC₁₋₄alkyloxy, morpholinylC₁₋₄alkyl, morpholinylC₁₋₄alkylamino, morpholinylC₁₋₄alkylaminoC₁₋₄alkyl, piperazinyl, C₁₋₄alkylpiperazinyl, C₁₋₄alkylpiperazinylC₁₋₄alkyloxy, piperazinylC₁₋₄alkyl, C₁₋₄alkylpiperazinylC₁₋₄alkyl, C₁₋₄alkylpiperazinylC₁₋₄alkylamino, C₁₋₄alkylpiperazinylC₁₋₄alkylaminoC₁₋₆alkyl, tetrahydropyrimidinylpiperazinyl, tetrahydropyrimidinylpiperazinylC₁₋₄alkyl, piperidinylaminoC₁₋₄alkylamino, piperidinylaminoC₁₋₄alkylaminoC₁₋₄alkyl, (C₁₋₄alkylpiperidinyl)(hydroxyC₁₋₄alkyl)aminoC₁₋₄alkylamino, (C₁₋₄alkylpiperidinyl)(hydroxyC₁₋₄alkyl)aminoC₁₋₄alkylaminoC₁₋₄alkyl, pyridinylC₁₋₄alkyloxy, hydroxyC₁₋₄alkylamino, hydroxyC₁₋₄alkylaminoC₁₋₄alkyl, di(C₁₋₄alkyl)aminoC₁₋₄alkylamino, aminothiadiazolyl, aminosulfonylpiperazinylC₁₋₄alkyloxy, or thiophenylC₁₋₄alkylamino;
the central 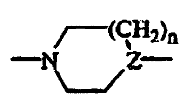 moiety may also be bridged (i.e. forming a bicyclic moiety) with a methylene, ethylene or propylene bridge; each R⁵ and R⁶ can be placed on the nitrogen in replacement of the hydrogen; aryl in the above is phenyl, or phenyl substituted with one or more substituents each independently selected from halo, C₁₋₆alkyl, C₁₋₆alkyloxy, trifluoromethyl, cyano or hydroxycarbonyl.

In one aspect, the inhibitor of histone deacetylase activity may be a trihalomethylcarbonyl compound, such as, for example, compounds disclosed in WO2003099760, having the general formula: 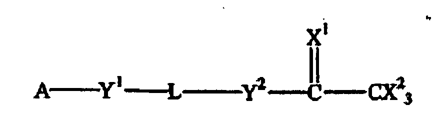
wherein A is a cyclic moiety selected from the group consisting of C₃₋₁₄cycloalkyl, 3-14 membered heterocycloalkyl, C₄₋₁₄cycloalkenyl, 3-8 membered heterocycloalkenyl, aryl, and heteroaryl; the cyclic moiety being optionally substituted with alkyl, alkenyl, alkynyl, alkoxy, hydroxyl, hydroxylalkyl, halo, haloalkyl, amino, thio, alkylthio, arylthio, aralkylthio, acylthio, alkylcarbonyloxy, alkyloxycarbonyl, alkylcarbonyl, alkylsulfonylamino, aminosulfonyl, or alkylsulfonyl; or A is a saturated branched C₃-₁₂ hydrocarbon chain or an unsaturated branched C₃-₁₂ hydrocarbon chain optionally interrupted by -O-, -S-, -N(R^{a})-, -C(O)- , -N(R^{a})-SO₂-, -SO₂-N(R^{a})-, -N(R^{a})-C(O)-O-, -O-C(O)-N(R^{a})-, -N(R^{a})-C(O)-N(R^{b})-, -O-C(O)-, -C(O)-O-, -O-SO₂-, -SO₂-O-, or -O-C(O)-O-, where each of R^{a} and R^{b}, characterized independently, is hydrogen, alkyl, alkenyl, alkynyl, alkoxy, hydroxylalkyl, hydroxyl, or haloalkyl; each of the saturated and the unsaturated branched hydrocarbon chain being optionally substituted with alkyl, alkenyl, alkynyl, alkoxy, hydroxyl, hydroxylalkyl, halo, haloalkyl, amino, thio, alkylthio, arylthio, aralkylthio, acylthio, alkylcarbonyloxy, alkyloxycarbonyl, alkylcarbonyl, alkylsulfonylamino, aminosulfonyl, or alkylsulfonyl;
each of Y¹ and Y², independently, is - O-, -S-, -N(R^{c})-, -N(R^{c})-C(O)-O-, -N(R^{c})-C(O)-,-C(O)-N(R^{c})-, -O-C(O)- N(R^{c})-, -N(R^{c})-C(O)-N(R^{d})-, -O-C(O)-O-, or a bond;
each of R^{c} and R^{d}, independently, being hydrogen, alkyl, alkenyl, alkynyl, alkoxy, hydroxylalkyl, hydroxyl, or haloalkyl; L is a straight C₃₋₁₂ hydrocarbon chain optionally containing at least one double bond, at least one triple bond, or at least one double bond and one triple bond; the hydrocarbon chain being optionally substituted with C₁₋₄alkyl, C₂₋₄alkenyl, C₂₋₄alkynyl, C₁₋₄alkoxy, hydroxyl, halo, amino, thio, alkylthio, arylthio, aralkylthio, acylthio, nitro, cyano, C₃₋₅cycloalkyl, 3-5 membered heterocycloalkyl, monocyclic aryl, 5-6 membered heteroaryl, C₁₋₄alkylcarbonyloxy, C₁₋₄alkyloxycarbonyl, C₁₋₄alkylcarbonyl, or formyl; and further being optionally interrupted by -O-, -N(R^{e})-, - N(R^{e})-C(O)-O-, -O-C(O)-N(R^{e})-, - N(R^{e})-C(O)-N(R^{f})-, or -O-C(O)-O-; each of R^{e} and
R^{f}, independently, being hydrogen, alkyl, alkenyl, alkynyl, alkoxy, hydroxylalkyl, hydroxyl, or haloalkyl, and X¹ isOorS; X² is a halogen; provided that when Y¹ and Y² are each a bond, L is a C₆₋₁₂ hydrocarbon chain containing at least one double bond at Cl, C2, C3 or C5 of the hydrocarbon chain from C=X¹, at least one triple bond, or at least one double bond and one triple bond, the hydrocarbon chain being optionally substituted with C₁₋₄alkyl, C₂₋₄alkenyl, C₂₋₄alkynyl, C₁₋₄alkoxy, hydroxyl, halo, amino, thio, alkylthio, arylthio, aralkylthio, acylthio, nitro, cyano, C₃₋₅cycloalkyl, 3-5 membered heterocycloalkyl, monocyclic characterized aryl, 5-6 membered heteroaryl, C₁₋₄alkylcarbonyloxy, C₁₋₄alkyloxycarbonyl, C₁₋₄alkylcarbonyl, or formyl; and further being optionally interrupted by -O-, -N(R^{e})-, -N(R^{e})-C(O)-O-, -O-C(O)-N(R^{e})-, -N(R^{e})-C(O)-N(R^{f})-, or -O-C(O)-O-; each of R^{e} and R^{f} independently, being hydrogen, alkyl, alkenyl, alkynyl, alkoxy, hydroxylalkyl, hydroxyl, or haloalkyl; or a salt thereof.

In one aspect, the inhibitor of histone deacetylase activity may be an alpha-chalcogenmethylcarbonyl compound, such as, for example, compounds disclosed in WO2003099789, having the general formula: 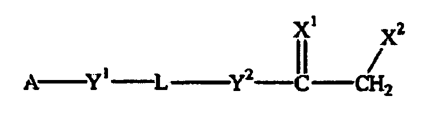 wherein A is a cyclic moiety selected from the group consisting of C₁₋₄cycloalkyl, 3-14 membered heterocycloalkyl, C₄₋₁₄cycloalkenyl, 3-8 membered heterocycloalkenyl, aryl, and heteroaryl; the cyclic moiety being optionally substituted with alkyl, alkenyl, alkynyl, alkoxy, hydroxyl, hydroxylalkyl, halo, haloalkyl, amino, thio, alkylthio, arylthio, aralkylthio, acylthio, alkylcarbonyloxy, alkyloxycarbonyl, alkylcarbonyl, alkylsulfonylamino, aminosulfonyl, or alkylsulfonyl; orA is a saturated branched C₃₋₁₂ hydrocarbon chain or an unsaturated i branched C₃₋₁₂ hydrocarbon chain optionally interrupted by -O-, -S-, -N(R^{a}) -, -C(O)-, -N(R^{a})-SO₂-, -SO₂-N(R^{a})-, -N(R^{a})-C(O)-O-, -O-C(O)-N(R^{a})-, -N(R^{a})-C(O)-N(R^{b})-, -O-C(O)-, -C(O)-O-, -O-SO₂-, -SO₂-O-, or -O-C(O)-O-, where each of R^{a} and R^{b}, characterized independently, is hydrogen, alkyl, alkenyl, alkynyl, alkoxy, hydroxylalkyl, hydroxyl, or haloalkyl; each of the saturated and the unsaturated branched hydrocarbon chain being optionally substituted with alkyl, alkenyl, alkynyl, alkoxy, hydroxyl, hydroxylalkyl, halo, haloalkyl, amino, thio, alkylthio, arylthio, aralkylthio, acylthio, alkylcarbonyloxy, alkyloxycarbonyl, alkylcarbonyl, alkylsulfonylamino, aminosulfonyl, or alkylsulfonyl; each of Y¹ and Y², independently, is -CH₂-, -O-, -S-, -N(R^{c})-, -N(R^{c})-C(O)-O-, -N(R^{c})-C(O)-, -C(O)-N(R^{c})-, -O- C(O)-N(R^{c})-, -N(R^{c})-C(O)-N(R^{d})-, -O-C(O)-O-, or a bond; each of R^{c} and R^{d} independently, being hydrogen, alkyl, alkenyl, alkynyl, alkoxy, hydroxylalkyl, hydroxyl, or haloalkyl; L is a straight C₃₋₁₂ hydrocarbon chain optionally containing at least one double bond, at least one triple bond, or at least one double bond and one triple bond; the hydrocarbon chain being optionally substituted with C₁₋₄alkyl, C₂₋₄alkenyl, C₁₋₄allcynyl, C₁₋₄alkoxy, hydroxyl, halo, amino, thio, alkylthio, arylthio, aralkylthio, acylthio, nitro, cyano, C₃₋₅cycloalkyl, 3-5 membered heterocycloalkyl, monocyclic aryl, 5-6 membered heteroaryl, C₁₋₄alkylcarbonyloxy, C₁₋₄alkyloxycarbonyl, C₁₋₄alkylcarbonyl, or formyl; and further being optionally interrupted by -O-, -N(R^{e})-, -N(R^{e}-C(O)-O-, -O-C(O)-N(R^{e})-, - N(R^{e})-C(O)-N(R^{f})-, or -O-C(O)-O-; each of R^{e} and 5 R^{f} independently, being hydrogen, alkyl, alkenyl, alkynyl, alkoxy, hydroxylalkyl, hydroxyl, or haloalkyl; X¹ is O or S.; and X² is - OR, -SR¹, or-SeR¹, wherein R is hydrogen, alkyl, acyl, aryl or aralkyl; provided that when Y¹ is a bond and L is saturated, the carbon adjacent to Y¹ is not substituted with C₁₋₄alkoxy or hydroxyl; or a salt thereof.

In one aspect, the inhibitor of histone deacetylase activity may be bicyclic hydroxamate derivative, such as, for example, compounds disclosed in WO2003066579, having the general formula:

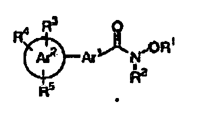

Wherein R¹ is hydrogen or alkyl; R² is hydrogen; Ar¹ is phenylene or a six membered heteroarylene ring containing one or two nitrogen ring atoms, the rest of the ring atoms being carbon; wherein said Ar¹ group is optionally substituted with one or two groups independently selected from alkyl, halo, hydroxy, alkoxy, haloalkoxy, or haloalkyl; Ar² is aryl, benzimidazol-2-yl, cycloalkyl or heterocycloalkyl; R³ is hydrogen, alkyl, halo, hydroxy, or alkoxy; and R⁴ and R⁵ are independently selected from the group consisting of hydrogen, alkyl, halo, haloalkyl, nitro, cyano, carboxy, carboxyalkyl, alkoxycarbonyl, optionally substituted phenyl, optionally substituted heteroaryl, optionally substituted heterocycloalkyl, cycloalkyl, heterocycloaminoalkyl, -X-R⁶, or -(C₁₋₆alkylene)-Y-R⁷ where X and Y are independently -O-, -S-,-SO-, -SO₂-, -NRs-,-CO- -NR⁹CO- -CONR¹⁰-,-NR¹¹SO₂-, -SO₂NR¹²-, -NHC(O)O-, -OC(O)NH-, -NR¹³CONR¹⁴-, or-NR¹⁵SO₂NR¹⁶-where R⁶ and R⁷ are independently hydrogen, alkyl, hydroxyalkyl, optionally substituted phenyl, optionally substituted heteroaryl, optionally substituted heterocycloalkyl, cycloalkyl, optionally substituted phenylalkyl, optionally substituted phenoxyalkyl, optionally substituted phenylalkenyl, optionally substituted phenylaminoalkyl, optionally substituted heteroaralkyl, optionally substituted heteroaryloxyalkyl, optionally substituted heterocycloalkylalkyl, or cycloalkylalkyl, R⁸, R⁹, R¹¹, R¹³, and R¹⁵ are independently hydrogen, alkyl, hydroxyalkyl, alkoxyalkyl, or optionally substituted phenylalkyl; R¹⁰, R¹², R¹⁴, and R¹⁶ are independently hydrogen, alkyl, optionally substituted phenylalkyl, alkoxy, hydroxyalkyl, haloalkyl, alkoxyalkyl, carboxyalkyl, cyanoalkyl, aminoalkyl, aminocarbonylalkyl, alkylaminocarbonylalkyl, dialkylaminocarbonylalkyl, or acyl or R⁴ and R⁵ together form methylenedioxy; and individual isomers, mixtures of isomers; or a pharmaceutically acceptable salt thereof provided that: (i) at least one of R³, R⁴ and R⁵ is not hydrogen; (ii) when Ar² is cycloalkyl, then at least two of R³, R⁴ and R⁵ are hydrogen; (iii) when R¹ and R³ are hydrogen, Ar¹ is phenylene and Ar² is phenyl, and one of R⁴ and R⁵ is methoxy, then the other of R⁴ and R⁵ is not -OR⁶ where R⁶ is S cyclopentyl or phenylpentyl; (iv) when Ar¹ is phenylene and Ar² is phenyl then at least one of R³, R⁴ and R⁵ is not alkyl; (v) when Ar¹ is phenylene, Ar² is aryl and is located at the 3-position of the phenylene ring, then Ar² is not substituted with an optionally substituted phenyl; (vi) when Ar¹ is phenylene and Ar² is phenyl, and R⁴ or R⁵ is - CONR¹⁰R⁶ or-(C₁₋₆alkylene)-CONR¹⁰R⁷ then said R⁴ or R⁵ is not located at the 4-position of the phenyl ring; and (vii) when Ar¹ is phenylene and Ar² is phenyl and two of R³, R⁴ and R⁵ are hydrogen, then the remaining of R³, R⁴ and R⁵ is not nitro.

In one aspect, the inhibitor of histone deacetylase activity may be a compound of the general formula: 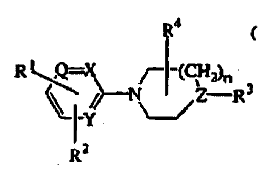
the N- oxide forms, the pharmaceutically acceptable addition salts and the stereo chemically isomeric forms thereof, wherein n is 0, 1, 2 or 3 and when n is 0 then a direct bond is intended;
each Q is nitrogen or 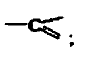
each X is nitrogen or 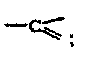
each Y is nitrogen or 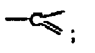
each Z is nitrogen or 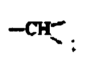
R¹ is -C(O)NR⁵R⁶, -N(H)C(O)R⁷, -C(O)-C₁₋₆alkanediylSR⁷, -NR⁸C(O)N(OH)R⁷, - NR⁸C(O)C₁₋₆alkanediylSR⁷, -NR⁸C(O)C=N(OH)R⁷ or another Zn- chelating-group wherein R⁵ and R⁶ are each independently selected from hydrogen, hydroxy, C₁₋₆alkyl, hydroxyC₁₋₆alkyl, aminoC₁₋₆alkyl or aminoaryl; R⁷ is independently selected from hydrogen, C₁₋₆alkyl, C₁₋₆alkylcarbonyl, arylC₁₋₆alkyl, C₁₋₆alkylpyrazinyl, pyridinone, pyrrolidinone or methylimidazolyl; R⁸ is independently selected from hydrogen or C₁₋₆alkyl; R² is hydrogen, halo, hydroxy, amino, nitro, C₁₋₆alkyl, C₁₋₆alkyloxy, trifluoromethyl, di(C₁₋₆alkyl)amino, hydroxyamino or naphtalenylsulfonylpyrazinyl; R³ is hydrogen, C₁₋₆alkyl, arylC₂₋₆alkenediyl, furanylcarbonyl, naphtalenylcarbonyl, - C(O)phenylR⁹, C₁₋₆alkylaminocarbonyl, aminosulfonyl, arylaminosulfonyl, aminosulfonylamino, di(C₁₋₆alkyl)aminosulfonylamino, arylaminosulfonylamino, aminosulfonylaminoC₁₋₆alkyl, di(C₁₋₆alkyl)aminosulfonylaminoC₁₋₆alkyl, arylaminosulfonylaminoC₁₋₆alkyl, di(C₁₋₆alkyl)aminoC₁₋₆alkyl, C₁₋₁₂alkylsulfonyl, di(C₁₋₆alkyl)aminosulfonyl, trihaloC₁₋₆alkylsulfonyl, di(aryl)C₁₋₆alkylcarbonyl, thiophenylC_{1- 6}alkylcarbonyl, pyridinylcarbonyl or arylC₁₋₆alkylcarbonyl wherein each R⁹ is independently selected from phenyl; phenyl substituted with one, two or three substituents independently selected from halo, amino, C₁₋₆alkyl, C₁₋₆alkyloxy, hydroxyC₁₋₄alkyl, hydroxyC₁₋₄alkyloxy, aminoC₁₋₄alkyloxy, di(C₁₋₄alkyl)aminoC₁₋₄alkyloxy, di(C₁₋₆alkyl)aminoC₁₋₆alkyl, di(C₁₋₆alkyl)aminoC₁₋₆alkyl(C₁₋₆alkyl)aminoC₁₋₆alkyl, hydroxyC₁₋₄alkylpiperazinylC₁₋₄alkyl, C₁₋₄alkyloxypiperidinylC₁₋₄alkyl, hydroxyC₁₋₄alkyloxyC₁₋₄alkylpiperazinyl, C₁₋₄alkylpiperazinylC₁₋₄alkyl, di(hydroxyC₁₋₄alkyl)aminoC₁₋₄alkyl, pyrrolidinylC₁₋₄alkyloxy, morpholinylC₁₋₄alkyloxy, or morpholinylC₁₋₄alkyl; thiophenyl; or thiophenyl substituted with di(C₁₋₄alkyl)aminoC₁₋₄alkyloxy, di(C₁₋₆alkyl)aminoC₁₋₆alkyl, di(C₁₋₆alkyl)aminoC₁₋₆alkyl(C₁₋₆alkyl)aminoC₁₋₆alkyl, pyrrolidinylC₁₋₄alkyloxy, C₁₋₄alkylpiperazinylC₁₋₄alkyl, di(hydroxyC₁₋₄alkyl)aminoC₁₋₄alkyl or morpholinylC₁₋₄alkyloxy. R⁴ is hydrogen, hydroxy, amino, hydroxyC₁₋₆alkyl, C₁₋₆alkyl, C₁₋₆alkyloxy, arylC₁₋₆alkyl, aminocarbonyl, hydroxycarbonyl, aminoC₁₋₆alkyl, aminocarbonylC₁₋₆alkyl, hydroxycarbonylC₁₋₆alkyl, hydroxyaminocarbonyl, C₁₋₆alkyloxycarbonyl, C₁₋₆alkylaminoC₁₋₆alkyl or di(C₁₋₆alkyl)aminoC₁₋₆alkyl; when R³ and R⁴ are present on the same carbon atom, R³ and R⁴ together may form a bivalent radical of formula -C(O)-NH-CH₂-NR¹⁰-, wherein R¹⁰ is hydrogen or aryl; when R³ and R⁴ are present on adjacent carbon atoms, R³ and R⁴ together may form a bivalent radical of formula =CH-CH=CH-CH=; aryl in the above is phenyl, or phenyl substituted with one or more substituents each independently selected from halo, C₁₋₆alkyl, C₁₋₆alkyloxy, trifluoromethyl, cyano or hydroxycarbonyl. See for example, compounds disclosed in WO2003075929.

In one aspect, the inhibitor of histone deacetylase activity may be a carbonylamino derivative, such as, for example, compounds disclosed in W02003076395, having the general formula: 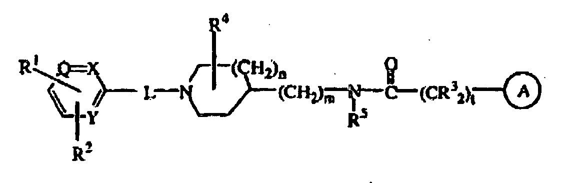
the N-oxide forms, the pharmaceutically acceptable addition salts and the stereo chemically isomeric forms thereof, wherein n is 0, 1, 2 or 3 and when n is 0 then a direct bond is intended; m is 0 or 1 and when m is 0 then a direct bond is intended; t is 0, 1, 2, 3 or 4 and when t is 0 then a direct bond is intended;
each Q is nitrogen or 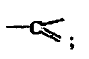
each X is nitrogen or 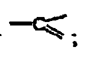
each Y is nitrogen or 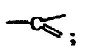
R¹ is-C(O)NR⁸R⁹, -NHC(O)R¹⁰, -C(O)-C₁₋₆alkanediylSR¹⁰, -NR¹¹C(O)N(OH)R¹⁰, - NR¹¹C(O)C₁₋₆alkanediylSR¹⁰, -NR¹¹C(O) C=N(OH)R¹⁰ or another Zn-chelating group wherein R⁸ and R⁹ are each independently selected from hydrogen, hydroxy, C₁₋₆alkyl, hydroxyC₁₋₆alkyl, aminoC₁₋₆alkyl or aminoaryl; R¹⁰ is hydrogen, C₁₋₆alkyl, C₁₋₆alkylcarbonyl, arylC₁₋₆alkyl, C₁₋₆alkylpyrazinyl, pyridinone, pyrrolidinone or methylimidazolyl; R¹¹ is hydrogen or C₁₋₆alkyl; R² is hydrogen, halo, hydroxy, amino, nitro, C₁₋₆alkyl, C₁₋₆alkyloxy, trifluoromethyl, di(C₁₋₆alkyl)amino, hydroxyamino or naphtalenylsulfonylpyrazinyl; -L- is a direct bond or a bivalent radical selected from C₁₋₆alkanediyl, C₁₋₆alkanediyloxy, amino, carbonyl or aminocarbonyl; each R³ independently represents a hydrogen atom and one hydrogen atom can be replaced by a substituent selected from aryl; R⁴ is hydrogen, hydroxy, amino, hydroxyC₁₋₆alkyl, C₁₋₆alkyl, C₁₋₆alkyloxy, arylC₁₋₆alkyl, aminocarbonyl, hydroxycarbonyl, aminoC₁₋₆alkyl, aminocarbonylC₁₋₆alkyl, hydroxycarbonylC₁₋₆alkyl, hydroxyaminocarbonyl, C₁₋₆alkyloxycarbonyl, C₁₋₆alkylaminoC₁₋₆alkyl or di(C₁₋₆alkyl)aminoC₁₋₆alkyl; R⁵ is hydrogen, C₁₋₆alkyl, C₁₋₁₀cycloalkyl, hydroxyC₁₋₆alkyl, C₁₋₆alkyloxyC₁₋₆alkyl, di(C₁₋₆alkyl)aminoC₁₋₆alkyl or aryl; 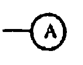 is a radical selected from 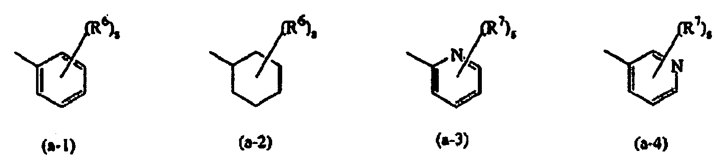 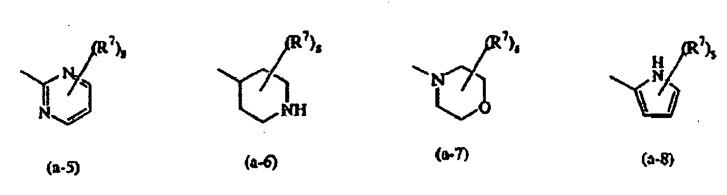 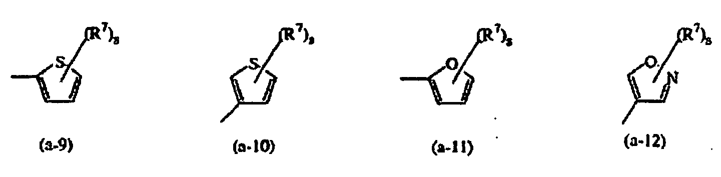 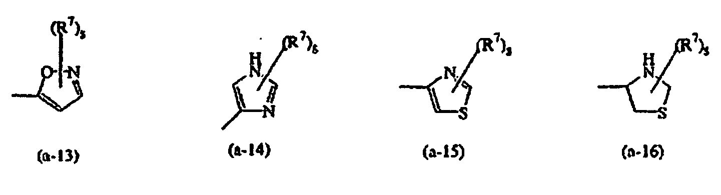 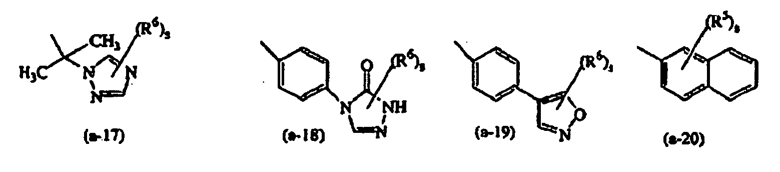 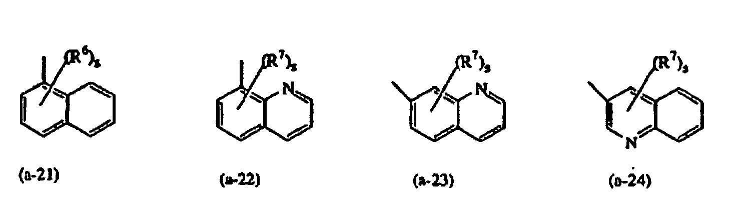 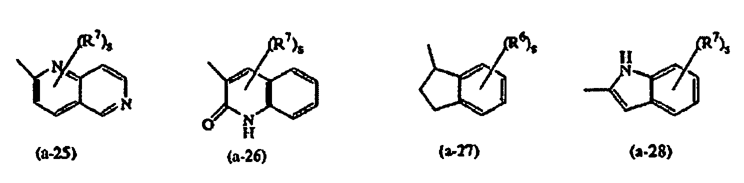 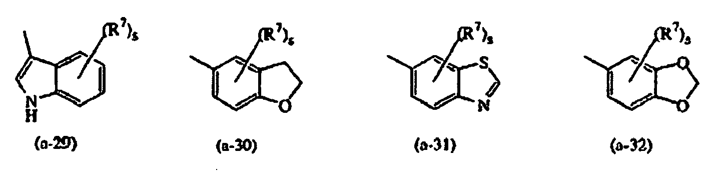 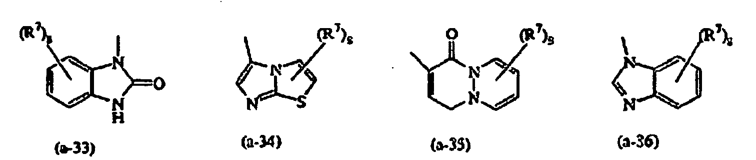 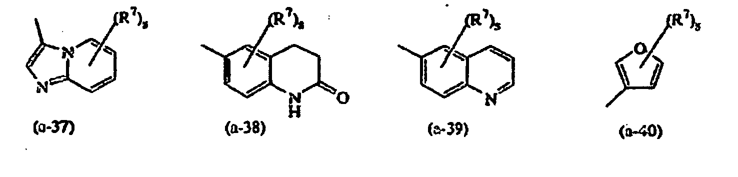 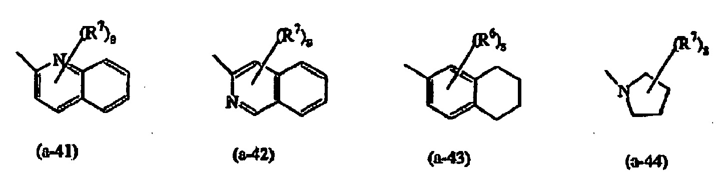 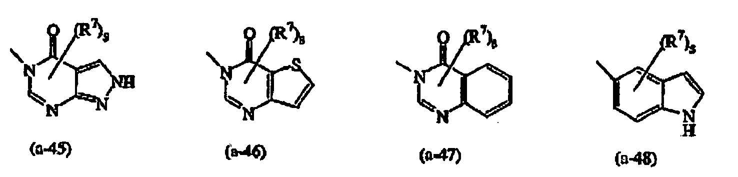 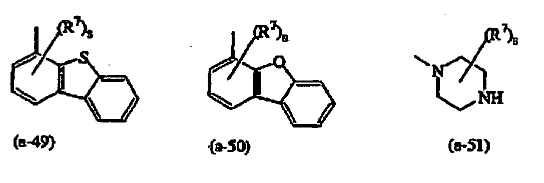
wherein each s is independently 0, 1, 2, 3, 4 or 5; each R⁶ and R⁷ are independently selected from hydrogen; halo; hydroxy; amino; nitro; trihaloC₁₋₆alkyl; trihaloC₁₋₆alkyloxy; C₁₋₆alkyl; C₁₋₆alkyl substituted with aryl and C₃₋₁₀cycloalkyl; C₁₋₆alkyloxy; C₁₋₆alkyloxyC₁₋₆alkyloxy; C₁₋₆alkylcarbonyl; C₁₋₆alkyloxycarbonyl; C₁₋₆alkylsulfonyl; cyanoC₁₋₆alkyl; hydroxyC₁₋₆alkyl; hydroxyC₁₋₆alkyloxy; hydroxyC₁₋₆alkylamino; aminoC₁₋₆alkyloxy; di(C₁₋₆alkyl)aminocarbonyl; di(hydroxyC₁₋₆alkyl)amino; (aryl)(C₁₋₆alkyl)amino; di(C₁₋₆alkyl)aminoC₁₋₆alkyloxy; di(C₁₋₆alkyl)aminoC₁₋₆alkylamino; di(C₁₋₆alkyl)aminoC₁₋₆alkylaminoC₁₋₆alkyl; arylsulfonyl; arylsulfonylamino; aryloxy; aryloxyC₁₋₆alkyl; arylC₂₋₆alkenediyl; di(C₁₋₆alkyl)amino; di(C₁₋₆alkyl)aminoC₁₋₆alkyl; di(C₁₋₆alkyl)amino(C₁₋₆alkyl) amino; di(C₁₋₆alkyl)amino(C₁₋₆alkyl)aminoC₁₋₆alkyl; di(C₁₋₆alkyl)aminoC₁₋₆alkyl(C₁₋₆alkyl)amino; di(C₁₋₆alkyl)aminoC₁₋₆alkyl(C₁₋₆alkyl)aminoC₁₋₆alkyl; aminosulfonylamino(C₁₋₆alkyl)amino; aminosulfonylamino(C₁₋₆alkyl)aminoC₁₋₆alkyl; di(C₁₋₆alkyl)aminosulfonylamino(C₁₋₆alkyl)amino; di(C₁₋₆alkyl) aminosulfonylamino(C₁₋₆alkyl)aminoC₁₋₆alkyl; cyano; thiophenyl; thiophenyl substituted with di(C₁₋₆alkyl)aminoC₁₋₆alkyl(C₁₋₆alkyl)aminoC₁₋₆alkyl, di(C₁₋₆alkyl)aminoC₁₋₆alkyl, C₁₋₆alkyl piperazinylC₁₋₆alkyl, hydroxyC₁₋₆alkylpiperazinylC₁₋₆alkyl, hydroxyC₁₋₆alkyloxyC₁₋₆alkylpiperazinylC₁₋₆alkyl, di(C₁₋₆alkyl)aminosulfonylpiperazinylC₁₋₆alkyl, C₁₋₆alkyloxypiperidinyl, C₁₋₆alkyloxypiperidinylC₁₋₆alkyl, morpholinylC₁₋₆alkyl, hydroxyC₁₋₆alkyl(C₁₋₆alkyl)aminoC₁₋₆alkyl, or di(hydroxyC₁₋₆alkyl) aminoC₁₋₆alkyl; furanyl; furanyl substituted with hydroxyC₁₋₆alkyl; benzofuranyl; imidazolyl; oxazolyl; oxazolyl substituted with aryl and C₁₋₆alkyl; C₁₋₆alkyltriazolyl; tetrazolyl; pyrrolidinyl; pyrrolyl; piperidinylC₁₋₆alkyloxy; morpholinyl; C₁₋₆alkylmorpholinyl; morpholinylC₁₋₆alkyloxy; morpholinylC₁₋₆alkyl; morpholinylC₁₋₆alkylamino; morpholinylC₁₋₆alkylaminoC₁₋₆alkyl; piperazinyl; C₁₋₆alkylpiperazinyl; C₁₋₆alkylpiperazinylC₁₋₆alkyloxy; piperazinylC₁₋₆alkyl; naphtalenylsulfonylpiperazinyl; naphtalenylsulfonylpiperidinyl; naphtalenylsulfonyl; C₁₋₆alkylpiperazinylC₁₋₆alkyl; C₁₋₆alkylpiperazinylC₁₋₆alkylamino; CvalkylpiperazinylC₁₋₆alkylaminoC₁₋₆alkyl; C₁₋₆alkylpiperazinylsulfonyl; aminosulfonylpiperazinylC₁₋₆alkyloxy; aminosulfonylpiperazinyl; aminosulfonylpiperazinylC₁₋₆alkyl; di(C₁₋₆alkyl) aminosulfonylpiperazinyl; di(C₁₋₆alkyl)aminosulfonylpiperazinylC₁₋₆alkyl; hydroxyC₁₋₆alkylpiperazinyl; hydroxyC₁₋₆alkylpiperazinylC₁₋₆alkyl; C₁₋₆alkyloxypiperidinyl; C₁₋₆alkyloxypiperidinylC₁₋₆alkyl; piperidinylaminoC₁₋₆alkylamino; piperidinylaminoC₁₋₆alkylaminoC₁₋₆alkyl; (C₁₋₆alkylpiperidinyl)(hydroxyC₁₋₆alkyl)aminoC₁₋₆alkylamino, (C₁₋₆alkylpiperidinyl)(hydroxyC₁₋₆alkyl)aminoC₁₋₆alkylaminoC₁₋₆alkyl; hydroxyC₁₋₆alkyloxyC₁₋₆alkylpiperazinyl; hydroxyC₁₋₆alkyloxyC₁₋₆alkylpiperazinylC₁₋₆alkyl; (hydroxyC₁₋₆alkyl)(C₁₋₆alkyl)amino; (hydroxyC₁₋₆alkyl)(C₁₋₆alkyl) aminoC₁₋₆alkyl; hydroxyC₁₋₆alkylaminoC₁₋₆alkyl; di(hydroxyC₁₋₆alkyl) aminoC₁₋₆alkyl; pyrrolidinylC₁₋₆alkyl; pyrrolidinylC₁₋₆alkyloxy; pyrazolyl; thiopyrazolyl; pyrazolyl substituted with two substituents selected from C₁₋₆alkyl or trihaloC₁₋₆alkyl; pyridinyl; pyridinyl substituted with C₁₋₆alkyloxy, aryloxy or aryl; pyrimidinyl; tetrahydropyrimidinylpiperazinyl; tetrahydropyrimidinylpiperazinylC₁₋₆alkyl; quinolinyl; indole; phenyl; phenyl substituted with one, two or three substituents independently selected from halo, amino, nitro, C₁₋₆alkyl, C₁₋₆alkyloxy, hydroxyC₁₋₆alkyl, trifluoromethyl, trifluoromethyloxy, hydroxyC₁₋₄alkyloxy, C₁₋₄alkylsulfonyl, C₁₋₄alkyloxyC₁₋₄alkyloxy, C₁₋₄alkyloxycarbonyl, aminoC₁₋₄alkyloxy, di(C₁₋₄alkyl)aminoC₁₋₄alkyloxy, di(C₁₋₄alkyl)amino, di(C₁₋₄alkyl)aminocarbonyl, di(C₁₋₄alkyl)aminoC₁₋₄alkyl, di(C₁₋₄alkyl)aminoC₁₋₄alkylaminoC₁₋₄alkyl, di(C₁₋₄alkyl)amino(c₁₋₄alkyl)amino, di(C₁₋₄alkyl)amino(C₁₋₄alkyl)aminoC₁₋₄alkyl, di(C₁₋₄alkyl)aminoC₁₋₄alkyl(C₁₋₄alkyl)amino, di(C₁₋₄alkyl)aminoC₁₋₄alkyl(C₁₋₄alkyl)aminoC₁₋₄alkyl, aminosulfonylamino(C₁₋₄alkyl)amino, aminosulfonylamino(C_{1- 4}alkyl) aminoC₁₋₄alkyl, di(C₁₋₄alkyl)aminosulfonylamino(C₁₋₄alkyl)amino, di(C₁₋₄alkyl)aminosulfonylamino(C₁₋₄alkyl)aminoC₁₋₆alkyl, cyano, piperidinylC₁₋₄alkyloxy, pyrrolidinylC₁₋₄alkyloxy, aminosulfonylpiperazinyl, aminosulfonylpiperazinylC₁₋₄alkyl, di(C₁₋₄alkyl)aminosulfonylpiperazinyl, di(C₁₋₄alkyl)aminosulfonylpiperazinylC₁₋₄alkyl, hydroxyC₁₋₄alkylpiperazinyl, hydroxyC₁₋₄alkylpiperazinylC₁₋₄alkyl, C₁₋₄alkyloxypiperidinyl, C₁₋₄alkyloxypiperidinylC₁₋₄alkyl, hydroxyC₁₋₄alkyloxyC₁₋₄alkylpiperazinyl, hydroxyC₁₋₄alkyloxyC₁₋₄alkylpiperazinylC₁₋₄alkyl, (hydroxyC₁₋₄alkyl)(C₁₋₄alkyl)amino, (hydroxyC₁₋₄alkyl)(C₁₋₄alkyl) aminoC₁₋₄alkyl, di(hydroxyC₁₋₄alkyl)amino, di(hydroxyC₁₋₄alkyl)aminoC₁₋₄alkyl, furanyl, furanyl substituted with-CH=CH-CH=CH-, pyrrolidinylC₁₋₄alkyl, pyrrolidinylC₁₋₄alkyloxy, morpholinyl, morpholinylC₁₋₄alkyloxy, morpholinylC₁₋₄alkyl, morpholinylC₁₋₄alkylamino, morpholinylC₁₋₄alkylaminoC₁₋₄alkyl, piperazinyl, C₁₋₄alkylpiperazinyl, C₁₋₄alkylpiperazinylC₁₋₄alkyloxy, piperazinylC₁₋₄alkyl, C₁₋₄alkylpiperazinylC₁₋₄alkyl, C₁₋₄alkylpiperazinylC₁₋₄alkylamino, C₁₋₄alkylpiperazinylC₁₋₄alkylaminoC₁₋₄alkyl, tetrahyfropyrimidinylpiperazinyl, tetrahydropyrimidinylpiperazinylC₁₋₄alkyl, piperidinylaminoC₁₋₄alkylamino, piperidinylaminoC₁₋₄alkylaminoC₁₋₄alkyl, (C₁₋₄alkylpiperidinyl)(hydroxyC₁₋₄alkyl)aminoC₁₋₄alkylamino, (C₁₋₄alkylpiperidinyl)(hydroxyC₁₋₄alkyl)aminoC₁₋₄alkylaminoC₁₋₄alkyl, pyridinylC₁₋₄alkyloxy, hydroxyC₁₋₄alkylamino, hydroxyC₁₋₄alkylaminoC₁₋₄alkyl, di(C₁₋₄alkyl)aminoC₁₋₄alkylamino, aminothiadiazolyl, aminosulfonylpiperazinylC₁₋₄alkyloxy, or thiophenylC₁₋₄alkylamino; each R⁶ and R⁷ can be placed on the nitrogen in replacement of the hydrogen; aryl in the above is phenyl, or phenyl substituted with one or more substituents each independently selected from halo, C₁₋₄alkyl, C₁₋₆alkyloxy, trifluoromethyl, cyano or hydroxycarbonyl.

In one aspect, the inhibitor of histone deacetylase activity may be a sulfonylamino derivative, such as, for example, compounds disclosed in WO2003076401, having the general formula: 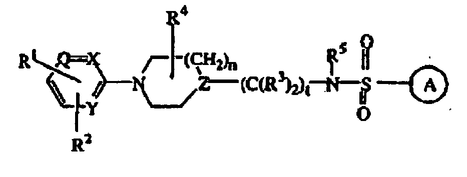
the N-oxide forms, the pharmaceutically acceptable addition salts and the stereo chemically isomeric forms thereof, wherein n is 0, 1, 2 or 3 and when n is 0 then a direct bond is intended; t is 0, 1, 2, 3 or 4 and when t is 0 then a direct bond is intended;
each Q is nitrogen or 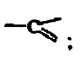
each X is nitrogen or 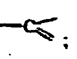
each Y is nitrogen or 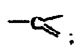
each Z is nitrogen or 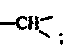
R¹ is-C(O)NR⁸R⁹, -N(H)C(O)R¹⁰, -C(O)-C₁₋₆alkanediylSR¹⁰, -NRC(O)N(OH)R¹⁰, - NR¹¹C(O)C₁₋₆alkanediylSR¹⁰, -NR¹¹C(O)C=N(OH)R¹⁰ or another Zn-chelating group wherein R⁸ and R⁹ are each independently selected from hydrogen, hydroxy, C₁₋₆alkyl, hydroxyC₁₋₆alkyl, aminoC₁₋₆alkyl or aminoaryl; R¹⁰ is independently selected from hydrogen, C₁₋₆alkyl, C₁₋₆alkylcarbonyl, arylC₁₋₆alkyl, C₁₋₆alkylpyrazinyl, pyridinone, pyrrolidinone or methylimidazolyl;: R¹¹ is independently selected from hydrogen or C₁₋₆alkyl; R² is hydrogen, halo, hydroxy, amino nitro, C₁₋₆alkyl, C₁₋₆alkyloxy, trifluoromethyl, di(C₁₋₆alkyl)amino, hydroxyamino or naphtalenylsulfonylpyrazinyl; each R³ independently represents a hydrogen atom and one hydrogen atom can be replaced by a substituent selected from aryl; R⁴ is hydrogen, hydroxy, amino, hydroxyC₁₋₆alkyl, Cl₁₋₆alkyl, C₁₋₆alkyloxy, arylC₁₋₆alkyl, aminocarbonyl, hydroxycarbonyl, aminoC₁₋₆alkyl, aminocarbonylC₁₋₆alkyl, hydroxycarbonylC₁₋₆alkyl, hydroxyaminocarbonyl, C₁₋₆alkyloxycarbonyl, C₁₋₆alkylaminoC₁₋₆alkyl or di(C₁₋₆alkyl)aminoC₁₋₆alkyl; R⁵ is hydrogen, C₁₋₆alkyl, C₃₋₁₀cycloalkyl, hydroxyC₁₋₆alkyl, C₁₋₆alkyloxyC₁₋₆alkyl, di(C₁₋₆alkyl)aminoC₁₋₆alkyl or aryl; 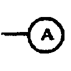 is a radical selected from 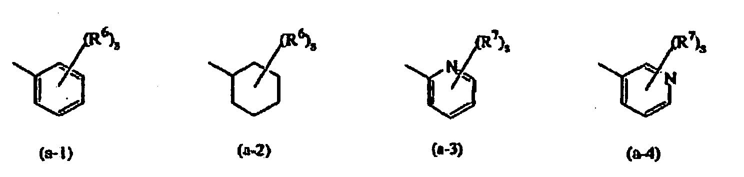 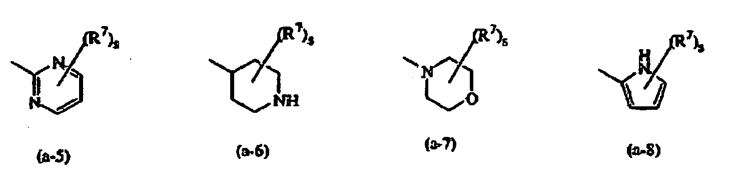 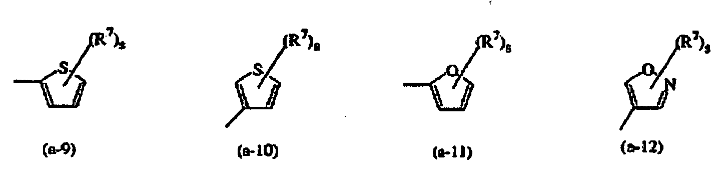 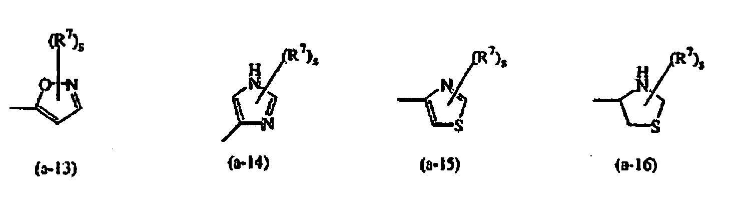 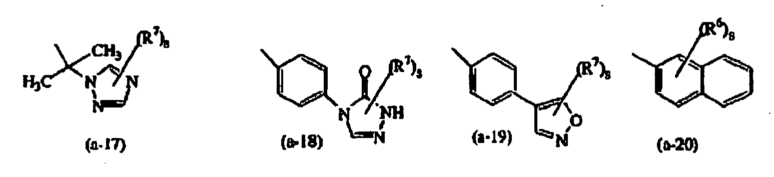 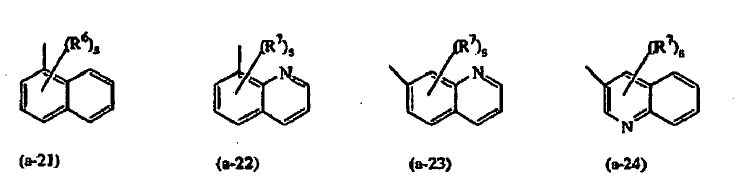 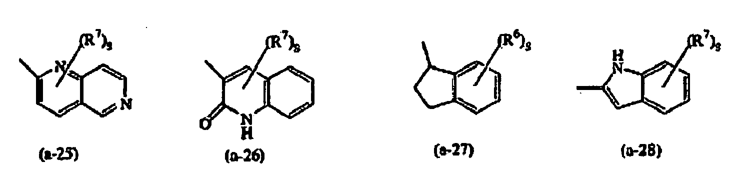 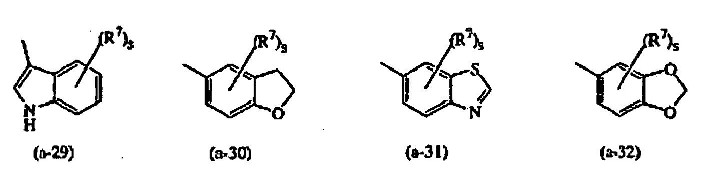 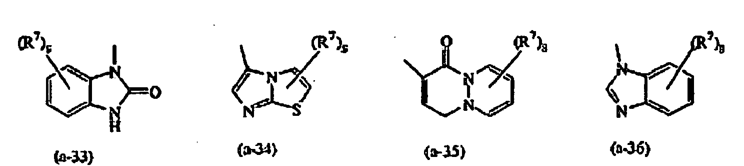 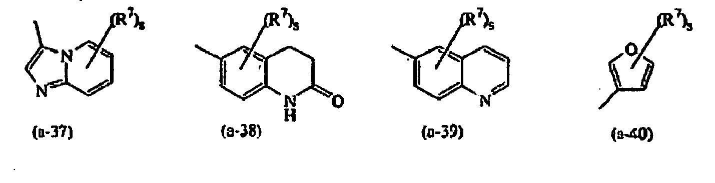 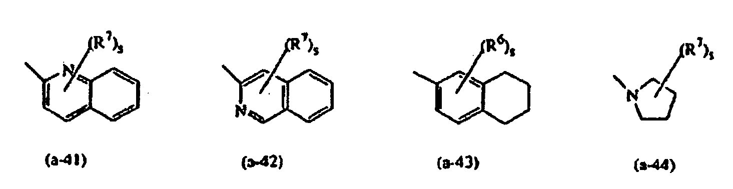 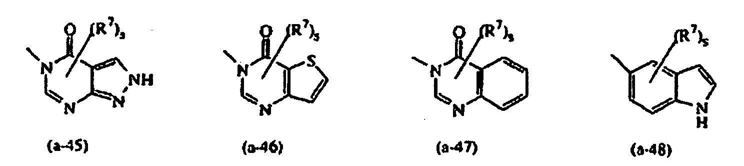 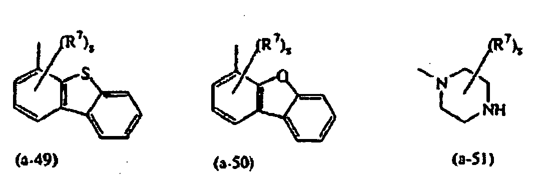
wherein each s is independently 0, 1, 2, 3, 4 or 5; each R⁶ and R⁷ are independently selected from hydrogen; halo; hydroxy; amino; nitro; trihaloC₁₋₆alkyl; trihaloC₁₋₆alkyloxy; C₁₋₆alkyl; C₁₋₆alkyl substituted with aryl and C₃₋₁₀cycloalkyl; C₁₋₆alkyloxy; C₁₋₆alkyloxyC₁₋₆alkyloxy; C₁₋₆alkylcarbonyl; C₁₋₆alkyloxycarbonyl; C₁₋₆alkylsulfonyl; cyanoC₁₋₆alkyl; hydroxyC₁₋₆alkyl; hydroxyC₁₋₆alkyloxy; hydroxyC₁₋₆alkylamino; aminoC₁₋₆alkyloxy; di(C₁₋₆alkyl)aminocarbonyl; di(hydroxyC₁₋₆alkyl)amino; (aryl)(C₁₋₆alkyl)amino; di(C₁₋₆alkyl)aminoC₁₋₆alkyloxy; di(C₁₋₆alkyl)aminoC₁₋₆alkylamino; di(C₁₋₆alkyl)aminoC₁₋₆alkylaminoC₁₋₆alkyl; arylsulfonyl; arylsulfonylamino; aryloxy; aryloxyC₁₋₆alkyl; arylC₂₋₆alkenediyl; di(C₁₋₆alkyl)amino; di(C₁₋₆alkyl)aminoC₁₋₆alkyl; di(C₁₋₆alkyl)amino(C₁₋₆alkyl)amino; di(C₁₋₆alkyl)amino(C₁₋₆alkyl)aminoC₁₋₆alkyl; di(C₁₋₆alkyl)aminoC₁₋₆alkyl(C₁₋₆alkyl)amino; di(C₁₋₆alkyl)aminoC₁₋₆alkyl(C₁₋₆alkyl)aminoC₁₋₆alkyl; aminosulfonylamino(C₁₋₆alkyl)amino; aminosulfonylamino(C₁₋₆alk-yl)aminoC₁₋₆alkyl; di(C₁₋₆alkyl)aminosulfonylamino(C₁₋₆alkyl)amino; di(C₁₋₆alkyl) aminosulfonylamino(C₁₋₆alkyl)aminoC₁₋₆alkyl; cyano; thiophenyl; thiophenyl substituted with di(C₁₋₆alkyl)aminoC₁₋₆alkyl(C₁₋₆alkyl)aminoC₁₋₆alkyl, di(C₁₋₆alkyl)aminoC₁₋₆alkyl, C₁₋₆alkylpiperazinylC₁₋₆alkyl, hydroxyC₁₋₆alkylpiperazinylC₁₋₆alkyl, hydroxyC₁₋₆alkyloxyC₁₋₆alkylpiperazinylC₁₋₆alkyl, di(C₁₋₆alkyl)aminosulfonylpiperazinylC₁₋₆alkyl, C₁₋₆alkyloxypiperidinyl, C₁₋₆alkyloxypiperidinylC₁₋₆alkyl), morphohnylC₁₋₆alkyl, hydroxyC₁₋₆alkyl(C₁₋₆alkyl)aminoC₁₋₆alkyl, or di(hydroxyC₁₋₆alkyl) aminoC₁₋₆alkyl; furanyl; furanyl substituted with hydroxyC₁₋₆alkyl; benzofuranyl; imidazolyl; oxazolyl; oxazolyl substituted with aryl and C₁₋₆alkyl; C₁₋₆alkyltriazolyl; tekazolyl; pyrrolidinyl; pyrrolyl; piperidinylC₁₋₆alkyloxy; morpholinyl; C₁₋₆alkylmorpholinyl; morpholinylC₁₋₆alkyloxy; morpholinylC₁₋₆alkyl; morpholinylC₁₋₆alkylamino; morpholinylC₁₋₆alkylaminoC₁₋₆alkyl; piperazinyl; C₁₋₆alkylpiperazinyl; C₁₋₆alkylpiperazinylC₁₋₆alkyloxy; piperazinylC₁₋₆alkyl; naphtalenylsulfonylpiperazinyl; naphtalenylsulfonylpiperidinyl; naphtalenylsulfonyl; C₁₋₆alkylpiperazinylC₁₋₆alkyl; C₁₋₆alkylpiperazinylC₁₋₆alkylamino; C₁₋₆alkylpiperazinylC₁₋₆alkylaminoC₁₋₆alkyl; C₁₋₆alkylpiperazinylsulfonyl; aminosulfonylpiperazinylC₁₋₆alkyloxy; aminosulfonylpiperazinyl; aminosulfonylpiperazinylC₁₋₆alkyl; di(C₁₋₆alkyl) aminosulfonylpiperazinyl; di(C₁₋₆alkyl)aminosulfonylpiperazinylC₁₋₆alkyl; hydroxyC₁₋₆alkylpiperazinyl; hydroxyC₁₋₆alkylpiperazinylC₁₋₆alkyl; C₁₋₆alkyloxypiperidinyl; C₁₋₆alkyloxypiperidinylC₁₋₆alkyl; piperidinylaminoC₁₋₆alkylamino; piperidinylaminoC₁₋₆alkylaminoC₁₋₆alkyl; (C₁₋₆alkylpiperidinyl)(hydroxyC₁₋₆alkyl)aminoC₁₋₆alkylamino; (C₁₋₆alkylpiperidinyl)(hydroxyC₁₋₆alkyl)aminoC₁₋₆alkylaminoC₁₋₆alkyl; hydroxyC₁₋₆alkyloxyC₁₋₆alkylpiperazinyl; hydroxyC₁₋₆alkyloxyC₁₋₆alkylpiperazinylC₁₋₆alkyl; (hydroxyC₁₋₆alkyl)(C₁₋₆alkyl)amino; (hydroxyC₁₋₆alkyl)(C₁₋₆alkyl)aminoC₁₋₆alkyl; hydroxy₁₋₆ alkylaminoC₁₋₆alkyl; di(hydroxyC₁₋₆alkyl)aminoC₁₋₆alkyl; pyrrolidinylC₁₋₆alkyl; pyrrolidinylC₁₋₆alkyloxy; pyrazolyl; thiopyrazolyl; pyrazolyl substituted with two substituents selected from C₁₋₆alkyl or trihaloC₁₋₆alkyl; pyridinyl; pyridinyl substituted with C₁₋₆alkyloxy, aryloxy or aryl; pyrimidinyl; tetrahydropyrimidinylpiperazinyl; tetrahydropyrimidinylpiperazinylC₁₋₆alkyl; quinolinyl; indole; phenyl; phenyl substituted with one, two or three substituents independently selected from halo, amino, nitro, C₁₋₆alkyl, C₁₋₆alkyloxy, hydroxyC₁₋₄alkyl, trifluoromethyl, trifluoromethyloxy, hydroxyC₁₋₄alkyloxy, C₁₋₄alkylsulfonyl, C₁₋₄alkyloxyC₁₋₄alkyloxy, C₁₋₄alkyloxycarbonyl, aminoC₁₋₄alkyloxy, di(C₁₋₄alkyl)aminoC₁₋₄alkyloxy, di(C₁₋₄alkyl)amino, di(C₁₋₄alkyl)aminocarbonyl, di(C₁₋₄alkyl)aminoC₁₋₄alkyl, di(C₁₋₄alkyl) aminoC₁₋₄alkylaminoC₁₋₄alkyl, di(C₁₋₄alkyl)amino(C₁₋₄alkyl)amino, di(C₁₋₄alkyl)amino(C₁₋₄alkyl)aminoC₁₋₄alkyl, di(C₁₋₄alkyl)aminoC₁₋₄alkyl(C₁₋₄alkyl)amino, di(C₁₋₄alkyl)aminoC₁₋₄alkyl(C₁₋₄alkyl)aminoC₁₋₄alkyl, aminosulfonylamino(C₁₋₄alkyl)amino, aminosulfonylamino(C_{1- 4}alkyl)aminoC₁₋₄alkyl, di(C₁₋₄alkyl)aminosulfonylamino(C₁₋₄alkyl)amino, di(C₁₋₄alkyl) aminosulfonylamino(C₁₋₄alkyl)aminoC₁₋₆alkyl, cyano, piperidinylC₁₋₄alkyloxy, pyrrolidinylC₁₋₄alkyloxy, aminosulfonylpiperazinyl, aminosulfonylpiperazinylC₁₋₄alkyl, di(C₁₋₄alkyl)aminosulfonylpiperazinyl, di(C₁₋₄alkyl)aminosulfonylpiperazinylC₁₋₄alkyl, hydroxyC₁₋₄alkylpiperazinyl, hydroxyC₁₋₄alkylpiperazinylC₁₋₄alkyl, C₁₋₄alkyloxypiperidinyl, C₁₋₄alkyloxypiperidinylC₁₋₄alkyl, hydroxyC₁₋₄alkyloxyC₁₋₄alkylpiperazinyl, hydroxyC₁₋₄alkyloxyC₁₋₄alkylpiperazinylC₁₋₄alkyl, (hydroxyC₁₋₄alkyl)(C₁₋₄alkyl)amino, (hydroxyC₁₋₄alkyl)(C₁₋₄alkyl)aminoC₁₋₄alkyl, di(hydroxyC₁₋₄alkyl)amino, di(hydroxyC₁₋₄alkyl)aminoC 4alkyl, furanyl, furanyl substituted with - CH=CH-CH=CH-, pyrrolidinylC₁₋₄alkyl, pyrrolidinylC₁₋₄alkyloxy, morpholinyl, morpholinylC₁₋₄alkyloxy, morpholinylC₁₋₄alkyl, morpholinylC₁₋₄alkylamino, morpholinylC₁₋₄alkylaminoC₁₋₄alkyl, piperazinyl, C₁₋₄alkylpiperazinyl, C₁₋₄alkylpiperazinylC₁₋₄alkyloxy, piperazinylC₁₋₄alkyl, C₁₋₄alkylpiperazinylC₁₋₄alkyl, C₁₋₄alkylpiperazinylC₁₋₄alkylamino, C₁₋₄alkylpiperazinylC₁₋₄alkylaminoC₁₋₆alkyl, tetrahydropyrimidinylpiperazinyl, tetrahydropyrimidinylpiperazinylC₁₋₄alkyl, piperidinylaminoC₁₋₄alkylamino, piperidinylaminoC₁₋₄alkylaminoC₁₋₄alkyl, (C₁₋₄alkylpiperidinyl)(hydroxyC₁₋₄alkyl)aminoC₁₋₄alkylamino, (C₁₋₄alkylpiperidinyl)(hydroxyC₁₋₄alkyl)aminoC₁₋₄alkylaminoC₁₋₄alkyl, pyridinylC₁₋₄alkyloxy, hydroxyC₁₋₄alkylamino, hydroxyC₁₋₄alkylaminoC₁₋₄alkyl, di(C₁₋₄alkyl)aminoC₁₋₄alkylamino, aminothiadiazolyl, aminosulfonylpiperazinylC₁₋₄alkyloxy, or thiophenylC₁₋₄alkylamino; each R⁶ and R⁷ can be placed on the nitrogen in replacement of the hydrogen; aryl in the above is phenyl, or phenyl substituted with one or more substituents each independently selected from halo, C₁₋₆alkyl, C₁₋₆alkyloxy, trifluoromethyl, cyano or hydroxycarbonyl.

In one aspect, the inhibitor of histone deacetylase activity may be a compound of the general formula: 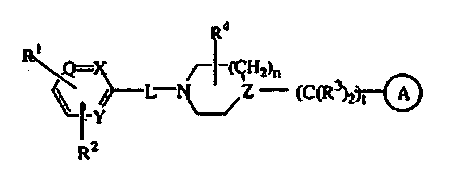
the N-oxide forms, the pharmaceutically acceptable addition salts and the stereo-chemically isomeric forms thereof, wherein n is 0, 1, 2 or 3 and when n is 0 then a direct bond is intended; t is 0, 1, 2, 3 or 4 and when t is 0 then a direct bond is intended;
each Q is nitrogen or 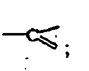
each X is nitrogen or 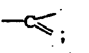
each Y is nitrogen or 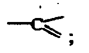
each Z is nitrogen or 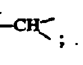
R¹ is-C(O)NR⁷R⁸, -NHC(O)R⁹, -C(O)-C₁₋₆alkanediylSR⁹, -NR¹⁰OC(O)N(OH)R⁹, - NR¹⁰C(O)C₁₋₆alkanediylSR⁹, -NR¹⁰C(O) C=N(OH)R⁹ or another Zn-chelating group wherein R⁷ and R⁸ are each independently selected from hydrogen, hydroxy, C₁₋₆alkyl, hydroxyC₁₋₆alkyl, amino₁₋₆alkyl or aminoaryl; R⁹ is independently selected from hydrogen, C₁₋₆alkyl, C₁₋₆alkylcarbonyl, arylC₁₋₆alkyl, C₁₋₆alkylpyrazinyl, pyridinone, pyrrolidinone or methylimidazolyl; R¹⁰ is independently selected from hydrogen or C₁₋₆alkyl; R² is hydrogen, halo, hydroxy, amino, nitro, C₁₋₆alkyl, C₁₋₆alkyloxy, trifluoromethyl, di(C₁₋₆alkyl)amino, hydroxyamino or naphtalenylsulfonylpyrazinyl; -L-is a direct bond or a bivalent radical selected from C₁₋₆alkanediyl, C₁₋₆alkanediyloxy, amino, carbonyl or aminocarbonyl; each R³ independently represents a hydrogen atom and one hydrogen atom can be replaced by a substituent selected from aryl; R⁴ is hydrogen, hydroxy, amino, hydroxyC₁₋₆alkyl, C₁₋₆alkyl, C₁₋₆alkyloxy, 1 arylC₁₋₆alkyl, aminocarbonyl, hydroxycarbonyl, aminoC₁₋₆alkyl, aminocarbonylC₁₋₆alkyl, hydroxycarbonylC₁₋₆alkyl, hydroxyaminocarbonyl, C₁₋₆alkyloxycarbonyl, C₁₋₆alkylaminoC₁₋₆alkyl or di(C₁₋₆alkyl)aminoC₁₋₆alkyl; 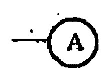 is a radical selected from 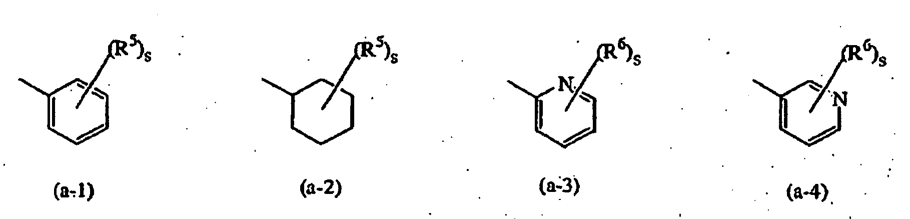 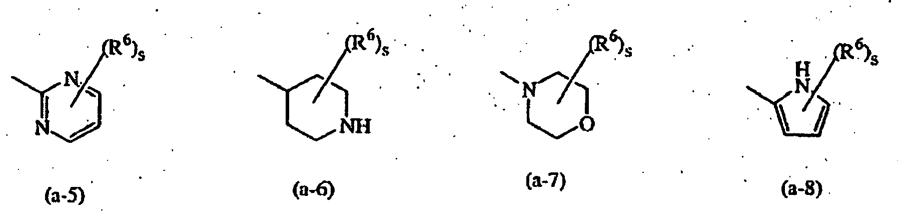 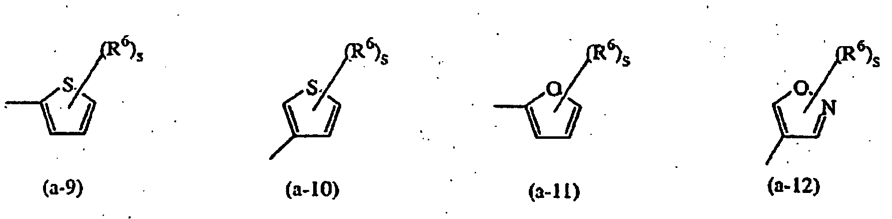 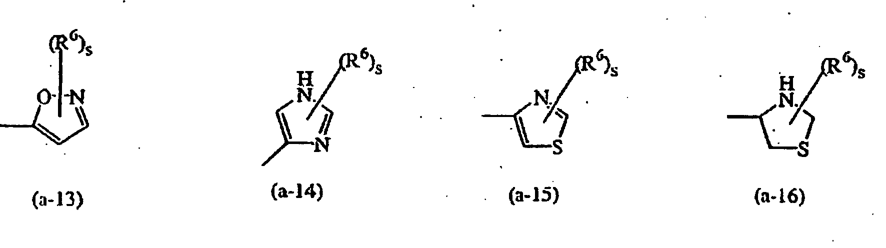 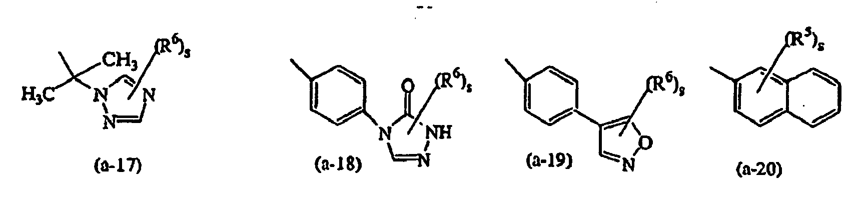 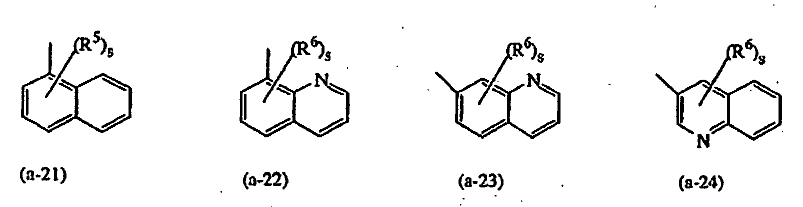 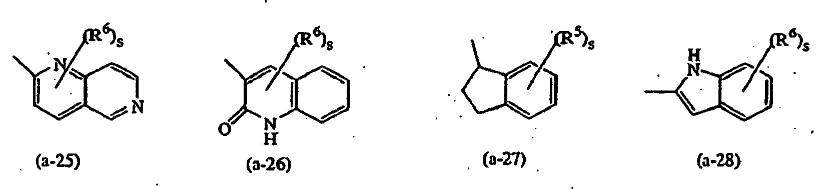 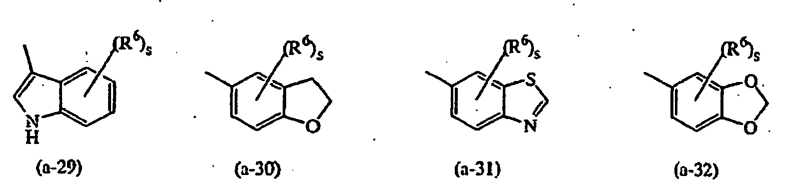 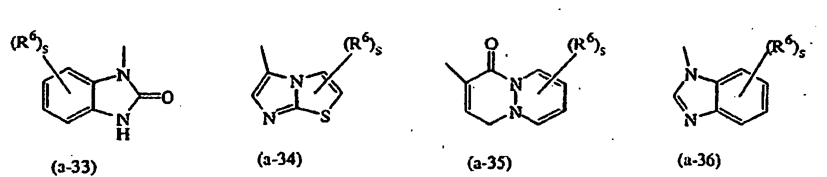 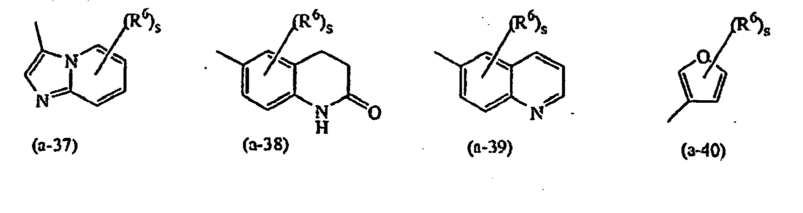 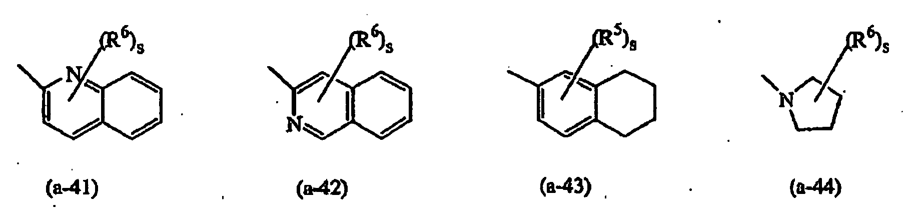 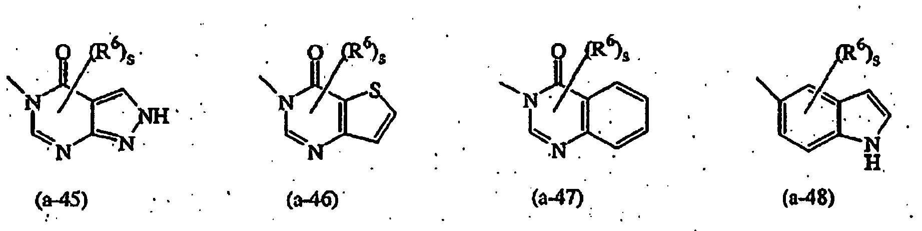 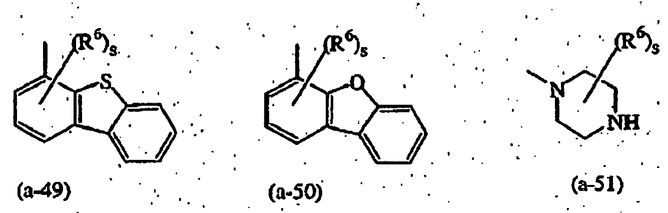
wherein each s is independently 0, 1, 2, 3, 4 or 5; each R⁵ and R⁶ are independently selected from hydrogen; halo; hydroxy; amino; nitro; trihaloC₁₋₆alkyl; trihaloC₁₋₆alkyloxy; C₁₋₆alkyl; C₁₋₆alkyl substituted with aryl and C₃₋₁₀cycloalkyl; C₁₋₆alkyloxy; C₁₋₆alkyloxyC₁₋₆alkyloxy; C₁₋₆alkylcarbonyl; C₁₋₆alkyloxycarbonyl, C₁₋₆alkylsulfonyl; cyanoC₁₋₆alkyl; hydroxyC₁₋₆alkyl; hydroxyC₁₋₆alkyloxy; hydroxyC₁₋₆alkylamino; aminoC₁₋₆alkyloxy; di(C₁₋₆alkyl)aminocarbonyl; di(hydroxyC₁₋₆alkyl)amino; (aryl)(C₁₋₆alkyl)amino; di(C₁₋₆alkyl) aminoC₁₋₆alkyloxy; di(C₁₋₆alkyl)aminoC₁₋₆alkylamino;; di(C₁₋₆alkyl) aminoC₁₋₆alkylaminoC₁₋₆alkyl; arylsulfonyl; arylsulfonylamino; aryloxy; aryloxyC₁₋₆alkyl; arylC₂₋₆alkenediyl; di(C₁₋₆alkyl)amino; di(C₁₋₆alkyl) aminoC₁₋₆alkyl; di(C₁₋₆alkyl)amino(C₁₋₆alkyl)amino, di(C₁₋₆alkyl)amino(C₁₋₆alkyl)aminoC₁₋₆alkyl; di(C₁₋₆alkylaminoC₁₋₆alkyl(C₁₋₆alkyl)amino; di(C₁₋₆alkyl)aminoC₁₋₆alkyl(C₁₋₆alkyl)aminoC₁₋₆alkyl; aminosulfonylamino(C₁₋₆alkyl)amino; aminosulfonylamino(C₁₋₆alkyl) aminoC₁₋₆alkyl; di(C₁₋₆alkyl)aminosulfonylamino(C₁₋₆alkyl)amino; di(C₁₋₆alkyl)aminosulfonylamino(C₁₋₆alkyl)aminoC₁₋₆alkyl; cyano; thiophenyl; thiophenyl substituted with di(C₁₋₆alkyl)aminoC₁₋₆alkyl(C₁₋₆alkyl) aminoC₁₋₆alkyl, di(C_{1- 6}alkyl)aminoC₁₋₆alkyl, C₁₋₆alkylpiperazinylC₁₋₆alkyl, hydroxyC₁₋₆alkylpiperazinylC₁₋₆alkyl, hydroxyC₁₋₆alkyloxyC₁₋₆alkylpiperazinylC₁₋₆alkyl, di(C₁₋₆alkyl) aminosulfonylpiperazinylC₁₋₆alkyl, C₁₋₆alkyloxypiperidinyl, C₁₋₆alkyloxypiperidinylC₁₋₆alkyl, morpholinylC₁₋₆alkyl, hydroxyC₁₋₆alkyl(C₁₋₆alkyl)aminoC₁₋₆alkyl, or di(hydroxyC₁₋₆alkyl)aminoC₁₋₆alkyl; furanyl; furanyl substituted with hydroxyC₁₋₆alkyl; benzofuranyl; imidazolyl; oxazolyl; oxazolyl substituted with aryl and C₁₋₆alkyl; C₁₋₆alkyltriazolyl; tetrazolyl; pyrrolidinyl; pyrrolyl; piperidinylC₁₋₆alkyloxy; morpholinyl; C₁₋₆alk-ylmorpholinyl; morpholinylC₁₋₆alkyloxy; morpholinylC₁₋₆alkyl; morpholinylC₁₋₆alkylamino; morpholinylC₁₋₆alkylaminoC₁₋₆alkyl; piperazinyl; C₁₋₆alkylpiperazinyl; C₁₋₆alkylpiperazinylC₁₋₆alkyloxy; piperazinylC₁₋₆alkyl; naphtalenylsulfonylpiperazinyl; naphtalenylsulfonylpiperidinyl; naphtalenylsulfonyl; C₁₋₆alkylpiperazinylC₁₋₆alkyl; C₁₋₆alkylpiperazinylC₁₋₆alkylamino; C₁₋₆alkylpiperazinylC₁₋₆alkylaminoC₁₋₆alkyl; C₁₋₆alkylpiperazinylsulfonyl; aminosulfonylpiperazinylC₁₋₆alkyloxy; aminosulfonylpiperazinyl; aminosulfonylpiperazinylC₁₋₆alkyl; di(C₁₋₆alkyl)aminosulfontylpiperazinyl; di(C₁₋₆alkyl)aminosulfonylpiperazinylC₁₋₆alkyl; hydroxyC₁₋₆alkylpiperazinyl; hydroxyC₁₋₆alkylpiperazinylC₁₋₆alkyl; C₁₋₆alkyloxypiperidinyl; C₁₋₆alkyloxypiperidinylC₁₋₆alkyl; piperidinylaminoC₁₋₆alkylamino; piperidinylaminoC₁₋₆alkylaminoC₁₋₆alkyl; : (C₁₋₆alkylpiperidinyl)(hydroxyC₁₋₆alkyl)aminoC₁₋₆alkylamino; (C₁₋₆alkylpiperidinyl)(hydroxyC₁₋₆alkyl)aminoC₁₋₆alkylaminoC₁₋₆alkyl; hydroxyC₁₋₆alkyloxyC₁₋₆alkylpiperazinyl; hydroxyC₁₋₆alkyloxyC₁₋₆alkylpiperazinylC₁₋₆alkyl; (hydroxyC₁₋₆alkyl)(C₁₋₆alkyl)amino; (hydroxyC₁₋₆alkyl)(C₁₋₆alkyl)aminoC₁₋₆alkyl; hydroxyC₁₋₆alkylaminoC₁₋₆alkyl; di(hydroxyC₁₋₆alkyl)aminoC₁₋₆alkyl; pyrrolidinylC₁₋₆alkyl; pyrrolidinytC₁₋₆alkyloxy; pyrazolyl; thiopyrazolyl; pyrazolyl substituted with two substituents selected from C₁₋₆alkyl or trihaloC₁₋₆alkyl; pyridinyl; pyridinyl substituted with C₁₋₆alkyloxy, aryloxy or aryl; pyrimidinyl; tetrahydropyrimidinylpiperazinyl; tetrahydropyrimidinylpiperazinylC₁₋₆alkyl; quinolinyl; indole; phenyl; phenyl substituted with one, two or three substituents independently selected from halo, amino, nitro, C₁₋₆alkyl, C₁₋₆alkyloxy, hydroxyC₁₋₄alkyl, trifluoromethyl, trifluoromethyloxy, hydroxyC₁₋₄alkyloxy, C₁₋₄alkylsulfonyl, C₁₋₄alkyloxyC₁₋₄alkyloxy, C₁₋₄alkyloxycarbonyl, aminoC₁₋₄alkyloxy, di(C₁₋₄alkyl)aminoC₁₋₄alkyloxy, di(C₁₋₄alkyl)amino, di(C₁₋₄alkyl)aminocarbonyl, di(C₁₋₄alkyl)aminoC₁₋₄alkyl, di(C₁₋₄alkyl) aminoC₁₋₄alkylaminoC₁₋₄alkyl, di(C₁₋₄alkyl)amino(C₁₋₄alkyl)amino, di(C₁₋₄alkyl)amino(C₁₋₄alkyl)aminoC₁₋₄alkyl, di(C₁₋₄alkyl)aminoC₁₋₄alkyl(C₁₋₄alkyl)amino, di(C1₋₄alkyl)aminoC₁₋₄alkyl(C₁₋₄alkyl)aminoC₁₋₄alkyl, aminosulfonylamino(C₁₋₄alkyl)amino, aminosulfonylamino(C₁₋₄alkyl)aminoC₁₋₄alkyl, di(C₁₋₄alkyl) aminosulfonylamino(C₁₋₄alkyl)amino, di(C₁₋₄alkyl)aminosulfonylamino(C₁₋₄alkyl)aminoC₁₋₆alkyl, cyano, piperidinylC₁₋₄alkyloxy, pyrrolidinylC₁₋₄alkyloxy, aminosulfonylpiperazinyl, aminosulfonylpiperazinylC₁₋₄alkyl, di(C₁₋₄alkyl)aminosulfonylpiperazinyl, di(C₁₋₄alkyl) aminosulfonylpiperazinylC₁₋₄alkyl, hydroxyC₁₋₄alkylpiperazinyl, hydroxyC₁₋₄alkylpiperazinylC₁₋₄alkyl, C₁₋₄alkyloxypiperidinyl, C₁₋₄alkyloxypiperidinylC₁₋₄alkyl, hydroxyC₁₋₄alkyloxyC₁₋₄alkylpiperazinyl, hydroxyC₁₋₄alkyloxyC₁₋₄alkylpiperazinylC₁₋₄alkyl, (hydroxyC₁₋₄alkyl)(C₁₋₄alkyl)amino, (hydroxyC₁₋₄alkyl)(C₁₋₄alkyl)aminoC₁₋₄alkyl,; di(hydroxyC₁₋₄alkyl)amino, di(hydroxyC₁₋₄alkyl)aminoC₁₋₄alkyl, furanyl, furanyl substituted with -CH-CH-CH=CH-, pyrrolidinylC₁₋₄alkyl, pyrrolidinylC₁₋₄alkyloxy, morpholinyl, morpholinylC₁₋₄alkyloxy, morpholinylC₁₋₄alkyl, morpholinylC₁₋₄alkylamino, morphotinylC₁₋₄alkylaminoC₁₋₄alkyl, piperazinyl, C₁₋₄alkylpiperazinyl, C₁₋₄alkylpiperazinylC₁₋₄alkyloxy, piperazinylC₁₋₄alkyl, C₁₋₄alkylpiperazinylC₁₋₄alkyl, C₁₋₄alkylpiperazinylC₁₋₄alkylamino, C₁₋₄alkylpiperazinylC₁₋₄alkylaminoC₁₋₆alkyl, tetrahydropyrimidinylpiperazinyl, tetrahydropyrimidinylpiperazinylC₁₋₄alkyl, piperidinytaminoC₁₋₄atkylamino, piperidinylaminoC₁₋₄alkylaminoC₁₋₄alkyl, (C₁₋₄alkylpiperidinyl)(hydroxyC₁₋₄alkyl)aminoC₁₋₄alkylamino, (C₁₋₄alkylpiperidinyl)(hydroxyC₁₋₄alkyl)aminoC₁₋₄alkylaminoC₁₋₄alkyl, pyridinylC₁₋₄alkyloxy, hydroxyC₁₋₄alkylamino, hydroxyC₁₋₄alkylaminoC₁₋₄alkyl, di(C₁₋₄alkyl)aminoC₁₋₄alkylamino, aminothiadiazolyl, aminosulfonylpiperazinylC₁₋₄alkyloxy, or thiophenylC₁₋₄alkylamino; each R⁵ and R⁶ can be placed on the nitrogen in replacement of the hydrogen; aryl in the above is phenyl, or phenyl substituted with one or more substituents each independently selected from halo, C₁₋₆alkyl, C₁₋₆alkyloxy, trifluoromethyl, cyano or hydroxycarbonyl. See, for example, compounds disclosed in W02003076400.

In one aspect, the inhibitor of histone deacetylase activity may be an aminocarbonyl derivative, such as, for example, compounds disclosed in W02003076421, having the general formula: 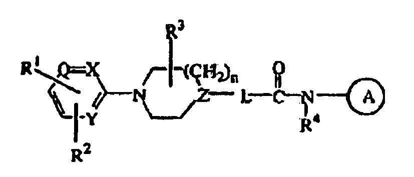
the *N*- oxide forms, the pharmaceutically acceptable addition salts and the stereo-chemically isomeric forms thereof, wherein n is 0, 1, 2 or 3 and when n is 0 then a direct bond is intended;
each Q is nitrogen or 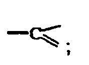
each X is nitrogen or 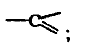
each Y is nitrogen or 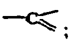
each Z is nitrogen or 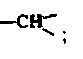
R¹ is-C(O)NR⁷R⁸,-N(H)C(O)R⁹,-C(O)-C₁₋₆alkanediylSR⁹,-NR¹⁰C(O)N(OH)R⁹, -N R¹⁰C(O)C₁₋₆alkanediylSR⁹,-NR¹⁰C(O)C=N(OH)R⁹ or another Zn-chelating group wherein R⁷ and R⁸ are each independently selected from hydrogen, hydroxy, C₁₋₆alkyl, hydroxyC₁₋₆alkyl, aminoC₁₋₆alkyl or aminoaryl; R⁹ is independently selected from hydrogen, C₁₋₆alkyl, C₁₋₆alkylcarbonyl, arylC₁₋₆alkyl, C₁₋₆alkylpyrazinyl, pyridinone, pyrrolidinone or methylimidazolyl; R¹⁰ is independently selected from hydrogen or C₁₋₆alkyl; R² is hydrogen, halo, hydroxy, amino, nitro, C₁₋₆alkyl, C₁₋₆alkyloxy, trifluoromethyl, di(C₁₋₆alkyl)amino, hydroxyamino or naphtalenylsulfonylpyrazinyl; R³ is hydrogen, hydroxy, amino, hydroxyC₁₋₆alkyl, C₁₋₆alkyl, C₁₋₆6alkyloxy, arylC₁₋₆alkyl, aminocarbonyl, hydroxycarbonyl, aminoC₁₋₆alkyl, aminocarbonylC₁₋₆alkyl, hydroxycarbonylC₁₋₆alkyl, hydroxyaminocarbonyl, C₁₋₆alkyloxycarbonyl, C₁₋₆alkylaminoC₁₋₆alkyl or di(C₁₋₆alkyl)aminoC₁₋₆alkyl; when Z is equal to nitrogen, then-L-is a direct bond; when Z is equal to 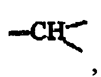 then -L- is -NH- or the bivalent radical -C₁₋₆alkanedlylNH-; R⁴ is hydrogen, C₁₋₆alkyl, C₃₋₆cycloalkyl, hydroxyC₁₋₆alkyl, C₁₋₆alkyloxyC₁₋₆alkyl, di(C₁₋₆alkyl)aminoC₁₋₆alkyl or aryl; 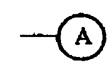 is a radical selected from 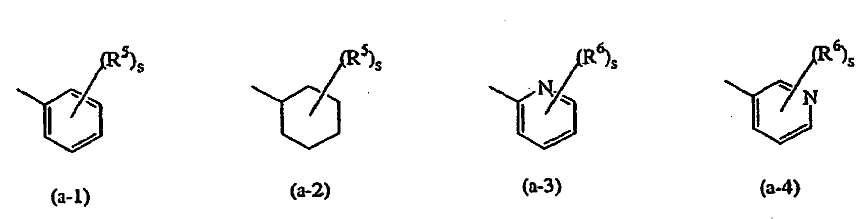 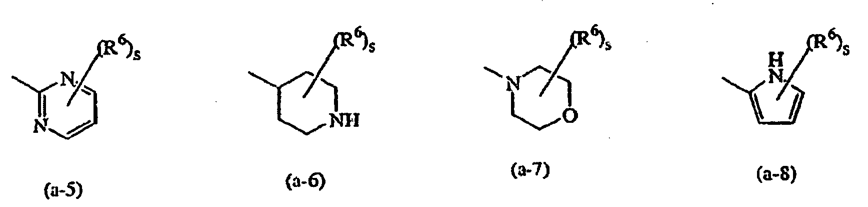 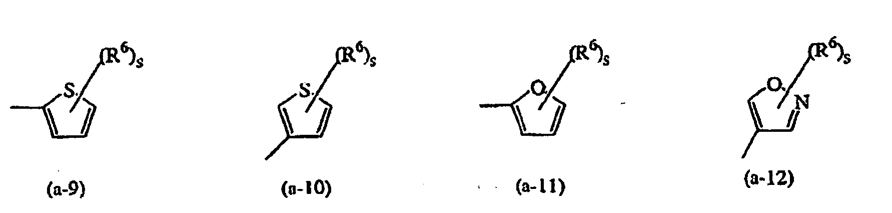 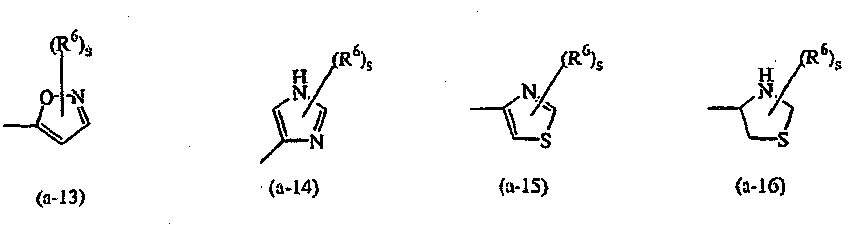 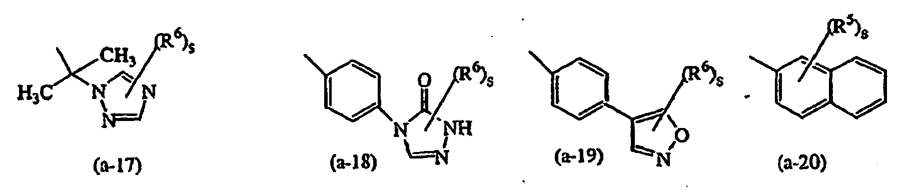 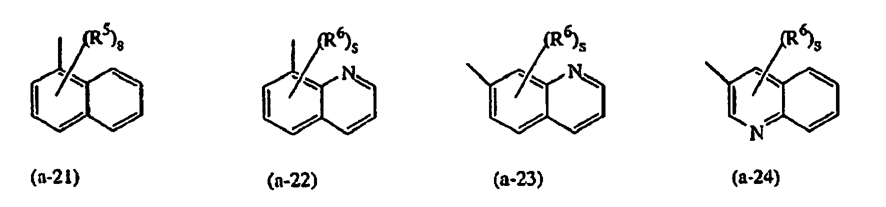 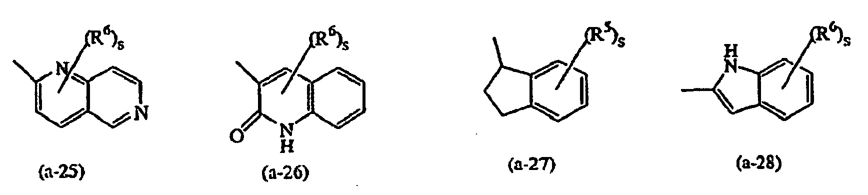 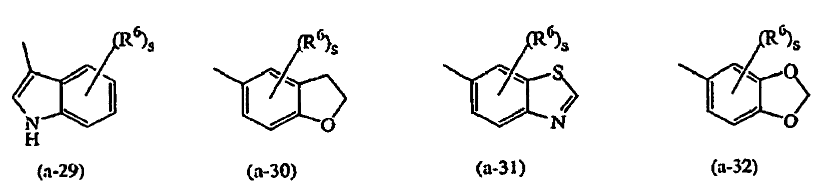 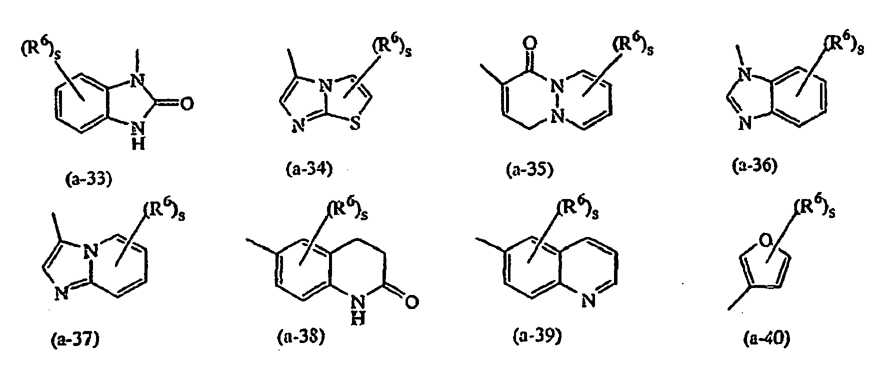 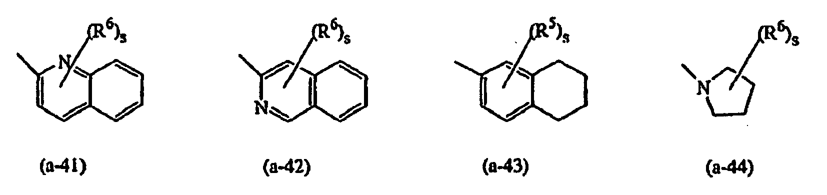 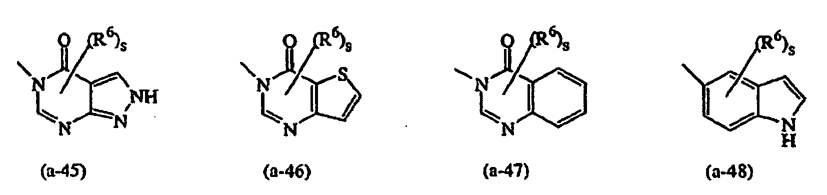 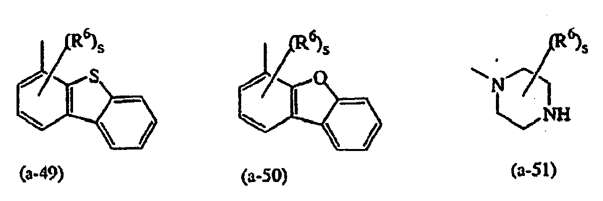
wherein each s is independently 0, 1, 2, 3, 4 or 5; : each R⁵ and R⁶ are independently selected from hydrogen; halo; hydroxy; amino; nitro; trihaloC₁₋₆alkyl; trihaloC₁₋₆alkyloxy; C₁₋₆alkyl; C₁₋₆alkyl substituted with aryl and C₃₋₁₀cycloalkyl; C₁₋₆alkyloxy; C₁₋₆alkyloxyC₁₋₆alkyloxy; C₁₋₆alkylcarbonyl; C₁₋₆alkyloxycarbonyl; C₁₋₆alkylsulfonyl; cyanoC₁₋₆alkyl; hydroxyC₁₋₆alkyl; hydroxyC₁₋₆alkyloxy; hydroxyC₁₋₆alkylamino; aminoC₁₋₆alkyloxy; di(C₁₋₆alkyl)aminocarbonyl; di(hydroxyC₁₋₆alkyl)amino; (aryl)(C₁₋₆alkyl)amino; di(C₁₋₆alkyl)aminoC₁₋₆alkyloxy; di(C₁₋₆alkyl)aminoC₁₋₆alkylamino; di(C₁₋₆alkyl)aminoC₁₋₆alkylaminoC₁₋₆alkyl; arylsulfonyl; arylsulfonylamino; aryloxy; aryloxyC₁₋₆alkyl; arylC₂-₆alkenediyl; di(C₁₋₆alkyl)amino; di(C₁₋₆alkyl)aminoC₁₋₆alkyl; di(C₁₋₆alkyl)amino(C₁₋₆alkyl) amino; di(C₁₋₆alkyl)amino(C₁₋₆alkyl)aminoC₁₋₆alkyl; di(C₁₋₆alkyl) aminoC₁₋₆alkyl(C₁₋₆alkyl)amino; di(C₁₋₆alkyl)aminoC₁₋₆alkyl(C₁₋₆alkyl)aminoC₁₋₆alkyl; aminosulfonylamino(C₁₋₆alkyl)amino; aminosulfonylamino(C₁₋₆alkyl)aminoC₁₋₆alkyl; di(C₁₋₆alkyl)aminosulfonylamino(C₁₋₆alkyl)amino; di(C₁₋₆alkyl)aminosulfonylamino(C₁₋₆alkyl)aminoC₁₋₆alkyl; cyano; thiophenyl; thiophenyl substituted with di(C₁₋₆alkyl)aminoC₁₋₆alkyl(C₁₋₆alkyl)aminoC₁₋₆alkyl, di(C₁₋₆alkyl)aminoC₁₋₆alkyl, C₁₋₆alkylpiperazinylC₁₋₆alkyl, hydroxyC₁₋₆alkylpiperazinylC₁₋₆alkyl, hydroxyC₁₋₆alkyloxyC₁₋₆alkylpiperazinylC₁₋₆alkyl, di(C_{1- 6}alkyl)aminosulfonylpiperazinylC₁₋₆alkyl, C₁₋₆alkyloxypiperidinyl, C₁₋₆alkyloxypiperidinylC₁₋₆alkyl, morpholinylC₁₋₆alkyl, hydroxyC₁₋₆alkyl(C₁₋₆alkyl)aminoC₁₋₆alkyl, or di(hydroxyC₁₋₆alkyl)aminoC₁₋₆alkyl; furanyl; furanyl substituted with hydroxyC₁₋₆alkyl; benzofuranyl; imidazolyl; oxazolyl; oxazolyl substituted with aryl and C₁₋₆alkyl; C₁₋₆alkyltriazolyl; tetrazolyl; pyrrolidinyl; pyrrolyl; piperidinylC₁₋₆alkyloxy; morpholinyl; C₁₋₆alkylmorpholinyl; morpholinylC₁₋₆alkyloxy; morpholinylC₁₋₆alkyl; morpholinylC₁₋₆alkylamino; morpholinylC₁₋₆alkylaminoC₁₋₆alkyl; piperazinyl; C₁₋₆alkylpiperazinyl; C₁₋₆alkylpiperazinylC₁₋₆alkyloxy; piperazinylC₁₋₆alkyl; naphtalenylsulfonylpiperazinyl; naphtalenylsulfonylpiperidinyl; naphtalenylsulfonyl: C₁₋₆alkylpiperazinylC₁₋₆alkyl; C₁₋₆alkylpiperazinylC₁₋₆alkylamino; C₁₋₆alkylpiperazinylC₁₋₆alkylaminoC₁₋₆alkyl; C₁₋₆alkylpiperazinylsulfonyl; aminosulfonylpiperazinylC₁₋₆alkyloxy; aminosulfonylpiperazinyl; aminosulfonylpiperazinylC₁₋₆alkyl; di(C₁₋₆alkyl)aminosulfonylpiperazinyl; di(C₁₋₆alkyl)aminosulfonylpiperazinylC₁₋₆alkyl; hydroxyC₁₋₆alkylpiperazinyl; hydroxyC₁₋₆alkylpiperazinylC₁₋₆alkyl; C₁₋₆alkyloxypiperidinyl; C₁₋₆alkyloxypiperidinylC₁₋₆alkyl; piperidinylaminoC₁₋₆alkylamino; piperidinylaminoC₁₋₆alkylaminoC₁₋₆alkyl; (C₁₋₆alkylpiperidinyl)(hydroxyC₁₋₆alkyl)aminoC₁₋₆alkylamino; (C₁₋₆alkylpiperidinyl)(hydroxyC₁₋₆alkyl)aminoC₁₋₆alkylaminoC₁₋₆alkyl; hydroxyC₁₋₆alkyloxyC₁₋₆alkylpiperazinyl; hydroxyC₁₋₆alkyloxyC₁₋₆alkylpiperazinylC₁₋₆alkyl; (hydroxyC₁₋₆alkyl)(C₁₋₆alkyl)amino; (hydroxyC₁₋₆alkyl)(C₁₋₆alkyl)aminoC₁₋₆alkyl; hydroxyC₁₋₆alkylaminoC₁₋₆alkyl; di(hydroxyC₁₋₆alkyl)aminoC₁₋₆alkyl; pyrrolidinylC₁₋₆alkyl; pyrrolidinylC₁₋₆alkyloxy; pyrazolyl; thiopyrazolyl; pyrazolyl substituted with two substituents selected from C₁₋₆alkyl or trihaloC₁₋₆alkyl; pyridinyl; pyridinyl substituted with C₁₋₆alkyloxy, aryloxy or aryl; pyrimidinyl; tetrahydropyrimidinylpiperazinyl; tetrahydropyrimidinylpiperazinylC₁₋₆alkyl; quinolinyl; indolyl; phenyl; phenyl substituted with one, two or three substituents independently selected from halo, amino, nitro, C₁₋₆alkyl, C₁₋₆alkyloxy, hydroxyC₁₋₄alkyl, trifluoromethyl, trifluoromethyloxy, hydroxyC₁₋₄alkyloxy, C₁₋₄alkylsulfonyl, C₁₋₄alkyloxyC₁₋₄alkyloxy, C₁₋₄alkyloxycarbonyl, aminoC₁₋₄alkyloxy, di(C₁₋₄alkyl)amino_{C1-4}alkyloxy, di(C₁₋₄alkyl)amino, di(C₁₋₄alkyl)aminocarbonyl, di(C₁₋₄alkyl)aminoC₁₋₄alkyl, di(C₁₋₄alkyl)aminoC₁₋₄alkylaminoC₁₋₄alkyl, di(C₁₋₄alkyl)amino(C₁₋₄alkyl)amino, di(C₁₋₄alkyl) amino(C₁₋₄alkyl)aminoC₁₋₄alkyl, di(C₁₋₄alkyl)aminoC₁₋₄alkyl(C₁₋₄alkyl)amino, di(C₁₋₄alkyl)aminoC₁₋₄alkyl(C₁₋₄alkyl)aminoC₁₋₄alkyl, aminosulfonylamino(C₁₋₄alkyl)amino, aminosulfonylamino(C₁₋₄alkyl)aminoC₁₋₄alkyl, di(C₁₋₄alkyl)aminosulfonylamino(C₁₋₄alkyl)amino, di(C₁₋₄alkyl) aminosulfonylamino(C₁₋₄alkyl)aminoC₁₋₆alkyl, cyano, piperidinylC₁₋₄alkyloxy, pyrrolidinylC₁₋₄alkyloxy, aminosulfonylpiperazinyl, aminosulfonylpiperazinylC₁₋₄alkyl, di(C₁₋₄alkyl)aminosulfonylpiperazinyl, di(C₁₋₄alkyl)aminosulfonylpiperazinylC₁₋₄alkyl, hydroxyC₁₋₄alkylpiperazinyl, hydroxyC₁₋₄alkylpiperazinylC₁₋₄alkyl, C₁₋₄alkyloxypiperidinyl, C₁₋₄alkyloxypiperidinylC₁₋₄alkyl, hydroxyC₁₋₄alkyloxyC₁₋₄alkylpiperazinyl, hydroxyC₁₋₄alkyloxyC₁₋₄alkylpiperazinylC₁₋₄alkyl, (hydroxyC₁₋₄alkyl)(C₁₋₄alkyl)amino, (hydroxyC₁₋₄alkyl)(C₁₋₄alkyl) aminoC₁₋₄alkyl, di(hydroxyC₁₋₄alkyl)amino, di(hydroxyC₁₋₄alkyl)aminoC₁₋₄alkyl, furanyl, furanyl substituted with-CH=CH-CH=CH-, pyrrolidinylC₁₋₄alkyl, pyrrolidinylC₁₋₄alkyloxy, morpholinyl, morpholinylC₁₋₄alkyloxy, morpholinylC₁₋₄alkyl, morpholinylC₁₋₄alkylamino, morpholinylC₁₋₄alkylaminoC₁₋₄alkyl, piperazinyl, C₁₋₄alkylpiperazinyl, C₁₋₄alkylpiperazinylC₁₋₄alkyloxy, piperazinylC₁₋₄alkyl, C₁₋₄alkylpiperazinylC₁₋₄alkyl, C₁₋₄alkylpiperazinylC₁₋₄alkylamino, C₁₋₄alkylpiperazinylC₁₋₄alkylaminoC₁₋₆alkyl, tetrahydropyrimidinylpiperazinyl, tetrahydropyrimidinylpiperazinylC₁₋₄alkyl, piperidinylaminoC₁₋₄alkylamino, piperidinylaminoC₁₋₄alkylaminoC₁₋₄alkyl, (C₁₋₄alkylpiperidinyl)(hydroxyC₁₋₄alkyl)aminoC₁₋₄alkylamino, (C₁₋₄alkylpiperidinyl)(hydroxyC₁₋₄alkyl)aminoC₁₋₄alkylaminoC₁₋₄alkyl, pyridinylC₁₋₄alkyloxy, hydroxyC₁₋₄alkylamino, hydroxyC₁₋₄alkylaminoC₁₋₄alkyl, di(C₁₋₄alkyl)aminoC₁₋₄alkylamino, aminothiadiazolyl, aminosulfonylpiperazinylC₁₋₄alkyloxy, or thiophenylC₁₋₄alkylamino; each R⁵ and R⁶ can be placed on the nitrogen in replacement of the hydrogen; aryl in the above is phenyl, or phenyl substituted with one or more substituents each independently selected from halo, C₁₋₆alkyl, C₁₋₆alkyloxy, trifluoromethyl, cyano or hydroxycarbonyl.

In one aspect, the inhibitor of histone deacetylase activity may be a compound having the general formula: 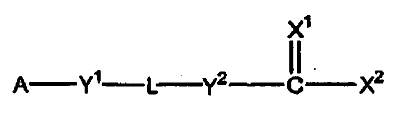 wherein A is a cyclic moiety selected from the group consisting of C3-14 cycloalkyl, 3-14 membered heterocycloalkyl, C4-14 cycloalkenyl, 3-8 membered heterocycloalkenyl, aryl, or heteroaryl; the cyclic moiety being optionally substituted with alkyl, alkenyl, alkynyl, alkoxy, hydroxyl, hydroxylalkyl, halo, haloalkyl, amino, alkylcarbonyloxy, alkyloxycarbonyl, alkylcarbonyl, alkylsulfonylamino, aminosulfonyl, or alkylsulfonyl; or A is a saturated branched C3-12 hydrocarbon chain or an unsaturated branched C3-12 hydrocarbon chain optionally interrupted by -0-, -S-, -N(R^{a})-, -C(O)-, -N(R^{a})-SO₂-, -SO₂-N(R^{a})-, -N(R^{a})-C(O)-O-, -O-C(O)-N(R^{a})-, -N(R^{a})-C(O)-N(R^{b})-, -O-C(O)-, -C(O)-O-, -O-SO₂-, -SO₂-O-, or -O-C(O)-O-, where each of R^{a} and R^{b}, independently, is hydrogen, alkyl, alkenyl, alkynyl, alkoxy, hydroxylalkyl, hydroxyl, or haloalkyl; each of the saturated and the unsaturated branched hydrocarbon chain being optionally substituted with alkyl, alkenyl, alkynyl, alkoxy, hydroxyl, hydroxylalkyl, halo, haloalkyl, amino, alkylcarbonyloxy, alkyloxycarbonyl, alkylcarbonyl, alkylsulfonylamino, aminosulfonyl, or alkylsulfonyl; each of Y¹ and Y², independently, is -CH₂-, -O-, -S-, -N(R)-, -N(R^{c})-C(O)-O-, -O-C(O)-N(R^{c})-, -N(R^{c})-C(O)-N(R^{d})-, -O-C(O)-O-, or a bond; each of R^{c} and R^{d}, independently, being hydrogen, alkyl, alkenyl, alkynyl, alkoxy, hydroxylalkyl, hydroxyl, or haloalkyl; L is a straight C2-12 hydrocarbon chain optionally containing at least one double bond, at least one triple bond, or at least one double bond and one triple bond; said hydrocarbon chain being optionally substituted with C1-4alkyl, C2-4alkenyl, C2-4alkynyl, C1-4alkoxy, hydroxyl, halo, amino, nitro, cyano, C3-5cycloalkyl, 3-5 membered heterocycloalkyl, monocyclic aryl, 5-6 membered heteroaryl, C1-4alkylcarbonyloxy, C1-4alkyloxycarbonyl, C1-4alkylcarbonyl, or formyl; and further being optionally interrupted by -O-, -N(R^{e})-, -N(R^{e})-C(O)-O-, -O-C(O)-N(R^{e})-, -N(R^{e})-C(O)-N(R^{f})-, or -O-C(O)-O-; each of R^{e} and R^{f} independently, being hydrogen, alkyl, alkenyl, alkynyl, alkoxy, hydroxylalkyl, hydroxyl, or haloalkyl; X¹ is O or S; and X² is - OR', -SR¹, -NR¹-OR¹, -NR-SR¹, -C(O)-OR₁, -CHR⁴-OR¹, -N=N-C(O)-N(R³)₂, or -O-CHR⁴-O-C(O)-R⁵, where each of R¹ and R², independently, is hydrogen, alkyl, hydroxylalkyl, haloalkyl, or a hydroxyl protecting group; R³ is hydrogen, alkyl, alkenyl, alkynyl, alkoxy, hydroxylalkyl, hydroxyl, haloalkyl, or an amino protecting group; R⁴ is hydrogen, alkyl, hydroxylalkyl, or haloalkyl; R⁵ is alkyl, hydroxylalkyl, or haloalkyl; and provided that when L is a C2-3 hydrocarbon containing no double bonds and X² is -OR¹, Y¹ is not a bond and Y² is not a bond; or a salt thereof.

### Therapeutic Use of The Cells of The Present Invention.

The present invention can be employed in a method for treating a patient suffering from, or at risk of developing Type 1 diabetes. This method involves isolating and culturing stem cells or progenitor cells, expanding the isolated population of cells, differentiating the cultured cells *in vitro* into a pancreatic lineage, or into a pancreatic hormone-producing cell, or into a β-cell lineage, and implanting the differentiated cells either directly or in a pharmaceutical carrier into the patient. If appropriate, the patient may be further treated with pharmaceutical agents or bioactives that facilitate the survival and function of the transplanted cells. These agents may include, for example, insulin, members of the TGF-β family, including TGF-β1, 2, and 3, bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-1, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, glucagon like peptide-I (GLP-1) and II, GLP-1 and 2 mimetibody, Exendin-4, retinoic acid, parathyroid hormone, MAPK inhibitors, such as, for example, compounds disclosed in U.S. Published Application 2004/0209901 and U.S. Published Application 2004/0132729.

This invention can also be employed in a method for treating a patient suffering from, or at risk of developing Type 2 diabetes. The method involves isolating and culturing stem cells or progenitor cells, expanding the isolated population of cells, differentiating the cultured cells *in vitro* into a pancreatic lineage, or into a pancreatic hormone-producing cell, or into a β-cell lineage, and implanting the differentiated cells either directly or in a pharmaceutical carrier into the patient.

The cells of the present invention may be genetically modified. For example, the cells may be engineered to over express markers characteristic of a cell of a β-cell lineage, such as, for example, NGN-3 (neurogenin-3), Hlxb9, Nkx6, Isl1, Pax6, NeuroD, Hnfla, Hnf6, Hnf3 Beta, and Mafa, or insulin. The cells may be engineered to over express with any suitable gene of interest. Techniques useful to genetically modify the amniotic fluid-derived cells of the present invention can be found, for example, in standard textbooks and reviews in cell biology. Methods in molecular genetics and genetic engineering are described, for example, in Molecular Cloning: A Laboratory Manual, 2nd Ed. (Sambrook et al., 1989); Oligonucleotide Synthesis (M. J. Gait, ed., 1984); Animal Cell Culture (R. I. Freshney, ed., 1987); the series Methods in Enzymology (Academic Press, Inc.); Gene Transfer Vectors for Mammalian Cells (I. M. Miller &M. P. Calos, eds., 1987); Current Protocols in Molecular Biology and Short Protocols in Molecular Biology, 3rd Edition (F. M. Ausubel et al., eds., 1987 & 1995); and Recombinant DNA Methodology II (R. Wu ed. , Academic Press 1995).

The nucleic acid molecule, encoding the gene of interest may be stably integrated into the genome of the cell, or the nucleic acid molecule may be present as an extrachromosomal molecule, such as a vector or plasmid. Such an extrachromosomal molecule may be auto- replicating. The term "transfection," as used herein, refers to a process for introducing heterologous nucleic acid into the host cell.

The cells, undifferentiated or otherwise, may be used as dispersed cells or formed into clusters that may be infused into the hepatic portal vein. Alternatively, the cells may be provided in biocompatible degradable polymeric supports, porous non-degradable devices or encapsulated to protect from host immune response. The cells may be implanted into an appropriate site in a recipient. The implantation sites include, for example, the liver, natural pancreas, renal subcapsular space, omentum, peritoneum, subserosal space or a subcutaneous pocket.

To enhance further differentiation, survival or activity of implanted cells, additional factors, such as growth factors, antioxidants or anti-inflammatory agents, can be administered before, simultaneously with, or after the administration of the cells. In certain embodiments, growth factors are utilized to differentiate the administered cells *in vivo.* These factors can be secreted by endogenous cells and exposed to the administered amniotic fluid-derived cells *in situ.* Implanted cells can be induced to differentiate by any combination of endogenous and exogenously administered growth factors known in the art.

The amount of cells used in implantation depends on a number of factors including the patient's condition and response to the therapy, and can be determined by one skilled in the art.

This invention can be employed in a method for treating a patient suffering from, or at risk of developing diabetes. The method includes isolating and culturing stem cells or progenitor cells, expanding the isolated population of cells, differentiating *in vitro* the cultured cells into a pancreatic lineage, or into a pancreatic hormone-producing cell, or into a β-cell lineage, and incorporating the cells into a three-dimensional support. The cells can be maintained *in vitro* on this support prior to implantation into the patient. Alternatively, the support containing the cells can be directly implanted in the patient without additional *in vitro* culturing. The support can optionally be incorporated with at least one pharmaceutical agent that facilitates the survival and function of the transplanted cells.

Support materials suitable for use for purposes of the present invention include tissue templates, conduits, barriers, and reservoirs useful for tissue repair. In particular, synthetic and natural materials in the form of foams, sponges, gels, hydrogels, textiles, and nonwoven structures, which have been used *in vitro* and *in vivo* to reconstruct or regenerate biological tissue, as well as to deliver chemotactic agents for inducing tissue growth, are suitable for use in practicing the methods of the present invention. See, e.g., the materials disclosed in U.S. Patent 5,770,417, U.S. Patent 6,022,743, U.S. Patent 5,567,612, U.S. Patent 5,759,830, U.S. Patent 6,626,950, U.S. Patent 6,534,084, U.S. Patent 6,306,424, U.S. Patent 6,365,149, U.S. Patent 6,599,323, U.S. Patent 6,656,488, and U.S. Patent 6,333,029. Exemplary polymers suitable for use in the present invention are disclosed in U.S. Published Application 2004/0062753 A1 and U.S. Patent 4,557,264. To form a support incorporated with a pharmaceutical agent, the pharmaceutical agent can be mixed with the polymer solution prior to forming the support. Alternatively, a pharmaceutical agent could be coated onto a fabricated support, preferably in the presence of a pharmaceutical carrier. The pharmaceutical agent may be present as a liquid, a finely divided solid, or any other appropriate physical form. Alternatively, excipients may be added to the support to alter the release rate of the pharmaceutical agent. In an alternate embodiment, the support is incorporated with at least one pharmaceutical compound that is an anti-inflammatory compound, such as, for example compounds disclosed in U.S. Patent 6,509,369.

In one embodiment, the support is incorporated with at least one pharmaceutical compound that is an anti-apoptotic compound, such as, for example, compounds disclosed in U.S. Patent 6,793,945.

In another embodiment, the support is incorporated with at least one pharmaceutical compound that is an inhibitor of fibrosis, such as, for example, compounds disclosed in U.S. Patent 6,331,298.

In a further embodiment, the support is incorporated with at least one pharmaceutical compound that is capable of enhancing angiogenesis, such as, for example, compounds disclosed in U.S. Published Application 2004/0220393 and U.S. Published Application 2004/0209901.

In still another embodiment, the support is incorporated with at least one pharmaceutical compound that is an immunosuppressive compound, such as, for example, compounds disclosed in U.S. Published Application 2004/0171623.

In a further embodiment, the support is incorporated with at least one pharmaceutical compound that is a growth factor, such as, for example, members of the TGF-β family, including TGF-β1, 2, and 3, bone morphogenic proteins (BMP-2, -3, -4, -5, -6, -7, -11,-12, and -13), fibroblast growth factors-1 and -2, platelet-derived growth factor-AA, and - BB, platelet rich plasma, insulin growth factor (IGF-I, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, hypoxia inducible factor 1-alpha, glucagon like peptide-I (GLP-1), GLP-1 and GLP-2 mimetibody, and II, Exendin-4, nodal, noggin, NGF, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in U.S. Published Application 2004/0209901 and U.S. Published Application 2004/0132729.

The incorporation of the cells of the present invention into a scaffold can be achieved by the simple depositing of cells onto the scaffold. Cells can enter into the scaffold by simple diffusion (J. Pediatr. Surg. 23 (1 Pt 2): 3-9 (1988)). Several other approaches have been developed to enhance the efficiency of cell seeding. For example, spinner flasks have been used in seeding of chondrocytes onto polyglycolic acid scaffolds (Biotechnol. Prog. 14(2): 193-202 (1998)). Another approach for seeding cells is the use of centrifugation, which yields minimum stress to the seeded cells and enhances seeding efficiency. For example, Yang *et al.* developed a cell seeding method (J. Biomed. Mater. Res. 55(3): 379-86 (2001)), referred to as Centrifugational Cell Immobilization (CCI).

The present invention is further illustrated, but not limited by, the following examples.

### Example 1

### The Establishment of Human Pancreatic Cell Lines.

*Pancreas Preparation -* Human pancreata not suitable for clinical transplantation were obtained from The National Disease Research Interchange (Philadelphia, PA) following appropriate consent for research use. The pancreas was transferred with organ preservation solution to a stainless steel pan on ice and trimmed of all extraneous tissue. The pancreatic duct was cannulated with an 18 gauge catheter and the pancreas was injected with an enzyme solution, which contained the LIBERASE HI™ enzyme (Roche 0.5 mg/ml, Roche) and DNase I (0.2 mg/ml) dissolved in Dulbecco's Phosphate Buffered Saline (DPBS).

*Rapid Mechanical Dissociation Followed by Enzymatic Digestion -* The enzyme infused pancreata were homogenized in a tissue processor, pulsed 3-5 times for 3-5 seconds/pulse, and the dissociated tissue was transferred to two 500 ml trypsinizing flasks (Bellco) containing magnetic stir bars. Thereafter, 50-100 ml of the enzyme solution was added to each flask. The flasks were placed in a 37°C water bath on submersible stir plates and allowed to incubate with an intermediate stir rate for 10 minutes. The stirring was stopped, and the finely digested tissue was removed from the flask and transferred into a 250 ml tube containing DPBS, 5% Fetal Bovine Serum (FBS) and 0.1 mg/ml DNase I (DPBS+) at 4°C to quench the digestion process. The flasks were replenished with 50-100 ml of the enzyme solution and returned to the water bath, and the stirring was re-initiated for an additional ten minutes. Again, the flasks were removed and the fine digest was collected and transferred to the 250 ml tubes on ice. This process was repeated for an additional 3-5 times until the pancreas was completely digested.

*Gradual Mechanical Dissociation with Simultaneous Enzyme Digestion -* The enzyme infused pancreata were processed according to methods as described in Diabetes 37:413-420 (1988). Briefly, the pancreata were cleaned of extraneous tissue and injected with the enzyme solution as described above. The pancreata were then placed into a Ricordi Chamber with beads and covered with a screen with a mesh size of 400-600 µm to retain larger clusters of tissue. The chamber was covered; the enzyme solution was circulated through the chamber at approximately 37°C, and the chamber was shaken to allow beads to disrupt pancreatic tissue during enzymatic digestion. Once adequate dissociation and digestion was achieved, the digestion was terminated and the tissue was collected.

*Tissue Separation -* The collected tissue was centrifuged at 150 x g for 5 minutes at 4°C. The supernatant was aspirated and the tissue was washed two additional times in DPBS+. Following the final wash, the tissue was applied to a discontinuous gradient for purification. The digested tissue was suspended in polysucrose (Mediatech, VA) with a density of 1.108 g/ml at a ratio of 1-2 ml tissue pellet per 10 ml of polysucrose solution. The tissue suspension was then transferred to round-bottom polycarbonate centrifuge tubes, and polysucrose solutions with densities of 1.096 and 1.037 were carefully applied to the tubes. A final layer of DMEM completed the discontinuous purification gradient. The gradient tubes were centrifuged at 2000 rpm for 20 minutes at 4°C with no brake applied. Following centrifugation, the tissue was collected from each interface (three interfaces total), washed several times in DPBS+ as described above, and collected in a 50 ml test tube.

*Further Cell Cluster Dissociation -* Optionally, one can further dissociate large cell clusters obtained using the above protocol into smaller clusters or single cell suspensions. After the final wash, the tissue from each fraction was suspended in 10 ml 1X trypsin/EDTA solution containing 200U/mL DNase I. The tubes were placed in the water bath and repeatedly aspirated and discharged from a 10 ml serological pipette for 5-6 minutes until a near single cell suspension was achieved. The digestion was quenched with the addition of 4°C DPBS+ and the tubes centrifuged at 800 rpm for 5 minutes. The cell suspensions were washed with DPBS+ and cultured as described below.

*Pancreatic Cell Culture -* Following the final wash, the cells from each interface were resuspended in DMEM, 2% FBS, 100 U/µg penicillin/streptomycin, ITS, 2 mM L-Glutamine, 0.0165 mM ZnSO₄ (Sigma), and 0.38 µM 2-mercaptoethanol (Invitrogen, CA) (hereinafter "the selection medium"). Six ml of the cell suspension was seeded in T-25 tissue culture flasks and 12 ml of the cell suspension was seeded into T-75 flasks. The flasks were placed in 37°C incubators with 5% CO₂. Following 2-4 weeks culture, a complete medium change was performed and adherent cells were returned to culture in DMEM (2750 mg/L D-glucose, 862 mg/L glutamine) (Gibco, CA) with 5% FBS (HyClone, UT), 1% P/S, 0.0165 mM ZnSO₄ (hereinafter "the growth medium") and allowed to reach near confluence (this stage is referred to as "passage 0" or "P0"), at which point they were passaged. Subsequent culturing of the cells was at 5000 cell/cm² in the growth medium. Cultures were passaged every 7-10 days at ~70-90% confluency.

### Example 2

### The Establishment of Human Amniotic Fluid-Derived Cell Lines.

Amniotic fluid used to isolate the cells of the present invention was taken from samples obtained through routine amniocentesis performed at 17-22 weeks of gestation for fetal karyotyping (Drexel University). The amniotic fluid was centrifuged for 7 minutes at 400 *x* g and the supernatant removed. The resulting cell pellet was resuspended in growth medium. The cells were cultured either on collagen type IV (1mg/100 mm plate) or fibronectin (10 micrograms/ml) coated plates. The cell yield from AF samples had a large variation (8000-300000 cell/sample), and some samples also contained a significant degree of red blood cell contamination. The cultures were left undisturbed for at least 5-10 days under hypoxic conditions (3% O₂). Thereafter, the cultures were fed with the same growth medium and cultured until the cultures reached 70-80% confluency. Cells at this stage were referred to as "P0". In some cultures, colonies of cells were isolated using a cloning ring and sub-cultured into a different culture plate. Cells were released from P0 culture by using TrypLE Express™ (Invitrogen) and seeded into fibronectin or collagen type IV coated flaks/dishes/plates at various densities (50-10,000 cell/cm²). Some of the P0 cells were used for serial dilution cloning. The population doubling time of the fastest growing cells was ∼24 hrs at early passages. Cells were typically split at 60% confluency and reseeded at 100-10000 cells/cm².

### Example 3

### PCR Analysis Of Cells.

RNA was extracted from cells cultured in the growth media. Total RNA from human pancreas (Ambion, INC) was included as a positive control.

*RNA extraction, purification, and cDNA synthesis.* RNA samples were purified through binding to a silica-gel membrane (Rneasy Mini Kit, Qiagen, CA) in the presence of an ethanol-containing, high-salt buffer while contaminants were washed away. High-quality RNA was then eluted in water. Yield and purity were assessed by A260 and A280 readings on the spectrophotometer. cDNA copies were made from purified RNA using the iScript cDNA synthesis kit (BioRad, CA).

*Real-time PCR amplification and quantitative analysis.* Unless otherwise stated, all reagents were purchased from Applied Biosystems. Real-time PCR reactions were performed using the ABI PRISM™ 7500 Sequence Detection System. TAQMAN™ FAST UNIVERSAL PCR MASTER MIX™ (ABI, CA) was used with 20 ng of reverse transcribed RNA in a total reaction volume of 20 µl. Each cDNA sample was run in duplicate to allow correction of pipetting errors. Primers and FAM-labeled TAQMANT™ probes were used at concentrations of 200 nM. The level of expression for each target gene was normalized using the pre-developed Applied Biosystem's human glyceraldehyde-3-phosphate dehydrogenase (GAPDH) endogenous control kit. Primers and probes were designed using either ABI PRISM PRIMER EXPRESS ™software or a pre-developed ABI gene analysis kit. For each gene, either one of the primers or the probe were designed to be exon-boundary spanning. This eliminated the possibility of the primers/probe binding to any genomic DNA present. The primer and probe sets are listed as follows: Pdx-1 (Hs00426216), Insulin (Hs00355773), glucagon (Hs00174967), and FoxA2 (HNF 3-beta) (Hs00232764). The remaining primers were designed using the PRIMERS program (ABI, CA). After an initial 95°C incubation for 20 sec, samples were cycled 40 times in two stages: a denaturation step at 95°C for 3 sec, followed by an annealing/extension step at 60°C for 30 sec.

For each primer/probe set, a cycle time value was determined as the cycle number at which the fluorescence intensity of the PCR reaction reached a specific value in the middle of the exponential region of amplification. An increase in expression of a gene corresponded to a decrease in the number of cycles required for the fluorescence intensity to reach this value.

### Example 4

### Fluorescence-Activated Cell Sorting (FACS) Analysis.

Adhered cells were removed from culture plates by five-minute incubation with the TRYPLE™ express solution (Gibco, CA). Released cells were resuspended in DMEM supplemented with 10% FBS and recovered by centrifugation, followed by washing and resuspending the cells in a staining buffer consisting of 2% BSA, 0.05% sodium azide (Sigma, MO) in PBS. If appropriate, the cells were Fc-receptor blocked using a 0.1% γ̃-globulin (Sigma) solution for 15 min. Aliquots (approximately 10⁵ cells) were incubated with either phycoerythirin (PE) or allophycocyanin (APC) conjugated monoclonal antibodies (5 µl antibody per 10⁶ cells), as indicated in **Table I**, or with an unconjugated primary antibody. Controls included appropriate isotype matched antibodies, non-stained cells, and cells only stained with secondary conjugated antibody. All incubations with antibodies were performed for 30 mins at 4°C after which the cells were washed with the staining buffer. Samples that were stained with unconjugated primary antibodies were incubated for an additional 30 mins at 4°C with secondary conjugated PE or -APC labeled antibodies. See **Table II** for a list of secondary antibodies used. Washed cells were pelleted and resuspended in the staining buffer and the cell surface molecules were identified by using a FACS Array (BD Biosciences) by collecting at least 10,000 events.

For intracellular staining, cells were first fixed for 10 mins with 4% paraformaldheyde, followed by two rinses in the staining buffer, centrifugation of cells and resuspension of the cells in a permeabilization buffer containing 0.5% Triton-X (Sigma) in PBS for 5 mins at room temperature (RT). The permeabilized cells were rinsed twice with a rinsing buffer, centrifuged, and resuspended in the staining buffer, and incubated with an appropriate conjugated antibody (5 µl antibody per 10⁶ cells) for 30 mins at 4°C. Samples that were stained with unconjugated primary antibodies were incubated for an additional 30 mins at 4°C with secondary conjugated PE or -APC labeled antibodies (**Table II**). Washed cells were pelleted and resuspended in the staining buffer and the internal proteins were identified by using a FACSArray (BD Biosciences) by collecting at least 10,000 events.

### Example 5

### Immunostaining of Cells.

10,000 cells/cm² cells were seeded into glass bottom 35 mm microwell dishes (Matek Corp, MA) in growth medium. Following three days in culture, the cells were fixed for 10 mins with 4% paraformaldheyde, followed by two rinses in PBS and addition of a permeabilization buffer containing 0.5% Triton-X (Sigma) for 5 mins at room temperature (RT), followed by an additional three rinses with PBS. The fixed and permeabilized cells were blocked with either 1% bovine serum albumin (BSA) or 4% serum from the species where the secondary antibody was raised in (goat, donkey, or rabbit). Control samples included reactions with the primary antibody omitted or where the primary antibody was replaced with corresponding immunoglobulins at the same concentration as the primary antibodies. Stained samples were rinsed with a PROLONG® antifade reagent (Invitrogen, CA) containing diamidino-2-phenylindole, dihydrochloride (DAPI) to counter-stain the nucleus. Images were acquired using a Nikon Confocal Eclipse C-1 inverted microscope (Nikon, Japan) and a 60X objective.

### Example 6

### Effects of Trichostatin A Treatment on Gene Expression in Panc-1 Cells and Neonatal Fibroblasts.

Neonatal fibroblasts, also designated Hs27, were derived from human foreskins and obtained from the American Type Culture Collection (ATCC). Panc-1 cells are a transformed cell line derived from a pancreatic epitheloid carcinoma of ductal origin, also obtained from ATCC.

Fibroblasts or Panc-1 cells were seeded into a 6-well tissue culture plate at a density of 50,000 cells/cm². Both cell types were cultured in medium containing 10%FBS and DMEM under standard cell culture conditions (37°C, 5%CO₂). After reaching confluence (2-3 days), trichostatin A diluted in dimethyl sulfoxide (DMSO) and medium was added at either 2.5 µM or 5 µM to the cultures. Parallel cultures were treated with an equivalent concentration of DMSO as a vehicle control.

RNA samples were obtained from the treated cultures 48 hours after the addition of trichostatin A or DMSO. The culture medium was removed, the cells were washed with phosphate buffered saline (PBS), and RLT Lysis buffer containing β-mercaptoethanol (Qiagen) was added. The samples were homogenized using Qiashredder columns

(Qiagen), and RNA was purified using the RNeasy Mini Kit (Qiagen). RNA quantity and quality was determined using a spectrophotometer, and cDNA was made using the iScript cDNA synthesis kit (BioRad).

The expression levels of Sox17, HNF-3 beta, Pdx-1, insulin, and glucagon were determined by Real-Time PCR (RT-PCR), as described in **Example 3.** Samples of 20ng cDNA were used in each reaction. RT-PCR reactions were performed on the Applied Biosystems 7500, and data was analyzed using the accompanying software. Human pancreas cDNA was included as a positive control. Results were normalized against GAPDH expression levels.

A basal level of expression for Sox17, HNF-3 beta, Pdx-1 and glucagon was detected in untreated Panc-1 cells. However, expression of these genes was not detectable in untreated neonatal fibroblasts. (**Table I**). Treatment of neonatal fibroblasts and Panc-1 cells with the HDAC inhibitor, trichostatin A, caused an increase in expression of Sox17, HNF-3 beta, Pdx-1, and glucagon (**Figure 1****, panels a-d & Table III**). Expression of insulin did not change relative to untreated controls in samples for either cell type under the conditions tested.

Basal expression levels of Sox17, HNF-3 beta, Pdx-1, and glucagon were higher in untreated Panc-1, compared to untreated neonatal fibroblast cells. Trichostatin A treatment evoked a more robust up-regulation of pancreatic gene expression in Panc-1 cells relative to fibroblasts, as measured for the representative endocrine and pancreas genes evaluated. Up-regulated expression of these genes also correlated in a dose-dependent manner with the concentration of trichostatin A used during treatment, again with a more robust effect noted in Panc-1 cells for these genes of interest. Panc-1 cells treated for 48 hours increased Sox-17 expression 60 times higher, Pdx-1 expression 11 times higher, and glucagon expression 5 times higher with 5.0 µM versus 2.5 µM trichostatin A. Similar dose response effects were noted for fibroblasts although the up-regulation was less pronounced overall (**Figure 1****, panels a-d & Table III**).

These data suggest that the potency of the HDAC inhibitor, trichostatin A, with respect to increasing expression of Sox17, HNF-3 beta, Pdx-1, and glucagon, is greater in Panc-1 cells that neonatal fibroblasts. This may also suggest that with a given treatment protocol, lineage specific gene expression can be enhanced to a greater extent in cells previously differentiated (or partially differentiated) along that same lineage pathway. However, the effect of treatment is not restricted to differentiated (or partially differentiated) cells of that pathway but may encompass cells from other lineage pathways.

### Example 7

**Effects of Trichostatin A on Gene Expression in Amniotic Fluid-Derived Cells.**

Amniotic fluid derived cells were seeded into 24-well tissue culture plates at a density of 5000/cm² and cultured in AMNIOMAX medium (Invitrogen) under standard cell culture conditions until confluent. Cells were obtained according to methods described in **Example 2.** After reaching confluence, 1.25µM trichostatin A diluted in DMSO and medium was added to sample wells; an equivalent concentration of DMSO was added to control wells as a no treatment control.

RNA samples were obtained from treated cultures at 30 minutes, 1.5 hours, 3 hours, 6 hours, 12 hours, and 24 hours following addition of trichostatin A or DMSO. Culture medium was removed, cells were washed with PBS, and RLT lysis buffer with β-mercaptoethanol (Qiagen) was added. RNA was purified using the RNeasy Mini Kit (Qiagen). RNA quantity and quality was determined using a spectrophotometer, and cDNA was made using the iScript cDNA synthesis kit (BioRad).

Expression levels of Sox17, HNF-3 beta, Pdx-1, insulin, and glucagon were determined by Real-Time PCR. Samples of 20ng cDNA were used in each reaction, performed on the Applied Biosystems 7500 according to methods described in **Example 3**. Data analysis was performed using the accompanying software. Human pancreas cDNA was included as a positive control, and results were normalized against GAPDH expression levels.

Basal expression of insulin and Sox-17 were consistently detected in untreated cells whereas low level expression of HNF-3 beta was detected intermittently at various time points in untreated cells. Glucagon and Pdx-1 gene expression were not detectable in untreated cells (**Table IV-A**).

Treatment of amniotic fluid-derived cells with trichostatin A caused a decrease in the gene expression of insulin, had relatively little or no effect on Sox-17 expression, but induced an increase in gene expression of glucagon, HNF-3 beta and Pdx-1 over time (**Figure 2****, panels a-d & Table IV-B**). HNF-3 beta gene expression in amniotic fluid-derived cells was detectable by RT-PCR at >35 cycles by 30 minutes after addition of trichostatin A, increasing with time to <35 cycles at 24 hours. Pdx-1 gene expression was undetectable 30 minutes after addition of trichostatin A, first detectable at >35 cycles by RT-PCR at 6 hours, increasing to <35 cycles or ∼0.1% of human pancreas levels at 24 hours. Glucagon expression was undetectable 30 minutes after addition of trichostatin A, first detectable at >35 cycles by RT-PCR at 12 hours, increasing to <24 cycles at 24 hours (**Figure 2****, panels c-d**).

These results, in conjunction with results from Panc-1 cells in **Example 6**, suggest a pattern in regulation of gene sets inherent in a particular cell lineage differentiation program. Differentiated cells treated with a chromatin-remodeling agent may down-regulate some genes and up-regulate other genes in response to both the presence of the HDAC inhibitor and environmental or other stimulatory signals.

### Example 8

### Effects of Trichostatin A on Gene Expression in Late Passage (P14) Pancreatic-Derived Stromal Cells.

Human pancreatic-derived stromal cells were obtained according to the methods described in **Example 1**. Cells were seeded into a 24-well tissue culture plate at a density of 5000/cm² and cultured in DMEM containing 10% fetal bovine serum under standard cell culture conditions until confluent. After the cells reached confluency, 2.5 µM trichostatin A, diluted in DMSO and medium was added to the wells. Parallel cultures were treated with an equivalent concentration of DMSO as a vehicle control.

RNA samples were obtained from treated cultures at 30 minutes, 1.5 hours, 3 hours, 6 hours, 12 hours and 24 hours following the addition of trichostatin A or DMSO. Culture medium was removed, the cells were washed with PBS, and RLT lysis buffer with β-mercaptoethanol (Qiagen) was added. RNA was purified using the RNeasy Mini Kit (Qiagen). RNA quantity and quality were determined using a spectrophotometer, and cDNA was made using the iScript cDNA synthesis kit (BioRad).

The expression levels of Sox17, HNF-3 beta, Pdx-1, insulin, and glucagon were determined by Real-Time PCR. Samples of 20ng cDNA were used in each reaction, which was performed on the Applied Biosystems 7500 according to the methods described in **Example 3**. Data were analyzed using the accompanying software. Human pancreas cDNA was included as a positive control, and results were normalized against GAPDH expression levels.

Expression of insulin, Pdx-1, glucagon or HNF-3 beta genes was not detected in untreated late passage pancreatic-derived stromal cells However, Sox-17, was detected at low levels intermittently in untreated cells at various time points during the course of the experiment (Table V-A). Treatment of late passage pancreatic-derived stromal cells with 2.5µM of trichostatin A caused an increase in gene expression of Sox-17, glucagon, HNF-3 beta, and Pdx-1 genes over time. However, no changes in insulin gene expression were observed (**Figure 3****, panels a-c & Table V-B**).

Sox-17 gene expression in late passage pancreatic-derived stromal cells was consistently detectable at >35 cycles by RT-PCR 3 hours after addition trichostatin A, increasing over time to levels <35 cycles or ∼3% of human pancreas levels at 24 hours (**Figure 3****, panel a**). HNF-3 beta gene expression was detectable at >35 cycles by RT-PCR by 6 hours after addition of trichostatin A, increasing to <35 cycles or ∼0.075% of human pancreas at 24 hours (**Figure 3****, panel b**). Pdx-1 gene expression was detectable at >35 cycles by RT-PCR by 12 hours after addition of trichostatin A, increasing to <35 cycles or ∼0.2% of human pancreas levels at 24 hours (**Figure 3****, panel b**). Glucagon expression was undetectable until 24 hours following addition of trichostatin A, reaching detectable levels of 35-40 cycles by RT-PCR at that time point (**Figure 3****, panel c**). Collectively these data indicate that treatment with the HDAC inhibitor agent trichostatin A can induce or enhance expression of endocrine pancreatic genes in cells that have low basal expression rates or that have lost previous expression patterns over time in culture.

### Example 9

### Effects of Chronic Trichostatin A Treatment on Gene Expression in Amniotic Fluid-Derived Cells.

Amniotic fluid derived cells were obtained according to the methods described in **Example 2**. Cells were seeded into a 24-well tissue culture plate at a density of 5000/cm² and cultured in AMNIOMAX (Invitrogen) under standard cell culture conditions until confluent. After the cells reached confluency, sample wells were treated with either 500 nM or 1.0 µM trichostatin A diluted in DMSO and medium; control wells were treated with an equivalent concentration of DMSO. At 24 hour intervals over the three day incubation period, cultures underwent a complete medium change, and a fresh dilution of trichostatin A or DMSO was added as appropriate.

RNA samples were obtained from the treated cultures at 24 hour intervals after the initial addition of trichostatin A or DMSO. The culture medium was removed, cells were washed with PBS, and RLT lysis buffer with β-mercaptoethanol (Qiagen) was added. RNA was purified using the RNeasy Mini Kit (Qiagen); RNA quantity and quality was determined using a spectrophotometer. cDNA was made using the iScript cDNA synthesis kit (BioRad).

Expression levels of Sox17, HNF-3 beta, Pdx-1, insulin, and glucagon were determined by RT-PCR. Samples of 20ng cDNA were used in each reaction, performed on an Applied Biosystems 7500 according to the methods described in **Example 3**. Data were analyzed using the accompanying software. Human pancreas cDNA was included as a control. Results were normalized against GAPDH expression levels.

Basal expression of insulin and Sox-17 genes was detectable in untreated amniotic fluid-derived cells. However, expression of glucagon and Pdx-1 genes was not detectable in untreated cells by RT-PCR up to cycle 40. HNF-3 beta was not detected in untreated cells at 24-hours in culture but was weakly expressed at 48 and 72 hours culture time.

Following treatment with trichostatin A, amniotic fluid-derived cells expressed glucagon, HNF-3 beta, and Pdx-1 above basal levels (**Figure 4****, panels a-e, Table VI**). Gene expression for glucagon and Pdx-1 increased in a dose and time dependent manner after treatment with trichostatin A, with highest expression seen at the final 72 hour time point and with the higher 1 µM treatment dose (**Figure 4****, panels a & d**). Similarly, HNF-3 beta gene expression in this example also increased in a time dependent manner with highest expression observed at the final 72 hour time point. However, HNF-3 beta expression was essentially equivalent with both treatment doses of 1 µM and 500nM trichostatin A (**Figure 4****, panel b**). In contrast, insulin gene expression decreased after treatment to non-detectable levels at all time points. Sox-17 expression was affected to a less significant degree relative to initial basal expression levels with both treatment doses of trichostatin A. This suggests that the extent of the induced gene expression in response to treatment with an HDAC inhibitor may be variable.

### Example 10

### Effects of Chronic Trichostatin A Treatment on Gene Expression in Late Passage Pancreatic-Derived Stromal Cells.

Pancreatic-derived stromal cells were obtained according to the methods described in **Example 1**. Cells were seeded into a 24-well tissue culture plate at a density of 5000/cm² and cultured in DMEM with 10% FBS under standard cell culture conditions until confluent. After the cells reached confluency, sample wells were treated with either 1.25 µM or 2.5 µM trichostatin A diluted in DMSO and medium; control wells received DMSO at an equivalent concentration. At 24 hour intervals over the three day culture period, cultures underwent a complete medium change, and a fresh dilution of trichostatin A or DMSO was added as appropriate. RNA samples were obtained from treated cultures daily after the initial addition of trichostatin A or DMSO. Culture medium was removed, cells were washed with PBS, and RLT lysis buffer with β-mercaptoethanol (Qiagen) was added. RNA was purified using the RNeasy Mini Kit (Qiagen), and RNA quantity and quality was determined using a spectrophotometer. cDNA was made using the iScript cDNA synthesis kit (BioRad).

Expression levels of Sox17, HNF-3 beta, Pdx-1, insulin, and glucagon were determined by RT-PCR. Samples of 20ng cDNA were used in each reaction, which was performed on the Applied Biosystems 7500, according to the methods described in **Example 3**. Data were analyzed using the accompanying software. Human pancreas cDNA was included as a control. Results were normalized against GAPDH expression levels.

Glucagon, HNF-3 beta, Pdx-1 and Sox-17 gene expression was not detectable by RT-PCR in untreated late passage pancreatic-derived stromal cells. After addition of trichostatin A, gene expression was detectable for all of the aforementioned genes. Insulin gene expression was undetectable prior to treatment and did not increase following trichostatin A treatment. Glucagon, HNF-3 beta, Pdx-1, and Sox-17 gene expression levels all increased in a time dependent manner with highest expression observed for all three genes at 72 hours. In some cases differences in expression levels could be seen when a higher concentration of trichostatin A was added to the cells (**Figure 5****, panels a-d & Table VII**). Increases in gene expression were equivalent with these two treatment concentrations of 1.25 and 2.5 µM trichostatin A; differences at each time point for each gene were minimal or did not appear to be significant. The data imply that for a given gene, there may be a maximal threshold level for treatment by an HDAC inhibitor to have an effect on gene expression at a given time point and that increasing the concentration of HDAC inhibitor has no added benefit. The data may also suggest that continuous replenished addition of trichostatin A during the treatment protocol may be necessary to sustain escalating increases in gene expression over time.

### Example 11

### Effects of Chronic Trichostatin A Treatment on Gene Expression in Amniotic Fluid-Cells and Pancreatic-Derived Stromal Cells.

Several cell lines obtained from different amniotic fluid specimens (see **Example 2**) and pancreas donors (see **Example 1**) were tested with similar results. Two lines at similar passage number but derived from different amniotic fluid specimens are shown in **Table VIII-A** and **Table VIII-B** for this example. One of these cell lines was also used in examples 2 and 4 above. In addition, this example also contains comparison data in **Table VIII-C** and **Table VIII-D** for a single pancreatic-derived stromal line grown to early and late passage number.

Amniotic fluid derived cells or pancreatic-derived stromal cells were seeded into 24-well tissue culture plates at a density of 5000/cm² and cultured in AMNIOMAX (Invitrogen) or DMEM with 10% FBS, respectively, under standard cell culture conditions until confluent. After the cells reached confluency, amniotic fluid derived cells were treated once at time 0 hours with 500 nM trichostatin A. Pancreas-derived stromal cells were treated once at time 0 hours with 1.25 µM trichostatin A. Samples for RT-PCR were taken daily from zero to six days at the times indicated in **Table VIII**.

RNA samples were obtained from the treated cultures up to 144 hours after the initial addition of trichostatin A or DMSO. Culture media was removed, the cells were washed with PBS, and RLT lysis buffer containing β-mercaptoethanol (Qiagen) was added. During culture, the medium was not changed nor was freshly prepared trichostatin A added. RNA was purified using the RNeasy Mini Kit (Qiagen), and RNA quantity and quality was determined using a spectrophotometer. cDNA was made using the iScript cDNA synthesis kit (BioRad).

Samples of 20ng cDNA were used in each reaction to determine the expression levels of the following genes in amniotic fluid-derived cells: Gata1, HNF-3 beta, Pdx-1, insulin, and Soxl7. Similarly, samples of 20ng cDNA were used in each reaction to determine the expression level of the following genes in pancreatic-derived stromal cells: glucagon, HNF-3 beta, insulin and Pdx-1. Real-Time PCR was performed on the Applied Biosystems 7500, and data was analyzed using the accompanying software according to the methods described in **Example 3.**

Analysis of samples obtained from the untreated amniotic fluid-derived cell line used in Examples 2 & 4 above showed that these cells did not express HNF-3 beta or Gata1 but did express Sox-17 and very low levels of insulin and Pdx-1 (**Table VIII-A**). After addition of trichostatin A, HNF-3 beta expression increased, but only during the first 48 hours after initial treatment. Pdx-1 was expressed continuously for 24 hours following treatment, but expression was not detectable after that time point. The insulin gene was not expressed for 48 hours following initial treatment but was detectable after 48 hours. There was no change in Sox17 or Gata1 expression observed (**Table VIII-A**). These data suggest that trichostatin A may inhibit insulin gene expression in these cells within the initial 24-48 hours of culture but is associated with up regulation of Pdx-1 and HNF-3 beta expression within the same time period, followed by a return to undetectable or very weak expression after 48 hours. Trichostatin A may degrade in culture and may not have a significant sustained impact on gene expression at later time points after 24-48 hours. Consequently, the net effect over long time periods using a single initial dose of trichostatin A may reflect the reversible nature of this reagent.

Analysis of samples obtained from an additional untreated amniotic fluid-derived cell line showed that these cells did not express basal levels of HNF-3 beta, Pdx-1 or Gata-1 but did express insulin and Sox-17. Following trichostatin A treatment, HNF-3 beta expression was detected at 48 hours, but as seen in the first amniotic fluid derived cell line, HNF-3 beta decreased to very weak expression levels after 48 hours (**Table VIII-B**). Pdx-1 gene expression was only detected at 24 hours following trichostatin A treatment and returned to undetectable levels for the remainder of the experiment. Insulin gene expression fell to undetectable levels following the addition of 500 nM trichostatin A was and then returned to detectable levels starting at 72 hours after the addition of trichostatin A (**Table VIII-B**). Expression of Sox17 and Gata1 genes did not change with the addition of trichostatin A. These data suggest that trichostatin A may be metabolized within 24-48 hours of addition to cell culture samples, that trichostatin A inhibits insulin gene expression in these cells but has a reversible stimulatory effect on other genes including HNF-3 beta and Pdx-1 (**Table VIII-B**).

Prior to treatment, early passage (P5) pancreatic-derived stromal cells expressed Pdx-1, while expression of the glucagon, HNF-3 beta and insulin genes was not detectable. At 24 hours following treatment with 2.5µM trichostatin A, HNF-3 beta, Pdx-1, and glucagon genes all demonstrated up-regulated expression, but the effect was not sustained and only glucagon expression was still detectable 48 hours after initial treatment, albeit decreased to a lower level than observed at 24 hours. Insulin gene expression remained undetectable and unchanged throughout the course of the experiment (**Table VIII-C**). These data suggest that following trichostatin A treatment, expression of some genes, e.g. transcription factors, may be short-lived depending on the inherent instability of the corresponding mRNA, and/or secondary effects of an HDAC inhibitor on mRNA stability, and/or additional negative regulatory pathways operating in these cells. More persistent expression of other genes over a similar time period may reflect greater message stability and/or additional positive regulatory effects.

Untreated late passage (P14) pancreatic-derived stromal cells did not express any of the genes of interest, suggesting that the expression of these endocrine pancreatic markers declines with time during in vitro culture maintenance. Following treatment with trichostatin A, insulin gene expression remained undetectable and unchanged throughout the course of the experiment (**Table VIII-D**). Glucagon, Pdx-1, and HNF-3 beta gene expression were detected by RT-PCR at 24 hours (**Table VIII-D**). However, only glucagon expression remained detectable 48 hours after initial treatment albeit at a lower expression level than observed at 24 hours and declined to undetectable levels thereafter. The glucagon gene was expressed for a shorter period of time in these late passage cells as compared to early passage pancreatic-derived stromal cells which may reflect a less plastic stage of response to the effects of HDAC inhibitors.

### Example 12

### Effects of Two Sequential Low Dose Trichostatin A Treatments on Gene Expression in Amniotic Fluid-Cells and Pancreatic-Derived Stromal Cells.

Several cell lines obtained from different amniotic fluid specimens (see **Example 2**) and pancreas donors (see **Example 1**) were tested with similar results. Two lines at similar passage number but derived from different amniotic fluid specimens are shown in **Table IX-A** and **Table IX-B** for this example. One of these cell lines was also used in **examples 7** and **9** above. In addition, this example also contains comparison data in **Table IX-C** and **Table IX-D** for a single pancreatic-derived stromal line grown to early and late passage number.

Amniotic fluid derived cells or pancreatic-derived stromal cells were seeded into 24-well tissue culture plates at a density of 5000/cm² and cultured in AMNIOMAX (Invitrogen) or DMEM with 10% FBS respectively under standard cell culture conditions until confluent. After the cells reached confluency, amniotic fluid derived cells were treated at time 0 hours with a dose of 500 nM trichostatin A, and pancreatic-derived stromal cells were treated with a dose of 1.25 µM trichostatin A solubilized in DMSO and medium. At time 24 hours, cell cultures underwent a complete medium change to remove all traces of trichostatin A. At time 96 hours, cell cultures were re-fed with fresh medium and received a second dose of trichostatin A at the same concentration as used previously. At time 120 hours, cell cultures again underwent a complete medium change and were cultured for an additional 24 hours or 148 hours total incubation time.

Samples were taken daily for RT-PCR at the times indicated in **Table IX-A to D**. The culture medium was removed, and samples were rinsed in PBS then collected in RLT with β-mercaptoethanol (Qiagen). RNA was purified using the RNeasy Mini Kit (Qiagen) and RNA quantity and quality were determined using a spectrophotometer. cDNA was made using the iScript cDNA synthesis kit (BioRad).

Samples of 20ng cDNA were used in each reaction to determine the expression levels of the following genes in amniotic fluid-derived cells: Gata1, HNF-3 beta, Pdx-1, insulin, and Sox17. Similarly, samples of 20ng cDNA were used in each reaction to determine the expression level of the following genes in pancreatic-derived cells: glucagon, HNF-3 beta, insulin and Pdx-1. Real-Time PCR was performed on the Applied Biosystems 7500, and data was analyzed using the accompanying software according to the methods described in **Example 3**.

Prior to treatment with trichostatin A, the amniotic fluid-derived cell line used in **Examples 7 & 9** did not express Gata-1 or HNF-3 beta. However, expression of the insulin and Pdx-1 and Sox-17 genes was detected by RT-PCR at 37 cycles and 27 cycles respectively (**Table IX-A**). Following treatment with 0.5µM trichostatin A, Sox-17 and Gata-1 gene expression levels did not change at any subsequent time point throughout the experiment. Insulin expression was detected by RT-PCR at 36 cycles prior to trichostatin A treatment, decreased to undetectable levels at 24 hours following the first addition of trichostatin A, reappeared at detectable levels of 36 cycles by 48 hours, and remained at 36 cycles for the duration of the experiment.. HNF-3 beta was detected by RT-PCR at 33 cycles following the first trichostatin A treatment, decreasing to undetectable levels following the first medium change, reappearing at 37 cycles following the second treatment with trichostatin A, and remaining for the duration of the experiment. Pdx-1 gene expression was detected at 37 cycles by RT-PCR, continuing at this level for 24 hours following the addition of trichostatin A, but was not detectable after that time point for the remainder of the experiment (**Table IX-A**).

Prior to treatment with trichostatin A, the amniotic fluid-derived cell line used in **Example 11** did not express Gata-1, HNF-3 beta or Pdx-1. Expression of insulin and Sox-17 was detected by RT-PCR at cycle 35 and cycle 27 respectively (**Table IX-B**). Following treatment with 0.5µM trichostatin A, HNF-3 beta was expressed at 34 cycles by RT-PRC until 72 hours; thereafter the expression level declined for the remainder of the experiment to cycle 37 as detected by RT-PCR. Pdx-1 gene expression was detected by 24 hours at cycle 39 by RT-PCR following trichostatin A treatment, but thereafter declined and was undetectable for the remainder of the experiment. Insulin gene expression was not detectable for the first 48 hours of the experiment following trichostatin A treatment but was detected by RT-PCR at cycle 34 at 72 hours and for the remainder of the experiment. Sox-17 and Gata-1 gene expression levels did not change throughout the course of the experiment (**Table IX-B**).

Early passage (P5) pancreatic-derived stromal cells did not express glucagon, HNF-3 beta or insulin prior to treatment with trichostatin A. However, Pdx-1 expression was detected by RT-PCR at cycle 38. Following treatment with 1.25 µM trichostatin A, there was no change in insulin expression. Pdx-1 and HNF-3 beta gene expression were detectable by RT-PCR at cycle 34 for both genes 24 hours following the addition of trichostatin A; thereafter expression fell to undetectable levels until after the second addition of trichostatin A, after which detection was at cycle 33 for Pdx-1 and HNF-3 beta. Glucagon gene expression was detected by RT-PCR at cycle 34 for the first 48 hours after addition of trichostatin A but fell to undetectable levels until after the second treatment cycle of trichostatin A, at which time the expression level increased to cycle 37 by RT-PCR (**Table IX-C**).

Similar results were seen for late passage (P11) pancreatic-derived stromal cells: Glucagon, HNF-3 beta, insulin, and Pdx-1 were undetectable prior to treatment. Following treatment with 1.25uM trichostatin A there was no change in insulin gene expression; however, expression of glucagon, HNF-3β and Pdx-1 genes could be detected by RT-PCR at cycle 33 for all three genes. Gene expression decreased thereafter or was undetectable until after the second treatment cycle of trichostatin A, where expression levels were detected by RT-PCR to be at cycle 36 for glucagon gene and 34 for HNF-3 beta and Pdx-1 genes (**Table IX-D**).

Collectively these data imply that effects of trichostatin A on gene expression are not sustained after its removal from culture. However, repeat applications of trichostatin A can restore it's effects on gene expression. The overall pattern of gene expression evoked by trichostatin A may depend in part on the dose concentration used, the duration of treatment, and the interval between treatment periods.

### Example 13

### Effects of Two Sequential High Dose Trichostatin A Treatments on Gene Expression in Amniotic Fluid-Cells and Pancreatic-Derived Stromal Cells.

Several cell lines obtained from different amniotic fluid specimens (see **Example 2**) and pancreas donors (see **Example 1**) were tested with similar results. Two lines at similar passage number but derived from different amniotic fluid specimens are shown in **Table X-A** and **Table X-B** for this example. One of these cell lines was also used in examples 2 and 4 above. In addition, this example also contains comparison data in **Table X-C** and **Table X-D** for a single pancreatic-derived stromal line grown to early and late passage number.

Amniotic fluid derived cells or pancreatic-derived stromal cells were seeded into 24-well tissue culture plates at a density of 5000/cm² and cultured in AMNIOMAX (Invitrogen) or DMEM with 10% FBS respectively under standard cell culture conditions until confluent. After the cells reached confluency, amniotic fluid derived cells were treated at time 0 hours with a dose of 1.25 µM trichostatin A, and pancreatic-derived stromal cells were treated with a dose of 5.0 µM trichostatin A solubilized in DMSO and medium. At time 24 hours, cell cultures underwent a complete medium change to remove all traces of trichostatin A. At time 96 hours, cell cultures were re-fed with fresh medium and received a second dose of trichostatin A at the same concentration as used previously. At time 120 hours, cell cultures again underwent a complete medium change and were cultured for an additional 24 hours or 148 hours total incubation time.

Samples were taken daily for RT-PCR at the times indicated in **Table X-A to D**. The culture media was removed, and samples were rinsed in PBS then collected in RLT with β-mercaptoethanol (Qiagen). RNA was purified using the RNeasy Mini Kit (Qiagen) and RNA quantity and quality were determined using a spectrophotometer. cDNA was made using the iScript cDNA synthesis kit (BioRad).

Samples of 20ng cDNA were used in each reaction to determine the expression levels of the following genes in amniotic fluid-derived cells: Gata1, HNF-3 beta, Pdx-1, insulin, and Soxl7. Similarly, samples of 20ng cDNA were used in each reaction to determine the expression level of the following genes in pancreatic-derived cells: glucagon, HNF-3 beta, insulin and Pdx-1. Real-Time PCR was performed on the Applied Biosystems 7500, and data was analyzed using the accompanying software according to the methods described in **Example 3**.

Prior to treatment with trichostatin A, the amniotic fluid-derived cell line used in **Examples 7 & 9** did not express Gata-1 or HNF-3 beta. Gene expression for insulin, Pdx-1, and Sox-17 was detected by RT-PCR at 36 cycles, 37 cycles, and 27 cycles, respectively. Treatment with 1.25 µM trichostatin A caused HNF-3 beta expression, detected at 32 cycles; however, this expression was transient as HNF-3 beta gene expression levels declined following the initial treatment with 1.25µM trichostatin A to undetectable levels. A second treatment with 1.25 µM trichostatin A caused HNF-3 beta gene expression to be detected at 32 cycles as measured by RT-PCR (**Table X-A**). Similar results were observed for Pdx-1 gene expression. Treatment with 1.25 µM trichostatin A caused an increase in Pdx-1 from 37 cycles to 32 cycles, as measured by RT-PCR. This increase was transient, however, as Pdx-1 gene expression levels declined to undetectable levels following the initial treatment with 1.25 µM trichostatin A. A second treatment with 1.25 µM trichostatin A caused Pdx-1 gene expression to be detected at 34 cycles as measured by RT-PCR (**Table X-A**). Gata-1 expression was only detected at 37 cycles by RT-PCR following the first addition of trichostatin A. Sox-17 gene expression did not change with the addition of trichostatin A. Insulin gene expression was undetectable following 1.25 µM trichostatin A treatment but was detected at 36 cycles as measured by RT-PCR following the media change (**Table X-A**).

Prior to treatment with trichostatin A, the amniotic fluid-derived cell line used in **Example 11** did not express Gata-1, HNF-3 beta and Pdx-1. However, expression of insulin and Sox-17 was detected by RT-PCR at 35 cycles and 27 cycles respectively. The initial 1.25 µM trichostatin A treatment stimulated expression of HNF-3 beta and Pdx-1. HNF-3 beta expression was detected at 35 cycles as measured by RT-PCR but that level of expression decreased to 38 cycles prior to the second addition of trichostatin A. Following the second 1.25 µM trichostatin A treatment HNF-3 beta gene expression returned to detection at 32 cycles as measured by RT-PCR. Pdx-1 expression was detected by RT-PCR at 33 cycles after the initial 1.25 µM trichostatin A treatment. This level of expression was not detectable after the trichostatin A was removed form the medium but returned to detectable levels at cycle 32 as measured by RT-PCR, following the second 1.25 µM trichostatin A treatment (**Table X-B**). Gata-1 gene expression did not change throughout the course of the experiment. While insulin gene expression was detected prior to trichostatin A treatment at 35 cycles as measured by RT-PCR, it was not detectable immediately following treatment. After trichostatin A was washed away, detection of insulin gene expression returned and was detectable at 36 cycles as measured by RT-PCR. Following the second addition of trichostatin A, insulin gene expression was once again undetectable. Sox-17 gene expression was unchanged for the duration of the experiment (**Table X-B**). These data suggest that 1.25 µM trichostatin A is a better concentration to use than 0.5 µM trichostatin A for increasing HNF-3β and Pdx-1 gene expression in this cell. The effects of 1.25 µM trichostatin A seem have a prolonged effect after a change of medium, indicating that the effect on gene expression persists beyond the actual time the compound is present. It also appears that trichostatin A may have an inhibitory effect on insulin gene expression.

Expression of glucagon, HNF-3 beta and insulin genes was not detected in early passage (P5) pancreatic-derived stromal cells prior to treatment with 5.0µM trichostatin A although Pdx-1 expression was observed at 38 cycles as measured by RT-PCR (**Table X-C**). Following treatment with 5.0 µM trichostatin A, HNF-3 beta and glucagon expression was detected at 34 cycles and 35 cycles respectively as measured by RT-PCR. HNF-3 beta gene expression was not detectable after trichostatin A was removed, but gene expression was restored and detectable at cycle 36 by RT-PCR following a second treatment with trichostatin A,. The level of expression of glucagon also decreased when trichostatin A was removed and was not detectable following the second addition of TSA until the end of the experiment when it was detected at 38 cycles by RT-PCR. Pdx-1 gene expression was detected only after the second treatment of trichostatin A at cycle 32 as measured by RT-PCR. Insulin gene expression did not change throughout the course of the experiment (**Table X-C**). These data suggest that 24 hours of treatment with 5.0 µM trichostatin A was sufficient to increase gene expression of HNF-3 beta and Pdx-1 but was not sufficient to increase insulin gene expression in early passage (P5) pancreatic-derived stromal cells.

Results for late passage (P11) pancreatic-derived stromal cells were similar to those seen for early passage (P5) pancreatic-derived stromal cells. Prior to trichostatin A treatment, no genes of interest were detectable, but following treatment with 5.0 µM trichostatin A, glucagon was detected at cycle 33, HNF-3 beta was detected at cycle 32 and Pdx-1 expression was detected at cycle 31 as measured by RT-PCR. Following the medium change, glucagon expression was still detectable by RT-PCR but the levels observed decreased to 38 cycles as measured by RT-PCR and did not increase with the second addition of trichostatin A. HNF-3 beta gene expression was detectable following the first trichostatin A treatment at 32 cycles but was undetectable following the change of medium; expression was not detectable again until after the second treatment of trichostatin A at 36 cycles as measured by RT-PCR. Pdx-1 was expressed at 31 cycles following the initial treatment with trichostatin A, but this level of expression was not detectable after the trichostatin A was removed. Pdx-1 gene expression was detected by RT-PCR at 34 cycles following the second addition of trichostatin A. There was no change in insulin gene expression following trichostatin A treatment or withdrawal (**Table X-D**). These data suggest that 24 hours of treatment with 5.0 µM trichostatin A was not sufficient to increase insulin gene expression in late passage (P 11) pancreatic-derived stromal cells, but that 5.0 µM trichostatin A was sufficient to increase gene expression of HNF-3 beta and Pdx-1.

### Example 14

### Effects of Two High Dose Trichostatin A Treatments on Gene Expression in Amniotic Fluid-Cells and Pancreatic-Derived Stromal Cells.

Several cell lines obtained from different amniotic fluid specimens (see **Example 2**) and pancreas donors (see **Example 1**) were tested with similar results. Two lines at similar passage number but derived from different amniotic fluid specimens are shown in **Table VIII-A** and **Table VIII-B** for this example. One of these cell lines was also used in **Examples 2** and **4** above. In addition, this example also contains comparison data in **Table X-C** and **Table X-D** for a single pancreatic-derived stromal line grown to early and late passage number.

Amniotic fluid derived cells or pancreatic-derived stromal cells were seeded into 24-well tissue culture plates at a density of 5000/cm² and cultured in AMNIOMAX (Invitrogen) or DMEM with 10% FBS respectively under standard cell culture conditions until confluent. After confluent, amniotic fluid derived cells were treated at time 0 hours with a dose of 1.25 µM trichostatin A, and pancreatic-derived stromal cells were treated with a dose of 5.0 µM trichostatin A solubilized in DMSO and medium. At time 48 hours, cell cultures underwent a complete medium change to remove all traces of trichostatin A. At time 96 hours, cell cultures were re-fed with fresh medium and received a second dose of trichostatin A at the same concentration as used previously. At time 120 hours, cell cultures again underwent a complete medium change and were cultured for an additional 24 hours or 148 hours total incubation time.

Samples were taken for RT-PCR at the times indicated in **Table XI-A to D.** RNA samples were obtained daily from the start of the experiment. Culture medium was removed, and cells were washed with PBS then collected in RLT with β-mercaptoethanol (Qiagen). RNA was purified using the RNeasy Mini Kit (Qiagen) and RNA quantity and quality was determined using a spectrophotometer. cDNA was made using the iScript cDNA synthesis kit (BioRad). Human pancreas cDNA was included as a control. Results were normalized against GAPDH expression levels.

Gene expression levels of Sox17, HNF-3 beta, Pdx-1, insulin, and glucagon were analyzed in amniotic-derived cells, while expression levels of glucagon, insulin, HNF-3 beta, and Pdx-1 were analyzed in pancreas-derived cells. Samples of 20ng cDNA was used in each Real-Time PCR reaction. RT-PCR was performed on the Applied Biosystems 7500, according to the methods described in **Example 3**. The data were analyzed using the accompanying software.

Prior to treatment with trichostatin A, the amniotic fluid-derived cell line used in **Examples 7 & 9** did not express Gata-1 and HNF-3 beta. Insulin and Pdx-1 were expressed at cycle 36 and cycle 37, respectively, as detected by RT-PCR, and Sox-17 expression was detected by RT-PCR at cycle 27. Following treatment with 1.25µM trichostatin A, Gata-1 gene expression was detected at cycle 37 by RT-PCR but was not detected for the remainder of the experiment. HNF-3 beta gene expression was detected by RT-PCR at cycle 31 following the addition of 1.25µM trichostatin A but this level of expression decreased to undetectable levels until the second addition of trichostatin A. Following the second treatment with trichostatin A, HNF-3 beta gene expression increased to 35 cycles as detected by RT-PCR. Insulin gene expression was not detectable by RT-PCR following the initial addition of 1.25µM trichostatin A. Pdx-1 gene expression was detected by RT-PCR at cycle 32 following the addition of 1.25µM trichostatin A, which decreased following the medium change but increased again after the second treatment with trichostatin A to 37 cycles as detected by RT-PCR. Sox-17 gene expression levels did not change throughout the course of the experiment (**Table XI-A**). These data provide further support that continued treatment with trichostatin A causes the increase of HNF-3 beta and Pdx-1 gene expression, that a more robust effect on gene expression can be measured with exposure to a higher concentration of trichostatin A for a longer time period, and that insulin gene expression may be inhibited in this cell type by the addition of trichostatin A.

Prior to treatment with trichostatin A, the amniotic fluid-derived cell line used in **Example 11** did not express Gata-1, HNF-3 beta and Pdx-1 prior to trichostatin A treatment. Sox-17 and insulin were expressed at cycles 27 and cycles 35, respectively, as detected by RT-PCR, prior to trichostatin A treatment. Following treatment with 1.25 µM trichostatin A, Gata-1 expression was detected at cycle 38 by RT-PCR, though this did not persist for more than 24 hours. Gata-1 expression was again detected at cycle 38 by RT-PCR following the second treatment with trichostatin A. HNF-3 beta expression was detected by RT-PCR at cycle 34 for the first 72 hours with a decrease to cycle 37 by 96 hours and an increase to cycle 33 following the second treatment with trichostatin A. Insulin expression was undetectable following treatment with 1.25 µM trichostatin A, and expression did not change for the duration of the experiment. Pdx-1 expression was detected at cycle 35 by RT-PCR following the initial 48-hour treatment with 1.25 µM trichostatin A. This level was undetectable once trichostatin A was removed but was detected by RT-PCR at cycle 34 following the second treatment of trichostatin A. Sox-17 gene expression levels did not change over the course of the experiment (**Table XI-B**). These data provide further support that continued treatment with trichostatin A causes an increase of HNF-3 beta and Pdx-1 gene expression, that a more robust effect on gene expression can be measured with exposure to a higher concentration of trichostatin A for a longer time period, and that insulin gene expression may be inhibited in this cell type by the addition of trichostatin A.

Early passage (P5) pancreatic-derived stromal cells expressed Pdx-1 at cycle 38 as detected by RT-PCR but do not express glucagon, HNF-3 beta and insulin prior to treatment with 5.0 µM trichostatin A. Following treatment with trichostatin A, glucagon gene expression was detected by RT-PCR at cycle 35. This gene expression level increased to cycle 33 as detected by RT-PCR at 48 hours but decreased to 38 cycles after the trichostatin A was removed. Following the second addition of trichostatin A, glucagon gene expression increased to 37 cycles. HNF-3 beta gene expression was detectable at 34 cycles by RT-PCR following trichostatin A treatment, and this level of expression persisted until the removal of trichostatin A. When trichostatin A was added to the cells, expression was detected by RT-PCR at cycle 33. Pdx-1 gene expression was not detectable following the initial treatment with trichostatin A but was detected by RT-PCR at cycle 32 at 48 hours. Once trichostatin A was removed from the medium, gene expression was undetectable (**Table XI-C**). Pdx-1 gene expression was detected at 32 cycles following the second addition of trichostatin as measured by RT-PCR. Insulin gene expression was undetectable while trichostatin A remained in the medium but was detected at 33 cycles by RT-PCR following the medium change. After trichostatin A was added to the medium again, insulin gene expression was undetectable (**Table XI-C**). These data provide further support that continued treatment with trichostatin A causes an increase of HNF-3 beta and Pdx-1 gene expression that a more robust effect on gene expression can be measured with exposure to a higher concentration of trichostatin A for a longer time period, and that insulin gene expression may be inhibited in this cell type by the addition of trichostatin A.

Results for late passage (P11) pancreatic-derived stromal were similar to those recorded for early passage (P5) pancreatic-derived stromal cells. No genes of interest were detectable prior to treatment with 5.0µM trichostatin A, but after addition of trichostatin A, glucagon was detected at cycle 33 by RT-PCR and HNF-3 beta was detected at 32 cycles by RT-PCR. Pdx-1 was detected by RT-PCR at cycle 31. Insulin gene expression did not change throughout the course of the experiment. Glucagon gene expression was detected at 33 cycles by RT-PCR for 48 hours following initial treatment with 5.0 µM trichostatin A. This level of glucagon gene expression decreased to 37 cycles following the removal of trichostatin A and was undetectable prior to second treatment with trichostatin A, at which point it was detected at cycle 35 by RT-PCR. HNF-3 beta and Pdx-1 gene expression followed the same pattern. Expression was detected for 48 hours following addition of 5.0 µM trichostatin A at cycles 33 and 32 respectively but was undetectable by RT-PCR after the medium was changed and trichostatin A was removed. Once trichostatin A was added again, HNF-3 beta gene expression increased to 34 cycles and Pdx-1 gene expression increased to 33 cycles as detected by RT-PCR (**Table XI-D**). These data provide further support that continued treatment with trichostatin A causes an increase of HNF-3 beta and Pdx-1 gene expression, that a more robust effect on gene expression can be measured with exposure to a higher concentration of trichostatin A for a longer time period, and that insulin gene expression may be inhibited in this cell type by the addition of trichostatin A.

### Example 15

### Effects of a 6 Hour Trichostatin A Treatment on Gene Expression in Amniotic Fluid-Cells and Pancreatic-Derived Stromal Cells.

Amniotic fluid derived cells or pancreas-derived cells were seeded into 24-well tissue culture plates at a density of 5000/cm² and cultured in AMNIOMAX (Invitrogen) or DMEM with 10% FBS respectively under standard cell culture conditions until confluency was reached. Upon reaching confluency, amniotic fluid derived cells were treated with 1.25 µM trichostatin A and pancreas-derived stromal cells were treated with 5.0 µM trichostatin A. The media was changed 6 hours following the addition of trichostatin A and cultures were maintained for the remainder of the experiment.. Several cell lines obtained from amniotic fluid (see **Example 2**) and pancreas (see **Example 1**) were tested. Samples were taken for RT-PCR at the times indicated in **Table XIIA-D.**

RNA samples were obtained at the time the trichostatin A was removed and 24 hours from the start of the experiment. The culture media was removed and cells were washed with PBS then collected in RLT Lysis Buffer with β-mercaptoethanol (Qiagen). RNA was purified using the RNeasy Mini Kit (Qiagen) and RNA quantity and quality was determined using a spectrophotometer. cDNA was made using the iScript cDNA synthesis kit (BioRad). Human pancreas cDNA was included as a control. Results were normalized against GAPDH expression levels.

The expression levels of Sox17, HNF-3 beta, Pdx-1, Insulin, and Gata-1 were analyzed in amniotic-derived cells while the expression levels of glucagon, insulin, pdx-1 and HNF-3 beta were analyzed in pancreas-derived cells. 20ng of cDNA was used in each RT-PCR reaction, which was performed on the Applied Biosystems 7500, according to the methods described in **Example 3.** Data was analyzed using the accompanying software.

Prior to treatment with trichostatin A, the amniotic fluid-derived cell line used in **Examples 7 & 9** did not express Gata-1 and HNF-3 beta. Following treatment with 1.25 µM trichostatin A, HNF-3 beta expression was detected by RT-PCR cycle 32, but following the wash, increased to 35 cycles. Insulin gene expression decreased from 36 cycles to undetectable levels as determined by RT-PCR following the addition of trichostatin A (**Table XII-A**). Pdx-1 expression was detected at cycle 37 by RT-PCR prior to treatment with trichostatin A. This level of expression decreased to 35 cycles after treatment with trichostatin A but was undetectable once trichostatin A was removed. Expression of Gata-1 and sox-17 remained unchanged with the addition of trichostatin A (**Table XII-A**).

Prior to treatment with trichostatin A, the amniotic fluid-derived cell line used in **Example 11** did not express Gata-1, HNF-3 beta or Pdx-1. Insulin and sox-17 were expressed at cycle 35 and cycle 27 respectively by RT-PCR. 6 hours of treatment with 1.25 µM trichostatin A was sufficient to increase the expression of HNF-3 beta and Pdx-1 to cycles 31 and 35 respectively, as detected by RT-PCR. Following removal of trichostatin A, HNF-3 beta expression was detected at cycle 35 by RT-PCR and Pdx-1 was undetectable. Insulin gene expression was not detectable following addition of trichostatin A. Gata-1 and Sox-17 gene expression levels remained unchanged following treatment of 1.25µM trichostatin A **(Table XII-B).** These data provide support that insulin gene expression may be inhibited by trichostatin A treatment in these cells and that 6 hours of treatment was sufficient to see an increase in gene expression of HNF-3 beta and Pdx-1.

Early passage (P5) pancreatic-derived stomal cells did not express glucagon, HNF-3 beta or insulin prior to addition of 5.0 µM trichostatin A. Following trichostatin A treatment glucagon gene expression was detected by RT-PCR at cycle 37, HNF-3 beta gene expression was detected by RT-PCR at cycle 35. Following trichostatin A removal, glucagon gene expression remained the same but HNF-3 beta was undetectable. Insulin gene expression remained undetectable until the trichostatin A was removed from the media, where it was then detected at cycle 32 by RT-PCR. Pdx-1 gene expression was detected at 38 cycles by RT-PCR prior to trichostatin A treatment, but following treatment that level of expression increased to 35 cycles **(Table XII-C).** These data suggest that treatment with 5.0µm trichostatin A for 6 hours was not sufficient for increasing expression of Pdx-1, but continues to suppress insulin gene expression as noted previously.

Late passage (P11) pancreatic-derived stromal cells did not express any of the genes of interest prior to treatment with 5.0 µM trichostatin A. Following trichostatin A treatment, glucagon was detected at 37 cycles, HNF-3 beta was detected at 36 cycles and Pdx-1 was detected at 37 cycles by RT-PCR. There was no change in insulin gene expression following the addition of trichostatin A. Following the removal of trichostatin A, glucagon gene expression was detected at 35 cycles by RT-PCR, while HNF-3 beta and Pdx-1 gene expression was undetectable **(Table XII-D).** These data suggests that trichostatin A is necessary to up-regulate HNF-3 beta and Pdx-1 gene expression.

### Example 16

### HDAC Inhibitor Screening for Cellular Differentiation

A library of small molecule inhibitors was made available for purposes of screening. The library was representative of a focused set of chemical structures designed for potential enzymatic inhibition. Compounds were solubilized in DMSO and diluted to 1mM stocks in 30% DMSO/50mM HEPES, plated in 96-well format, and frozen dessicated at -80°C prior to use.

Amniotic fluid derived cells were seeded into flat bottom 96-well tissue culture plates at a density of 15,000 cells/well and cultured until confluent in completely supplemented AMNIOMAX (Invitrogen) under standard cell culture conditions. After confluent, cells were treated at time 0 hours with 5µM HDAC inhibitor diluted in culture medium (0.5% DMSO final assay concentration). Negative control wells received diluent alone. Positive control wells received 5uM TSA. At time 24 hours, samples were taken for RT-PCR. Culture medium was removed, cells were washed with PBS, and a lysate was collected in RLT with β-mercaptoethanol (Qiagen). RNA was purified using the RNeasy Mini Kit (Qiagen), and RNA quantity and quality was determined using a spectrophotometer. cDNA was made using the iScript cDNA synthesis kit (BioRad). Human pancreas cDNA was included as a control. Results were normalized against GAPDH expression levels.

Gene expression levels of Pdx-1 were analyzed using 20ng cDNA in each Real-Time PCR reaction. RT-PCR was performed on the Applied Biosystems 7500 according to the methods described in **Example 15.** Data analysis was performed using the accompanying software. Results for all primary screening are presented in **Table XIII-A.**

Compounds that caused an increase in Pdx-1 gene expression to a level at least 80% of that induced by TSA were considered "hits" and were retested in the above protocol. Of 572 compounds tested, 56 were considered "hits" (9.8% hit rate). In the confirmation assays, all 56 hits were able to increase Pdx-1 gene expression to a level at least 80% of that caused by TSA (100% confirmation rate). Results for confirmation screening are presented in **Table XIII-B.**

Confirmed compounds were also screened in triplicate for cytotoxic effects using an MTS tetrazolium assay (Promega; Cell Titer 96 AQᵤₑₒᵤₛ). Parallel cultures of amniotic fluid-derived cells in 96-well plates were exposed to 5µM compound for 24 or 72 hours under standard culture conditions. MTS reagent was added to each well per manufacturer's instructions and cultured at 37°C for one hour then read at 490nm on a standard spectrophotometer. Numerical readings from treated wells were compared to untreated control wells and used to calculate a % viability index correlating with cellular metabolic activity. Summary results for all compounds are presented in **Figures 6** **and** **7** comparing viability measures for both exposure times.

The top ten most effective compounds were selected based on highest induction of Pdx-1 gene expression and least toxicity. These compounds were further tested in a two-fold dose titration assay using amniotic fluid derived cells **(Table XIII-C and** **Figure 8****)** or pancreatic derived cells **(Table XIII-D and** **Figure 9****).** Compound concentrations were tested over a range of 0.15 to 10µM for an exposure time of 24hrs prior to analysis of Pdx I gene expression. An MTS cytotoxicity assay was also repeated in triplicate with a dose titration of each compound in both cell types. Summary results are presented in **Figures 10** **and** **11****.** **Figure 12** compares the potency of enzyme inhibition for a dose titration of selected hits to TSA, a known HDAC inhibitor.

### Example 17

### Effects of a Combination of HDAC inhibitor and MG-132 Treatment on Gene Expression in an Adult Pancreatic-Derived Cell line.

Pancreatic derived cells were treated with trichostatin A (TSA), at varying concentrations either alone or in sequential combination with a cell permeable proteasome inhibitor, MG-132, also added at varying concentrations. Briefly, cells were plated and grown to a confluent monolayer in 6-well cluster plates in normal culture medium composed of DMEM high glucose with 10% FCS. At time 0 hrs, a sample of untreated cells was harvested for a basal control. MG-132 (EMD Biosciences) solubilized in DMSO was diluted to a final concentration of 1µM or 10 µM in fresh medium and allowed to incubate with the cells for 24 hours under standard culture conditions. After 24 hours, the culture medium was removed, replaced with fresh medium containing a final dilution of 1.25 µM or 2.5 µM TSA, and allowed to incubate for an additional 24 hours. Negative control wells contained an equivalent volume of medium and DMSO diluent but without treatment compound.

RNA samples were obtained from the treated cultures 48 hours after initiating treatment. The culture medium was removed, the cells were washed with phosphate buffered saline (PBS), and RLT Lysis buffer containing β-mercaptoethanol (Qiagen) was added. The samples were homogenized using Qiashredder columns (Qiagen), and RNA was purified using the RNeasy Mini Kit (Qiagen). RNA quantity and quality was determined using a spectrophotometer, and cDNA was made using the iScript cDNA synthesis kit (BioRad).

The expression levels of Pdx-1, HNF-3 beta, insulin, and Nkx6.1 were determined by Real-Time PCR (RT-PCR) using 20ng cDNA per reaction, as described in **Example 3.** RT-PCR reactions were performed on the Applied Biosystems 7500 with data analysis using the accompanying software. Human pancreas cDNA was included as a positive control. Results were normalized against GAPDH expression levels.

Results in **Table XIV- Table XVI** demonstrate that TSA can induce a low level of gene expression for Pdx-1, HNF-3 beta, and Nkx6.1 in adult pancreatic-derived cells after 24 hours of treatment. Untreated cells consistently demonstrate no basal expression level of these genes. The proteasome inhibitor MG-132 was not able to induce gene expression when added alone to cultures (with the exception of 10 µM MG-132 which induced a low level of Nkx6.1 expression) nor did pre-treatment with the MG-132 compound prior to TSA addition enhance gene expression levels. Insulin gene expression was absent in adult pancreatic-derived cells and did not change relative to untreated controls in any of the samples tested.

The sequence of addition for MG-132 and TSA to cells in culture was reversed for results shown in **Table XV**. TSA alone was able to induce a low level of gene expression for Pdx-1, HNF-3 beta, and Nkx6.1 in adult pancreatic-derived cells, and this observed gene expression was not enhanced by pre- or post-treatment of cells with the MG-132 compound. In some cases, induced gene expression decreased if the TSA was removed in the final 24 hours of culture, suggesting that effects of an HDAC inhibitor on chromatin remodeling are reversible over time after washout and recovery.

The ability of TSA to induce gene expression was tested in different passages of adult pancreatic-derived cells, alone or in combination with MG-132, to determine if length of time in culture is a factor in their susceptibility to a differentiation stimulus. Results in **Table XVI** show gene expression levels for passage 10 and passage 19 cells after treatment with TSA, alone or in combination with MG-132. Overall, late passage (P19) cells demonstrate slightly better gene expression compared to early passage (P10) cells in this example, but results are not consistent or statistically significant for individual genes or treatments.

A range of dose concentrations and exposure times for treatment with the MG-132 compound followed by sequential addition of TSA (1.25 µM or 2.5 µM for 24 hours) was tested for improved induction of gene expression in adult pancreatic-derived cells. Results are shown in **Table XVII** and **Table XVII**. A slight additive effect was observed for combination treatment conditions, but there was no robust improvement in gene expression over TSA treatment alone for Pdx-1, HNF-3 beta, or Nkx6.1. Insulin gene expression was absent in adult pancreatic-derived cells and did not change relative to untreated controls in any of the samples tested.

Adult pancreatic-derived cells were also exposed to sequential treatments with MG-132 followed by TSA followed by a differentiation medium in an effort to enhance differentiation and lineage specific gene expression. Results for Pdx-1 gene expression in adult pancreatic-derived cells are shown in **Table XVIII** for a range of compound doses and exposure times in one exemplary differentiation medium. Differentiation medium #2 contained DMEM low glucose, 1% FCS, 20ng/ml activin A, 10ng/ml betacellulin, 20ng/ml bFGF, 10mM nicotinamide, and 50nM GLP-1 receptor agonist. As was observed previously **(Table XVII),** induced gene expression decreased after TSA was removed from culture, suggesting that effects of an HDAC inhibitor on chromatin remodeling are reversible over time after washout and recovery. Gene expression was not improved over TSA treatment alone for all treatment categories.

Additional differentiation agents were tried in combination with TSA in an attempt to enhance differentiation and lineage specific gene expression. GSI-IX and GSI-X (EMD Biosciences) are two gamma secretase inhibitors that block Notch activation and signaling, thereby modulating cellular differentiation. **Table XIX** shows gene expression results for cells treated with various concentrations of each gamma secretase inhibitor, alone and in sequential combination with TSA. Neither GSI-IX or GSI-X were able to induce Pdx-1, HNF-3 beta, or Nkx6.1 gene expression when added alone to adult pancreatic-derived cell cultures nor did pre-treatment with the either GSI compound prior to TSA addition enhance gene expression levels. Insulin gene expression was absent in adult pancreatic-derived cells and did not change relative to untreated controls in any of the samples tested.

A series of proprietary HDAC inhibitors was identified from a cell-based screen as having significant ability to induce gene expression relative to TSA **(Example 16).** These compounds were tested in adult pancreatic-derived cells, alone or in sequential addition with the proteasome inhibitor MG-132. Results are shown for the Pdx-1 gene in **Table XX** and the insulin gene in **Table XXI.** Untreated adult pancreatic-derived cells consistently demonstrated no basal expression level of Pdx-1. Each HDAC inhibitor induced a low level of Pdx-1 gene expression. However, pre-treatment with MG-132 did not significantly enhance Pdx-1 gene expression beyond what was observed with individual HDAC inhibitors alone. Insulin gene expression was absent in adult pancreatic-derived cells and did not change relative to untreated controls in any of the samples tested.

### Example 18

### Dose Titration of Chromatin Remodeling or DNA Demethylating Agents at Various Times.

Amniotic fluid cells were plated in duplicate culture plates with multiple replicate sets. After reaching confluency (2-3 days), 5-azacytidine (5-Aza-2'-deoxycytidine; EMD Biosciences) was added to each replicate set at a range of 0.156 µM to 2.5 µM, final concentration. An equivalent amount of solvent was added to the no treatment control cultures. Cells were returned to standard culture conditions for 24 hr after which medium was removed, cells were washed with phosphate buffered saline (PBS), and RLT lysis buffer containing β-mercaptoethanol (Qiagen) was added to each well. Samples were homogenized using Qiashredder columns (Qiagen) and RNA was purified using the RNeasy Mini Kit (Qiagen). RNA quantity and quality was determined using a spectrophotometer, and cDNA was made using the iScript cDNA synthesis kit (BioRad). Samples of 20ng cDNA were used in each reaction to determine expression levels of Pdx-1 or HNF-3 beta. Real-Time PCR was performed on an Applied Biosystems 7500 system, and data was analyzed using the accompanying software.

**Table XXIII** shows that untreated amniotic fluid cells have no basal expression level of Pdx-1 or HNF-3 beta. 5-Aza-2'deoxycytidine (5-Aza) is a DNA methyltransferase inhibitors which can affect chromatin remodeling. Treatment of Amniotic fluid cells with 5-Aza alone did not induce Pdx-1 or HNF-3 beta. In contrast, treatment with the HDAC inhibitor TSA was able to induce Pdx-1 and HNF-3 beta gene expression.

### Example 19

### Changes in Gene Expression by the Simultaneous Addition of a Histone Deacetylase Inhibitor and a DNA Demethylating Agent.

Amniotic fluid cells were plated and allowed to reach confluency (2-3 days). Both trichostatin A and 5-aza-2'-deoxcytidine were added to the culture at an optimal, nontoxic dose titration for 24 hours. At the conclusion of the treatment period, medium was removed, cells were washed with phosphate buffered saline (PBS), and RLT lysis buffer containing β-mercaptoethanol (Qiagen) was added to each well. Samples were homogenized using Qiashredder columns (Qiagen), and RNA was purified using the RNeasy Mini Kit (Qiagen). RNA quantity and quality were determined using a spectrophotometer, and cDNA was made using the iScript cDNA synthesis kit (BioRad). Samples of 20ng cDNA were used in each reaction to determine expression levels of Pdx-1 and HNF-3 beta. Real-Time PCR was performed on an Applied Biosystems 7500 system, and data was analyzed using the accompanying software.

**Table XXIV** shows that untreated amniotic fluid cells have no basal expression of Pdx-1 or HNF-3 beta, but gene expression can be induced after 24 hrs treatment with 1.25uM TSA. Treatment of amniotic fluid cells with 5-Aza alone did not induce Pdx-1 or HNF-3 beta nor did it enhance gene expression when used in combination with the HDAC inhibitor TSA.

A sequential treatment with 5-Aza-2'-deoxycytidine followed by TSA was also used to induce gene expression in amniotic fluid cells. Confluent monolayers were pre-treated for 6 hours with 5-Aza after which TSA was added in combination for the final 18 hours of culture. Cell lysates, RNA extraction, cDNA synthesis and PCR were performed as described above. Results as shown in **Table XXV** also indicate that 5-Aza alone did not induce Pdx-1 or HNF-3 beta gene expression nor did it demonstrate an additive effect when used as a pre-treatment in combination with the HDAC inhibitor TSA.

### Example 20

### The Effects of Trichostatin A Treatment and Inhibition of the Sonic Hedgehog Pathway on Gene Expression in Early Passage (P5) Pancreatic-Derived Stromal Cells and Amniotic Fluid-Derived Cells.

Pancreatic-derived stromal cells are obtained according to the methods described in **Example 1**. Cells are seeded into a 24-well tissue culture plate at a density of 5000/cm2/well and cultured in DMEM with 10% FBS under standard cell culture conditions until confluent. Amniotic fluid obtained from National Disease Research Interchange (NDRI) is processed according to the methods described in **Example 2**. Cells are seeded into a 24-well tissue culture plate at a density of 5000/cm2/well and cultured in AMNIOMAX (Invitrogen) under standard cell culture conditions until confluent. After the cells reach confluency, sample wells are treated with 1.25 mM trichostatin A diluted in DMSO and medium; control wells receive DMSO at an equivalent concentration. At 24 hours, sample wells receive another dose of 1.25 µM trichostatin A. A 10-µM dose of Cyclopamine (Sigma) and 10 mM Nicotinamide (Sigma) are added to the cell culture medium. The following day, medium is removed and cells are washed with PBS. New medium is added once every other day including similar doses of Nicotinamide and Cyclopamine. On day 7, Cells are collected for real time PCR analysis as described in **Example 3.**

### Example 21

### The Effect of Trichostatin A on Gene Expression in Peripheral Blood Mononuclear Cells.

Peripheral blood mononuclear cells are isolated by density gradient sedimentation and plated in culture at a density of 0.5-2 x 10⁶ per ml. An activation mitogen, such as for example PHA, is added at a final concentration of 10µg/ml. Controls omit the addition of PHA. Cells will be cultured for 3 days after which cells will be collected, washed, counted, resuspended and replated at a density of 1-2 x 10⁶ per ml. Trichostatin A (or an alternative histone deacetylase inhibitor) and/or 5-azacytidine (or an alternative demethylating agent) is added to the culture at an optimal, nontoxic concentration and for a preferred time period to induce appropriate gene expression, as determined from **Examples 5-15** above. Untreated control wells receive a similar dilution of vehicle or diluent. At the conclusion of the culture time period (for example, 24 hours), medium will be removed, and cells are washed with phosphate buffered saline (PBS). RLT lysis buffer containing β-mercaptoethanol (Qiagen) is added to each well. Samples are homogenized using Qiashredder columns (Qiagen) and RNA is purified using the RNeasy Mini Kit (Qiagen). RNA quantity and quality is determined using a spectrophotometer, and cDNA is made using the iScript cDNA synthesis kit (BioRad). Samples of 20ng cDNA are used in each reaction to determine expression levels of PDX-1, insulin, glucagon, somatostatin, sox17, gata4, globin, beta-2-microglobulin. Real-Time PCR is performed on an Applied Biosystems 7500 system, and data is analyzed using the accompanying software.

### Example 22

### Differentiation of Cells into Non-Pancreatic Cell types.

Amniotic fluid or pancreatic progenitor cells or neonatal fibroblasts are plated and allowed to reach confluency (2-3 days). Alternatively, peripheral blood mononuclear cells are isolated by density gradient sedimentation and plated in culture at a density of 1-2 x 10⁶ per ml. Trichostatin A (or an alternative histone deacetylase inhibitor) and/or 5-azacytidine (or an alternative demethylating agent) is added to the culture at an optimal, nontoxic concentration and for a preferred time period to induce appropriate gene expression, as determined from **Examples 5-15 above**. At the conclusion of the time period (for example, 24 hours), medium is removed; cells are washed with phosphate buffered saline (PBS), resuspended and plated at a seeding density as above. A differentiation protocol for adipocytes, known to those skilled in the art (Chemicon cat. #ECM950), is applied to cultured cells over a time period of 7 days or as appropriate. After culture is concluded, cells are stained with Oil Red O to detect lipid droplets in differentiated adipocytes. Alternatively, RLT lysis buffer containing β-mercaptoethanol (Qiagen) is added to each well. Samples are homogenized using Qiashredder columns (Qiagen) and RNA is purified using the RNeasy Mini Kit (Qiagen). RNA quantity and quality is determined using a spectrophotometer, and cDNA is made using the iScript cDNA synthesis kit (BioRad). Samples of 20ng cDNA are used in each reaction to determine expression levels of PPARγ. Real-Time PCR will be performed on an Applied Biosystems 7500 system, and data will be analyzed using the accompanying software.

**TABLE I-A. ANTIBODIES TO SURFACE RECEPTORS**

| Antibody | Supplier | Isotype | Clone |
|---|---|---|---|
| Alkaline phosphatase | R&D systems (MN) | Mouse IgG1 | B4-78 |
| ATP binding cassette transporter (ABCG2) | BD Pharmingen (CA) | Mouse IgG2b, Kappa | 5D3 |
| CD10 | BD Pharmingen (CA) | Mouse IgG1, Kappa | HI10a |
| CD29 (Beta I integrin) | BD Pharmingen (CA) | Mouse IgG1, Kappa | MAR4 |
| CD44 | BD Pharmingen (CA) | Mouse IgG2b, Kappa | G44-26 |
| CD45 | BD Pharmingen (CA) | Mouse IgG1, Kappa | Hi30 |
| CD49f | BD Pharmingen (CA) | Rat IgG2A, Kappa | G0H3 |
| CD49b (Alpha 2 integrin) | BD Pharmingen (CA) | Mouse IgG2a, Kappa | 121-H6 |
| CD56 (NCAM) | BD Pharmingen (CA) | Mouse IgG1, Kappa | B 159 |
| CD73 | BD Pharmingen (CA) | Mouse IgG1, Kappa | AD2 |
| CD90 | BD Pharmingen (CA) | Mouse IgG1, kappa | 5E10 |
| CD95 | BD Pharmingen (CA) | Mouse IgG1, Kappa | DX2 |
| CD105 (endoglin) | Santa Cruz Biotechnology (CA) | Mouse IgG1 | P3D1 |
| CD117 (c-Kit) | BD Pharmingen (CA) | Mouse IgG1, kappa | YB5.B8 |
| CD133 | Miltenyi Biotec (CA) | Mouse IgG1 | Ac133 |
| Epithelial adhesion molecule (EpCAM) | BD Pharmingen (CA) | Mouse IgG1 | EBA-1 |
| Hepatocyte growth factor receptor (HGF or c-Met) | R&D systems (MN) | Mouse IgG2A | 95309 |
| Platelet/endothelial cell adhesion molecule-1 (PECAM-1) | Santa Cruz Biotechnology (CA) | Mouse IgG1 | WM-59 |
| CD49b (Alpha 2 integrin) | BD Pharmingen (CA) | Mouse IgG2a, Kappa | 121-H6 |
| Alpha 3 integrin | Santa Cruz (CA) | Mouse IgG1 | P1B5 |
| Alpha 5 intgerin | Santa Cruz (CA) | Mouse IgG3 | P1D6 |
| Beta 3 integrin | Santa Cruz (CA) | Mouse IgG1 | Y2/51 |
| Alpha V Beta 3 integrin (CD51/61) | BD Pharmingen (CA) | Mouse IgG1, Kappa | 23C6 |
| SSEA-3 | Chemicon (CA) | Mouse IgG3 | MC-631 |
| SSEA-4 | Chemicon (CA) | Rat IgM | MC-813-70 |
| TRA 1-60 | Chemicon (CA) | Mouse IgM | TRA 1-60 |
| TRA 1-81 | Chemicon (CA) | Mouse IgM | TRA 1-81 |
| TRA 1-85 | Chemicon (CA) | Mouse IgG1 | TRA 1-85 |
| TRA 2-54 | Chemicon (CA) | Mouse IgG1 | TRA 2-54 |
| EGF r | BD Pharmingen (CA) | Mouse IgG2b, Kappa | EGFR1 |
| HLA ABC | BD Pharmingen (CA) | Mouse IgG1, Kappa | G46-2.6 |
| HLA DR | BD Pharmingen (CA) | Mouse IgG2b, Kappa | TU36 |

**TABLE I-B. LIST OF ANTIBODIES USED FOR IDENTIFICATION OF INTRACELLULAR MARKERS**

| Antibody | Supplier | Isotype | Clone |
|---|---|---|---|
| Nestin | R&D systems (MN) | Mouse IgG1 | HSG02 |
| Cytokeratin 5/8 | Santa Cruz Biotechnology (CA) | Mouse IgG1 | C50 |
| Vimentin | Santa Cruz Biotechnology (CA) | Mouse IgG1 | V9 |
| Pan-Cytokeratin (4, 5, 6, 8, 10, 13, 18) | Santa Cruz Biotechnology (CA) | Mouse IgG 1 | C11 |
| Peripherin | Santa Cruz Biotechnology (CA) | Goat Polyclonal | C 19 |
| Gilial fibrillary acidic protein (GFAP) | Santa Cruz Biotechnology (CA) | Goat polyclonal | N-18 |
| Pan-Cytokeratin (14, 15, 16, and 19) | BD Pharmingen (CA) | Mouse IgG1, Kappa | KA4 |
| Beta III tubulin | Chemicon International (CA) | Mouse IgG1 | TU-20 |

**TABLE II. LIST OF SECONDARY CONJUGATED ANTIBODIES USED FOR FACS AND IMMUNOSTAINININGANALYSIS.**

| Secondary conjugated antibody | Supplier | Dilution |
|---|---|---|
| Goat Anti-Mouse IgG APC conjugated | Jackson ImmunoResearch (PA) | 1:200 |
| Goat Anti-Mouse IgG PE conjugated | Jackson ImmunoResearch (PA) | 1:200 |
| Donkey anti-rabbit PE or - APC conjugated | Jackson ImmunoResearch (PA) | 1:200 |
| Donkey anti-goat PE or - APC conjugated | Jackson ImmunoResearch (PA) | 1:200 |
| Goat anti-mouse IgM PE | SouthernBiotech (AL) | 1:200 |
| Goat anti-Rat IgM PE | SouthernBiotech (AL) | 1:200 |
| Goat anti-mouse IgG3 PE | SouthernBiotech (AL) | 1:200 |

**TABLE III. THE EFFECTS OF HISTONE DEACETYLASE INHIBITOR TREATMENT ON GENE EXPRESSION IN PANC-1 CELLS AND NEONATAL FIBROBLASTS.**

| | GAPDH | glucagon | HNF-3beta | Insulin | pdx-1 | Sox17 |
|---|---|---|---|---|---|---|
| untreated fibroblasts | +++ | nd | nd | nd | nd | nd |
| fibroblasts with 2.5µM trichostatin A after 48 hours | +++ | +++ | +++ | nd | + | +++ |
| Fibroblasts with 5.0µM trichostatin A after 48 hours | +++ | +++ | +++ | nd | + | +++ |
| untreated Panc-1 | +++ | nd | +++ | nd | +++ | +++ |
| Panc-1 with 2.5µM trichostatin A after 48 hours | +++ | +++ | +++ | + | +++ | +++ |
| panc-1 with 5.0µM trichostatin A after 48 hours | +++ | +++ | +++ | + | +++ | +++ |
| human pancreas | +++ | +++ | +++ | ++ | +++ | +++ |
| | | | | | | |
| | | nd | not detectable at >40 cycles by RT-PCR | | | |
| | | + | detectable at greater than 35-40 cycles by RT-PCR | | | |
| | | ++ | detectable at less than 35 cycles by RT-PCR with SE greater than 0.9 | | | |
| | | +++ | detectable at less than 35 cycles by RT-PCR with SE less than 0.9 | | | |
| All RT-PCR was performed on the Applied Biosystems 7500 Real-Time PCR System | | | | | | |

**TABLE IV-A. GENE EXPRESSION IN THE UNTREATED AMNIOTIC FLUID-DERIVED CELL LINE AFCA009-A.**

| | Untreated | | | | |
|---|---|---|---|---|---|
| Time (hours) | glucagon | HNF-3beta | Insulin | pdx-1 | Sox17 |
| 0 | nd | nd | + | nd | +++ |
| 0.5 | nd | + | + | nd | +++ |
| 1.5 | nd | nd | + | nd | +++ |
| 3 | nd | nd | +++ | nd | +++ |
| 6 | nd | + | +++ | nd | +++ |
| 12 | nd | nd | +++ | nd | +++ |
| 24 | nd | nd | +++ | nd | +++ |
| | | | | | |
| nd | not detectable at >40 cycles by RT-PCR | | | | |
| + | detectable between 35 and 40 cycles by RT-PCR | | | | |
| ++ | detectable at less than 35 cycles by RT-PCR with std error greater than 0.9 | | | | |
| +++ | detectable at less than 35 cycles by RT-PCR with std error less than 0.9 | | | | |
| All RT-PCR was performed on the Applied Biosystems 7500 Real-Time PCR System | | | | | |

**TABLE IV-B. GENE EXPRESSION IN THE AMNIOTIC FLUID-DERIVED CELL LINE AFCA009-A TREATED WITH TRICHOSTATIN A FOR 24 HOURS.**

| | Treated | | | | |
|---|---|---|---|---|---|
| Time (hours) | glucagon | HNF-3beta | Insulin | pdx-1 | Sox17 |
| 0.5 | nd | + | + | nd | +++ |
| 1.5 | nd | + | + | nd | +++ |
| 3 | nd | +++ | + | nd | +++ |
| 6 | nd | +++ | nd | + | +++ |
| 12 | + | +++ | + | +++ | +++ |
| 24 | +++ | +++ | + | +++ | +++ |
| | | | | | |
| nd | not detectable at >40 cycles by RT-PCR | | | | |
| + | detectable between 35 and 40 cycles by RT-PCR | | | | |
| ++ | detectable at less than 35 cycles by RT-PCR with std error greater than 0.9 | | | | |
| +++ | detectable at less than 35 cycles by RT-PCR with std error less than 0.9 | | | | |
| All RT-PCR was performed on the Applied Biosystems 7500 Real-Time PCR System | | | | | |

**TABLE V-A. GENE EXPRESSION IN LATE PASSAGE PANCREATIC-DERIVED STROMAL CELLS.**

| | untreated | | | | |
|---|---|---|---|---|---|
| Time (hours) | glucagon | HNF-3beta | Insulin | pdx-1 | Sox17 |
| 0 | nd | nd | nd | nd | + |
| 0.5 | nd | nd | nd | nd | nd |
| 1.5 | nd | nd | nd | nd | nd |
| 3 | nd | nd | nd | nd | + |
| 6 | nd | nd | nd | nd | + |
| 12 | nd | nd | nd | nd | nd |
| 24 | nd | nd | nd | nd | + |
| | | | | | |
| nd | not detectable at >40 cycles by RT-PCR | | | | |
| + | detectable between 35 and 40 cycles by RT-PCR | | | | |
| ++ | detectable at less than 35 cycles by RT-PCR with std error greater than 0.9 | | | | |
| +++ | detectable at less than 35 cycles by RT-PCR with std errorless than 0.9 | | | | |
| All RT-PCR was performed on the Applied Biosystems 7500 Real-Time PCR System | | | | | |

**TABLE V-B. GENE EXPRESSION IN LATE PASSAGE PANCREATIC-DERIVED STROMAL CELLS TREATED WITH TRICHOSTATIN A FOR 24 HOURS.**

| | Treated | | | | |
|---|---|---|---|---|---|
| Time (hours) | glucagon | HNF-3beta | Insulin | pdx-1 | Sox17 |
| 0.5 | nd | nd | nd | nd | + |
| 1.5 | nd | nd | nd | nd | nd |
| 3 | nd | nd | nd | nd | + |
| 6 | nd | + | nd | nd | + |
| 12 | nd | + | nd | + | + |
| 24 | + | +++ | nd | +++ | +++ |
| | | | | | |
| nd | not detectable at >40 cycles by RT-PCR | | | | |
| + | detectable between 35 and 40 cycles by RT-PCR | | | | |
| ++ | detectable at less than 35 cycles by RT-PCR with std error greater than 0.9 | | | | |
| +++ | detectable at less than 35 cycles by RT-PCR with std error less than 0.9 | | | | |
| All RT-PCR was performed on the Applied Biosystems 7500 Real-Time PCR System | | | | | |

**TABLE VI. THE EFFECT OF CHRONIC TRICHOSTATIN A TRATMENT ON GENE EXPRESSION IN AMNIOTIC FLUID-DERIVED CELLS.**

| 24 hours | glucagon | HNF-3beta | Insulin | pdx-1 | Soxl7 | |
|---|---|---|---|---|---|---|
| untreated | nd | nd | + | nd | +++ | |
| 500 nM | + | +++ | nd | +++ | +++ | |
| 1 µM | +++ | +++ | nd | +++ | +++ | |
| | | | | | | |
| 48 hours | glucagon | HNF-3b | Insulin | PDX-1 | Sox17 | |
| untreated | nd | + | +++ | nd | +++ | |
| 500 nM | + | +++ | nd | + | +++ | |
| 1 µM | ++ | +++ | nd | +++ | +++ | |
| 72 hours | glucagon | HNF-3b | Insulin | PDX-1 | Sox17 | |
| untreated | nd | + | +++ | nd | +++ | |
| 500 nM | + | +++ | nd | +++ | +++ | |
| 1 µM | +++ | +++ | nd | +++ | +++ | |
| | | | | | | |
| nd | not detectable at >40 cycles by RT-PCR | | | | | |
| + | detectable between 35 and 40 cycles by RT-PCR | | | | | |
| ++ | detectable at less than 35 cycles by RT-PCR with std error greater than 0.9 | | | | | |
| +++ | detectable at less than 35 cycles by RT-PCR with std error less than 0.9 | | | | | |
| All RT-PCR was performed on the Applied Biosystems 7500 Real-Time PCR System | | | | | | |

**TABLE VII. THE EFFECT OF CHRONIC TRICHOSTATIN A TRATMENT ON GENE EXPRESSION IN LATE PASSAGE PANCREATIC-DERIVED STROMAL CELLS.**

| 24 hours | glucagon | HNF-3beta | Insulin | pdx-1 | Sox17 |
|---|---|---|---|---|---|
| untreated | nd | nd | nd | nd | nd |
| 1.25 µM | + | +++ | nd | + | +++ |
| 2.5 µM | nd | + | nd | +++ | +++ |
| | | | | | |
| 48 hours | glucagon | HNF-3b | Insulin | PDX-1 | Sox17 |
| untreated | nd | nd | nd | nd | + |
| 1.25 µM | + | ++ | nd | +++ | +++ |
| 2.5 µM | + | + | nd | +++ | +++ |
| 72 hours | glucagon | HNF-3b | Insulin | PDX-1 | Soxl7 |
| untreated | nd | nd | nd | nd | + |
| 1.25 µM | + | +++ | nd | +++ | +++ |
| 2.5 µM | nd | +++ | nd | + | +++ |
| | | | | | |
| nd | not detectable at >40 cycles by RT-PCR | | | | |
| + | detectable between 35 and 40 cycles by RT-PCR | | | | |
| ++ | detectable at less than 35 cycles by RT-PCR with std error greater than 0.9 | | | | |
| +++ | detectable at less than 35 cycles by RT-PCR with std error less than 0.9 | | | | |
| All RT-PCR was performed on the Applied Biosystems 7500 Real-Time PCR System | | | | | |

**TABLE VIII-A. THE EFFECT OF A SINGLE CHRONIC TRICHOSTATIN A DOSE ON GENE EXPRESSION IN AN AMNIOTIC FLUID-DERIVED CELL LINE.**

| sample | Gata 1 | HNF | insulin | Pdx-1 | sox17 |
|---|---|---|---|---|---|
| untreated | nd | nd | + | + | +++ |
| 24 hrs | nd | +++ | nd | + | +++ |
| 48 hrs | nd | +++ | nd | nd | +++ |
| 72 hrs | nd | + | + | nd | +++ |
| 96 hrs | nd | nd | + | nd | +++ |
| 120 hrs | nd | + | + | nd | +++ |
| 144 hrs | nd | nd | + | nd | +++ |
| | | nd | not detectable at >40 cycles by RT-PCR | | |
| | | + | detectable between 35 cycles and 40 cycles by RT-PCR | | |
| | | ++ | detectable at less than 35 cycles by RT-PCR with SE greater than 0.9 | | |
| | | +++ | detectable at less than 35 cycles by RT-PCR with SE less than 0.9 | | |
| All RT-PCR was performed on the Applied Biosystems 7500 Real-Time PCR System | | | | | |

**TABLE VIII-B. THE EFFECT OF A SINGLE CHRONIC TRICHOSTATIN A DOSE ON GENE EXPRESSION IN A SECOND AMNIOTIC FLUID-DERIVED CELL LINE.**

| sample | Gata1 | HNF | insulin | Pdx-1 | sox17 |
|---|---|---|---|---|---|
| untreated | nd | nd | +++ | nd | +++ |
| 24 hrs | nd | +++ | nd | + | +++ |
| 48 hrs | nd | +++ | nd | nd | +++ |
| 72 hrs | nd | + | + | nd | +++ |
| 96 hrs | nd | + | ++ | nd | +++ |
| 120 hrs | nd | + | ++ | nd | +++ |
| 144 hrs | nd | + | ++ | nd | +++ |
| | | nd | not detectable at >40 cycles by RT-PCR | | |
| | | + | detectable between 35 and 40 cycles by RT-PCR | | |
| | | ++ | detectable at less than 35 cycles by RT-PCR with SE greater than 0.9 | | |
| | | +++ | detectable at less than 35 cycles by RT-PCR with SE less than 0.9 | | |
| All RT-PCR was performed on the Applied Biosystems 7500 Real-Time PCR System | | | | | |

**TABLE VIII-C. THE EFFECT OF A SINGLE CHRONIC TRICHOSTATIN A DOSE ON GENE EXPRESSION IN AN EARLY PASSAGE (P5) PANCREATIC-DERIVED STROMAL CELL LINE.**

| sample | glucagon | HNF | Insulin | Pdx-1 |
|---|---|---|---|---|
| untreated | nd | nd | nd | + |
| 24 hrs | +++ | +++ | nd | +++ |
| 48 hrs | + | nd | nd | nd |
| 72 hrs | + | nd | nd | nd |
| 96 hrs | +++ | nd | nd | nd |
| 120 hrs | nd | nd | nd | nd |
| 144 hrs | nd | nd | nd | nd |
| | | nd | not detectable at >40 cycles by RT-PCR | |
| | | + | detectable between 35 and 40 cycles by RT-PCR | |
| | | ++ | detectable at less than 35 cycles by RT-PCR with SE greater than 0.9 | |
| | | +++ | detectable at less RT-PCR with than 35 cycles by SE less than 0.9 | |
| All RT-PCR was performed on the Applied Biosystems 7500 Real-Time PCR System | | | | |

**TABLE VIII-D. THE EFFECT OF A SINGLE CHRONIC TRICHOSTATIN A DOSE ON GENE EXPRESSION IN AN LATE PASSAGE (P14) PANCREATIC-DERIVED STROMAL CELL LINE.**

| sample | glucagon | HNF | Insulin | Pdx-1 |
|---|---|---|---|---|
| untreated | nd | nd | nd | nd |
| 24 hrs | +++ | + | nd | +++ |
| 48 hrs | + | nd | nd | nd |
| 72 hrs | nd | nd | nd | nd |
| 96 hrs | nd | nd | nd | nd |
| 120 hrs | nd | nd | nd | nd |
| 144 hrs | nd | nd | nd | nd |
| | | nd | not detectable at >40 cycles by RT-PCR | |
| | | + | detectable between 35 and 40 cycles RT-PCR | |
| | | ++ | detectable at less than 35 cycles RT-PCR with SE greater than 0.9 | |
| | | +++ | detectable at less than 35 cycles RT-PCR with SE less than 0.9 | |
| All RT-PCR was performed on the Applied Biosystems 7500 Real-Time PCR System | | | | |

**TABLE IX-A. THE EFFECT OF TWO 500 NM TRICHOSTATIN A DOSES ON GENE EXPRESSION IN AN AMNIOTIC FLUID-DERIVED CELL LINE.**

| sample | Gatal | HNF | insulin | Pdx-1 | sox17 |
|---|---|---|---|---|---|
| untreated | nd | nd | + | + | +++ |
| 24 hrs | nd | +++ | nd | + | +++ |
| 48 hrs | nd | +++ | + | nd | +++ |
| 72 hrs | nd | + | + | nd | +++ |
| 96 hrs | nd | nd | + | nd | +++ |
| 120 hrs | nd | + | + | nd | +++ |
| 144 hrs | nd | + | + | nd | +++ |
| | | nd | not detectable at >40 cycles by RT-PCR | | |
| | | + | detectable between 35 and 40 cycles by RT-PCR | | |
| | | ++ | detectable at less than 35 cycles by RT-PCR with SE greater than 0.9 | | |
| | | +++ | detectable at less than 35 cycles by RT-PCR with SE less than 0.9 | | |
| All RT-PCR was performed on the Applied Biosystems 7500 Real-Time PCR System | | | | | |

**TABLE IX-B. THE EFFECT OF TWO 500 NM TRICHOSTATIN A DOSES ON GENE EXPRESSION IN A SECOND AMNIOTIC FLUID-DERIVED CELL LINE.**

| sample | Gatal | HNF | insulin | Pdx-1 | sox17 |
|---|---|---|---|---|---|
| untreated | nd | nd | +++ | nd | +++ |
| 24 hrs | nd | +++ | nd | + | +++ |
| 48 hrs | nd | +++ | nd | nd | +++ |
| 72 hrs | nd | +++ | +++ | nd | +++ |
| 96 hrs | nd | + | +++ | nd | +++ |
| 120 hrs | nd | + | + | nd | +++ |
| 144 hrs | nd | + | +++ | nd | +++ |
| | | nd | not detectable at >40 cycles by RT-PCR | | |
| | | + | detectable between 35 and 40 cycles RT-PCR | | |
| | | ++ | detectable at less than 35 cycles RT-PCR with SE greater than 0.9 | | |
| | | +++ | detectable at less than 35 cycles RT-PCR with SE less than 0.9 | | |
| All RT-PCR was performed on the Applied Biosystems 7500 Real-Time PCR System | | | | | |

**TABLE IX-C. THE EFFECT OF TWO 1.25 µM TRICHOSTATIN A DOSES ON GENE EXPRESSION IN AN EARLY PASSAGE (P5) PANCREATIC-DERIVED STROMAL CELL LINE.**

| sample | glucagon | HNF | Insulin | Pdx-1 |
|---|---|---|---|---|
| untreated | nd | nd | nd | + |
| 24 hrs | +++ | +++ | nd | +++ |
| 48 hrs | + | nd | nd | nd |
| 72 hrs | nd | nd | nd | nd |
| 96 hrs | nd | nd | nd | nd |
| 120 hrs | + | +++ | nd | +++ |
| 144 hrs | + | nd | nd | nd |
| | | nd | not detectable at >40 cycles by RT-PCR | |
| | | + | detectable between 35 and 40 cycles by RT-PCR | |
| | | ++ | detectable at less than 35 cycles by RT-PCR with SE greater than 0.9 | |
| | | +++ | detectable at less than 35 cycles by RT-PCR with SE less than 0.9 | |
| All RT-PCR was performed on the Applied Biosystems 7500 Real-Time PCR System | | | | |

**TABLE IX-D. THE EFFECT OF TWO 1.25 µM TRICHOSTATIN A DOSES ON GENE EXPRESSION IN A LATE PASSAGE (P14) PANCREATIC-DERIVED STROMAL CELL LINE.**

| sample | glucagon | HNF | Insulin | Pdx-1 |
|---|---|---|---|---|
| untreated | nd | nd | nd | nd |
| 24 hrs | +++ | + | nd | +++ |
| 48 hrs | +++ | nd | +- | nd |
| 72 hrs | + | nd | nd | nd |
| 96 hrs | nd | nd | nd | nd |
| 120 hrs | = | +++ | nd | ++ |
| 144 hrs | + | nd | nd | nd |
| | | nd | not detectable at >40 cycles by RT-PCR | |
| | | + | detectable between 35 and 40 cycles by RT-PCR | |
| | | ++ | detectable at less than 35 cycles by RT-PCR with SE greater than 0.9 | |
| | | +++ | detectable at less than 35 cycles by RT-PCR with SE less than 0.9 | |
| All RT-PCR was performed on the Applied Biosystems 7500 Real-Time PCR System | | | | |

**TABLE X-A. THE EFFECT OF TWO 1.25 µM TRICHOSTATIN A DOSES ON GENE EXPRESSION IN AN AMNIOTIC FLUID-DERIVED CELL LINE.**

| sample | Gatal | HNF-3 beta | insulin | Pdx-1 | sox17 |
|---|---|---|---|---|---|
| untreated | nd | nd | + | + | +++ |
| 24 hrs | + | +++ | nd | +++ | +++ |
| 48 hrs | nd | +++ | nd | nd | +++ |
| 72 hrs | nd | + | + | nd | +++ |
| 96 hrs | nd | + | + | nd | +++ |
| 120 hrs | nd | +++ | nd | +++ | +++ |
| 144 hrs | nd | +++ | nd | nd | +++ |
| | | nd | not detectable at >40 cycles by RT-PCR | | |
| | | + | detectable between 35 and 40 cycles by RT-PCR | | |
| | | ++ | detectable at less than 35 cycles by RT-PCR with SE greater than 0.9 | | |
| | | +++ | detectable at less than 35 cycles by RT-PCR with SE less than 0.9 | | |
| All RT-PCR was performed on the Applied Biosystems 7500 Real-Time PCR System | | | | | |

**TABLE X-B. THE EFFECT OF TWO 1.25 µM TRICHOSTATIN A DOSES ON GENE EXPRESSION IN A SECOND AMNIOTIC FLUID-DERIVED CELL LINE.**

| sample | Gata1 | HNF-3 beta | insulin | Pdx-1 | sox17 |
|---|---|---|---|---|---|
| untreated | nd | nd | +++ | nd | +++ |
| 24 hrs | + | +++ | nd | +++ | +++ |
| 48 hrs | nd | +++ | nd | nd | +++ |
| 72 hrs | nd | +++ | + | nd | +++ |
| 96 hrs | nd | + | + | nd | +++ |
| 120 hrs | + | +++ | nd | +++ | +++ |
| 144 hrs | nd | +++ | nd | nd | +++ |
| | | nd | not detectable at >40 cycles by RT-PCR | | |
| | | + | detectable between 35 and 40 cycles by RT-PCR | | |
| | | ++ | detectable at less than 35 cycles by RT-PCR with SE greater than 0.9 | | |
| | | +++ | detectable at less than 35 cycles by RT-PCR with SE less than 0.9 | | |
| All RT-PCR was performed on the Applied Biosystems 7500 Real-Time PCR System | | | | | |

**TABLE X-C. THE EFFECT OF TWO 5 µM TRICHOSTATIN A DOSES ON GENE EXPRESSION IN AN EARLY PASSAGE (P5) PANCREATIC-DERIVED STROMAL CELL LINE.**

| sample | glucagon | HNF-3 beta | Insulin | Pdx-1 |
|---|---|---|---|---|
| untreated | nd | nd | nd | + |
| 24 hrs | +++ | +++ | nd | ++ |
| 48 hrs | + | nd | nd | nd |
| 72 hrs | + | nd | nd | nd |
| 96 hrs | nd | nd | nd | nd |
| 120 hrs | nd | + | nd | +++ |
| 144 hrs | + | nd | nd | nd |
| | | nd | not detectable at >40 cycles by RT-PCR | |
| | | + | detectable between 35 and 40 cycles by RT-PCR | |
| | | ++ | detectable at less than 35 cycles by RT-PCR with SE greater than 0.9 | |
| | | +++ | detectable at less than 35 cycles by RT-PCR with SE less than 0.9 | |
| All RT-PCR was performed on the Applied Biosystems 7500 Real-Time PCR System | | | | |

**TABLE X-D. THE EFFECT OF TWO 5 µM TRICHOSTATIN A DOSES ON GENE EXPRESSION IN A LATE PASSAGE (P11) PANCREATIC-DERIVED STROMAL CELL LINE.**

| sample | glucagon | HNF-3 beta | Insulin | Pdx-1 |
|---|---|---|---|---|
| untreated | nd | nd | nd | nd |
| 24 hrs | +++ | +++ | nd | + |
| 48 hrs | + | nd | + | nd |
| 72 hrs | + | nd | nd | nd |
| 96 hrs | + | nd | nd | nd |
| 120 hrs | + | + | nd | +++ |
| 144 hrs | + | nd | nd | nd |
| | | nd | not detectable | |
| | | - | detectable at greater than 35 cycles | |
| | | + | detectable at less than 35 cycles with SE greater than 0.9 | |
| | | ++ | detectable at less than 35 cycles with SE less than 0.9 | |
| All RT-PCR was performed on the Applied Biosystems 7500 Real-Time PCR System | | | | |

**TABLE XI-A. THE EFFECT OF TWO 1.25 µM TRICHOSTATIN A DOSES ON GENE EXPRESSION IN AN AMNIOTIC FLUID-DERIVED CELL LINE.**

| sample | Gatal | HNF-3 beta | insulin | Pdx-1 | sox17 |
|---|---|---|---|---|---|
| untreated | nd | nd | + | + | +++ |
| 24 hrs | + | +++ | nd | +++ | +++ |
| 48 hrs | nd | +++ | nd | +++ | +++ |
| 72 hrs | nd | + | nd | nd | +++ |
| 96 hrs | nd | nd | nd | nd | +++ |
| 120 hrs | nd | + | nd | + | +++ |
| | | nd | not detectable at >40 cycles by RT-PCR | | |
| | | + | detectable between 35 and 40 cycles by RT-PCR | | |
| | | ++ | detectable at less than 35 cycles by RT-PCR with SE greater than 0.9 | | |
| | | +++ | detectable at less than 35 cycles by RT-PCR with SE less than 0.9 | | |
| All RT-PCR was performed on the Applied Biosystems 7500 Real-Time PCR System | | | | | |

**TABLE XI-B. THE EFFECT OF TWO 1.25 µM TRICHOSTATIN A DOSES ON GENE EXPRESSION IN A SECOND AMNIOTIC FLUID-DERIVED CELL LINE.**

| sample | Gata1 | HNF-3 beta | insulin | Pdx-1 | sox17 |
|---|---|---|---|---|---|
| untreated | nd | nd | +++ | nd | +++ |
| 24 hrs | + | +++ | nd | ++ | +++ |
| 48 hrs | nd | +++ | nd | + | +++ |
| 72 hrs | nd | +++ | nd | nd | +++ |
| 96 hrs | nd | + | nd | nd | +++ |
| 120 hrs | + | +++ | nd | +++ | +++ |
| | | nd | not detectable at >40 cycles by RT-PCR | | |
| | | + | detectable between 35 and 40 cycles by RT-PCR | | |
| | | ++ | detectable at less than 35 cycles by RT-PCR with SE greater than 0.9 | | |
| | | +++ | detectable at less than 35 cycles by RT-PCR with SE less than 0.9 | | |
| All RT-PCR was performed on the Applied Biosystems 7500 Real-Time PCR System | | | | | |

**TABLE XI-C. THE EFFECT OF TWO 5 µM TRICHOSTATIN A DOSES ON GENE EXPRESSION IN AN EARLY PASSAGE (P5) PACNREATIC-DERIVED STROMAL CELL LINE.**

| sample | glucagon | HNF-3 beta | Insulin | Pdx-1 |
|---|---|---|---|---|
| untreated | nd | nd | nd | + |
| 24 hrs | +++ | +++ | nd | +++ |
| 48 hrs | +++ | +++ | nd | +++ |
| 72 hrs | + | nd | +++ | nd |
| 96 hrs | + | nd | nd | nd |
| 120 hrs | + | +++ | nd | +++ |
| | | nd | not detectable at >40 cycles by RT-PCR | |
| | | + | detectable between 35 and 40 cycles by RT-PCR | |
| | | ++ | detectable at less than 35 cycles by RT-PCR with SE greater than 0.9 | |
| | | +++ | detectable at less than 35 cycles by RT-PCR with SE less than 0.9 | |
| All RT-PCR was performed on the Applied Biosystems 7500 Real-Time PCR System | | | | |

**TABLE XI-D. THE EFFECT OF TWO 5 µM TRICHOSTATIN A DOSES ON GENE EXPRESSION IN A LATE PASSAGE (P11) PACNREATIC-DERIVED STROMAL CELL LINE.**

| sample | glucagon | HNF-3 beta | Insulin | Pdx-1 |
|---|---|---|---|---|
| untreated | nd | nd | nd | Nd |
| 24 hrs | +++ | +++ | nd | + |
| 48 hrs | +++ | +++ | nd | +++ |
| 72 hrs | + | nd | nd | Nd |
| 96 hrs | nd | nd | nd | Nd |
| 120 hrs | + | +++ | + | +++ |
| | | nd | not detectable at >40 cycles by RT-PCR | |
| | | + | detectable between 35 and 40 cycles by RT-PCR | |
| | | ++ | detectable at less than 35 cycles by RT-PCR with SE greater than 0.9 | |
| | | +++ | detectable at less than 35 cycles by RT-PCR with SE less than 0.9 | |
| All RT-PCR was performed on the Applied Biosystems 7500 Real-Time PCR System | | | | |

**TABLE XII-A. THE EFFECT OF A 6 HOUR 1.25 µM TRICHOSTATIN A TREATMENT ON GENE EXPRESSION IN AN AMNIOTIC FLUID-DERIVED CELL LINE.**

| sample | Gatal | HNF-3 beta | insulin | Pdx-1 | sox17 |
|---|---|---|---|---|---|
| untreated | nd | nd | + | + | +++ |
| 6 hrs | nd | +++ | nd | + | +++ |
| 24 hrs | nd | +++ | nd | nd | +++ |
| | | nd | not detectable at >40 cycles by RT-PCR | | |
| | | + | detectable between 35 and 40 cycles by RT-PCR | | |
| | | ++ | detectable at less than 35 cycles by RT-PCR with SE greater than 0.9 | | |
| | | +++ | detectable at less than 35 cycles by RT-PCR with SE less than 0.9 | | |
| All RT-PCR was performed on the Applied Biosystems 7500 Real-Time PCR System | | | | | |

**TABLE XII-B. THE EFFECT OF A 6 HOUR 1.25 µM TRICHOSTATIN A TREATMENT ON GENE EXPRESSION IN A SECOND AMNIOTIC FLUID-DERIVED CELL LINE.**

| sample | Gatal | HNF-3 beta | insulin | Pdx-1 | sox17 |
|---|---|---|---|---|---|
| untreated | nd | nd | +++ | nd | +++ |
| 6 hrs | nd | +++ | nd | +++ | +++ |
| 24 hrs | nd | + | nd | nd | +++ |
| | | nd | not detectable at >40 cycles by RT-PCR | | |
| | | + | detectable between 35 and 40 cycles by RT-PCR | | |
| | | ++ | detectable at less than 35 cycles by RT-PCR with SE greater than 0.9 | | |
| | | +++ | detectable at less than 35 cycles by RT-PCR with SE less than 0.9 | | |
| All RT-PCR was performed on the Applied Biosystems 7500 Real-Time PCR System | | | | | |

**TABLE XII-C. THE EFFECT OF A 6 HOUR 5 µM TRICHOSTATIN A TREATMENT ON GENE EXPRESSION IN AN EARLY PASSAGE (P5) PANCREATIC-DERIVED STOMAL CELL LINE.**

| sample | glucagon | HNF-3 beta | Insulin | Pdx-1 |
|---|---|---|---|---|
| untreated | nd | nd | Nd | + |
| 6 hrs | + | +++ | Nd | + |
| 24 hrs | + | nd | +++ | nd |
| | | nd | not detectable at >40 cycles by RT-PCR | |
| | | + | detectable between 35 and 40 cycles by RT-PCR | |
| | | ++ | detectable at less than 35 cycles by RT-PCR with SE greater than 0.9 | |
| | | +++ | detectable at less than 35 cycles by RT-PCR with SE less than 0.9 | |
| All RT-PCR was performed on the Applied Biosystems 7500 Real-Time PCR System | | | | |

**TABLE XII-D. THE EFFECT OF A 6 HOUR 5 µM TRICHOSTATIN A TREATMENT ON GENE EXPRESSION IN A LATE PASSAGE (P11) PANCREATIC-DERIVED STOMAL CELL LINE.**

| sample | glucagon | HNF-3 beta | Insulin | Pdx-1 |
|---|---|---|---|---|
| untreated | nd | nd | Nd | nd |
| 6 hrs | + | + | Nd | + |
| 24 hrs | + | nd | Nd | nd |
| | | nd | not detectable at >40 cycles by RT-PCR | |
| | | + | detectable between 35 and 40 cycles by RT-PCR | |
| | | ++ | detectable at less than 35 cycles by RT-PCR with SE greater than 0.9 | |
| | | +++ | detectable at less than 35 cycles by RT-PCR with SE less than 0.9 | |
| All RT-PCR was performed on the Applied Biosystems 7500 Real-Time PCR System | | | | |

**TABLE XIII-A. THE EFFECT OF A 24 HOUR 5 µM HDAC INHIBITOR TREATMENT ON GENE EXPRESSION IN AN AMNIOTIC FLUID-DERIVED CELL LINE.**

| **Sample ID** | **GAPDH** | | | | | **PDX1** | | | | | **RQ** |
|---|---|---|---|---|---|---|---|---|---|---|---|
| | **replicate 1** | **replicate 2** | **replicate 3** | **Avg Ct** | **Ct std error** | **replicate 1** | **replicate 2** | **replicate 3** | **Avg Ct** | **Ct std error** | |
| 26996723 | 24.260 | 23.821 | 23.707 | 23.929 | 0.169 | 34.809 | 35.903 | 35.334 | 35.349 | 0.316 | 0.4 |
| 18042557 | 23.212 | 23.018 | 22.979 | 23.070 | 0.072 | Undet. | Undet. | Undet. | | | |
| 26979355 | 23.319 | 23.349 | 23.400 | 23.356 | 0.023 | Undet. | Undet. | Undet. | | | |
| 18042583 | 23.638 | 22.907 | 22.955 | 23.167 | 0.236 | Undet. | Undet. | | Undet. | | |
| 27019850 | 23.187 | 23.108 | 23.388 | 23.228 | 0.083 | Undet. | Undet. | Undet. | | | |
| 27054313 | 23.844 | 23.374 | 23.754 | 23.657 | 0.144 | Undet. | 37.615 | Undet. | 37.615 | | 0.1 |
| 27129986 | 22.500 | 22.395 | 22.720 | 22.539 | 0.096 | Undet. | Undet. | Undet. | | | |
| 28635932 | 23.066 | 23.193 | 22.783 | 23.014 | 0.121 | Undet. | Undet. | Undet. | | | |
| 27140191 | 22.741 | 22.310 | 23.831 | 22.961 | 0.452 | Undet. | Undet. | Undet. | | | |
| 28543944 | 23.396 | 23.309 | 24.072 | 23.592 | 0.241 | 33.558 | 33.199 | 34.116 | 33.624 | 0.267 | 1.0 |
| 37271637 | 22.937 | 22.716 | 23.892 | 23.182 | 0.361 | Undet. | Undet. | Undet. | | | |
| 28596555 | 24.069 | 23.590 | 24.194 | 23.951 | 0.184 | Undet. | Undet. | Undet. | | | |
| 37271559 | 23.616 | 22.356 | 23.402 | 23.125 | 0.389 | Undet. | Undet. | Undet. | | | |
| 26972088 | 23.138 | 22.635 | 23.270 | 23.014 | 0.194 | Undet. | Undet. | Undet. | | | |
| 37271650 | 23.544 | 22.678 | 23.153 | 23.125 | 0.251 | Undet. | Undet. | Undet. | | | |
| 26977808 | 23.451 | 22.408 | 23.290 | 23.050 | 0.324 | Undet. | Undet. | Undet. | | | |
| 26142415 | 23.215 | 23.242 | 23.298 | 23.251 | 0.024 | Undet. | Undet. | Undet. | | | |
| untreated | 23.521 | 23.394 | 22.812 | 23.242 | 0.218 | Undet. | Undet. | Undet. | | | |
| 27422863 | 23.729 | 23.950 | 23.895 | 23.858 | 0.066 | Undet. | Undet. | Undet. | | | |
| TSA | 23.783 | 23.132 | 23.772 | 23.562 | 0.215 | 33.408 | 33.067 | 34.151 | 33.542 | 0.320 | 1.0 |
| 27652677 | 23.799 | 23.698 | 24.276 | 23.924 | 0.178 | 33.890 | 33.157 | 34.960 | 34.002 | 0.524 | 0.9 |
| 28507193 | 23.845 | 23.847 | 23.292 | 23.661 | 0.185 | 34.271 | 35.198 | 34.328 | 34.599 | 0.300 | 0.5 |
| 37653265 | 23.047 | 23.007 | 23.298 | 23.118 | 0.091 | Undet. | Undet. | Undet. | | | |
| 28571699 | 23.474 | 23.664 | 23.202 | 23.447 | 0.134 | 33.700 | 34.438 | 33.901 | 34.013 | 0.220 | 0.7 |
| 37791156 | 24.088 | 23.389 | 24.155 | 23.877 | 0.245 | 34.803 | 33.520 | 34.063 | 34.129 | 0.372 | 0.8 |
| 28584842 | 23.796 | 23.644 | 23.524 | 23.655 | 0.079 | 38.374 | 38.530 | Undet. | 38.452 | 0.078 | 0.0 |
| 37791104 | 23.669 | 23.319 | 23.563 | 23.517 | 0.104 | 34.057 | 34.571 | 34.617 | 34.415 | 0.180 | 0.5 |
| 28649465 | 24.300 | 23.501 | 23.729 | 23.844 | 0.238 | 37.693 | 36.510 | 37.504 | 37.236 | 0.367 | 0.1 |
| 27027026 | 23.856 | 24.250 | 23.063 | 23.723 | 0.349 | 34.025 | 34.238 | 33.121 | 33.795 | 0.342 | 0.9 |
| 29232073 | 23.800 | 23.643 | 23.260 | 23.568 | 0.160 | 33.011 | 32.785 | 33.056 | 32.951 | 0.084 | 1.5 |
| 27059331 | 23.980 | 23.479 | 23.577 | 23.679 | 0.153 | 34.639 | 35.004 | 35.340 | 34.994 | 0.202 | 0.4 |
| 32404580 | 23.398 | 23.067 | 23.213 | 23.226 | 0.096 | Undet. | Undet. | Undet. | | | |
| 27628250 | 24.575 | 24.101 | 23.936 | 24.204 | 0.192 | Undet. | Undet. | Undet. | | | |
| 27051895 | 23.535 | Undet. | 23.851 | 23.693 | 0.158 | Undet. | Undet. | Undet. | | | |
| 27075945 | 25.120 | 24.170 | 24.520 | 24.604 | 0.277 | Undet. | Undet. | Undet. | | | |
| 27070160 | 23.647 | Undet. | 23.682 | 23.665 | 0.018 | Undet. | Undet. | Undet. | | | |
| 27083537 | 23.952 | 23.591 | 23.511 | 23.685 | 0.136 | Undet. | Undet. | Undet. | | | |
| 27263366 | 23.251 | Undet. | 23.128 | 23.189 | 0.061 | Undet. | Undet. | Undet. | | | |
| 27132391 | 24.200 | 23.126 | 22.964 | 23.430 | 0.388 | Undet. | Undet. | Undet. | | | |
| 28571530 | 22.535 | Undet. | 23.435 | 22.985 | 0.450 | 33.766 | 33.452 | 34.341 | 33.853 | 0.260 | 0.6 |
| 27554553 | 23.624 | 22.718 | 24.634 | 23.659 | 0.553 | Undet. | Undet. | Undet. | | | |
| 28596503 | 22.792 | Undet. | 24.440 | 23.616 | 0.824 | Undet. | Undet. | Undet. | | | |
| 26900289 | 23.948 | 23.001 | 23.988 | 23.645 | 0.322 | Undet. | Undet. | Undet. | | | |
| 28600650 | 22.837 | Undet. | 23.425 | 23.131 | 0.294 | 34.063 | 34.555 | 35.507 | 34.708 | 0.424 | 0.3 |
| 26994461 | 23.777 | 23.129 | 23.634 | 23.514 | 0.197 | 34.337 | 34.593 | 35.308 | 34.746 | 0.291 | 0.4 |
| 37791065 | 23.222 | Undet. | 23.680 | 23.451 | 0.229 | Undet. | Undet. | Undet. | | | |
| 28549118 | 23.725 | 23.522 | 23.683 | 23.643 | 0.062 | 33.354 | 34.022 | 33.765 | 33.714 | 0.194 | 1.0 |
| 28427139 | 23.377 | Undet. | 23.768 | 23.572 | 0.195 | Undet. | Undet. | Undet. | | | |
| 33300943 | 26.317 | 25.666 | 25.677 | 25.887 | 0.215 | 35.984 | 34.695 | 36.702 | 35.794 | 0.587 | 1.1 |
| untreated | 25.963 | 24.642 | 24.596 | 25.067 | 0.448 | Undet. | Undet. | Undet. | | | |
| 33297888 | 25.322 | 24.894 | 25.139 | 25.118 | 0.124 | 34.115 | 34.145 | 35.206 | 34.489 | 0.359 | 1.6 |
| TSA | 25.224 | 24.046 | 25.952 | 25.074 | 0.555 | 34.884 | 33.285 | 37.095 | 35.088 | 1.104 | 1.0 |
| 27617655 | 24.911 | 24.921 | 24.659 | 24.830 | 0.086 | Undet. | Undet. | Undet. | | | |
| 37653330 | 25.412 | 24.899 | 25.249 | 25.186 | 0.151 | 34.251 | 34.336 | 37.049 | 35.212 | 0.919 | 1.0 |
| 28427113 | 23.593 | 25.653 | 24.239 | 24.495 | 0.608 | Undet. | Undet. | Undet. | | | |
| 37804806 | 25.101 | 24.947 | 24.651 | 24.899 | 0.132 | 34.074 | 34.378 | 33.528 | 33.993 | 0.249 | 1.9 |
| 29784001 | 25.297 | 23.826 | 25.277 | 24.800 | 0.487 | 34.381 | 33.130 | 35.162 | 34.224 | 0.592 | 1.5 |
| 37786138 | 25.704 | 24.545 | 25.075 | 25.108 | 0.335 | 36.068 | 35.269 | 35.080 | 35.473 | 0.303 | 0.8 |
| 33300839 | 24.859 | 24.349 | 25.424 | 24.878 | 0.311 | 34.667 | 34.031 | 34.291 | 34.330 | 0.184 | 1.5 |
| 38117820 | 25.256 | 25.088 | 25.574 | 25.306 | 0.142 | 37.712 | 38.096 | Undet. | 37.904 | | 0.2 |
| 26960167 | 25.043 | 24.157 | 26.281 | 25.160 | 0.616 | Undet. | Undet. | Undet. | | | |
| 38117859 | 25.102 | 24.401 | 24.947 | 24.816 | 0.213 | 34.452 | 33.525 | 34.524 | 34.167 | 0.322 | 1.6 |
| 27263158 | 25.050 | 25.327 | 26.236 | 25.538 | 0.358 | Undet. | Undet. | Undet. | | | |
| 38134395 | 26.227 | 24.421 | 24.750 | 25.133 | 0.555 | 35.286 | 34.709 | 34.664 | 34.886 | 0.200 | 1.2 |
| 27018576 | 25.059 | 26.422 | 26.338 | 25.939 | 0.441 | Undet. | Undet. | Undet. | | | |
| 26988312 | Undet. | 25.334 | 25.043 | 25.188 | 0.145 | Undet. | Undet. | Undet. | | | |
| 27019590 | 24.966 | 25.597 | 25.371 | 25.311 | 0.184 | Undet. | Undet. | Undet. | | | |
| 27121120 | Undet. | 25.838 | 24.550 | 25.194 | 0.644 | Undet. | Undet. | Undet. | | | |
| 37398855 | 25.106 | 25.585 | 25.667 | 25.453 | 0.175 | 34.258 | 35.682 | 36.410 | 35.450 | 0.632 | 1.1 |
| 27527305 | Undet. | 24.701 | 24.457 | 24.579 | 0.122 | Undet. | Undet. | Undet. | | | |
| 37398920 | 24.880 | 24.721 | 25.962 | 25.187 | 0.390 | 34.095 | 34.318 | 38.141 | 35.518 | 1.313 | 0.9 |
| 28540954 | Undet. | 24.599 | 25.218 | 24.909 | 0.309 | Undet. | 34.346 | 35.022 | 34.684 | 0.338 | 1.3 |
| 37398803 | 24.882 | 24.801 | 25.211 | 24.965 | 0.125 | Undet. | Undet. | Undet. | | | |
| 38153895 | Undet. | 24.610 | 25.201 | 24.906 | 0.296 | Undet. | 34.806 | 33.621 | 34.213 | 0.592 | 1.7 |
| 26989664 | 24.773 | 25.105 | 24.903 | 24.927 | 0.096 | Undet. | Undet. | Undet. | | | |
| 38153882 | Undet. | 26.274 | 25.062 | 25.668 | 0.606 | Undet. | 37.247 | 38.152 | 37.700 | 0.452 | 0.3 |
| 26981149 | 24.448 | 24.504 | 24.713 | 24.555 | 0.081 | Undet. | Undet. | Undet. | | | |
| 38151243 | Undet. | 24.350 | 24.916 | 24.633 | 0.283 | Undet. | Undet. | Undet. | | | |
| 26980174 | 24.031 | 24.519 | 24.410 | 24.320 | 0.148 | Undet. | Undet. | Undet. | | | |
| 27151813 | Undet. | 24.158 | 24.158 | 24.158 | 0.000 | Undet. | Undet. | Undet. | | | |
| 28557425 | 25.178 | 26.164 | 25.943 | 25.762 | 0.299 | Undet. | Undet. | Undet. | | | |
| untreated | 25.006 | 25.206 | 24.299 | 24.837 | 0.275 | Undet. | Undet. | Undet. | | | |
| 28427165 | 25.814 | 25.849 | 25.246 | 25.636 | 0.196 | Undet. | Undet. | Undet. | | | |
| TSA | 24.505 | 24.926 | 25.230 | 24.887 | 0.210 | 34.728 | 35.053 | 35.205 | 34.995 | 0.141 | 1.0 |
| 31046587 | 25.779 | 26.267 | 25.659 | 25.901 | 0.186 | Undet. | Undet. | Undet. | | | |
| 27147133 | 24.411 | 24.561 | 24.534 | 24.502 | 0.046 | Undet. | Undet. | Undet. | | | |
| 29776266 | 24.154 | 24.116 | 24.860 | 24.376 | 0.242 | 35.133 | 35.538 | 36.006 | 35.559 | 0.252 | 0.5 |
| 28296463 | 24.977 | 24.747 | 24.891 | 24.872 | 0.067 | 34.283 | 34.543 | 34.843 | 34.556 | 0.162 | 1.3 |
| 31051462 | 24.556 | 26.036 | 25.128 | 25.240 | 0.431 | 34.094 | 35.728 | 35.226 | 35.016 | 0.483 | 1.3 |
| 37804793 | 24.852 | 24.631 | 25.010 | 24.831 | 0.110 | 36.305 | 35.229 | 37.367 | 36.300 | 0.617 | 0.4 |
| 31046626 | 25.476 | 25.949 | 25.315 | 25.580 | 0.190 | 34.367 | 36.453 | 34.852 | 35.224 | 0.630 | 1.4 |
| 37786151 | 24.284 | 24.435 | 24.630 | 24.450 | 0.100 | Undet. | Undet. | Undet. | | | |
| 26913757 | 24.957 | 25.645 | 25.250 | 25.284 | 0.199 | 34.973 | 37.589 | 36.757 | 36.440 | 0.772 | 0.5 |
| 26949403 | 24.466 | 24.798 | 25.277 | 24.847 | 0.235 | 34.294 | 34.565 | 35.856 | 34.905 | 0.482 | 1.0 |
| 26949481 | 25.813 | 24.921 | 25.412 | 25.382 | 0.258 | Undet. | 36.352 | 37.228 | 36.790 | 0.438 | 0.4 |
| 26949312 | 24.320 | 24.803 | 24.744 | 24.622 | 0.152 | 35.021 | 34.216 | 34.846 | 34.694 | 0.245 | 1.0 |
| 38134330 | 24.475 | 24.365 | 24.648 | 24.496 | 0.082 | 34.122 | 34.272 | 34.485 | 34.293 | 0.105 | 1.0 |
| 27054729 | 24.948 | 23.766 | 24.321 | 24.345 | 0.341 | Undet. | Undet. | Undet. | | | |
| 26949273 | 24.375 | 24.987 | 24.188 | 24.517 | 0.241 | Undet. | Undet. | 38.272 | 38.272 | 13.756 | 0.1 |
| 27032057 | 24.798 | 23.366 | 23.645 | 23.936 | 0.438 | Undet. | Undet. | Undet. | | | |
| 26949338 | 23.566 | 24.222 | 24.548 | 24.112 | 0.289 | Undet. | Undet. | Undet. | | | |
| 27137032 | 23.410 | 23.417 | 23.655 | 23.494 | 0.080 | Undet. | Undet. | Undet. | | | |
| 26949442 | 22.969 | 23.638 | 23.092 | 23.233 | 0.206 | Undet. | Undet. | Undet. | | | |
| 27136863 | 23.630 | 23.623 | 23.350 | 23.534 | 0.092 | Undet. | Undet. | Undet. | | | |
| 26949455 | 23.411 | 23.404 | 23.496 | 23.437 | 0.029 | 38.003 | Undet. | Undet. | 38.003 | 14.566 | 0.0 |
| 27136889 | 23.470 | 23.073 | 23.395 | 23.313 | 0.122 | Undet. | Undet. | Undet. | | | |
| 26949416 | 23.911 | 24.119 | 24.837 | 24.289 | 0.280 | Undet. | Undet. | Undet. | | | |
| 27136993 | 22.996 | 23.892 | 23.618 | 23.502 | 0.265 | 39.778 | Undet. | Undet. | | | |
| 26949390 | 24.275 | 24.257 | 24.422 | 24.318 | 0.052 | Undet. | 38.129 | Undet. | 38.129 | 13.811 | 0.1 |
| 27131546 | 23.753 | 23.765 | 23.305 | 23.608 | 0.151 | Undet. | Undet. | Undet. | | | |
| 28571504 | 23.541 | 23.601 | 24.039 | 23.727 | 0.157 | 33.308 | 33.000 | 33.285 | 33.198 | 0.099 | 1.2 |
| 38222964 | 24.179 | 24.730 | 23.718 | 24.209 | 0.292 | 36.820 | 37.173 | 35.371 | 36.454 | 0.551 | 0.2 |
| 26909636 | 25.050 | 24.647 | 25.047 | 24.915 | 0.134 | Undet. | 37.496 | Undet. | 37.496 | 12.581 | 0.1 |
| untreated | 24.256 | 24.568 | 23.648 | 24.157 | 0.270 | Undet. | Undet. | Undet. | | | |
| 27142674 | 24.391 | 24.247 | 24.322 | 24.320 | 0.042 | 33.969 | 33.143 | 34.450 | 33.854 | 0.382 | 1.2 |
| TSA | 23.984 | 24.219 | 24.240 | 24.148 | 0.082 | 34.305 | 33.750 | 33.714 | 33.923 | 0.191 | 1.0 |
| 27142401 | 24.025 | 23.977 | 24.376 | 24.126 | 0.126 | 33.905 | 33.759 | 33.382 | 33.682 | 0.156 | 1.2 |
| 26951249 | 24.389 | 24.749 | 24.816 | 24.651 | 0.133 | Undet. | 38.406 | Undet. | 38.406 | 13.755 | 0.1 |
| 26910897 | 23.739 | 23.279 | 23.606 | 23.541 | 0.137 | Undet. | Undet. | Undet. | | | |
| 28584777 | 23.515 | 24.366 | 23.736 | 23.872 | 0.255 | Undet. | Undet. | Undet. | | | |
| 27122225 | 23.209 | 23.726 | 24.422 | 23.786 | 0.351 | Undet. | Undet. | Undet. | | | |
| 27147081 | 23.214 | 24.310 | 23.750 | 23.758 | 0.317 | Undet. | Undet. | Undet. | | | |
| 29232541 | 24.254 | 24.722 | 23.970 | 24.315 | 0.219 | 35.196 | 36.290 | 35.380 | 35.622 | 0.338 | 0.3 |
| 27142323 | 23.457 | 24.960 | 23.902 | 24.106 | 0.446 | Undet. | Undet. | Undet. | | | |
| 29381378 | 24.546 | 24.326 | 23.773 | 24.215 | 0.230 | Undet. | Undet. | Undet. | | | |
| 27621126 | 23.510 | 24.315 | 23.849 | 23.892 | 0.233 | 34.405 | 36.442 | 35.120 | 35.322 | 0.597 | 0.3 |
| 35807343 | 24.368 | 24.136 | 24.203 | 24.236 | 0.069 | Undet. | Undet. | Undet. | | | |
| 28577068 | 24.063 | 24.943 | 23.943 | 24.316 | 0.315 | Undet. | Undet. | Undet. | | | |
| 27580670 | 23.352 | 23.782 | 23.059 | 23.398 | 0.210 | Undet. | Undet. | Undet. | | | |
| 27018225 | 23.053 | 22.978 | 23.054 | 23.028 | 0.025 | Undet. | Undet. | Undet. | | | |
| 36278853 | 22.947 | 23.720 | 23.930 | 23.532 | 0.299 | 34.557 | 35.220 | 35.618 | 35.132 | 0.310 | 0.3 |
| 27018199 | 23.661 | 23.099 | 23.405 | 23.388 | 0.162 | 36.020 | Undet. | 35.971 | 35.995 | 0.024 | 0.2 |
| 27274468 | 22.037 | 23.412 | 23.484 | 22.978 | 0.471 | 33.015 | 34.377 | 34.210 | 33.868 | 0.429 | 0.6 |
| 27017159 | 22.920 | 23.738 | 23.472 | 23.377 | 0.241 | Undet. | Undet. | Undet. | | | |
| 27267279 | 22.643 | 22.592 | 22.679 | 22.638 | 0.025 | Undet. | Undet. | Undet. | | | |
| 27017198 | 23.103 | 23.130 | 22.990 | 23.074 | 0.043 | Undet. | Undet. | Undet. | | | |
| 27266343 | 23.106 | 23.736 | 23.830 | 23.558 | 0.227 | Undet. | Undet. | Undet. | | | |
| 27019291 | 24.182 | 24.023 | 23.403 | 23.869 | 0.238 | Undet. | Undet. | Undet. | | | |
| 27385098 | 23.383 | 23.388 | 23.347 | 23.372 | 0.013 | Undet. | Undet. | Undet. | | | |
| 28313220 | 22.945 | 23.484 | 23.016 | 23.148 | 0.169 | Undet. | Undet. | Undet. | | | |
| 26633763 | 22.814 | 23.950 | 23.333 | 23.366 | 0.328 | 34.310 | 34.330 | 34.681 | 34.440 | 0.120 | 0.5 |
| 27059357 | 23.035 | 23.341 | 23.262 | 23.213 | 0.092 | Undet. | Undet. | Undet. | | | |
| 27018212 | 23.303 | 23.834 | 23.869 | 23.669 | 0.183 | 35.327 | 36.291 | 36.004 | 35.874 | 0.286 | 0.2 |
| 29380936 | 24.078 | 23.260 | 23.143 | 23.494 | 0.294 | Undet. | Undet. | Undet. | | | |
| 26933374 | 22.736 | 23.306 | 22.857 | 22.967 | 0.173 | 38.795 | 37.135 | Undet. | 37.965 | 0.830 | 0.0 |
| untreated | 22.489 | 23.195 | 22.871 | 22.852 | 0.204 | 37.202 | Undet. | Undet. | 37.202 | | 0.1 |
| 26933296 | 23.747 | 23.482 | 23.207 | 23.479 | 0.156 | 38.026 | 36.423 | 38.190 | 37.546 | 0.564 | 0.1 |
| TSA | 23.044 | 23.134 | 22.950 | 23.043 | 0.053 | 33.199 | 32.871 | 33.190 | 33.087 | 0.108 | 1.0 |
| 26984152 | 24.221 | 23.739 | 23.754 | 23.905 | 0.158 | 36.175 | 35.407 | Undet. | 35.791 | 0.384 | 0.3 |
| 28658149 | 23.203 | 23.604 | 23.657 | 23.488 | 0.143 | Undet. | Undet. | Undet. | | | |
| 27418183 | 23.324 | 24.440 | 23.318 | 23.694 | 0.373 | 36.060 | Undet. | 35.063 | 35.561 | 0.499 | 0.3 |
| 27136811 | 23.266 | 23.336 | 23.615 | 23.406 | 0.106 | 37.985 | Undet. | Undet. | 37.985 | | 0.0 |
| 27557478 | 23.593 | 23.572 | 23.836 | 23.667 | 0.085 | 34.039 | 35.024 | 33.497 | 34.187 | 0.447 | 0.7 |
| 38171757 | 23.458 | 23.471 | 23.592 | 23.507 | 0.042 | 33.467 | 33.686 | 33.451 | 33.535 | 0.076 | 1.0 |
| 27587807 | 23.431 | 24.064 | 23.991 | 23.829 | 0.200 | 33.198 | 34.015 | 34.464 | 33.892 | 0.371 | 1.0 |
| 38176073 | 23.276 | 23.870 | 24.020 | 23.722 | 0.227 | 34.055 | 34.403 | 34.346 | 34.268 | 0.107 | 0.7 |
| 37851398 | 23.690 | 23.464 | 23.981 | 23.712 | 0.150 | Undet. | Undet. | Undet. | | | |
| 27136668 | 23.367 | 23.216 | 23.431 | 23.338 | 0.064 | Undet. | Undet. | Undet. | | | |
| 27447654 | 23.027 | 23.724 | 23.329 | 23.360 | 0.202 | Undet. | Undet. | Undet. | | | |
| 26956163 | 25.497 | 25.072 | 24.804 | 25.124 | 0.201 | Undet. | Undet. | Undet. | | | |
| 28309411 | 24.832 | 24.120 | 25.468 | 24.807 | 0.389 | 37.671 | Undet. | Undet. | 37.671 | | 0.3 |
| 27570972 | 25.572 | 37.680 | 25.164 | 29.472 | 4.106 | Undet. | Undet. | Undet. | | | |
| 28576860 | 25.598 | 26.164 | 24.807 | 25.523 | 0.393 | Undet. | Undet. | Undet. | | | |
| 28313272 | 24.416 | 35.980 | 25.458 | 28.618 | 3.693 | Undet. | Undet. | Undet. | | | |
| 28571868 | 24.355 | 24.539 | 25.931 | 24.941 | 0.498 | Undet. | Undet. | Undet. | | | |
| 28315326 | 23.921 | 35.649 | 24.443 | 28.004 | 3.825 | Undet. | Undet. | Undet. | | | |
| 28587208 | 23.747 | 24.105 | 24.690 | 24.181 | 0.275 | Undet. | 39.157 | Undet. | 39.157 | | 0.1 |
| 28314000 | 24.075 | 24.004 | 24.521 | 24.200 | 0.162 | Undet. | Undet. | Undet. | | | |
| 28536118 | 24.526 | 24.904 | 25.315 | 24.915 | 0.228 | Undet. | Undet. | Undet. | | | |
| 26949299 | 23.771 | 24.068 | 24.483 | 24.107 | 0.206 | 38.670 | Undet. | Undet. | 38.670 | | 0.1 |
| 28536157 | 24.000 | 23.824 | 24.423 | 24.082 | 0.178 | Undet. | Undet. | Undet. | | | |
| 27669330 | 24.327 | 24.087 | 24.542 | 24.319 | 0.131 | Undet. | Undet. | Undet. | | | |
| 27347892 | 24.539 | 24.615 | 23.645 | 24.266 | 0.311 | Undet. | Undet. | Undet. | | | |
| 28536092 | 23.794 | 24.954 | 25.249 | 24.666 | 0.444 | Undet. | Undet. | Undet. | | | |
| 27520155 | 24.100 | 23.580 | 24.579 | 24.087 | 0.288 | 35.568 | 34.329 | 36.791 | 35.563 | 0.711 | 0.7 |
| 27522482 | 24.761 | 24.913 | 25.147 | 24.940 | 0.112 | Undet. | Undet. | Undet. | | | |
| untreated | 25.264 | 24.183 | 24.749 | 24.732 | 0.312 | Undet. | Undet. | Undet. | | | |
| 27522703 | 25.332 | 25.431 | 24.898 | 25.220 | 0.164 | Undet. | Undet. | Undet. | | | |
| TSA | 25.183 | 24.692 | 25.296 | 25.057 | 0.185 | 35.293 | 36.354 | 36.306 | 35.984 | 0.346 | 1.0 |
| 27522378 | 25.032 | 24.780 | 24.553 | 24.788 | 0.138 | 38.019 | 36.490 | 37.004 | 37.171 | 0.449 | 0.4 |
| 27527383 | 25.080 | 24.931 | 25.157 | 25.056 | 0.066 | Undet. | Undet. | 38.764 | 38.764 | | 0.1 |
| 27522716 | 24.724 | 24.145 | 24.981 | 24.616 | 0.247 | Undet. | 38.139 | Undet. | 38.139 | | 0.2 |
| 28557555 | 25.237 | 24.088 | 24.799 | 24.708 | 0.335 | Undet. | 39.126 | 38.095 | 38.611 | 0.515 | 0.1 |
| 27522612 | 25.272 | 23.862 | 25.055 | 24.729 | 0.438 | Undet. | Undet. | Undet. | | | |
| 29232515 | 25.033 | 24.774 | 24.774 | 24.860 | 0.086 | 37.030 | 35.977 | 35.802 | 36.270 | 0.383 | 0.7 |
| 26909584 | 24.014 | 24.215 | 25.134 | 24.454 | 0.345 | 35.777 | 37.044 | 37.375 | 36.732 | 0.487 | 0.4 |
| 28978209 | 25.070 | 24.805 | 25.000 | 24.958 | 0.079 | 36.014 | 36.358 | 36.036 | 36.136 | 0.111 | 0.8 |
| 28427152 | 25.599 | 25.110 | 24.974 | 25.228 | 0.190 | 36.227 | 37.351 | 36.245 | 36.608 | 0.372 | 0.7 |
| 28297919 | 24.506 | 23.866 | 24.850 | 24.407 | 0.288 | Undet. | Undet. | Undet. | | | |
| 28831777 | 25.136 | 25.295 | 25.699 | 25.377 | 0.168 | 37.044 | 36.832 | 38.013 | 37.297 | 0.363 | 0.5 |
| 26980395 | 24.611 | 24.138 | 25.222 | 24.657 | 0.314 | 39.393 | 38.003 | Undet. | 38.698 | | 0.1 |
| 27625156 | 25.349 | 25.726 | 25.303 | 25.459 | 0.134 | Undet. | Undet. | Undet. | | | |
| untreated | 25.801 | 25.810 | 24.698 | 25.436 | 0.369 | Undet. | Undet. | Undet. | | | |
| 27639573 | 23.900 | 25.355 | 24.942 | 24.732 | 0.433 | Undet. | Undet. | Undet. | | | |
| TSA | 25.220 | 25.127 | 25.504 | 25.284 | 0.113 | 34.288 | 33.753 | 33.952 | 33.997 | 0.156 | 1.0 |
| 28641171 | 24.440 | 24.748 | 25.337 | 24.842 | 0.263 | 33.387 | 33.788 | 34.383 | 33.852 | 0.289 | 0.8 |
| 28629822 | 24.933 | 25.202 | 25.199 | 25.112 | 0.089 | Undet. | Undet. | Undet. | | | |
| 32681584 | 23.907 | 24.326 | 24.567 | 24.267 | 0.193 | 33.154 | 34.016 | 33.469 | 33.546 | 0.252 | 0.7 |
| 35772893 | 24.988 | 25.125 | 25.294 | 25.136 | 0.089 | Undet. | Undet. | Undet. | | | |
| 33300852 | 25.032 | 25.190 | 24.345 | 24.855 | 0.259 | 34.273 | 34.816 | 33.615 | 34.235 | 0.347 | 0.6 |
| 38134356 | 25.588 | 25.085 | 24.635 | 25.102 | 0.275 | 34.633 | 33.744 | 33.952 | 34.110 | 0.269 | 0.8 |
| 27643148 | 24.420 | 25.258 | 25.184 | 24.954 | 0.268 | Undet. | Undet. | Undet. | | | |
| 27632709 | 24.923 | 24.174 | 24.470 | 24.522 | 0.218 | Undet. | Undet. | Undet. | | | |
| 27643330 | 25.370 | 25.206 | 24.466 | 25.014 | 0.278 | Undet. | Undet. | Undet. | | | |
| 27632891 | 24.657 | 24.099 | 25.055 | 24.603 | 0.277 | Undet. | Undet. | Undet. | | | |
| 28439970 | 24.294 | 25.096 | 24.570 | 24.653 | 0.235 | Undet. | Undet. | Undet. | | | |
| 28309346 | 25.621 | 25.246 | 25.551 | 25.473 | 0.115 | Undet. | Undet. | Undet. | | | |
| 26913679 | 23.366 | 24.257 | 24.610 | 24.078 | 0.370 | Undet. | Undet. | Undet. | | | |
| 27447589 | 24.177 | 24.302 | 23.512 | 23.997 | 0.245 | Undet. | Undet. | Undet. | | | |
| 26951691 | 23.534 | 24.481 | 24.633 | 24.216 | 0.344 | Undet. | Undet. | Undet. | | | |
| 27291030 | 23.754 | 24.116 | 23.847 | 23.905 | 0.108 | 33.526 | 34.257 | 34.041 | 33.941 | 0.217 | 0.4 |
| 27142687 | 24.349 | 24.083 | 24.865 | 24.432 | 0.230 | Undet. | Undet. | Undet. | | | |
| 27136720 | 23.685 | 24.244 | 23.653 | 23.860 | 0.192 | Undet. | Undet. | Undet. | | | |
| 27667250 | 22.955 | 23.025 | 23.809 | 23.263 | 0.274 | Undet. | Undet. | Undet. | | | |
| 27136798 | 23.505 | 23.978 | 23.863 | 23.782 | 0.142 | Undet. | Undet. | Undet. | | | |
| 28309294 | 24.163 | 23.419 | 23.525 | 23.702 | 0.232 | Undet. | 39.313 | Undet. | 39.313 | | 0.0 |
| 27571024 | 24.241 | 24.288 | 24.038 | 24.189 | 0.077 | Undet. | Undet. | Undet. | | | |
| 27122160 | 23.067 | 23.603 | 23.803 | 23.491 | 0.220 | 39.409 | Undet. | Undet. | 39.409 | | 0.0 |
| 28576808 | 23.808 | 23.752 | 23.531 | 23.697 | 0.084 | Undet. | Undet. | Undet. | | | |
| 28407028 | 23.820 | 23.592 | 24.291 | 23.901 | 0.206 | Undet. | Undet. | Undet, | | | |
| 27528566 | 23.499 | 23.324 | 23.521 | 23.448 | 0.062 | Undet. | Undet. | Undet. | | | |
| 35278321 | 24.524 | 24.128 | 24.400 | 24.351 | 0.117 | Undet. | Undet. | Undet. | | | |
| 27632982 | 24.483 | 24.300 | 25.170 | 24.651 | 0.265 | 34.941 | 35.332 | 35.065 | 35.113 | 0.115 | 0.3 |
| 37125335 | 25.400 | 25.753 | 25.171 | 25.441 | 0.169 | 36.379 | 37.855 | 36.226 | 36.820 | 0.519 | 0.7 |
| 28606643 | 25.577 | 25.336 | 25.026 | 25.313 | 0.159 | 36.854 | Undet. | 37.622 | 37.238 | 0.384 | 0.5 |
| 26975390 | 24.244 | 24.642 | 24.569 | 24.485 | 0.122 | Undet. | Undet. | Undet. | | | |
| 27039857 | 24.096 | 25.170 | 24.919 | 24.728 | 0.324 | Undet. | Undet. | Undet. | | | |
| 27136772 | 23.531 | 24.142 | 24.691 | 24.121 | 0.335 | Undet. | Undet. | Undet. | | | |
| 28315300 | 25.210 | 24.255 | 24.285 | 24.583 | 0.313 | 37.049 | 35.189 | 35.295 | 35.844 | 0.603 | 0.8 |
| 27580501 | 22.909 | 24.055 | 23.447 | 23.470 | 0.331 | 36.408 | 37.492 | 38.392 | 37.431 | 0.574 | 0.1 |
| 28338089 | 24.651 | 23.823 | 24.375 | 24.283 | 0.243 | 36.158 | 34.465 | 35.045 | 35.223 | 0.497 | 1.0 |
| 27580566 | 24.085 | 23.855 | 25.288 | 24.409 | 0.444 | Undet. | Undet. | Undet. | | | |
| 28345213 | 24.095 | 24.166 | 25.308 | 24.523 | 0.393 | 36.572 | 38.152 | 39.734 | 38.153 | 0.913 | 0.2 |
| 26906997 | 23.873 | 24.037 | 25.062 | 24.324 | 0.372 | 36.113 | 36.913 | 36.674 | 36.567 | 0.237 | 0.4 |
| 28376686 | 23.841 | 24.449 | 24.781 | 24.357 | 0.275 | 34.588 | 37.510 | 37.156 | 36.418 | 0.921 | 0.5 |
| 27017406 | 24.003 | 24.700 | 24.430 | 24.377 | 0.203 | Undet. | Undet. | Undet. | | | |
| 28407275 | 25.100 | 24.919 | 25.010 | 25.010 | 0.052 | 38.940 | 38.991 | 38.011 | 38.648 | 0.319 | 0.2 |
| 27025583 | 24.295 | 24.049 | 24.476 | 24.273 | 0.124 | Undet. | Undet. | Undet. | | | |
| 28427087 | 25.151 | 24.365 | 24.577 | 24.698 | 0.235 | Undet. | Undet. | Undet. | | | |
| 28518919 | 25.348 | 26.388 | 25.303 | 25.680 | 0.354 | 37.862 | 37.800 | 37.565 | 37.742 | 0.090 | 0.5 |
| untreated | 25.642 | 25.490 | 24.786 | 25.306 | 0.264 | Undet. | Undet. | Undet. | | | |
| 27122147 | 25.551 | 25.801 | 25.514 | 25.622 | 0.090 | Undet. | Undet. | Undet. | | | |
| TSA | 25.742 | 24.269 | 25.113 | 25.041 | 0.427 | 36.363 | 35.589 | 35.919 | 35.957 | 0.224 | 1.0 |
| 28616289 | 24.986 | 25.793 | 25.125 | 25.301 | 0.249 | Undet. | Undet. | 39.841 | 39.841 | | 0.1 |
| 27191021 | 24.613 | 24.822 | 24.770 | 24.735 | 0.063 | Undet. | Undet. | Undet. | | | |
| 28296424 | 24.516 | 24.286 | 24.612 | 24.471 | 0.097 | 38.876 | Undet. | Undet. | 38.876 | | 0.1 |
| 31040217 | 25.833 | 25.010 | 24.614 | 25.152 | 0.359 | 36.613 | 36.255 | 35.410 | 36.093 | 0.357 | 1.0 |
| 31035940 | 24.718 | 25.087 | 24.753 | 24.853 | 0.118 | Undet. | Undet. | Undet. | | | |
| 26960674 | 25.234 | 24.897 | 25.346 | 25.159 | 0.135 | Undet. | Undet. | Undet. | | | |
| 31040776 | 25.467 | 25.545 | 25.078 | 25.363 | 0.144 | 38.215 | Undet. | 38.619 | 38.417 | 0.202 | 0.2 |
| 27639677 | 24.960 | 24.639 | 24.662 | 24.754 | 0.103 | Undet. | Undet. | Undet. | | | |
| 31040763 | 24.456 | 25.210 | 25.013 | 24.893 | 0.226 | 34.445 | 37.223 | 36.715 | 36.128 | 0.854 | 0.8 |
| 26930943 | 25.188 | 25.187 | 24.711 | 25.029 | 0.159 | Undet. | 39.275 | Undet. | 39.275 | | 0.1 |
| 31040074 | 24.736 | 24.986 | 25.398 | 25.040 | 0.193 | 35.196 | 35.623 | 35.911 | 35.577 | 0.208 | 1.3 |
| 27589328 | 25.622 | 25.004 | 24.638 | 25.088 | 0.287 | Undet. | Undet. | Undet. | | | |
| 27136980 | 24.727 | 24.850 | 24.967 | 24.848 | 0.069 | Undet. | Undet. | Undet. | | | |
| untreated | 24.474 | 25.112 | 23.651 | 24.412 | 0.423 | Undet. | Undet. | Undet. | | | |
| 27136967 | 24.620 | 25.024 | 24.796 | 24.813 | 0.117 | Undet. | Undet. | Undet. | | | |
| TSA | 25.524 | 24.816 | 24.525 | 24.955 | 0.297 | 34.079 | 34.137 | 34.838 | 34.351 | 0.244 | 1.0 |
| 27136941 | 24.103 | 24.558 | 24.049 | 24.237 | 0.162 | Undet. | Undet. | Undet. | | | |
| 27552421 | 24.033 | 24.462 | 23.705 | 24.066 | 0.219 | Undet. | Undet. | Undet. | | | |
| 27136915 | 23.741 | 24.085 | 24.156 | 23.994 | 0.128 | Undet. | Undet. | Undet. | | | |
| 28549144 | 23.835 | 24.102 | 23.953 | 23.964 | 0.077 | Undet. | Undet. | Undet. | | | |
| 27137045 | 24.891 | 24.158 | 24.374 | 24.474 | 0.218 | Undet. | Undet. | Undet. | | | |
| 31002439 | 24.376 | 24.549 | 24.140 | 24.355 | 0.118 | 34.143 | 34.913 | 33.826 | 34.294 | 0.323 | 0.7 |
| 27136759 | 25.201 | 24.229 | 24.374 | 24.602 | 0.303 | Undet. | Undet. | Undet. | | | |
| 28338102 | 25.329 | 25.197 | 24.361 | 24.962 | 0.303 | 34.412 | 35.402 | 34.838 | 34.884 | 0.287 | 0.7 |
| 27263379 | 24.560 | 24.145 | 24.388 | 24.364 | 0.120 | Undet. | Undet. | Undet. | | | |
| 28427100 | 24.269 | 24.822 | 24.783 | 24.625 | 0.178 | 34.893 | 34.936 | 36.372 | 35.400 | 0.486 | 0.4 |
| 27263392 | 24.642 | 24.421 | 24.629 | 24.564 | 0.071 | Undet. | Undet. | Undet. | | | |
| 28549079 | 24.907 | 24.903 | 25.208 | 25.006 | 0.101 | 34.961 | 35.212 | 36.292 | 35.488 | 0.409 | 0.5 |
| 37827634 | 25.598 | 24.690 | 25.369 | 25.219 | 0.273 | Undet. | 39.182 | Undet. | 39.182 | | 0.0 |
| 26996710 | 25.215 | 25.482 | 25.429 | 25.375 | 0.082 | 35.942 | 35.427 | 35.921 | 35.763 | 0.168 | 0.5 |
| 37844664 | 24.908 | 25.119 | 25.158 | 25.062 | 0.078 | Undet. | 39.783 | 39.602 | 39.693 | 0.091 | 0.0 |
| 26996736 | 25.125 | 25.864 | 25.606 | 25.532 | 0.217 | 38.536 | 37.573 | 38.540 | 38.216 | 0.322 | 0.1 |
| 26996645 | 24.567 | 25.174 | 25.949 | 25.230 | 0.400 | 34.780 | 35.082 | 37.356 | 35.739 | 0.813 | 0.5 |
| 26996749 | 24.563 | 24.917 | 25.081 | 24.854 | 0.153 | 37.187 | 36.188 | 38.134 | 37.170 | 0.562 | 0.1 |
| 26996866 | 24.168 | 24.633 | 25.345 | 24.716 | 0.342 | 36.779 | 37.676 | 37.350 | 37.268 | 0.262 | 0.1 |
| 28651012 | 24.177 | 25.858 | 25.082 | 25.039 | 0.486 | Undet. | Undet. | Undet. | | | |
| 26996892 | 24.733 | 25.659 | 25.230 | 25.207 | 0.268 | Undet. | 38.929 | Undet. | 38.929 | | 0.1 |
| 28663973 | 25.447 | 24.777 | 25.495 | 25.240 | 0.232 | 36.413 | 35.620 | 37.239 | 36.424 | 0.468 | 0.3 |
| 26996905 | 24.717 | 24.767 | 26.120 | 25.201 | 0.460 | 37,053 | 38.627 | Undet. | 37.840 | | 0.1 |
| 28324036 | 24.445 | 25.078 | 24.774 | 24.765 | 0.183 | Undet. | Undet. | Undet. | | | |
| 26996671 | 25.088 | 24.652 | 25.361 | 25.034 | 0.206 | 34.953 | 34.577 | 35.472 | 35.001 | 0.259 | 0.7 |
| 31046600 | 25.059 | 25.378 | 25.243 | 25.227 | 0.092 | 35.053 | 34.542 | 35.433 | 35.009 | 0.258 | 0.8 |
| 26996918 | 24.998 | 24.642 | 25.391 | 25.010 | 0.216 | 36.120 | 39.263 | 37.122 | 37.501 | 0.927 | 0.1 |
| 26977847 | 25.343 | 25.159 | 25.020 | 25.174 | 0.093 | 36.414 | 35.872 | 36.448 | 36.245 | 0.187 | 0.3 |
| 27632917 | 25.292 | 25.074 | 24.977 | 25.114 | 0.093 | Undet. | Undet. | Undet. | | | |
| untreated | 24.567 | 24.317 | 24.320 | 24.401 | 0.083 | Undet. | Undet. | Undet. | | | |
| 36721698 | 24.869 | 24.496 | 25.066 | 24.810 | 0.167 | Undet. | Undet. | Undet. | | | |
| TSA | 25.242 | 24.688 | 24.362 | 24.764 | 0.257 | 34.017 | 33.986 | 34.600 | 34.201 | 0.200 | 1.0 |
| 26909688 | 24.749 | 24.470 | 24.655 | 24.624 | 0.082 | Undet. | Undet. | Undet. | | | |
| 27190124 | 24.170 | 24.162 | 24.112 | 24.148 | 0.018 | Undet. | Undet. | Undet. | | | |
| 27628198 | 23.994 | 23.750 | 23.435 | 23.726 | 0.162 | Undet. | Undet. | Undet. | | | |
| 27213719 | 23.657 | 24.136 | 24.006 | 23.933 | 0.143 | Undet. | Undet. | Undet. | | | |
| 27625182 | 25.294 | 24.715 | 24.419 | 24.809 | 0.257 | Undet. | Undet. | Undet. | | | |
| 27633073 | 24.361 | 24.411 | 24.020 | 24.264 | 0.123 | Undet. | Undet. | Undet. | | | |
| 27632670 | 24.416 | 23.905 | 24.541 | 24.287 | 0.194 | Undet. | Undet. | Undet. | | | |
| 26909727 | 23.951 | 24.121 | 23.907 | 23.993 | 0.065 | Undet. | Undet. | Undet. | | | |
| 27625260 | 25.338 | 24.521 | 25.385 | 25.081 | 0.280 | Undet. | Undet. | Undet. | | | |
| 26909753 | 24.241 | 24.759 | 24.811 | 24.604 | 0.182 | Undet. | Undet. | Undet. | | | |
| 33297914 | 24.778 | 24.863 | 25.238 | 24.960 | 0.141 | Undet. | Undet. | Undet. | | | |
| 26909766 | 24.152 | 24.337 | 24.474 | 24.321 | 0.093 | Undet. | Undet. | Undet. | | | |
| 31069415 | 26.046 | 25.168 | 25.487 | 25.567 | 0.257 | Undet. | Undet. | Undet. | | | |
| 28518568 | 25.205 | 25.745 | 24.773 | 25.241 | 0.281 | Undet. | Undet. | Undet. | | | |
| 37804546 | 25.980 | 25.100 | 25.904 | 25.661 | 0.282 | Undet. | 37.203 | 37.663 | 37.433 | 0.230 | 0.2 |
| 31064072 | 24.451 | 25.196 | 24.636 | 24.761 | 0.224 | 38.184 | Undet. | Undet. | 38.184 | | 0.1 |
| 27477788 | 24.889 | 25.131 | 25.142 | 25.054 | 0.083 | 34.645 | 33.999 | 35.341 | 34.662 | 0.387 | 0.9 |
| 28309385 | 24.313 | 23.666 | 23.861 | 23.947 | 0.192 | Undet. | Undet. | Undet. | | | |
| 28557464 | 24.010 | 24.051 | 24.259 | 24.107 | 0.077 | Undet. | Undet. | Undet. | | | |
| 28309320 | 23.977 | 24.768 | 24.233 | 24.326 | 0.233 | Undet. | Undet. | Undet. | | | |
| 31053828 | 25.368 | 24.710 | 25.074 | 25.050 | 0.190 | 35.482 | 34.909 | 35.035 | 35.142 | 0.174 | 0.6 |
| 28309242 | 24.936 | 24.196 | 25.256 | 24.796 | 0.314 | Undet. | Undet. | Undet. | | | |
| 31056792 | 25.531 | 24.944 | 25.251 | 25.242 | 0.170 | 34.006 | 34.183 | 35.584 | 34.591 | 0.499 | 1.1 |
| 27635556 | 23.806 | 24.328 | 24.648 | 24.261 | 0.246 | Undet. | Undet. | Undet. | | | |
| 28531516 | 24.913 | 25.111 | 24.518 | 24.847 | 0.174 | Undet. | Undet. | Undet. | | | |
| 26963105 | 24.191 | 25.137 | 24.288 | 24.539 | 0.300 | Undet. | Undet. | Undet. | | | |
| 28309255 | 24.481 | 24.642 | 24.879 | 24.667 | 0.116 | Undet. | Undet. | Undet. | | | |
| 26986076 | 24.344 | 25.435 | 25.115 | 24.965 | 0.324 | Undet. | Undet. | Undet. | | | |
| 35772568 | 25.492 | 24.814 | 25.236 | 25.181 | 0.198 | Undet. | Undet. | Undet. | | | |
| 33155811 | Undet. | 25.223 | 24.620 | 24.922 | 0.301 | Undet. | 34.716 | 34.473 | 34.594 | 0.122 | 1.2 |
| 28404844 | 25.613 | 25.084 | 24.326 | 25.007 | 0.373 | 33.790 | 34.994 | 33.859 | 34.214 | 0.390 | 1.6 |
| 27016223 | 24.120 | 24.419 | 24.296 | 24.278 | 0.087 | Undet. | Undet. | Undet. | | | |
| 27137006 | 24.342 | 24.182 | 23.564 | 24.029 | 0.237 | Undet. | Undet. | Undet. | | | |
| 27022307 | 23.655 | 23.699 | 23.532 | 23.629 | 0.050 | Undet. | Undet. | Undet. | | | |
| 28507271 | 23.898 | 24.106 | 23.715 | 23.906 | 0.113 | 34.305 | 34.073 | 33.170 | 33.849 | 0.346 | 1.0 |
| 27534455 | 23.609 | 24.152 | 23.101 | 23.621 | 0.303 | Undet. | Undet. | Undet. | | | |
| 27136733 | 23.810 | 24.356 | 24.478 | 24.214 | 0.205 | Undet. | Undet. | Undet. | | | |
| 27422889 | 23.609 | 23.523 | 24.619 | 23.917 | 0.352 | Undet. | Undet. | Undet. | | | |
| 27136850 | 23.687 | 24.632 | 24.870 | 24.396 | 0.361 | Undet. | Undet. | Undet. | | | |
| 28532946 | 24.465 | 24.924 | 25.496 | 24.962 | 0.298 | Undet. | Undet. | Undet. | | | |
| 27136824 | 23.913 | 24.738 | 23.786 | 24.146 | 0.299 | Undet. | Undet. | Undet. | | | |
| 27482286 | 24.941 | 25.019 | 24.783 | 24.914 | 0.069 | Undet. | Undet. | Undet. | | | |
| 27136785 | 23.789 | 24.405 | 24.810 | 24.335 | 0.297 | Undet. | Undet. | Undet. | | | |
| 27513252 | 23.440 | 25.004 | 24.486 | 24.310 | 0.460 | Undet. | Undet. | Undet. | | | |
| 27519635 | 24.468 | 24.346 | 24.049 | 24.288 | 0.124 | Undet. | Undet. | Undet. | | | |
| 27016236 | 24.552 | 24.527 | 24.409 | 24.496 | 0.044 | Undet. | Undet. | Undet. | | | |
| untreated | 23.644 | 24.704 | 23.909 | 24.085 | 0.319 | Undet. | Undet. | Undet. | | | |
| 27570933 | 25.399 | 25.309 | 25.590 | 25.433 | 0.083 | Undet. | 39.987 | Undet. | 39.987 | | 0.1 |
| TSA | 23.550 | 24.827 | 25.394 | 24.590 | 0.545 | 33.973 | 35.214 | 36.156 | 35.115 | 0.632 | 1.0 |
| 38113595 | 25.447 | 25.194 | 25.985 | 25.542 | 0.233 | Undet. | Undet. | Undet. | | | |
| 28547103 | 23.600 | 24.933 | 24.122 | 24.218 | 0.388 | Undet. | Undet. | Undet. | | | |
| 38107862 | 24.508 | 24.397 | 25.429 | 24.778 | 0.327 | 35.763 | 34.588 | 35.360 | 35.237 | 0.345 | 1.0 |
| 27572441 | 24.503 | 24.497 | 24.826 | 24.609 | 0.108 | 37.178 | 36.165 | Undet. | 36.671 | | 0.3 |
| 37125426 | 24.243 | 26.276 | 25.208 | 25.242 | 0.587 | Undet. | Undet. | Undet. | | | |
| 27587781 | 23.711 | 24.806 | 24.422 | 24.313 | 0.321 | 34,627 | 35.384 | 35.423 | 35.145 | 0,259 | 0.8 |
| 37827803 | 24.882 | 26.378 | 25.923 | 25.728 | 0.443 | Undet. | Undet. | Undet. | | | |
| 27580449 | 24.246 | 24.694 | 24.624 | 24.522 | 0.139 | 39.133 | 39.534 | Undet. | 39.334 | | 0.1 |
| 27151787 | 24.130 | 24.315 | 24.967 | 24.471 | 0.254 | Undet. | Undet. | Undet. | | | |
| 27643356 | 23.498 | 23.871 | 24.253 | 23.874 | 0.218 | Undet. | Undet. | Undet. | | | |
| 27151696 | 24.762 | 26.717 | 26.051 | 25.843 | 0.574 | Undet. | Undet. | Undet. | | | |
| 28577055 | 24.660 | 25.028 | 25.290 | 24.993 | 0.183 | 36.317 | 38.005 | 38.243 | 37.522 | 0.606 | 0.2 |
| 27039935 | 25.747 | 25.241 | 25.546 | 25.511 | 0.147 | Undet. | Undet. | Undet. | | | |
| 5526079 | 24.378 | 24.744 | 24.253 | 24.458 | 0.147 | 38.468 | Undet. | Undet. | 38.468 | | 0.1 |
| 27054586 | 25.661 | 24.876 | 26.091 | 25.543 | 0.356 | Undet. | 35.540 | 36.810 | 36.175 | 0.635 | 0.9 |
| 28663986 | 25.660 | 25.063 | 24.651 | 25.125 | 0.293 | 35.763 | 36.539 | 37.168 | 36.490 | 0.406 | 0.6 |
| 27643317 | 24.596 | 24.477 | 25.159 | 24.744 | 0.210 | Undet. | Undet. | Undet. | | | |
| 27122173 | 25.897 | 24.232 | 24.055 | 24.728 | 0.587 | Undet. | Undet. | Undet. | | | |
| 26962962 | 24.519 | 25.148 | 25.253 | 24.973 | 0.229 | 35.172 | 36.500 | 35.870 | 35.847 | 0.384 | 0.8 |
| 28846740 | 24.635 | 24.978 | 24.362 | 24.658 | 0.178 | Undet. | Undet. | Undet. | | | |
| 27054547 | 24.505 | 25.174 | 25.158 | 24.946 | 0.220 | Undet. | Undet. | Undet. | | | |
| 28846870 | 25.841 | 25.207 | 24.813 | 25.287 | 0.299 | 38.785 | Undet. | 38.368 | 38.577 | 0.208 | 0.1 |
| 27075750 | 25.220 | 26.054 | 24.696 | 25.324 | 0.395 | Undet. | Undet. | Undet. | | | |
| 28847052 | 25.234 | 25.158 | 25.346 | 25.246 | 0.054 | 37.174 | 39.129 | 37.205 | 37.836 | 0.647 | 0.2 |
| 26981201 | 25.062 | 26.784 | 25.954 | 25.933 | 0.497 | 34.594 | 39.419 | 37.627 | 37.213 | 1.408 | 0.6 |
| 26996684 | 25.765 | 25.868 | 25.291 | 25.641 | 0.178 | 36.277 | 38.770 | Undet. | 37.523 | | 0.4 |
| 26163605 | 24.085 | 26.517 | 24.262 | 24.955 | 0.783 | Undet. | Undet. | Undet. | | | |
| 26996840 | 24.788 | 24.936 | 24.829 | 24.851 | 0.044 | 37.526 | 38.023 | Undet. | 37.774 | | 0.2 |
| 28625207 | 26.500 | 26.148 | 25.262 | 25.970 | 0.368 | Undet. | Undet. | Undet. | | | |
| 26996762 | 26.116 | 25.977 | 25.417 | 25.837 | 0.214 | Undet. | Undet. | Undet. | | | |
| 26962923 | 25.212 | 24.611 | 24.295 | 24.706 | 0.269 | Undet. | Undet. | Undet. | | | |
| 28543983 | 24.136 | 24.752 | 25.596 | 24.828 | 0.423 | 34.584 | 35.008 | 36.088 | 35.227 | 0.448 | 0.6 |
| 27117259 | 23.577 | 24.012 | 24.643 | 24.078 | 0.309 | Undet. | Undet. | Undet. | | | |
| 26996775 | 24.312 | 24.521 | 25.027 | 24.620 | 0.212 | Undet. | Undet. | Undet. | | | |
| 27062698 | 24.055 | 23.029 | 24.546 | 23.877 | 0.447 | Undet. | Undet. | Undet. | | | |
| 28540863 | 24.266 | 24.013 | 24.682 | 24.320 | 0.195 | Undet. | Undet. | Undet. | | | |
| 27558414 | 25.079 | 24.977 | 25.312 | 25.123 | 0.099 | Undet. | Undet. | Undet. | | | |
| 28625207 | 26.500 | 26.148 | 25.262 | 25.970 | 0.368 | Undet. | Undet. | Undet. | | | |
| 26996762 | 26.116 | 25.977 | 25.417 | 25.837 | 0.214 | Undet. | Undet. | Undet. | | | |
| 26962923 | 25.212 | 24.611 | 24.295 | 24.706 | 0.269 | Undet. | Undet. | Undet. | | | |
| 28543996 | 24.371 | 24.567 | 25.262 | 24.733 | 0.270 | 35.957 | 35.930 | 38.742 | 36.876 | 0.933 | 0.2 |
| 27620710 | 25.632 | 25.183 | 24.982 | 25.265 | 0.192 | 36.320 | 35.495 | 37.583 | 36.466 | 0.607 | 0.3 |
| 31013086 | 23.843 | 24.466 | 24.015 | 24.108 | 0.186 | Undet. | Undet. | Undet. | | | |
| 27639287 | 26.097 | 25.083 | 24.981 | 25.387 | 0.356 | 37.354 | 35.099 | 36.341 | 36.265 | 0.652 | 0.4 |
| 27570946 | 25.740 | 25.036 | 24.087 | 24.954 | 0.479 | Undet. | Undet. | Undet. | | | |
| 31012904 | 26.105 | 25.203 | 26.042 | 25.783 | 0.291 | Undet. | 36.764 | 39.046 | 37.905 | 1.141 | 0.2 |
| 27580657 | 25.303 | 25.266 | 24.355 | 24.975 | 0.310 | Undet. | Undet. | Undet. | | | |
| 26996853 | 25.404 | 26.458 | 26.147 | 26.003 | 0.313 | 37.940 | Undet. | 36.666 | 37.303 | 0.637 | 0.3 |
| untreated | 25.027 | 25.201 | 24.805 | 25.011 | 0.115 | Undet. | Undet. | Undet. | | | |
| 27014377 | 25.373 | 26.058 | 25.358 | 25.596 | 0.231 | 36.538 | Undet. | Undet. | 36.538 | | 0.4 |
| TSA | 25.625 | 24.740 | 25.526 | 25.297 | 0.280 | 34.243 | 34.713 | 35.604 | 34.853 | 0.399 | 1.0 |
| 27422746 | 24.376 | 25.133 | 24.956 | 24.822 | 0.229 | Undet. | Undet. | 38.571 | 38.571 | | 0.1 |
| 27580592 | 24.154 | 24.132 | 24.715 | 24.333 | 0.191 | Undet. | Undet. | Undet. | | | |
| 27572428 | 23.983 | 23.999 | 23.941 | 23.974 | 0.017 | Undet. | Undet. | Undet. | | | |
| 30999683 | 23.961 | 23.904 | 24.853 | 24.239 | 0.307 | Undet. | Undet. | Undet. | | | |
| 31005871 | 25.092 | 25.266 | 24.433 | 24.930 | 0.253 | Undet. | Undet. | Undet. | | | |
| 30997824 | 24.736 | 25.301 | 25.304 | 25.114 | 0.189 | 38.106 | Undet. | Undet. | 38.106 | | 0.1 |
| 26909714 | 25.747 | 25.089 | 24.654 | 25.163 | 0.318 | Undet. | Undet. | Undet. | | | |
| 30997915 | 24.016 | 23.826 | 25.500 | 24.447 | 0.529 | 35.120 | 34.484 | 38.055 | 35.886 | 1.100 | 0.3 |
| 26933283 | 24.967 | 25.399 | 25.611 | 25.326 | 0.189 | Undet. | Undet. | Undet. | | | |
| 38222951 | 24.001 | 24.253 | 24.267 | 24.174 | 0.086 | 34.263 | 35.078 | 35.148 | 34.829 | 0.284 | 0.5 |
| 31053854 | 24.259 | 24.657 | 25.685 | 24.867 | 0.425 | 34.290 | 34.889 | 34.671 | 34.617 | 0.175 | 0.9 |
| 31068778 | 24.068 | 23.556 | 25.024 | 24.216 | 0.430 | Undet. | Undet. | Undet. | | | |
| 28315365 | 24.320 | 24.653 | 24.780 | 24.584 | 0.137 | Undet. | Undet. | Undet. | | | |
| untreated | 23.910 | 23.797 | 24.101 | 23.936 | 0.089 | Undet. | Undet. | Undet. | | | |
| 28313207 | 24.002 | 23.687 | 24.403 | 24.031 | 0.207 | Undet. | Undet. | Undet. | | | |
| TSA | 23.623 | 23.860 | 24.215 | 23.899 | 0.172 | 33.638 | 33.531 | 34.048 | 33.739 | 0.158 | 1.0 |
| 28323906 | 23.687 | 24.909 | 24.801 | 24.466 | 0.391 | 34.367 | 35.284 | 35.552 | 35.068 | 0.359 | 0.6 |
| 31069428 | 23.493 | 24.477 | 24.423 | 24.131 | 0.319 | 34.340 | 35.238 | 34.369 | 34.649 | 0.294 | 0.6 |
| 33300969 | 23.168 | 23.676 | 23.517 | 23.454 | 0.150 | Undet. | Undet. | Undet. | | | |
| 27387373 | 23.898 | 24.070 | 24.399 | 24.122 | 0.147 | Undet. | Undet. | Undet. | | | |
| 38163957 | 25.357 | 26.004 | 25.081 | 25.481 | 0.273 | 35.864 | 36.126 | 35.613 | 35.868 | 0.148 | 0.7 |
| 31064020 | 24.732 | 24.652 | 24.144 | 24.509 | 0.184 | Undet. | Undet. | Undet. | | | |
| 38168078 | 27.754 | 27.511 | 27.049 | 27.438 | 0.207 | 39.974 | 36.909 | Undet. | 38.442 | | 0.4 |
| 26988858 | 25.779 | 26.112 | 24.955 | 25.615 | 0.344 | Undet. | Undet. | Undet. | | | |
| 28631590 | 27.344 | 26.515 | 26.052 | 26.637 | 0.378 | 35.928 | 35.746 | 35.839 | 35.838 | 0.053 | 1.6 |
| 26988884 | 26.272 | 26.550 | 26.196 | 26.339 | 0.107 | Undet. | Undet. | Undet. | | | |
| 28848469 | 27.748 | 27.488 | 26.734 | 27.323 | 0.304 | 38.450 | 37.563 | 36.819 | 37.610 | 0.471 | 0.7 |
| 28323945 | 27.625 | 27.522 | 26.844 | 27.330 | 0.245 | Undet. | Undet. | Undet. | | | |
| 27117480 | 25.467 | Undet. | 25.406 | 25.436 | 0.030 | Undet. | Undet. | Undet. | | | |
| 28502240 | 23.784 | Undet. | 24.697 | 24.241 | 0.457 | 34.980 | Undet. | 34.441 | 34.710 | 0.269 | 0.6 |
| 27217333 | 24.447 | 25.299 | 25.554 | 25.100 | 0.335 | Undet. | Undet. | Undet. | | | |
| 38107849 | 24.724 | 23.958 | 24.695 | 24.459 | 0.251 | 36.717 | 34.464 | 35.626 | 35.602 | 0.650 | 0.4 |
| 27217541 | 24.459 | 24.017 | 25.843 | 24.773 | 0.550 | Undet. | Undet. | Undet. | | | |
| 38107992 | 26.261 | 25.144 | 23.985 | 25.130 | 0.657 | 35.134 | 34.835 | 34.154 | 34.707 | 0.290 | 1.2 |
| 26986453 | 24.457 | 24.750 | 24.861 | 24.689 | 0.120 | 34.993 | 35.054 | 34.562 | 34.870 | 0.155 | 0.8 |
| 28540902 | 24.714 | 23.666 | 24.235 | 24.205 | 0.303 | Undet. | Undet. | Undet. | | | |
| 27132352 | 25.096 | 24.767 | 25.817 | 25.227 | 0.310 | Undet. | Undet. | Undet. | | | |
| 28600871 | 25.439 | 25.562 | 25.282 | 25.427 | 0.081 | Undet. | Undet. | Undet. | | | |
| 28532933 | 26.627 | 26.705 | 27.149 | 26.827 | 0,163 | Undet. | Undet. | Undet. | | | |
| 27599728 | 25.695 | 26.209 | 25.925 | 25.943 | 0.149 | Undet. | Undet. | Undet. | | | |
| 37852763 | 30.123 | 29.119 | 29.119 | 29.119 | 0.000 | Undet. | Undet. | Undet. | | | |
| 31064111 | 28.294 | 27.951 | 26.350 | 27.532 | 0.599 | Undet. | Undet. | 39.010 | 39.010 | | 0.3 |
| 28502435 | 28.975 | 28.291 | 26.821 | 28.029 | 0.635 | Undet. | 39.597 | 38.139 | 38.868 | 0.729 | 0.5 |
| 27635465 | 26.206 | 26.620 | 26.167 | 26.331 | 0.145 | 38.682 | Undet. | 38.220 | 38.451 | 0.231 | 0.2 |
| 27589315 | 25.450 | 24.672 | 24.901 | 25.008 | 0.231 | Undet. | Undet. | 36.379 | 36.379 | | 0.2 |
| untreated | 24.029 | 24.992 | 23.594 | 24.205 | 0.413 | Undet. | Undet. | Undet. | | | |
| 28323984 | 24.654 | 26.129 | 25.638 | 25.474 | 0.434 | 34.091 | 36.059 | 35.084 | 35.078 | 0.568 | 0.7 |
| TSA | 24.014 | 24.850 | 24.240 | 24.368 | 0.250 | 33.284 | 33.710 | 33.332 | 33.442 | 0.135 | 1.0 |
| 28414061 | 23.654 | 24.116 | 24.593 | 24.121 | 0.271 | 35.522 | 33.277 | 33.701 | 34.167 | 0.689 | 0.5 |
| 38163983 | 24.587 | 24.897 | 24.382 | 24.622 | 0.150 | Undet. | Undet. | Undet. | | | |
| 28482285 | 23.943 | 23.882 | 24.218 | 24.014 | 0.104 | Undet. | Undet. | Undet. | | | |
| 38163970 | 24.030 | 24.092 | 24.096 | 24.073 | 0.021 | Undet. | Undet. | Undet. | | | |
| 27147406 | 24.134 | 24.401 | 25.040 | 24.525 | 0.269 | 35.747 | 36.206 | 36.608 | 36.187 | 0.249 | 0.2 |
| 30999670 | 24.431 | 24.134 | 23.965 | 24.177 | 0.136 | Undet. | Undet. | Undet. | | | |
| 27521052 | 24.472 | 24.650 | 25.097 | 24.740 | 0.186 | Undet. | Undet. | Undet. | | | |
| 27075607 | 24.055 | 24.648 | 23.970 | 24.224 | 0.213 | Undet. | Undet. | Undet. | | | |
| 27521533 | 24.063 | 23.796 | 24.721 | 24.193 | 0.275 | 33.516 | 33.076 | 33.208 | 33.267 | 0.130 | 1.0 |
| 27274260 | 24.185 | 23.504 | 23.583 | 23.757 | 0.215 | Undet. | Undet. | Undet. | | | |
| 27580709 | 24.897 | 24.378 | 25.523 | 24.933 | 0.331 | 35.794 | 33.983 | 39.014 | 36.264 | 1.471 | 0.2 |
| 27267292 | 23.461 | 23.517 | 24.181 | 23.720 | 0.231 | Undet. | Undet. | Undet. | | | |
| 28324010 | 25.433 | 25.572 | 25.040 | 25.348 | 0.159 | Undet. | Undet. | Undet. | | | |
| 27142765 | 25.437 | 24.838 | 24.863 | 25.046 | 0.196 | Undet. | Undet. | Undet. | | | |
| 28606656 | 25.074 | 24.740 | 24.497 | 24.770 | 0.167 | Undet. | Undet. | Undet. | | | |
| 27143272 | 24.408 | 23.986 | 24.879 | 24.424 | 0.258 | 35.276 | 33.385 | 37.026 | 35.229 | 1.051 | 0.3 |
| 27482559 | 23.841 | 24.003 | 23.997 | 23.947 | 0.053 | Undet. | Undet. | Undet. | | | |
| 27143246 | 25.112 | 24.525 | 24.891 | 24.843 | 0.171 | Undet. | Undet. | Undet. | | | |
| 27557374 | 23.926 | 23.923 | 24.054 | 23.968 | 0.043 | Undet. | Undet. | 37.020 | 37.020 | | 0.1 |
| 28635919 | 24.710 | 24.515 | 24.508 | 24.578 | 0.066 | 33.726 | 33.326 | 33.992 | 33.681 | 0.193 | 1.0 |
| 28439905 | 24.137 | 24.009 | 24.235 | 24.127 | 0.065 | Undet. | Undet. | Undet. | | | |
| 36722621 | 23.753 | 24.563 | 24.280 | 24.199 | 0.237 | Undet. | Undet. | Undet. | | | |
| 26142428 | 24.903 | 24.792 | 24.548 | 24.748 | 0.105 | Undet. | Undet. | Undet. | | | |
| 26933387 | 25.084 | 24.556 | 24.950 | 24.863 | 0.159 | Undet. | Undet. | Undet. | | | |
| 27131169 | 24.427 | 25.309 | 24.194 | 24.643 | 0.339 | Undet. | Undet. | Undet. | | | |
| 38168052 | 24.136 | 24.915 | 25.218 | 24.756 | 0.322 | Undet. | Undet. | Undet. | | | |
| 27143441 | 25.343 | 25.314 | 24.262 | 25.329 | 0.014 | Undet. | Undet. | Undet. | | | |
| 28553720 | 24.815 | 24.094 | 24.503 | 24.471 | 0.209 | Undet. | Undet. | Undet. | | | |
| 28658071 | 25.281 | 25.598 | 24.812 | 25.230 | 0.228 | 37.497 | 37.936 | Undet. | 37.716 | | 0.1 |
| untreated | 24.387 | 24.309 | 24.265 | 24.320 | 0.036 | Undet. | Undet. | Undet. | | | |
| 27244529 | 24.884 | 25.074 | 24.298 | 24.752 | 0.233 | Undet. | Undet. | Undet. | | | |
| TSA | 24.628 | 24.966 | 24.373 | 24.656 | 0.172 | 33.969 | 34.009 | 33.683 | 33.887 | 0.103 | 1.0 |
| 28571751 | 25.372 | 24.185 | 24.093 | 24.550 | 0.412 | Undet. | Undet. | Undet. | | | |
| 26909662 | 25.180 | 24.673 | 24.269 | 24.707 | 0.264 | Undet. | 36.055 | 35.932 | 35.993 | 0.061 | 0.2 |
| 28407340 | 25.068 | 25.649 | 25.037 | 25.251 | 0.199 | Undet. | Undet. | Undet. | | | |
| 26909675 | 26.118 | 24.533 | 24.626 | 25.092 | 0.513 | 37.641 | 34.960 | 33.868 | 35.489 | 1.121 | 0.4 |
| 28407054 | 23.938 | 24.905 | 25.185 | 24.676 | 0.378 | Undet. | Undet. | Undet. | | | |
| 27576770 | 24.828 | 25.195 | 24.257 | 24.760 | 0.273 | Undet. | Undet. | 38.592 | 38.592 | | 0.0 |
| 26949429 | 25.408 | 25.469 | 25.470 | 25.449 | 0.020 | Undet. | Undet. | 38.613 | 38.613 | | 0.1 |
| 37853842 | 24.449 | 24.559 | 24.622 | 24.543 | 0.050 | 35.513 | 35.069 | 35.341 | 35.308 | 0.129 | 0.3 |
| 27120171 | 25.299 | 23.545 | 23.554 | 24.133 | 0.583 | Undet. | Undet. | Undet. | | | |
| 27136902 | 24.747 | 23.931 | 23.997 | 24.225 | 0.262 | Undet. | Undet. | Undet. | | | |
| 27632683 | 24.762 | 25.095 | 23.968 | 24.608 | 0.334 | Undet. | Undet. | Undet. | | | |
| 27136681 | 25.169 | 24.614 | 24.131 | 24.638 | 0.300 | Undet. | Undet. | Undet. | | | |
| 27265004 | Undet. | 25.264 | 25.439 | 25.352 | 0.087 | Undet. | Undet. | Undet. | | | |
| 26905008 | 24.664 | 25.278 | 25.048 | 24.997 | 0.179 | Undet. | Undet. | Undet. | | | |
| 27418144 | Undet. | 24.947 | 26.084 | 25.516 | 0.569 | Undet. | Undet. | Undet. | | | |
| 27527552 | 24.880 | 25.556 | 23.546 | 24.661 | 0.590 | Undet. | Undet. | Undet. | | | |
| 27413854 | Undet. | 24.643 | 25.382 | 25.013 | 0.370 | Undet. | Undet. | Undet. | | | |
| 27556971 | 25.449 | 24.362 | 24.318 | 24.710 | 0.370 | Undet. | Undet. | Undet. | | | |
| 27418612 | 23.997 | 24.979 | 25.410 | 24.795 | 0.418 | Undet. | Undet. | Undet. | | | |
| 27632722 | 24.629 | 23.710 | 24.122 | 24.154 | 0.266 | Undet. | Undet. | Undet. | | | |
| 34661913 | 25.498 | 24.633 | 25.360 | 25.164 | 0.268 | Undet. | Undet. | Undet. | | | |
| 27392703 | 25.526 | 25.245 | 25.312 | 25.361 | 0.085 | Undet. | Undet. | Undet. | | | |
| 34661848 | 26.227 | 24.864 | 25.535 | 25.542 | 0.393 | Undet. | Undet. | Undet. | | | |
| 28841982 | 25.344 | 26.234 | 25.035 | 25.538 | 0.359 | Undet. | Undet. | Undet. | | | |
| 27478867 | 25.568 | 25.563 | 25.118 | 25.416 | 0.149 | 36.495 | 33.605 | 34.360 | 34.820 | 0.865 | 0.9 |
| 27392612 | 24.422 | 25.385 | 24.544 | 24.783 | 0.303 | Undet. | Undet. | Undet. | | | |
| 27059409 | 25.222 | 25.057 | 25.501 | 25.260 | 0.129 | 34.569 | 33.627 | 36.027 | 34.741 | 0.698 | 0.8 |
| 27418404 | 23.728 | 24.229 | 24.549 | 24.169 | 0.239 | Undet. | Undet. | Undet. | | | |

**TABLE XIII-B. CONFIRMATION OF THE EFFECT OF A 24 HOUR 5 µM HDAC INHIBITOR TREATMENT ON GENE EXPRESSION IN AN AMNIOTIC FLUID-DERIVED CELL LINE.**

| **Sample ID** | **GAPDH** | | | | | **PDX1** | | | | | **RQ** |
|---|---|---|---|---|---|---|---|---|---|---|---|
| | **replicate 1** | **replicate 2** | **replicate 3** | **Avg Ct** | **Ct std error** | **replicate 1** | **replicate 2** | **replicate 3** | **Avg Ct** | **Ct std error** | |
| 37398920 | 25.420 | 25.256 | 25.038 | 25.238 | 0.111 | 34.787 | 35.062 | 35.895 | 35.248 | 0.333 | 1.0 |
| 27142401 | 24.445 | 24.541 | 23.877 | 24.288 | 0.207 | 33.966 | 34.259 | 33.941 | 34.055 | 0.102 | 1.2 |
| 28540954 | 24.890 | 25.342 | 25.265 | 25.166 | 0.139 | 35.228 | 36.267 | 36.141 | 35.879 | 0.327 | 0.6 |
| 38171757 | 24.268 | 24.387 | 24.016 | 24.224 | 0.109 | 34.805 | 35.029 | 35.087 | 34.974 | 0.086 | 0.6 |
| 38153895 | 24.200 | 24.280 | 25.079 | 24.520 | 0.281 | 34.173 | 34.383 | 35.762 | 34.773 | 0.498 | 0.9 |
| 27587807 | 23.876 | 24.208 | 23.798 | 23.961 | 0.126 | 33.965 | 34.302 | 33.760 | 34.009 | 0.158 | 1.0 |
| 28641171 | 23.773 | 24.227 | 24.073 | 24.024 | 0.133 | 34.095 | 34.477 | 34.528 | 34.367 | 0.136 | 0.8 |
| 27418378 | 24.342 | 24.198 | 24.202 | 24.247 | 0.047 | 33.631 | 34.547 | 34.349 | 34.176 | 0.278 | 1.1 |
| 38134356 | 24.419 | 24.312 | 24.273 | 24.335 | 0.044 | 34.489 | 34.059 | 33.708 | 34.085 | 0.226 | 1.2 |
| 31017090 | 24.113 | 23.911 | 23.722 | 23.916 | 0.113 | 34.240 | 34.105 | 33.911 | 34.085 | 0.096 | 0.9 |
| 38134330 | 24.507 | 23.751 | 24.189 | 24.149 | 0.219 | 34.412 | 34.092 | 34.241 | 34.248 | 0.093 | 1.0 |
| 31034679 | 24.344 | 24.604 | 23.901 | 24.283 | 0.205 | 34.516 | 35.932 | 33.805 | 34.751 | 0.625 | 0.7 |
| 28571504 | 24.909 | 24.076 | 24.204 | 24.397 | 0.259 | 35.080 | 34.224 | 34.253 | 34.519 | 0.281 | 0.9 |
| untreated | 23.912 | 24.202 | 22.841 | 23.652 | 0.414 | Undet. | Undet. | Undet. | | | |
| 27142674 | 25.358 | 24.614 | 24.272 | 24.748 | 0.321 | 36.420 | 37.104 | 35.083 | 36.202 | 0.594 | 0.4 |
| TSA | 24.046 | 24.166 | 23.900 | 24.037 | 0.077 | 33.642 | 34.068 | 34.487 | 34.066 | 0.244 | 1.0 |
| 28543944 | 25.532 | 24.894 | 24.619 | 25.015 | 0.270 | 34.071 | 35.442 | 33.854 | 34.456 | 0.497 | 1.5 |
| 33297888 | 25.068 | 25.317 | 24.260 | 24.882 | 0.319 | 35.468 | 35.794 | 34.191 | 35.151 | 0.489 | 0.8 |
| 27652677 | 25.472 | 26.026 | 25.114 | 25.537 | 0.265 | 35.421 | 35.305 | 35.791 | 35.506 | 0.147 | 1.0 |
| 29784001 | 24.145 | 25.498 | 24.319 | 24.654 | 0.425 | 34.405 | 35.296 | 34.552 | 34.751 | 0.276 | 1.0 |
| 37791156 | 24.846 | 24.772 | 24.478 | 24.699 | 0.112 | 34.664 | 34.717 | 35.086 | 34.822 | 0.132 | 0.9 |
| 33300839 | 23.936 | 24.567 | 23.526 | 24.009 | 0.303 | 34.030 | 35.349 | 33.397 | 34.259 | 0.575 | 0.9 |
| 27027026 | 24.178 | 24.228 | 24.189 | 24.198 | 0.015 | 35.047 | 34.665 | 35.347 | 35.020 | 0.198 | 0.6 |
| 37653330 | 24.026 | 23.846 | 24.328 | 24.067 | 0.141 | 35.112 | 34.216 | 34.437 | 34.588 | 0.269 | 0.7 |
| 28296463 | 24.339 | 25.009 | 24.222 | 24.524 | 0.245 | 34.855 | 35.179 | 33.676 | 34.570 | 0.457 | 1.0 |
| 37804806 | 23.630 | 24.794 | 24.507 | 24.311 | 0.350 | 33.884 | 34.958 | 34.779 | 34.540 | 0.332 | 0.9 |
| 29232073 | 25.770 | 24.392 | 24.032 | 24.732 | 0.530 | 35.296 | 34.031 | 33.856 | 34.394 | 0.454 | 1.3 |
| 38117859 | 24.117 | 24.224 | 23.792 | 24.044 | 0.130 | 34.275 | 35.254 | 33.985 | 34.505 | 0.384 | 0.7 |
| 28549118 | 24.647 | 25.288 | 24.014 | 24.649 | 0.368 | 34.570 | 34.475 | 33.335 | 34.127 | 0.397 | 1.5 |
| 38134395 | 24.930 | 25.053 | 24.326 | 24.770 | 0.225 | 34.916 | 35.775 | 34.018 | 34.903 | 0.507 | 0.9 |
| 33300943 | 24.701 | 24.984 | 24.613 | 24.766 | 0.112 | 35.194 | 34.126 | 34.677 | 34.666 | 0.308 | 1.1 |
| 37398855 | 24.503 | 24.532 | 24.188 | 24.408 | 0.110 | 34.466 | 35.210 | 34.656 | 34.777 | 0.223 | 0.8 |
| 27477788 | 25.097 | 24.453 | 24.659 | 24.736 | 0.190 | 36.412 | 37.169 | 36.231 | 36.604 | 0.287 | 0.5 |
| 28978209 | 24.641 | 24.285 | 25.099 | 24.675 | 0.236 | 35.163 | 35.268 | 35.246 | 35.225 | 0.032 | 1.2 |
| 31056792 | 24.766 | 24.163 | 24.492 | 24.474 | 0.174 | 34.953 | 35.210 | 35.550 | 35.238 | 0.173 | 1.1 |
| 28338089 | 23.759 | 23.832 | 24.232 | 23.941 | 0.147 | 34.426 | 34.325 | 34.700 | 34.484 | 0.112 | 1.2 |
| 28404844 | 24.027 | 24.018 | 24.158 | 24.067 | 0.045 | 34.651 | 34.934 | 34.500 | 34.695 | 0.127 | 1.2 |
| 31040763 | 23.801 | 23.296 | 24.169 | 23.755 | 0.253 | 35.347 | 34.971 | 35.942 | 35.420 | 0.283 | 0.6 |
| 28507271 | 23.848 | 24.535 | 24.390 | 24.258 | 0.209 | 35.328 | 36.253 | 34.710 | 35.430 | 0.449 | 0.8 |
| 31040074 | 23.907 | 23.793 | 24.333 | 24.011 | 0.164 | 34.423 | 34.814 | 34.717 | 34.651 | 0.118 | 1.2 |
| 33155811 | 24.514 | 25.189 | 24.240 | 24.648 | 0.282 | 35.983 | 36.844 | 35.316 | 36.048 | 0.442 | 0.7 |
| 31040217 | 24.483 | 24.161 | 24.379 | 24.341 | 0.095 | 34.058 | 34.871 | 34.429 | 34.453 | 0.235 | 1.7 |
| 27054586 | 24.200 | 24.213 | 24.435 | 24.282 | 0.076 | Undet. | Undet. | Undet. | | | |
| 31053854 | 24.687 | 24.435 | 24.507 | 24.543 | 0.075 | 34.671 | 35.019 | 35.391 | 35.027 | 0.208 | 1.3 |
| 28107862 | 23.983 | 24.083 | 24.026 | 24.031 | 0.029 | 34.815 | 35.037 | 34.681 | 34.845 | 0.104 | 1.0 |
| 31056337 | 24.907 | 24.663 | 25.257 | 24.942 | 0.172 | 35.522 | 35.357 | 36.013 | 35.631 | 0.197 | 1.1 |
| 27587781 | 24.414 | 24.986 | 24.470 | 24.624 | 0.182 | 34.447 | 35.884 | 36.096 | 35.476 | 0.518 | 1.0 |
| 38107992 | 25.900 | 25.217 | 24.554 | 25.224 | 0.388 | 35.976 | 34.946 | 34.299 | 35.074 | 0.488 | 2.0 |
| 26949403 | 24.692 | 24.477 | 24.991 | 24.720 | 0,149 | 35.564 | 35.065 | 36.181 | 35.603 | 0.323 | 1.0 |
| 28631590 | 25.519 | 24.738 | 24.625 | 24.961 | 0.281 | 34.742 | 35.168 | 33.926 | 34.612 | 0.364 | 2.3 |
| 26949312 | 24.462 | 24.011 | 24.190 | 24.221 | 0.131 | 34.766 | 33.913 | 34.742 | 34.474 | 0.281 | 1.5 |
| 28635919 | 24.171 | 24.660 | 24.178 | 24.336 | 0.162 | 35.110 | 35.232 | 35.146 | 35.163 | 0.036 | 1.0 |
| TSA | 23.668 | 24.182 | 23.885 | 24.344 | 0.215 | 33.964 | 35.167 | 34.574 | 35.192 | 0.373 | 1.0 |
| TSA | 24.663 | 25.070 | 24.598 | 24.344 | 0.215 | 35.878 | 36.531 | 35.037 | 35.192 | 0.373 | 1.0 |
| 27521533 | 24.213 | 24.021 | 23.758 | 23.997 | 0.132 | 34.621 | 35.278 | 34.899 | 34.932 | 0.190 | 0.9 |
| untreated | 23.283 | 23.648 | 23.254 | 23.486 | 0.108 | Undet. | Undet. | | | | |
| untreated | 23.196 | 23.043 | 23.402 | 23.486 | 0.108 | Undet. | Undet. | Undet. | | | |
| untreated | Undet. | 24.249 | 23.703 | 23.486 | 0.108 | Undet. | Undet. | Undet. | | | |
| untreated | Undet. | 23.587 | 23.500 | 23.486 | 0.108 | | Undet. | Undet. | | | |
| 27478867 | 24.584 | 23.830 | 23.759 | 24.058 | 0.264 | 34.884 | 34.307 | 34.502 | 34.565 | 0.169 | 1.3 |
| 27059409 | 23.999 | 24.195 | 23.663 | 23.952 | 0.155 | 35.017 | 35.188 | 34.433 | 34.879 | 0.229 | 0.9 |
| 31051462 | 24.029 | 24.067 | 23.494 | 23.864 | 0.185 | 35.571 | 34.159 | 34.044 | 34.591 | 0.491 | 1.1 |
| 31046626 | 24.487 | 24.610 | 24.356 | 24.484 | 0.073 | 35.723 | 35.028 | 34.713 | 35.155 | 0.298 | 1.1 |

**TABLE XIII-C. EFFECTS OF A RANGE OF HDAC INHIBITOR CONCENTRATIONS ON PDX-1 GENE EXPRESSION IN AN ADULT PANCREATIC-DERIVED CELL LINE.**

| **Sample ID - [µM]** | **GAPDH** | | | | | **PDX1** | | | | | **RQ** |
|---|---|---|---|---|---|---|---|---|---|---|---|
| | **replicate 1** | **replicate 2** | **replicate 3** | **Avg Ct** | **Ct std error** | **replicate 1** | **replicate 2** | **replicate 3** | **Avg Ct** | **Ct std error** | |
| 38107992 - 10 | 25.854 | 26.295 | 25.885 | 26.012 | 0.142 | 34.811 | 35.401 | 35.570 | 35.261 | 0.230 | 29.7 |
| 38107992 - 5 | 26.793 | 26.371 | 25.049 | 26.071 | 0.525 | 35.651 | 35.655 | 35.354 | 35.553 | 0.100 | 25.3 |
| 38107992 - 2.5 | 25.028 | 24.963 | 25.887 | 25.293 | 0.298 | 34.518 | 34.498 | 35.615 | 34.877 | 0.369 | 23.6 |
| 38107992 - 1.25 | 24.753 | 24.509 | 24.937 | 24.733 | 0.124 | 35.203 | 34.412 | 35.291 | 34.969 | 0.279 | 15.0 |
| 38107992 - 0.625 | 26.371 | 24.940 | 24.678 | 25.330 | 0.526 | 35.141 | 34.417 | 34.300 | 34.620 | 0.263 | 28.9 |
| 38107992 - 0.3 | 26.148 | 26.441 | 26.342 | 26.311 | 0.086 | 35.099 | 36.077 | 37.448 | 36.208 | 0.681 | 19.0 |
| 38107992 - 0.15 | 25.871 | 26.539 | 26.269 | 26.227 | 0.194 | 36.197 | 38.658 | 37.448 | 37.434 | 0.710 | |
| TSA - 10 | 26.572 | 27.068 | 26.209 | 26.616 | 0.249 | 35.124 | 35.572 | 36.134 | 35.610 | 0.292 | 35.5 |
| TSA - 5 | 25.742 | 26.506 | 25.545 | 25.931 | 0.293 | 35.266 | 35.363 | 35.224 | 35.284 | 0.041 | 27.7 |
| TSA - 2.5 | 26.200 | 25.952 | 25.560 | 25.904 | 0.186 | 35.384 | 34.902 | 35.579 | 35.288 | 0.201 | 27.1 |
| TSA -1.25 | 25.632 | 25.667 | 25.592 | 25.630 | 0.022 | 36.420 | 34.667 | 36.585 | 35.890 | 0.613 | 14.8 |
| TSA - 0.625 | 26.069 | 26.049 | 26.170 | 26.096 | 0.037 | Undet. | Undet. | Undet. | | | |
| TSA - 0.3 | 25.510 | 25.856 | 25.253 | 25.539 | 0.175 | 39.154 | Undet. | Undet. | 39.154 | | 1.4 |
| TSA - 0.15 | 26.035 | 26.138 | 25.728 | 25.967 | 0.123 | Undet. | Undet. | Undet. | | 7.650 | |
| 0 | 26.049 | 26.028 | 25.849 | 25.618 | 0.205 | Undet. | 38.657 | Undet. | 38.657 | | 1.0 |
| 0 | 25.784 | 25.167 | 24.829 | 25.618 | 0.205 | Undet. | Undet. | Undet. | 38.657 | | 1.0 |
| 0 | 24.407 | 24.186 | 24.294 | 24.099 | 0.104 | Undet. | Undet. | Undet. | | | |
| 0 | 24.016 | 23.998 | 23.691 | 24.099 | 0.104 | Undet. | Undet. | Undet. | | | |
| 0 | 24.124 | 23.928 | 23.801 | 23.939 | 0.061 | Undet. | Undet. | Undet. | | | |
| 0 | 24.116 | 23.866 | 23.798 | 23.939 | 0.061 | Undet. | Undet. | Undet. | | | |
| 0 | 24.242 | 24.373 | Undet. | 23.772 | | 37.590 | 39.153 | Undet. | 38.417 | 0.489 | 1.0 |
| 0 | 23.378 | 23.623 | 23.243 | 23.772 | 0.228 | 39.369 | Undet. | 37.556 | 38.417 | 0.489 | 1.0 |
| 0 | 24.586 | 25.147 | 24.440 | 24.422 | 0.278 | 38.967 | Undet. | Undet. | 38.967 | | 1.0 |
| 0 | 23.601 | 23.640 | 25.115 | 24.422 | 0.278 | Undet. | Undet. | Undet. | 38.967 | | 1.0 |
| 31040217 - 10 | 25.132 | 24.790 | 25.542 | 25.155 | 0.217 | 36.209 | 36.919 | 37.671 | 36.933 | 0.422 | 5.2 |
| 31040217 - 5 | 24.547 | 24.930 | 24.847 | 24.775 | 0.117 | 35.345 | 36.324 | 36.883 | 36.184 | 0.449 | 6.7 |
| 31040217 - 2.5 | 23.785 | 24.335 | 25.267 | 24.462 | 0.433 | 35.688 | 35.971 | 36.523 | 36.061 | 0.245 | 5.8 |
| 31040217 - 1.25 | 23.522 | 23.924 | 24.384 | 23.944 | 0.249 | 35.989 | 36.590 | 35.831 | 36.137 | 0.231 | 3.9 |
| 31040217 - 0.625 | 24.268 | 24.709 | 24.875 | 24.618 | 0.181 | 35.589 | 37.628 | 36.186 | 36.468 | 0.605 | 4.9 |
| 31040217 - 0.3 | 24.443 | 24.789 | 24.625 | 24.619 | 0.100 | 37.833 | Undet. | 39.120 | 38.476 | 0.644 | 1.2 |
| 31040217 - 0,15 | 25.150 | 25.011 | 25.313 | 25.158 | 0.087 | Undet. | 38.691 | 38.734 | 38.712 | 0.022 | 1.5 |
| 31053854 - 10 | 24.777 | 24.193 | 24.641 | 24.537 | 0.176 | Undet. | Undet. | Undet. | | | |
| 31053854 - 5 | 24.637 | 24.385 | 24.691 | 24.571 | 0.094 | Undet. | Undet. | Undet. | | | |
| 31053854 - 2.5 | 24.051 | 24.396 | 24.033 | 24.160 | 0.118 | Undet. | Undet. | Undet. | | | |
| 31053854 - 1.25 | 23.994 | 23.713 | 24.133 | 23.947 | 0.123 | Undet. | Undet. | Undet. | | | |
| 31053854 - 0.625 | 24.437 | 24.220 | 23.870 | 24.175 | 0.165 | Undet. | Undet. | Undet. | | | |
| 31053854 - 0.3 | 31.550 | 24.336 | 24.094 | 26.660 | 2.446 | Undet. | Undet. | Undet. | | | |
| 31053854 - 0.15 | 23.928 | 24.158 | 23.990 | 24.025 | 0.069 | Undet. | Undet. | Undet. | | | |
| 27652677 - 10 | 25.250 | 24.378 | 25.188 | 24.939 | 0.281 | 34.816 | 34.818 | 35.259 | 34.964 | 0.148 | 17.4 |
| 27652677 - 5 | 24.566 | 25.182 | 25.147 | 24.965 | 0.200 | 36.169 | 36.202 | 35.943 | 36.105 | 0.082 | 8.0 |
| 27652677 - 2.5 | 23.947 | 24.313 | 24.718 | 24.326 | 0.223 | Undet. | 37.575 | 38.262 | 37.918 | 0.343 | 1.5 |
| 27652677 -1.25 | 23.626 | 23.432 | 24.218 | 23.759 | 0.237 | Undet. | Undet. | Undet. | | | |
| 27652677 - 0.625 | 24.175 | 24.157 | 24.390 | 24.241 | 0.075 | Undet. | Undet. | Undet. | | | |
| 27652677 - 0.3 | 24.168 | 24.024 | 24.093 | 24.095 | 0.041 | 38.126 | Undet. | Undet. | 38.126 | | 1.1 |
| 27652677 - 0.15 | 24.083 | 24.150 | 24.209 | 24.148 | 0.036 | Undet. | Undet. | Undet. | | | |
| 28404844 - 10 | 24.871 | 24.460 | 24.391 | 24.574 | 0.150 | 36.439 | 35.815 | 35.287 | 35.847 | 0.333 | 7.3 |
| 28404844-5 | 24.313 | 24.845 | 24.575 | 24.578 | 0.154 | 35.147 | 35.902 | 35.578 | 35.542 | 0.219 | 9.1 |
| 28404844 - 2.5 | 24.669 | 24.641 | 24.502 | 24.604 | 0.052 | 34.490 | 35.225 | 35.445 | 35.053 | 0.289 | 12.9 |
| 28404844 - 1.25 | 25.192 | 24.764 | 24.583 | 24.846 | 0.181 | 35.589 | 35.070 | 34.908 | 35.189 | 0.205 | 13.9 |
| 28404844 - 0.625 | 23.716 | 24.349 | 23.709 | 23.925 | 0.212 | 34.875 | 34.681 | 35.078 | 34.878 | 0.114 | 9.1 |
| 28404844 - 0.3 | 24.314 | 24.294 | 23.777 | 24.128 | 0.176 | 36.174 | 35.414 | 35.560 | 35.716 | 0.233 | 5.9 |
| 28404844 - 0.15 | 23.887 | 24.220 | 24.537 | 24.215 | 0.188 | 37.444 | 36.090 | Undet. | 36.767 | | 3.0 |
| 26949312 - 10 | 24.120 | 24.568 | 23.828 | 24.172 | 0.215 | 35.274 | 35.372 | 39.769 | 36.805 | 1.483 | 2.8 |
| 26949312 - 5 | 25.533 | 24.509 | 23.791 | 24.611 | 0.506 | 35.363 | 35.306 | 35.734 | 35.467 | 0.134 | 9.8 |
| 26949312 - 2.5 | 24.709 | 25.296 | 23.902 | 24.636 | 0.404 | 35.370 | 35.367 | 36.890 | 35.876 | 0.507 | 7.5 |
| 26949312 - 1.25 | 23.660 | 23.190 | 24.193 | 23.681 | 0.290 | 35.789 | 34.936 | 36.215 | 35.647 | 0.376 | 4.5 |
| 26949312 - 0.625 | 24.276 | 23.284 | 24.147 | 23.902 | 0.311 | 38.198 | 36.099 | 37.896 | 37.398 | 0.655 | 1.6 |
| 26949312 - 0.3 | 23.439 | 24.406 | 23.351 | 23.732 | 0.338 | 37.138 | 39.249 | 38.950 | 38.446 | 0.659 | 0.7 |
| 26949312 - 0.15 | 24.174 | 24.056 | 23.536 | 23.922 | 0.196 | 38.356 | Undet. | 38.433 | 38.394 | 0.039 | 0.8 |
| 28543944 - 10 | 25.306 | 26.435 | 24.692 | 25.477 | 0.510 | 35.410 | 37.512 | 35.734 | 36.219 | 0.653 | 10.6 |
| 28543944 - 5 | 24.657 | 24.080 | 25.402 | 24.713 | 0.383 | 34.878 | 35.863 | 36.609 | 35.783 | 0.501 | 8.4 |
| 28543944 - 2.5 | 23.736 | 24.072 | 25.806 | 24.538 | 0.642 | 34.559 | 35.559 | 37.287 | 35.802 | 0.797 | 7.4 |
| 28543944 - 1.25 | 23.897 | 23.331 | 24.122 | 23.783 | 0.235 | 35.466 | 35.389 | 36.261 | 35.705 | 0.279 | 4.7 |
| 28543944 - 0.625 | 24.217 | 24.081 | 24.581 | 24.293 | 0.149 | 36.082 | 38.200 | 37.353 | 37.212 | 0.615 | 2.3 |
| 28543944 - 0.3 | 24.994 | 24.387 | 26.001 | 25.127 | 0.471 | 38.644 | 38.248 | Undet. | 38.446 | | 1.8 |
| 28543944 - 0.15 | 25.266 | 24.555 | 24.912 | 24.911 | 0.205 | Undet. | Undet. | 39.187 | 39.187 | | 0.9 |
| 28631590 - 10 | 24.737 | 24.142 | 24.856 | 24.578 | 0.221 | 35.810 | 35.755 | 36.284 | 35.950 | 0.168 | 6.8 |
| 28631590 - 5 | 24.973 | 24.748 | 24.992 | 24.904 | 0.078 | 36.229 | 36.065 | 36.230 | 36.174 | 0.055 | 7.3 |
| 28631590 - 2.5 | 25.338 | 24.195 | 24.584 | 24.705 | 0.336 | 35.048 | 34.434 | 37.759 | 35.747 | 1.022 | 8.6 |
| 28631590-1.25 | 24.314 | 24.110 | 23.892 | 24.106 | 0.122 | 34.385 | 34.392 | 34.731 | 34.503 | 0.114 | 13.4 |
| 28631590 - 0.625 | 24.319 | 24.150 | 23.813 | 24.094 | 0.149 | 35.683 | 35.838 | 36.587 | 36.036 | 0.279 | 4.6 |
| 28631590 - 0.3 | 24.518 | 24.526 | 25.279 | 24.774 | 0.252 | 38.616 | Undet. | Undet. | 38.616 | | 1.2 |
| 28631590 - 0.15 | 25.046 | 25.180 | 24.530 | 24.919 | 0.198 | Undet. | Undet. | 38.393 | 38.393 | | 1.6 |

**TABLE XIII-D. EFFECTS OF A RANGE OF HDAC INHIBITOR CONCENTRATIONS ON PDX-1 GENE EXPRESSION IN AN AMNIOTIC FLUID-DERIVED CELL LINE.**

| **Sample ID** | **GAPDH** | | | | | **PDX1** | | | | | **RQ** |
|---|---|---|---|---|---|---|---|---|---|---|---|
| | **replicate** | **1 replicate 2** | **replicate** | **3 Avg Ct** | **Ct std error** | **replicate 1** | **replicate** | **2 replicate 3** | **Avg Ct** | **Ct std error** | |
| 26949312-10 | 26.448 | 25.057 | 26.003 | 25.836 | 0.41 | 34.880 | 34.463 | 34.407 | 34.583 | 0.149 | 1.5 |
| 26949312-5 | 26.255 | 26.023 | 25.642 | 25.973 | 0.179 | 34.994 | 35.507 | 35.068 | 35.190 | 0.160 | 1.7 |
| 26949312-2.5 | 25.467 | 25.567 | 25.04 | 25.358 | 0.162 | 34.855 | 34.585 | 34.175 | 34.538 | 0.198 | 2.5 |
| 26949312-1.25 | 25.255 | 25.919 | 25.182 | 25.452 | 0.235 | 35.501 | 35.418 | 36.239 | 35.719 | 0.261 | 1.5 |
| 26949312-0.6 | 25.894 | 24.872 | 25.982 | 25.582 | 0.356 | 36.874 | 37.669 | 37.358 | 37.301 | 0.231 | 3.5 |
| 26949312-0.3 | 25.766 | 26.482 | 26.42 | 26.223 | 0.229 | Undet. | Undet. | Undet. | | | |
| 26949312-0.15 | 26.148 | 25.651 | 26.007 | 25.935 | 0.148 | 39.610 | Undet. | Undet. | 39.610 | | |
| untreated | 25.155 | 25.282 | 24.491 | 25.012 | 0.177 | Undet. | Undet. | Undet. | | | |
| untreated | 24.518 | 25.032 | 25.591 | 25.012 | 0.177 | Undet. | Undet. | Undet. | | | |
| untreated | 23.971 | 23.902 | 23.626 | 23.713 | 0.13 | Undet. | Undet. | Undet. | | | |
| untreated | 23.343 | 24.079 | 23.356 | 23.713 | 0.13 | Undet. | Undet. | Undet. | | | |
| untreated | 23.665 | 23.605 | 23.351 | 23.54 | 0.096 | Undet. | Undet. | Undet. | | | |
| untreated | 22.711 | 24.132 | 23.102 | 23.157 | 0.228 | Undet. | Undet. | Undet. | | | |
| untreated | 22.619 | 23.429 | 22.946 | 23.157 | 0.228 | Undet. | Undet. | Undet. | | | |
| untreated | 25.115 | 24.958 | 24.442 | 24.948 | 0.142 | Undet. | Undet. | Undet. | | | |
| untreated | 24.642 | 25.386 | 25.145 | 24.948 | 0.142 | Undet. | Undet. | Undet. | | | |
| untreated | 24.434 | 23.244 | 24.613 | 24.293 | 0.211 | Undet. | Undet. | Undet. | | | |
| untreated | 24.494 | 24.471 | 24.5 | 24.293 | 0.211 | Undet. | Undet. | Undet. | | | |
| 28543944-10 | 24.996 | 25.183 | 25.384 | 25.187 | 0.112 | 34.373 | 34.171 | 34.239 | 34.261 | 0.059 | 1.2 |
| 28543944-5 | 25.587 | 25.215 | 24.7 | 25.167 | 0.257 | 34.541 | 34.324 | 33.963 | 34.276 | 0.169 | 1.8 |
| 28543944-2.5 | 24.629 | 25.097 | 24.898 | 24.874 | 0.136 | 34.987 | 34.578 | 34.544 | 34.703 | 0.142 | 1.6 |
| 28543944-1.25 | 24.503. | 24.213 | 24.673 | 24.463 | 0.134 | 34.645 | 34.463 | 35.010 | 34.706 | 0.161 | 1.6 |
| 28543944-0.6 | 24.818 | 25.068 | 25.269 | 25.052 | 0.13 | 35.881 | 36.647 | 37.432 | 36.654 | 0.448 | 3.8 |
| 28543944-0.3 | 25.534 | 24.953 | 25.368 | 25.285 | 0.173 | 37.046 | Undet. | 37.161 | 37.104 | 0.058 | 10.8 |
| 28543944-0.15 | 24.912 | 25.273 | 24.778 | 24.988 | 0.148 | Undet. | Undet. | 39.134 | 39.134 | | |
| 28631590-10 | 25.828 | 25.244 | 24.508 | 25.193 | 0.382 | 35.365 | 34.330 | 34.445 | 34.713 | 0.328 | 0.9 |
| 28631590-5 | 24.97 | 25.007 | 24.542 | 24.84 | 0.149 | 33.904 | 33.720 | 34.243 | 33.956 | 0.153 | 1.8 |
| 28631590-2.5 | 24.636 | 24.346 | 24.129 | 24.37 | 0.147 | 34.082 | 34.456 | 34.056 | 34.198 | 0.129 | 1.6 |
| 28631590-1.25 | 24.807 | 24.817 | 24.211 | 24.612 | 0.2 | 34.608 | 34.245 | 34.213 | 34.355 | 0.127 | 2.5 |
| 28631590-0.6 | 24.806 | 24.792 | 25.032 | 24.877 | 0.078 | 34.402 | 35.116 | 35.208 | 34.909 | 0.255 | 11.4 |
| 28631590-0.3 | 25.014 | 24.86 | 24.97 | 24.948 | 0.046 | 37.170 | 37.587 | 38.313 | 37.690 | 0.334 | 5.7 |
| 28631590-0.15 | 24.975 | 24.384 | 24.603 | 24.654 | 0.172 | Undet. | 38.059 | 38.341 | 38.200 | 0.141 | 4.8 |
| TSA 10 | 24.088 | 24.18 | 23.943 | 24.07 | 0.069 | 33.220 | 33.509 | 33.418 | 33.383 | 0.085 | |
| TSA 5 | 24.181 | 23.869 | 23.934 | 23.995 | 0.095 | 34.219 | 33.503 | 34.193 | 33.972 | 0.234 | |
| TSA 2.5 | 23.809 | 23.453 | 23.244 | 23.502 | 0.165 | 34.160 | 34.073 | 33.853 | 34.029 | 0.091 | |
| TSA 1.25 | 23.38 | 23.538 | 22.972 | 23.297 | 0.169 | 33.428 | 34.285 | 34.823 | 34.179 | 0.406 | |
| TSA 0.6 | 23.708 | 23.509 | 23.369 | 23.529 | 0.098 | 35.218 | 37.464 | 38.525 | 37.069 | 0.975 | |
| TSA 0.3 | 23.243 | 23.353 | 23.162 | 23.252 | 0.055 | 38.481 | 37.619 | 39.413 | 38.504 | 0.518 | |
| TSA 0.15 | 23.757 | 23.344 | 23.329 | 23.476 | 0.14 | 39.691 | 38.881 | Undet. | 39.286 | | |
| 31053854-10 | 23.95 | 24.213 | 24.164 | 24.109 | 0.081 | 34.456 | 34.060 | 35.137 | 34.551 | 0.315 | 0.5 |
| 31053854-5 | 24.213 | 23.982 | 23.683 | 23.959 | 0.153 | 33.752 | 34.593 | 35.331 | 34.559 | 0.456 | 0.6 |
| 31053854-2.5 | 23.458 | 23.25 | 23.656 | 23.455 | 0.117 | 35.529 | 37.419 | 37.660 | 36.869 | 0.674 | 0.1 |
| 31053854-1.25 | 23.12 | 23.307 | 23.179 | 23.202 | 0.055 | 38.802 | Undet. | Undet. | 38.802 | | |
| 31053854-0.6 | 23.538 | 23.383 | 23.064 | 23.328 | 0.14 | 38.685 | Undet. | Undet. | 38.685 | | |
| 31053854-0.3 | 23.615 | 23.955 | 23.548 | 23.706 | 0.126 | Undet. | Undet. | Undet. | | | |
| 31053854-0.15 | 23.692 | 23.374 | 23.677 | 23.581 | 0.104 | Undet. | Undet. | 38.037 | 38.037 | | |
| 38107992-10 | 23.412 | 23.343 | 23.425 | 23.393 | 0.025 | 34.350 | 34.777 | 34.222 | 34.450 | 0.168 | 0.3 |
| 38107992-5 | 23.572 | 23.435 | 23.575 | 23.527 | 0.046 | 34.937 | 35.065 | 35.059 | 35.020 | 0.042 | 0.3 |
| 38107992-2.5 | 24.128 | 23.576 | 23.108 | 23.604 | 0.295 | 34.948 | 34.541 | 34.008 | 34.499 | 0.272 | 0.8 |
| 38107992-1.25 | 23.583 | 23.279 | 23.341 | 23.401 | 0.093 | 34.621 | 34.064 | 33.672 | 34.119 | 0.275 | 1.1 |
| 38107992-0.6 | 23.62 | 23.347 | 23.752 | 23.573 | 0.119 | 35.001 | 33.830 | 34.246 | 34.359 | 0.343 | 6.7 |
| 38107992-0.3 | 23.552 | 23.329 | 23.828 | 23.569 | 0.144 | 34.862 | 34.486 | 35.081 | 34.810 | 0.174 | 16.1 |
| 38107992-0.15 | 24.006 | 23.779 | 23.658 | 23.814 | 0.102 | 35.970 | 36.013 | 36.826 | 36.270 | 0.278 | 10.2 |
| 31040074-6.25 | 26.192 | 25.514 | 25.223 | 25.643 | 0.287 | 34.777 | 34.683 | 34.357 | 34.605 | 0.127 | 1.3 |
| 31040074-3.1 | 25.313 | 25.833 | 25.942 | 25.696 | 0.194 | 33.880 | 34.662 | 34.821 | 34.454 | 0.291 | 2.3 |
| 31040074-1.56 | 24.947 | 25.173 | 25.52 | 25.213 | 0.166 | 34.942 | 34.885 | 35.638 | 35.155 | 0.242 | 1.5 |
| 31040074-0.78 | 24.609 | 24.91 | 25.24 | 24.919 | 0.182 | 34.957 | 34.464 | 35.647 | 35.023 | 0.343 | 1.7 |
| 31040074-0.39 | 25.807 | 25.252 | 25.518 | 25.526 | 0.16 | 35.811 | 36.152 | 38.097 | 36.687 | 0.712 | 5.2 |
| 31040074-0.2 | 25.462 | 25.424 | 25.405 | 25.43 | 0.017 | 38.438 | Undet. | Undet. | 38.438 | | |
| 31040074-0.1 | 25.444 | 25.12 | 25.157 | 25.24 | 0.102 | Undet. | Undet. | Undet. | | | |
| 31040217-10 | 25.645 | 26.643 | 25.023 | 25.771 | 0.472 | 34.905 | 36.254 | 34.118 | 35.092 | 0.624 | 1.0 |
| 31040217-5 | 24.899 | 25.387 | 25.103 | 25.129 | 0.141 | 34.277 | 34.507 | 35.221 | 34.669 | 0.284 | 1.4 |
| 31040217-2.5 | 24.928 | 25.136 | 25.108 | 25.057 | 0.065 | 34.061 | 34.010 | 33.576 | 33.882 | 0.154 | 3.3 |
| 31040217-1.25 | 25.737 | 25.45 | 25.269 | 25.485 | 0.136 | 35.123 | 34.764 | 35.203 | 35.030 | 0.135 | 2.5 |
| 31040217-0.6 | 25.143 | 25.711 | 25.642 | 25.499 | 0.179 | 35.127 | 35.967 | 35.948 | 35.681 | 0.277 | 8.5 |
| 31040217-0.3 | 25.342 | 25.486 | 25.586 | 25.471 | 0.071 | 37.404 | 38.012 | 37.484 | 37.633 | 0.191 | 8.5 |
| 31040217-0.15 | 25.374 | 25.842 | 24.962 | 25.393 | 0.254 | 38.193 | Undet. | 38.627 | 38.410 | 0.217 | 6.9 |
| 27652677-10 | 25.094 | 25.655 | 24.988 | 25.246 | 0.207 | 34.189 | 34.889 | 34.775 | 34.618 | 0.217 | 1.0 |
| 27652677-5 | 25.306 | 25.138 | 24.962 | 25.135 | 0.099 | 34.722 | 34.870 | 35.005 | 34.866 | 0.082 | 1.2 |
| 27652677-2.5 | 24.583 | 24.722 | 24.091 | 24.465 | 0.192 | 36.261 | 36.455 | 35.189 | 35.969 | 0.394 | 0.5 |
| 27652677-1.25 | 25.446 | 24.477 | 24.751 | 24.891 | 0.288 | Undet. | 36.790 | 39.614 | 38.202 | 1.412 | 0.2 |
| 27652677-0.6 | 25.024 | 25.316 | 24.827 | 25.056 | 0.142 | Undet. | Undet. | Undet. | | | |
| 27652677-0.3 | 24.997 | 25.472 | 24.259 | 24.909 | 0.353 | Undet. | Undet. | Undet. | | | |
| 27652677-0.15 | 25.428 | 24.982 | 24.833 | 25.081 | 0.179 | Undet. | Undet. | Undet. | | | |
| 28404844-10 | 24.37 | 24.307 | 25.196 | 24.624 | 0.287 | 34.508 | 33.938 | 34.491 | 34.313 | 0.187 | 0.8 |
| 28404844-5 | 25.704 | 24.338 | 24.919 | 24.987 | 0.396 | 35.323 | 34.233 | 34.390 | 34.649 | 0.340 | 1.2 |
| 28404844-2.5 | 24.817 | 25.298 | 24.498 | 24.871 | 0.232 | 35.107 | 34.032 | 35.117 | 34.752 | 0.360 | 1.2 |
| 28404844-1.25 | 24.805 | 25.06 | 24.541 | 24.802 | 0.15 | 35.403 | 37.184 | 34.947 | 35.845 | 0.682 | 0.9 |
| 28404844-0.6 | 24.478 | 25.252 | 25,057 | 24.929 | 0.233 | 36.445 | 36.404 | 35.967 | 36.272 | 0.153 | 4.6 |
| 28404844-0.3 | 25.166 | 24.565 | 24.399 | 24.71 | 0.233 | Undet. | 38.756 | 38.892 | 38.824 | 0.068 | 2.2 |
| 28404844-0.15 | 24.838 | 25.498 | 25.135 | 25.157 | 0.191 | 38.107 | Undet. | Undet. | 38.107 | | |

**TABLE XIV. EFFECTS OF A HDAC INHIBITOR AND MG-132 TREATMENT ON GENE EXPRESSION IN AN ADULT PANCREAS-DERIVED CELL LINE.**

| **H5F3P19 Cell Treatment** | **Pdx1 Sample Ct** | **Pancreas Pdx1 Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated cells | - | 29.144 | 22.091 | 27.162 | - | - | - | |
| medium treated control cells | - | 28.628 | 20.249 | 25.471 | - | - | - | |
| 1 uM MG132 for 24 hrs | - | 29.144 | 21.634 | 27.162 | - | - | - | |
| 10uM MG132 for 24 hrs | - | 29.144 | 22.384 | 27.162 | - | - | - | |
| 1.25uM TSA for 24 hrs | 34.557 | 28.628 | 21.045 | 25.471 | 10.355 | 0.001 | 0.076 | 0.108 |
| 2.5uM TSA for 24 hrs | 32.624 | 28.628 | 20.438 | 25.471 | 9.029 | 0.002 | 0.191 | 0.162 |
| 1 uM MG132 for 24 hrs followed by 1.25uM TSA for 24 hrs | 32.744 | 28.628 | 20.788 | 25.471 | 8.799 | 0.002 | | 0.225 0.258 |
| 1 uM MG1 32 for 24 hrs followed by 2.5uM TSA for 24 hrs | 32.316 | 28.628 | 20.784 | 25.471 | 8.375 | 0.003 | 0.301 | 0.143 |
| 10uM MG132 for 24 hrs followed by 1.25uM TSA for 24 hrs | 32.019 | 28.628 | 22.355 | 25.471 | 6.507 | 0.011 | 1.100 | 0.034 |
| 10uM MG132 for 24 hrs followed by 2.5uM TSA for 24 hrs | 31.582 | 28.628 | 22.272 | 25.471 | 6.153 | 0.014 | 1.405 | 0.077 |

| **H5F3P19 Cell Treatment** | **HNF3-β Sample Ct** | **Pancreas HNF3-β Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated cells | - | 27.251 | 22.091 | 27.162 | - | - | - | |
| medium treated control cells | - | 27.622 | 20.249 | 25.471 | - | - | - | |
| 1uM MG132 for 24 hrs | - | 27.251 | 21.634 | 27.162 | - | - | - | |
| 10uM MG132 for 24 hrs | - | 27.251 | 22.384 | 27.162 | - | - | - | |
| 1.25uM TSA for 24 hrs | 34.069 | 27.622 | 21.045 | 25.471 | 10.873 | 0.001 | 0.053 | 0.111 |
| 2.5uM TSA for 24 hrs | 32.883 | 27.622 | 20.438 | 25.471 | 10.294 | 0.001 | 0.080 | 0.170 |
| 1uM MG132 for 24 hrs followed by 1.25uM TSA for 24 hrs | 33.778 | 27.622 | 20.788 | 25.471 | 10.839 | 0.001 | 0.055 | 0.219 |
| 1 uM MG132 for 24 hrs followed by 2.5uM TSA for 24 hrs | 33.429 | 27.622 | 20.784 | 25.471 | 10.494 | 0.001 | 0.069 | 0.213 |
| 10uM MG132 for 24 hrs followed by 1.25uM TSA for 24 hrs | 31.678 | 27.622 | 22.355 | 25.471 | 7.172 | 0.007 | 0.693 | 0.078 |
| 10uM MG132 for 24 hrs followed by 2.5uM TSA for 24 hrs | 31.183 | 27.622 | 22.272 | 25.471 | 6.760 | 0.009 | 0.923 | 0.136 |

| **H5F3P19 Cell Treatment** | **Insulin Sample Ct** | **Pancreas Insulin Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated cells | - | 18.282 | 22.091 | 27.162 | - | - | - | - |
| medium treated control cells | - | 18.011 | 20.249 | 25.471 | - | - | - | - |
| 1uM MG132 for 24 hrs | - | 18.282 | 21.634 | 27.162 | - | - | - | - |
| 10uM MG132 for 24 hrs | - | 18.282 | 22.384 | 27.162 | - | - | - | - |
| 1.25uM TSA for 24 hrs | - | 18.011 | 21.045 | 25.471 | - | - | - | - |
| 2.5uM TSA for 24 hrs | - | 18.011 | 20.438 | 25.471 | - | - | - | - |
| 1uM MG132 for 24 hrs followed by 1.25uM TSA for 24 hrs | - | 18.011 | 20.788 | 25.471 | - | - | - | - |
| 1 uM MG132 for 24 hrs followed by 2.5uM TSA for 24 hrs | - | 18.011 | 20.784 | 25.471 | - | - | - | - |
| 10uM MG132 for 24 hrs followed by 1.25uM TSA for 24 hrs | - | 18.011 | 22.355 | 25.471 | - | - | - | - |
| 10uM MG132 for 24 hrs followed by 2.5uM TSA for 24 hrs | - | 18.011 | 22.272 | 25.471 | - | - | - | - |

| **H5F3P19 Cell Treatment** | **NKX6.1 Sample Ct** | **Pancreas NKX6.1 Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated cells | - | 29.679 | 22.091 | 27.162 | - | - | - | |
| medium treated control cells | - | 30.161 | 20.249. | 25.471 | - | - | - | |
| 1uM MG132 for 24 hrs | - | 29.679 | 21.634 | 27.162 | - | - | - | - |
| 10uM MG132 for 24 hrs | 36.281 | 29.679 | 22.384 | 27.162 | 11.380 | 0.000 | 0.038 | 0.211 |
| 1.25uM TSA for 24 hrs | 30.621 | 30.161 | 21.045 | 25.471 | 4.886 | 0.034 | 3.382 | 0.110 |
| 2.5uM TSA for 24 hrs | 29.323 | 30.161 | 20.438 | 25.471 | 4.195 | 0.055 | 5.460 | 0.113 |
| 1 uM MG132 for 24 hrs followed by 1.25uM TSA for 24 hrs | 29.718 | 30.161 | 20.788 | 25.471 | 4.240 | 0.053 | 5.292 | 0.080 |
| 1uM MG132 for 24 hrs followed by 2.5uM TSA for 24 hrs | 29.122 | 30.161 | 20.784 | 25.471 | 3.648 | 0.080 | 7.977 | 0.191 |
| 10uM MG132 for 24 hrs followed by 1.25uM TSA for 24 hrs | 29.704 | 30.161 | 22.355 | 25.471 | 2.659 | 0.158 | 15.833 | 0.017 |
| 10uM MG132 for 24 hrs followed by 2.5uM TSA for 24 hrs | 29.410 | 30.161 | 22.272 | 25.471 | 2.448 | 0.183 | 18.326 | 0.019 |

**TABLE XV. EFFECTS OF A HDAC INHIBITOR AND MG-132 TREATMENT ON GENE EXPRESSION IN AN ADULT PANCREAS-DERIVED CELL LINE.**

| **H5F3P9 Cell Treatment: MG132 followed by TSA** | **Pdx1 Sample Ct** | **Pdx1 Pancreas Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated control | - | 28.660 | 24.340 | 28.950 | - | - | - | - |
| 1 uM MG132 for 24 hrs | - | 28.660 | 23.680 | 28.950 | - | - | - | - |
| 10uM MG132 for 24 hrs | - | 28.660 | 23.680 | 28.950 | - | - | - | - |
| 2% DMEM for 24 hrs followed by 1.25uM TSA for 24 hrs | 35.010 | 28.660 | 23.690 | 28.950 | 11.610 | 0.000 | 0.032 | 0.100 |
| 2% DMEM for 24 hrs followed by 2.5uM TSA for 24 hrs | 34.060 | 28.660 | 23.780 | 28.950 | 10.570 | 0.001 | 0.066 | 0.200 |
| 1 uM MG132 for 24 hrs followed by 1.25uM TSA for 24 hrs | 31.770 | 28.660 | 24.040 | 28.950 | 8.020 | 0.004 | 0.385 | 0.130 |
| 1uM MG132 for 24 hrs followed by 2.5uM TSA for 24 hrs | 33.240 | 28.660 | 23.800 | 28.950 | 9.730 | 0.001 | 0.118 | 0.170 |
| 10uM MG132 for 24 hrs followed by 1.25uM TSA for 24 hrs | 32.120 | 28.660 | 25.880 | 28.950 | 6.530 | 0.011 | 1.082 | 0.400 |
| 10uM MG132 for 24 hrs followed by 2.5uM TSA for 24 hrs | 32.100 | 28.660 | 24.370 | 28.950 | 8.020 | 0.004 | 0.385 | 0.110 |

| **H5F3P9 Cell Treatment: TSA followed by MG132** | **Pdx1 Sample Ct** | **Pdx1 Pancreas Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated control | - | 28.660 | 22.680 | 28.950 | - | - | - | - |
| 1.25uM TSA for 24 hr | 34.940 | 28.660 | 22.900 | 28.950 | 12.330 | 0.000 | 0.019 | 0.260 |
| 2.5uM TSA for 24 hr | 34.940 | 28.660 | 24.310 | 28.950 | 10.920 | 0.001 | 0.052 | 0.060 |
| 2%DMEM for 24 hrs followed by 1uM MG132 for 24 hrs | - | 28.660 | 25.400 | 28.950 | - | - | - | - |
| 2%DMEM for 24 hrs followed by 10uM MG132 for 24 hrs | - | 28.660 | 24.070 | 28.950 | - | - | - | - |
| 1.25uM TSA for 24 hrs followed by 1uM MG132 for 24 hrs | - | 28.660 | 24.120 | 28.950 | - | - | - | - |
| 1.25uM TSA for 24 hrs followed by 10uM MG132 for 24 hrs | - | 28.660 | 24.250 | 28.950 | - | - | - | - |
| 2.5uM TSA for 24 hrs followed by 1uM MG132 for 24 hrs | - | 28.660 | 24.290 | 28.950 | - | - | - | - |
| 2.5uM TSA for 24 hrs followed by 10uM MG132 for 24 hrs | - | 28.660 | 26.670 | 28.950 | - | - | - | - |
| | | | | | | | | |

| **H5F3P9 Cell Treatment: MG132 followed by TSA** | **HNF3-β Sample Ct** | **HNF3-β Pancreas Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated control | - | 26.720 | 24.340 | 28.950 | - | - | - | - |
| 1uM MG132 for 24 hrs | 34.030 | 26.720 | 23.680 | 28.950 | 12.580 | 0.000 | 0.016 | 0.190 |
| 10uM MG132 for 24 hrs | 34.550 | 26.720 | 23.680 | 28.950 | 13.100 | 0.000 | 0.011 | 0.090 |
| 2% DMEM for 24 hrs followed by 1.25uM TSA for 24 hrs | 35.140 | 26.720 | 23.690 | 28.950 | 13.680 | 0.000 | 0.008 | 0.090 |
| 2% DMEM for 24 hrs followed by 2.5uM TSA for 24 hrs | 33.290 | 26.720 | 23.780 | 28.950 | 11.740 | 0.000 | 0.029 | 0.370 |
| 1uM MG132 for 24 hrs followed by 1.25uM TSA for 24 hrs | 31.900 | 26.720 | 24.040 | 28.950 | 10.090 | 0.001 | 0.092 | 0.220 |
| 1 uM MG132 for 24 hrs followed by 2.5uM TSA for 24 hrs | 34.030 | 26.720 | 23.800 | 28.950 | 12.460 | 0.000 | 0.018 | 0.350 |
| 10uM MG132 for 24 hrs followed by 1.25uM TSA for 24 hrs | 29.770 | 26.720 | 25.880 | 28.950 | 6.120 | 0.014 | 1.438 | 0.150 |
| 10uM MG132 for 24 hrs followed by 2.5uM TSA for 24 hrs | 30.290 | 26.720 | 24.370 | 28.950 | 8.150 | 0.004 | 0.352 | 0.040 |
| untreated control | - | 26.720 | 22.680 | 28.950 | - | - | - | - |
| 1.25uM TSA for 24 hr | 34.120 | 26.720 | 22.900 | 28.950 | 13.450 | 0.000 | 0.009 | 0.180 |
| 2.5uM TSA for 24 hr | 34.180 | 26.720 | 24.310 | 28.950 | 12.100 | 0.000 | 0.023 | 0.450 |
| 2%DMEM for 24 hrs followed by 1uM MG132 for 24 hrs | 32.830 | 26.720 | 25.400 | 28.950 | 9.660 | 0.001 | 0.124 | 0.250 |
| 2%DMEM for 24 hrs followed by 10uM MG132 for 24 hrs | 32.520 | 26.720 | 24.070 | 28.950 | 10.680 | 0.001 | 0.061 | 0.050 |
| 1.25uM TSA for24 hrs followed by 1uM MG132 for 24 hrs | 34.870 | 26.720 | 24.120 | 28.950 | 12.980 | 0.000 | 0.012 | 0.380 |
| 1.25uM TSA for 24 hrs followed by 10uM MG132 for 24 hrs | 35.070 | 26.720 | 24.250 | 28.950 | 13.050 | 0.000 | 0.012 | 0.230 |
| 2.5uM TSA for 24 hrs followed by 1uM MG132 for 24 hrs | 34.940 | 26.720 | 24.290 | 28.950 | 12.880 | 0.000 | 0.013 | 0.450 |
| 2.5uM TSA for 24 hrs followed by 10uM MG132 for 24 hrs | 35.670 | 26.720 | 26.670 | 28.950 | 11.230 | 0.000 | 0.042 | 0.400 |

| **H5F3P9 Cell Treatment: MG132 followed by TSA** | **Insulin Sample Ct** | **Insulin Pancreas Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated control | - | 17.940 | 24.340 | 28.950 | - | - | - | |
| 1uM MG132 for 24 hrs | - | 17.940 | 23.680 | 28.950 | - | - | - | |
| 10uM MG132 for 24 hrs | - | 17.940 | 23.680 | 28.950 | - | - | - | |
| 2% DMEM for 24 hrs followed by 1.25uM TSA for 24 hrs | - | 17.940 | 23.690 | 28.950 | - | - | - | |
| 2% DMEM for 24 hrs followed by 2.5uM TSA for 24 hrs | - | 17.940 | 23.780 | 28.950 | - | - | - | |
| 1uM MG132 for 24 hrs followed by 1.25uM TSA for 24 hrs | - | 17.940 | 24.040 | 28.950 | - | - | - | |
| 1 uM MG132 for 24 hrs followed by 2.5uM TSA for 24 hrs | - | 17.940 | 23.800 | 28.950 | - | - | - | |
| 10uM MG132 for 24 hrs followed by 1.25uM TSA for 24 hrs 10uM | 37.060 | 17.940 | 25.880 | 28.950 | 22.190 | 0.000 | 0.000 | 0.360 |
| 10 uM MG132 for 24 hrs followed by 2.5uM TSA for 24 hrs | - | 17.940 | 24.370 | 28.950 | - | - | - | |

| **H5F3P9 Cell Treatment: TSA2 followed by MG132** | **Insulin Sample Ct** | **InsulinPancreas Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated control | - | 17.940 | 22.680 | 28.950 | - | - | - | |
| 1.25uM TSA for 24 hr | - | 17.940 | 22.900 | 28.950 | - | - | - | |
| 2.5uM TSA for 24 hr | - | 17.940 | 24.310 | 28.950 | - | - | - | |
| 2%DMEM for 24 hrs followed by 1uM MG132 for 24 hrs | - | 17.940 | 25.400 | 28.950 | - | - | - | |
| 2%DMEM for 24 hrs followed by 10uM MG132 for 24 hrs | - | 17.940 | 24.070 | 28.950 | - | - | - | |
| 1.25uM TSA for 24 hrs followed by 1uM MG132 for 24 hrs | - | 17.940 | 24.120 | 28.950 | - | - | - | |
| 1.25uM TSA for 24 hrs followed by 10uM MG132 for 24 hrs | - | 17.940 | 24.250 | 28.950 | - | - | - | |
| 2.5uM TSA for 24 hrs followed by 1uM MG132 for 24 hrs | - | 17.940 | 24.290 | 28.950 | - | - | - | |
| 2.5uM TSA for 24 hrs followed by 10uM MG132 for 24 hrs | - | 17.940 | 26.670 | 28.950 | - | - | - | |

| **H5F3P9 Cell Treatment: MG132 followed by TSA** | **NKX6.1 Sample Ct** | **NKX6.1 Pancreas Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated control | - | 29.120 | 24.340 | 28.950 | - | - | - | - |
| 1uM MG132 for 24 hrs | 33.820 | 29.120 | 23.680 | 28.950 | 9.970 | 0.001 | 0.100 | 0.000 |
| 10uM MG132 for 24 hrs | 32.850 | 29.120 | 23.680 | 28.950 | 9.970 | 0.001 | 0.100 | 0.420 |
| 2% DMEM for 24 hrs followed by 1.25uM TSA for 24 hrs | 30.590 | 29.120 | 23.690 | 28.950 | 6.730 | 0.009 | 0.942 | 0.060 |
| 2% DMEM for 24 hrs followed by 2.5uM TSA for 24 hrs | 29.830 | 29.120 | 23.780 | 28.950 | 5.880 | 0.017 | 1.698 | 0.060 |
| 1 uM MG132 for 24 hrs followed by 1.25uM TSA for 24 hrs | 27.970 | 29.120 | 24.040 | 28.950 | 3.760 | 0.074 | 7.381 | 0.070 |
| 1 uM MG132 for 24 hrs followed by 2.5uM TSA for 24 hrs | 29.310 | 29.120 | 23.800 | 28.950 | 5.340 | 0.025 | 2.469 | 0.200 |
| 10uM MG132 for 24 hrs followed by 1.25uM TSA for 24 hrs | 29.000 | 29.120 | 25.880 | 28.950 | 2.950 | 0.129 | 12.941 | 0.050 |
| 10uM MG132 for 24 hrs followed 2.5uM TSA for 24 hrs | 28.860 | 29.120 | 24.370 | 28.950 | 4.320 | 0.050 | 5.007 | 0.100 |

| **H5F3P9 Cell Treatment: TSA followed by MG132** | **NKX6.1 Sample Ct** | **NKX6.1 Pancreas Ct** | **Sample GAPDH Ct** | **Pancreas GAPDH Ct** | **ΔΔCt** | **2^{∧}(-ΔΔCt)=RQ** | **% RQ** | **Std err** |
|---|---|---|---|---|---|---|---|---|
| untreated control | - | 29.120 | 22.680 | 28.950 | - | - | - | - |
| 1.25uM TSA for 24 hr | 29.920 | 29,120 | 22.900 | 28.950 | 6.850 | 0.009 | 0.867 | 0.000 |
| 2.5uM TSA for 24 hr | 30.130 | 29.120 | 24.310 | 28.950 | 5.650 | 0.020 | 1.992 | 0.130 |
| 2%DMEM for 24 hrs followed by 1uM MG132 for 24 hrs | 32.830 | 29.120 | 25.400 | 28.950 | 7.260 | 0.007 | 0.652 | 0.250 |
| 2%DMEM for 24 hrs followed by 10uM MG132 for 24 hrs | 33.200 | 29.120 | 24.070 | 28.950 | 8.960 | 0.002 | 0.201 | 0.610 |
| 1.25uM TSA for 24 hrs followed by 1uM MG132 for 24 hrs | 35.950 | 29.120 | 24.120 | 28.950 | 11.660 | 0.000 | 0.031 | 0.110 |
| 1.25uM TSA for 24 hrs followed by 10uM MG132 for 24 hrs | 34.700 | 29.120 | 24.250 | 28.950 | 10.280 | 0.001 | 0.080 | 0.160 |
| 2.5uM TSA for 24 hrs followed by 1uM MG132 for 24 hrs | 34.820 | 29.120 | 24.290 | 28.950 | 10.360 | 0.001 | 0.076 | 0.480 |
| 2.5uM TSA for 24 hrs followed by 10uM MG132 for 24 hrs | 35.400 | 29.120 | 26.670 | 28.950 | 8.560 | 0.003 | 0.265 | 0.060 |

**TABLE XVI. EFFECTS OF A HDAC INHIBITOR AND MG-132 TREATMENT ON GENE EXPRESSION IN AN ADULT PANCREAS-DERIVED CELL LINE.**

| **H5F3P10 Cell Treatment: MG132 followed by TSA** | **Pdx1 Sample Ct** | **Pdx1 Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 28.660 | 24.010 | 28.950 | - | - | - | - |
| 1uM MG132 for 24 hrs | - | 30.110 | 25.460 | 33.450 | - | - | - | - |
| 10uM MG132 for 24 hrs | - | 30.110 | 25.620 | 33.450 | - | - | - | - |
| 2% DMEM for 24 hrs followed by 1.25uM TSA for 24 hrs | 36.490 | 28.660 | 22.190 | 28.950 | 14.590 | 0.000 | 0.004 | 0.850 |
| 2% DMEM for 24 hrs followed by 2.5uM TSA for 24 hrs | 33.270 | 28.660 | 21.920 | 28.950 | 11.640 | 0.000 | 0.031 | 0.110 |
| 1uM MG132 for 24 hrs followed by 1.25uM TSA for 24 hrs | 33.810 | 30.110 | 26.470 | 33.450 | 10.680 | 0.001 | 0.061 | 0.120 |
| 1uM MG132 for 24 hrs followed by 2.5uM TSA for 24 hrs | 33.590 | 30.110 | 32.740 | 33.450 | 4.190 | 0.055 | 5.479 | 0.200 |
| 10uM MG132 for 24 hrs followed by 1.25uM TSA for 24 hrs | 31.530 | 30.110 | 25.950 | 33.450 | 8.920 | 0.002 | 0.206 | 0.010 |
| 10uM MG132 for 24 hrs followed by 2.5uM TSA for 24 hrs | 32.160. | 28.660 | 22.570 | 28.950 | 9.880 | 0.001 | 0.106 | 0.180 |

| **H5F3P19 Cell Treatment: MG132 followed by TSA** | **Pdx1 Sample Ct** | **Pdx1 Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 30.110 | 26.740 | 33.450 | - | - | - | - |
| 1uM MG132 for 24 hrs | - | 28.660 | 23.870 | 28.950 | - | - | - | - |
| 10uM MG132 for 24 hrs | - | 28.660 | 24.790 | 28.950 | - | - | - | - |
| 2% DMEM for 24 hrs followed by 1.25uM TSA for 24 hrs | 32.790 | 28.660 | 24.650 | 28.950 | 8.430 | 0.003 | 0.290 | 0.160 |
| 2% DMEM for 24 hrs followed by 2.5uM TSA for 24 hrs | 30.510 | 28.660 | 23.340 | 28.950 | 7.460 | 0.006 | 0.568 | 0.100 |
| 1 uM MG132 for 24 hrs followed by 1.25uM TSA for 24 hrs | 32.120 | 28.660 | 24.280 | 28.950 | 8.130 | 0.004 | 0.357 | 0.260 |
| 1uM MG132 for 24 hrs followed by 2.5uM TSA for 24 hrs | 32.740 | 28.660 | 24.710 | 28.950 | 8.320 | 0.003 | 0.313 | 0.060 |
| 10uM MG132 for 24 hrs followed by 1.25uM TSA for 24 hrs | 32.470 | 28.660 | 25.290 | 28.950 | 7.470 | 0.006 | 0.564 | 0.160 |
| 10uM MG132 for 24 hrs followed by 2.5uM TSA for 24 hrs | 32.310 | 28.660 | 25.100 | 28.950 | 7.500 | 0.006 | 0.552 | 0.040 |

| **H5F3P10 Cell Treatment: MG132 followed by TSA** | **HNF-β Sample Ct** | **HNF-β Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 26.720 | 24.010 | 28.950 | - | - | - | |
| 1 uM MG132 for 24 hrs | - | 27.920 | 25.460 | 33.450 | - | - | - | - |
| 10uM MG132 for 24 hrs | 37.510 | 27.920 | 25.620 | 33.450 | 17.420 | 0.000 | 0.001 | 0.730 |
| 2% DMEM for 24 hrs followed by 1.25uM TSA for 24 hrs | 36.000 | 26.720 | 22.190 | 28.950 | 16.040 | 0.000 | 0.001 | 1.200 |
| 2% DMEM for 24 hrs followed by 2.5uM TSA for 24 hrs | 33.500 | 26.720 | 21.920 | 28.950 | 13.810 | 0.000 | 0.007 | 0.300 |
| 1uM MG132 for 24 hrs followed by 1.25uM TSA for 24 hrs | 31.690 | 27.920 | 26.470 | 33.450 | 10.750 | 0.001 | 0.058 | 0.230 |
| 1uM MG132 for 24 hrs followed by 2.5uM TSA for 24 hrs | 31.840 | 27.920 | 32.740 | 33.450 | 4.630 | 0.040 | 4.039 | 0.300 |
| 10uM MG132 for 24 hrs followed by 1.25uM TSA for 24 hrs | 28.950 | 27.920 | 25.950 | 33.450 | 8.530 | 0.003 | 0.271 | 0.040 |
| 10uM MG132 for 24 hrs followed by 2.5uM TSA for 24 hrs | 30.410 | 26.720 | 22.570 | 28.950 | 10.070 | 0.001 | 0.093 | 0.100 |

| **H5F3P19 Cell Treatment: MG132 followed by TSA** | **HNF-β Sample Ct** | **HNF-β Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| | | | | | | | | |
| untreated control | - | 27.920 | 26.740 | 33.450 | - | - | - | |
| 1uM MG132 for 24 hrs | 33.270 | 26.720 | 23.870 | 28.950 | 11.630 | 0.000 | 0.032 | 0.170 |
| 10uM MG132 for 24 hrs | 34.110 | 26.720 | 24.790 | 28.950 | 11.550 | 0.000 | 0.033 | 0.280 |
| 2% DMEM for 24 hrs followed by 1.25uM TSA for 24 hrs | 32.190 | 26.720 | 24.650 | 28.950 | 9.770 | 0.001 | 0.115 | 0.160 |
| 2% DMEM for 24 hrs followed by 2.5uM TSA for 24 hrs | 31.720 | 26.720 | 23.340 | 28.950 | 10.610 | 0.001 | 0.064 | 0.080 |
| 1uM MG132 for 24 hrs followed by 1.25uM TSA for 24 hrs | 29.370 | 26.720 | 24.280 | 28.950 | 7.320 | 0.006 | 0.626 | 0.260 |
| 1 uM MG132 for 24 hrs followed by 2.5uM TSA for 24 hrs | 29.940 | 26.720 | 24.710 | 28.950 | 7.460 | 0.006 | 0.568 | 0.010 |
| 10uM MG132 for 24 hrs followed by 1.25uM TSA for 24 hrs | 29.310 | 26.720 | 25.290 | 28.950 | 6.250 | 0.013 | 1.314 | 0.210 |
| 10uM MG132 for 24 hrs followed by 2.5uM TSA for 24 hrs | 29.840 | 26.720 | 25.100 | 28.950 | 6.970 | 0.008 | 0.798 | 0.330 |

| **H5F3P10 Cell Treatment: MG132 followed by TSA** | **Insulin Sample Ct** | **Insulin Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 17.940 | 24.010 | 28.950 | - | - | - | - |
| 1 uM MG132 for 24 hrs | - | 19.080 | 25.460 | 33.450 | - | - | - | - |
| 10uM MG132 for 24 hrs | - | 19.080 | 25.620 | 33.450 | - | - | - | - |
| 2% DMEM for 24 hrs followed by 1.25uM TSA for 24 hrs | - | 17.940 | 22.190 | 28.950 | - | - | - | - |
| 2% DMEM for 24 hrs followed by 2.5uM TSA for 24 hrs | - | 17.940 | 21.920 | 28.950 | - | - | - | - |
| 1uM MG132 for 24 hrs followed by 1.25uM TSA for 24 hrs | - | 19.080 | 26.470 | 33.450 | - | - | - | - |
| 1uM MG132 for 24 hrs followed by 2.5uM TSA for 24 hrs | - | 19.080 | 32.740 | 33.450 | - | - | - | - |
| 10uM MG132 for 24 hrs followed by 1.25uM TSA for 24 hrs | - | 19.080 | 25.950 | 33.450 | - | - | - | - |
| 10uM MG132 for 24 hrs followed by 2.5uM TSA for 24 hrs | 36.000 | 17.940 | 22.570 | 28.950 | 24.440 | 0.000 | 0.000 | 0.280 |

| **H5F3P19 Cell Treatment: MG132 followed by TSA** | **Insulin Sample Ct** | **Insulin Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 19.080 | 26.740 | 33.450 | - | - | - | - |
| 1uM MG132 for 24 hrs | - | 17.940 | 23.870 | 28.950 | - | - | - | - |
| 10uM MG132 for 24 hrs | - | 17.940 | 24.790 | 28.950 | - | - | - | - |
| 2% DMEM for 24 hrs followed by 1.25uM TSA for 24 hrs | - | 17.940 | 24.650 | 28.950 | - | - | - | - |
| 2% DMEM for 24 hrs followed by 2.5uM TSA for 24 hrs | - | 17.940 | 23.340 | 28.950 | - | - | - | - |
| 1uM MG132 for 24 hrs followed by 1.25uM TSA for 24 hrs | 36.580 | 17.940 | 24.280 | 28.950 | 23.310 | 0.000 | 0.000 | 0.760 |
| 1 uM MG132 for 24 hrs followed by 2.5uM TSA for 24 hrs | 36.430 | 17.940 | 24.710 | 28.950 | 22.730 | 0.000 | 0.000 | 0.320 |
| 10uM MG132 for 24 hrs followed by 1.25uM TSA for 24 hrs | 36.060 | 17.940 | 25.290 | 28.950 | 21.780 | 0.000 | 0.000 | 1.100 |
| 10uM MG132 for 24 hrs followed by 2.5uM TSA for 24 hrs | - | 17.940 | 25.100 | 28.950 | - | - | - | - |

| **H5F3P10 Cell Treatment: MG132 followed by TSA** | **NKX6.1 Sample Ct** | **NKX6.1 Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 29.120 | 24.010 | 28.950 | - | - | - | |
| 1uM MG132 for 24 hrs | - | 30.240 | 25.460 | 33.450 | - | - | - | - |
| 10uM MG132 for 24 hrs | 34.550 | 30.240 | 25.620 | 33.450 | 12.140 | 0.000 | 0.022 | 0.380 |
| 2% DMEM for 24 hrs followed by 1.25uM TSA for 24 hrs | 30.640 | 29.120 | 22.190 | 28.950 | 8.280 | 0.003 | 0.322 | 0.060 |
| 2% DMEM for 24 hrs followed by 2.5uM TSA for 24 hrs | 29.380 | 29.120 | 21.920 | 28.950 | 7.290 | 0.006 | 0.639 | 0.040 |
| 1 uM MG132 for 24 hrs followed by 1.25uM TSA for 24 hrs | 30.690 | 30.240 | 26.470 | 33.450 | 7.430 | 0.006 | 0.580 | 0.070 |
| 1uM MG132 for 24 hrs followed by 2.5uM TSA for 24 hrs | 30.740 | 30.240 | 32.740 | 33.450 | 1.210 | 0.432 | 43.227 | 0.210 |
| 10uM MG132 for 24 hrs followed by 1.25uM TSA for 24 hrs | 28.930 | 30.240 | 25.950 | 33.450 | 6.190 | 0.014 | 1.370 | 0.200 |
| 10uM MG132 for 24 hrs followed by 2.5uM TSA for 24 hrs | 29.290 | 29.120 | 22.570 | 28.950 | 6.550 | 0.011 | 1.067 | 0.070 |

| **H5F3P19 Cell Treatment: MG132 followed by TSA** | **NKX6.1 Sample Ct** | **NKX6.1 Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 30.240 | 26.740 | 33.450 | - | - | - | |
| 1uM MG132 for 24 hrs | 31.930 | 29.120 | 23.870 | 28.950 | 7.890 | 0.004 | 0.422 | 0.090 |
| 10uM MG132 for 24 hrs | 32.660 | 29.120 | 24.790 | 28.950 | 7.700 | 0.005 | 0.481 | 0.350 |
| 2% DMEM for 24 hrs followed by 1.25uM TSA for 24 hrs | 29.070 | 29.120 | 24.650 | 28.950 | 4.250 | 0.053 | 5.256 | 0.210 |
| 2% DMEM for 24 hrs followed by 2.5uM TSA for 24 hrs | 28.690 | 29.120 | 23.340 | 28.950 | 5.180 | 0.028 | 2.758 | 0.300 |
| 1 uM MG132 for 24 hrs followed by 1.25uM TSA for 24 hrs | 28.290 | 29.120 | 24.280 | 28.950 | 3.840 | 0.070 | 6.983 | 0.210 |
| 1 uM MG132 for 24 hrs followed by 2.5uM TSA for 24 hrs | 29.430 | 29.120 | 24.710 | 28.950 | 4.550 | 0.043 | 4.269 | 0.230 |
| 10uM MG132 for 24 hrs followed by 1.25uM TSA for 24 hrs | 29.040 | 29.120 | 25.290 | 28.950 | 3.580 | 0.084 | 8.362 | 0.400 |
| 10uM MG132 for 24 hrs followed by 2.5uM TSA for 24 hrs | 29.240 | 29.120 | 25.100 | 28.950 | 3.970 | 0.064 | 6.381 | 0.020 |

**TABLE XVII. EFFECTS OF A HDAC INHIBITOR AND MG-132 TREATMENT ON GENE EXPRESSION IN AN ADULT PANCREAS-DERIVED CELL LINE.**

| **H5F3P10 Cell Treatment: MG132 followed by TSA** | **Pdx1 Sample Ct** | **Pdx1 Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control 3 hrs | - | 30.812 | 21.985 | 28.330 | - | - | - | - |
| 1uM MG132 for 3 hrs | - | 30.812 | 22.223 | 28.330 | - | - | - | - |
| 5uM MG132 for 3 hrs | - | 30.812 | 22.358 | 28.330 | - | - | - | - |
| 10uM MG132 for 3 hrs | - | 30.812 | 23.215 | 28.330 | - | - | - | - |
| untreated control 6 hrs | - | 30.812 | 22.845 | 28.330 | - | - | | |
| 1 uM MG132 for 6 hrs | - | 30.812 | 22.134 | 28.330 | - | - | - | - |
| 5uM MG132 for 6 hrs | - | 30.812 | 22.601 | 28.330 | - | - | - | - |
| 10uM MG132 for 6 hrs | - | 30.812 | 23.078 | 28.330 | - | - | - | - |
| 1uM MG132 for 9 hrs | - | 30.812 | 21.710 | 28.330 | - | - | - | - |
| 5uM MG132 for 9 hrs | - | 30.812 | 22.376 | 28.330 | - | - | - | - |
| 10uM MG132 for 9 hrs | - | 30.812 | 22.948 | 28.330 | - | - | - | - |
| untreated control 12 hrs | - | 30.812 | 22.332 | 28.330 | - | - | - | - |
| 1uM MG132 for 12 hrs | 39.852 | 30.812 | 22.163 | 28.330 | 15.207 | 0.000 | 0.003 | |
| 5uM MG132 for 12 hrs | 38.040 | 30.812 | 22.247 | 28.330 | 13.311 | 0.000 | 0.010 | |
| 10uM MG132 for 12 hrs | - | 30.812 | 22.265 | 28.330 | - | - | - | - |
| untreated control 24 hrs | - | 30.812 | 22.426 | 28.330 | - | - | - | - |
| 1uM MG132 for 24 hrs | - | 29.282 | 20.963 | 27.787 | - | - | - | - |
| 5uM MG132 for 24 hrs | - | 29.282 | 21.825 | 27.787 | - | - | - | - |
| 10uM MG132 for 24 hrs | - | 29.282 | 22.143 | 27.787 | - | - | - | - |
| 2.5uM TSA for 24 hrs | 35.425 | 29.282 | 22.023 | 27.787 | 11.907 | 0.000 | 0.026 | 0.978 |
| 1uM MG132 for 3 hrs followed by 2.5uM TSA for 24hrs | 37.429 | 30.812 | 23.465 | 28.330 | 11.482 | 0.000 | 0.035 | 0.173 |
| 5uM MG132 for 3 hrs followed by 2.5uM TSA for 24hrs | 35.465 | 30.812 | 23.507 | 28.330 | 9.476 | 0.001 | 0.140 | 0.346 |
| 10uM MG132 for 3 hrs followed by 2.5uM TSA for 24hrs | 33.821 | 30.812 | 23.684 | 28.330 | 7.655 | 0.005 | 0.496 | 0.159 |
| 1 uM MG132 for 6 hrs followed by 2.5uM TSA for 24hrs | 36.660 | 30.812 | 22.604 | 28.330 | 11.574 | 0.000 | 0.033 | 0.491 |
| 5uM MG132 for 6 hrs followed by 2.5uM TSA for 24hrs | 32.950 | 30.812 | 22.716 | 28.330 | 7.752 | 0.005 | 0.464 | 0.021 |
| 10uM MG132 for 6 hrs followed by 2.5uM TSA for 24hrs | 32.293 | 30.812 | 23.269 | 28.330 | 6.542 | 0.011 | 1.073 | 0.168 |
| 1uM MG132 for 9 hrs followed by 2.5uM TSA for 24hrs | 35.004 | 29.282 | 21.479 | 27.787 | 12.030 | 0.000 | 0.024 | 0.003 |
| 5uM MG132 for 9 hrs followed by 2.5uM TSA for 24hrs | 33.868 | 29.282 | 21.698 | 27.787 | 10.675 | 0.001 | 0.061 | 0.050 |
| 10uM MG132 for 9 hrs followed by 2.5uM TSA for 24hrs | 32.776 | 29.282 | 22.310 | 27.787 | 8.971 | 0.002 | 0.199 | 0.175 |
| 1 uM MG132 for 12 hrs followed by 2.5uM TSA for 24hrs | 35.127 | 29.282 | 22.395 | 27.787 | 11.237 | 0.000 | 0.041 | 0.969 |
| 5uM MG132 for 12 hrs followed by 2.5uM TSA for 24hrs | 33.794 | 29.282 | 22.898 | 27.787 | 9.401 | 0.001 | 0.148 | 0.342 |
| 10uM MG132 for 12 hrs followed by 2.5uM TSA for 24hrs | 33.325 | 29.282 | 23.707 | 27.787 | 8.123 | 0.004 | 0.359 | 0.112 |
| 1uM MG132 for 24 hrs followed by 2.5uM TSA for 24hrs | - | 29.282 | 20.116 | 27.787 | - | - | - | - |
| 5uM MG132 for 24 hrs followed by 2.5uM TSA for 24hrs | 34.520 | 29.282 | 19.742 | 27.787 | 13.283 | 0.000 | 0.010 | 0.235 |
| 10uM MG132 for 24 hrs followed by 2.5uM TSA for 24hrs | 33.216 | 29.282 | 20.421 | 27.787 | 11.300 | 0.000 | 0.040 | 0.029 |

| **H5F3P10 Cell Treatment: MG132 followed by TSA** | **HNF3-β Sample Ct** | **HNF3-β Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control 3 hrs | - | 28.3680 | 21.9850 | 28.3300 | - | - | - | - |
| 1 uM MG132 for 3 hrs | - | 28.3680 | 22.2230 | 28.3300 | - | - | - | - |
| 5uM MG132 for 3 hrs | - | 28.3680 | 22.3580 | 28.3300 | - | - | - | - |
| 10uM MG132 for 3 hrs | - | 28.3680 | 23.2150 | 28.3300 | - | - | - | - |
| untreated control 6 hrs | - | 28.3680 | 22.8450 | 28.3300 | - | - | - | - |
| 1 uM MG132 for 6 hrs | - | 28.3680 | 22.1340 | 28.3300 | - | - | - | - |
| 5uM MG132 for 6 hrs | - | 28.3680 | 22.6010 | 28.3300 | - | - | - | - |
| 10uM MG132 for 6 hrs | 37.2890 | 28.3680 | 23.0780 | 28.3300 | 14.1730 | 0.0001 | 0.0054 | |
| 1uM MG132 for 9 hrs | - | 28.3680 | 21.7100 | 28.3300 | - | - | - | - |
| 5uM MG132 for 9 hrs | - | 28.3680 | 22.3760 | 28.3300 | - | - | - | - |
| 10uM MG132 for 9 hrs | - | 28.3680 | 22.9480 | 28.3300 | - | - | - | - |
| untreated control 12 hrs | - | 28.3680 | 22.3320 | 28.3300 | - | - | - | - |
| 1uM MG132 for 12 hrs | - | 28.3680 | 22.1630 | 28.3300 | - | - | - | - |
| 5uM MG132 for 12 hrs | 36.9740 | 28.3680 | 22.2470 | 28.3300 | 14.6890 | 0.0000 | 0.0038 | - |
| 10uM MG132 for 12 hrs | 37.3820 | 28.3680 | 22.2650 | 28.3300 | 15.0790 | 0.0000 | 0.0029 | 0.1310 |
| untreated control 24 hrs | - | 28.3680 | 22.4260 | 28.3300 | - | - | - | - |
| 1uM MG132 for 24 hrs | - | 28.5450 | 20.9630 | 27.7870 | - | - | - | - |
| 5uM MG132 for 24 hrs | 35.8470 | 28.5450 | 21.8250 | 27.7870 | 13.2640 | 0.0001 | 0.0102 | 0.3366 |
| 10uM MG132 for 24 hrs | 33.3790 | 28.5450 | 22.1430 | 27.7870 | 10.4780 | 0.0007 | 0.0701 | 0.6053 |
| 2.5uM TSA for 24 hrs | 36.1230 | 28.5450 | 22.0230 | 27.7870 | 13.3420 | 0.0001 | 0.0096 | 0.5317 |
| 1 uM MG132 for 3 hrs followed by 2.5uM TSA for 24hrs | 37.1680 | 28.3680 | 23.4650 | 28.3300 | 13.6650 | 0.0001 | 0.0077 | |
| 5uM MG132 for 3 hrs followed by 2.5uM TSA for 24hrs | 37.6040 | 28.3680 | 23.5070 | 28.3300 | 14.0590 | 0.0001 | 0.0059 | 1.0580 |
| 10uM MG132 for 3 hrs followed by 2.5uM TSA for 24hrs | 34.5960 | 28.3680 | 23.6840 | 28.3300 | 10.8740 | 0.0005 | 0.0533 | 0.0290 |
| 1 uM MG132 for 6 hrs followed by 2.5uM TSA for 24hrs | 35.6510 | 28.3680 | 22.6040 | 28.3300 | 13.0090 | 0.0001 | 0.0121 | 0.4200 |
| 5uM MG132 for 6 hrs followed by 2.5uM TSA for 24hrs | 33.7310 | 28.3680 | 22.7160 | 28.3300 | 10.9770 | 0.0005 | 0.0496 | 0.0870 |
| 10uM MG132 for 6 hrs followed by 2.5uM TSA for 24hrs | 32.5800 | 28.3680 | 23.2690 | 28.3300 | 9.2730 | 0.0016 | 0.1616 | 0.2290 |
| 1 uM MG132 for 9 hrs followed by 2.5uM TSA for 24hrs | 35.3600 | 28.5450 | 21.4790 | 27.7870 | 13.1230 | 0.0001 | 0.0112 | 1.4927 |
| 5uM MG132 for 9 hrs followed by 2.5uM TSA for 24hrs | 34.3150 | 28.5450 | 21.6980 | 27.7870 | 11.8590 | 0.0003 | 0.0269 | 0.0007 |
| 10uM MG132 for 9 hrs followed by 2.5uM TSA for 24hrs | 34.0920 | 28.5450 | 22.3100 | 27.7870 | 11.0240 | 0.0005 | 0.0480 | 0.1626 |
| 1 uM MG132 for 12 hrs followed by 2.5uM TSA for 24hrs | 36.3570 | 28.5450 | 22.3950 | 27.7870 | 13.2040 | 0.0001 | 0.0106 | 1.0557 |
| 5uM MG132 for 12 hrs followed by 2.5uM TSA for 24hrs | 34.4540 | 28.5450 | 22.8980 | 27.7870 | 10.7980 | 0.0006 | 0.0562 | 0.6336 |
| 10uM MG132 for 12 hrs followed by 2.5uM TSA for 24hrs | 32.9030 | 28.5450 | 23.7070 | 27.7870 | 8.4380 | 0.0029 | 0.2883 | 0.1704 |
| 1uM MG132 for 24 hrs followed by 2.5uM TSA for 24hrs | 36.5240 | 28.5450 | 20.1160 | 27.7870 | 15.6500 | 0.0000 | 0.0019 | 0.6215 |
| 5uM MG132 for 24 hrs followed by 2,5uM TSA for 24hrs | 34.5550 | 28.5450 | 19.7420 | 27.7870 | 14.0550 | 0.0001 | 0.0059 | 0.8330 |
| 10uM MG132 for 24 hrs followed by 2.5uM TSA for 24hrs | 30.7860 | 28.5450 | 20.4210 | 27.7870 | 9.6070 | 0.0013 | 0.1282 | 0.6081 |

| **H5F3P10 Cell Treatment: MG132 followed by TSA** | **Insulin Sample Ct** | **Insulin Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control 3 hrs | - | 19.823 | 21.985 | 28.330 | - | - | - | - |
| 1 uM MG132 for 3 hrs | - | 19.823 | 22.223 | 28.330 | - | - | - | - |
| 5uM MG132 for 3 hrs | - | 19.823 | 22.358 | 28.330 | - | - | - | - |
| 10uM MG132 for 3 hrs | - | 19.823 23.215 | | 28.330 | - | - | - | - |
| untreated control 6 hrs | - | 19.823 | 22.845 | 28.330 | - | - | - | - |
| 1 uM MG1 32 for 6 hrs | - | 19.823 | 22.134 | 28.330 | - | - | - | - |
| 5uM MG132 for 6 hrs | - | 19.823 | 22.601 | 28.330 | - | - | - | - |
| 10uM MG132 for 6 hrs | - | 19.823 | 23.078 | 28.330 | - | - | - | - |
| 1 uM MG1 32 for 9 hrs | - | 19.823 | 21.710 | 28.330 | - | - | - | - |
| 5uM MG132 for 9 hrs | - | 19.823 | 22.376 | 28.330 | - | - | - | - |
| 10uM MG132 for 9 hrs | - | 19.823 | 22.948 | 28.330 | - | - | - | - |
| untreated control 12 hrs | - | 19.823 | 22.332 | 28.330 | - | - | - | - |
| 1uM MG132 for 12 hrs | - | 19.823 | 22.163 | 28.330 | - | - | - | - |
| 5uM MG132 for 12 hrs | - | 19.823 | 22.247 | 28.330 | - | - | - | - |
| 10uM MG132 for 12 hrs | - | 19.823 | 22.265 | 28.330 | - | - | - | - |
| untreated control 24 hrs | - | 19.823 | 22.426 | 28.330 | - | - | - | - |
| 1uM MG132 for 24 hrs | - | 19.135 | 20.963 | 27.787 | | | | |
| 5uM MG132 for 24 hrs | - | 19.135 | 21.825 | 27.787 | | | | |
| 10uM MG132 for 24 hrs | - | 19.135 | 22.143 | 27.787 | | | | |
| 2.5uM TSA for 24 hrs | - | 19.823 | 22.124 | 28.330 | - | - | - | - |
| 1uM MG132 for 3 hrs followed by 2.5uM TSA for 24hrs | - | 19.823 | 23.465 | 28.330 | - | - | - | - |
| 5uM MG132 for 3 hrs followed by 2.5uM TSA for 24hrs | - | 19.823 | 23.507 | 28.330 | - | - | - | - |
| 10uM MG132 for 3 hrs followed by 2.5uM TSA for 24hrs | - | 19.823 | 23.684 | 28.330 | - | - | - | - |
| 1 uM MG132 for 6 hrs followed by 2.5uM TSA for 24hrs | - | 19.823 | 22.604 | 28.330 | - | - | - | - |
| 5uM MG132 for 6 hrs followed by 2.5uM TSA for 24hrs | - | 19.823 | 22.716 | 28.330 | - | - | - | - |
| 10uM MG132 for 6 hrs followed by 2.5uM TSA for 24hrs | - | 19.823 | 23.269 | 28.330 | - | - | - | - |
| 1 uM MG132 for 9 hrs followed by 2.5uM TSA for 24hrs | - | 19.135 | 21.479 | 27.787 | | | | |
| 5uM MG132 for 9 hrs followed by 2.5uM TSA for 24hrs | - | 19.135 | 21.698 | 27.787 | | | | |
| 10uM MG132 for 9 hrs followed by 2.5uM TSA for 24hrs | - | 19.135 | 22.310 | 27.787 | | | | |
| 1 uM MG132 for 12 hrs followed by 2.5uM TSA for 24hrs | - | 19.135 | 22.395 | 27.787 | | | | |
| 5uM MG132 for 12 hrs followed by 2.5uM TSA for 24hrs | - | 19.135 | 22.898 | 27.787 | | | | |
| 10uM MG132 for 12 hrs followed by 2.5uM TSA for 24hrs | - | 19.135 | 23.707 | 27.787 | | | | |
| 1 uM MG132 for 24 hrs followed by 2.5uM TSA for 24hrs | - | 19.135 | 20.116 | 27.787 | | | | |
| 5uM MG132 for 24 hrs followed by 2.5uM TSA for 24hrs | - | 19.135 | 19.742 | 27.787 | | | | |
| 10uM MG132 for 24 hrs followed by 2.5uM TSA for 24hrs | - | 19.135 | 20.421 | 27.787 | | | | |

| **H5F3P10 Cell Treatment: MG132 followed by TSA** | **NKX6.1 Sample Ct** | **NKX6.1 Pancreas Ct** | **Sample GAPDH Ct** | **Pancreas GAPDH Ct** | **ΔΔCt** | **2^(ΔΔCt)=RQ** | **% RQ** | **Std err** |
|---|---|---|---|---|---|---|---|---|
| untreated control 3 hrs | - | 29.877 | 21.985 | 28.330 | | | | |
| 1uM MG132 for 3 hrs | - | 29.877 | 22.223 | 28.330 | - | - | - | - |
| 5uM MG132 for 3 hrs | 37.817 | 29.877 | 22.358 | 28.330 | 13.912 | 0.000 | 0.006 | 0.455 |
| 10uM MG132 for 3 hrs | 35.781 | 29.877 | 23.215 | 28.330 | 11.019 | 0.000 | 0.048 | |
| untreated control 6 hrs | | 29.877 | 22.845 | 28.330 | - | - | - | - |
| 1uM MG132 for 6 hrs | - | 29.877 | 22.134 | 28.330 | - | - | - | - |
| 5uM MG132 for 6 hrs | 36.014 | 29.877 | 22.601 | 28.330 | - | - | - | |
| 10uM MG132 for 6 hrs | 36.808 | 29.877 | 23.078 | 28.330 | 12.183 | 0.000 | 0.022 | 0.725 |
| untreated control 24 hrs | - | 29.877 | 22.426 | 28.330 | - | - | - | - |
| 2.5uM TSA for 24 hrs | 32.336 | 29.877 | 22.124 | 28.330 | 8.665 | 0.002 | 0.246 | 0.026 |
| 1 uM MG132 for 3 hrs followed by 2.5uM TSA for 24hrs | 31.999 | 29.877 | 23.465 | 28.330 | 6.987 | 0.008 | 0.788 | 0.060 |
| 5uM MG132 for 3 hrs followed by 2.5uM TSA for 24hrs | 31.784 | 29.877 | 23.507 | 28.330 | 6.730 | 0.009 | 0.942 | 0.050 |
| 10uM MG132 for 3 hrs followed by 2.5uM TSA for 24hrs | 30.182 | 29.877 | 23.684 | 28.330 | 4.951 | 0.032 | 3.233 | 0.037 |
| 1 uM MG132 for 6 hrs followed by 2.5uM TSA for 24hrs | 31.042 | 29.877 | 22.604 | 28.330 | 6.891 | 0.008 | 0.843 | 0.056 |
| 5uM MG132 for 6 hrs followed by 2.5uM TSA for 24hrs | 29.876 | 29.877 | 22.716 | 28.330 | 5.613 | 0.020 | 2.043 | 0.111 |
| 10uM MG132 for 6 hrs followed by 2.5uM TSA for 24hrs | 29.460 | 29.877 | 23.269 | 28.330 | 14.644 | 10.040 | 4.000 | 0.022 |

**TABLE XVIII. EFFECTS OF A HDAC INHIBITOR AND MG-132 TREATMENT ON GENE EXPRESSION IN AN ADULT PANCREAS-DERIVED CELL LINE.**

| **H5F3P19 Cell Treatment: MG132 followed by TSA** | **Pdx1 Sample Ct** | **Pdx1 Pancreas Ct** | **Sample GAPDH Ct** | **Pancreas GAPDH Ct** | **ΔΔCt** | **2^(ΔΔCt)=RQ** | **% RQ** | **Std err** |
|---|---|---|---|---|---|---|---|---|
| untreated control | - | 27.909 | 19.927 | 25.975 | - | - | - | - |
| 1uM MG132 for 6 hrs | - | 27.909 | 19.311 | 25.975 | - | - | - | - |
| 5uM MG132 for 6 hrs | - | 27.909 | 19.494 | 25.975 | - | - | - | - |
| 10uM MG132 for 6 hrs | - | 27.909 | 19.657 | 25.975 | - | - | - | - |
| 1uM MG132 for 24 hrs | - | 27.909 | 19.446 | 25.975 | - | - | - | - |
| 10uM MG132 for 24 hrs | - | 27.909 | 19.417 | 25.975 | - | - | - | - |
| 1.25uM TSA for 24 hrs | 33.243 | 27.909 | 19.373 | 25.975 | 11.936 | 0.000 | 0.03 | 0.357 |
| 2.5uM TSA for 24 hrs | 32.933 | 27.909 | 21.176 | 25.975 | 9.823 | 0.001 | 0.11 | 0.095 |
| 1uM MG132 for 6 hrs followed by 2.5uM TSA for 24 hrs | - | 27.909 | 22.139 | 25.975 | - | - | - | - |
| 5uM MG132 for 6 hrs followed by 2.5uM TSA for 24 hrs | 32.011 | 27.909 | 21.967 | 25.975 | 8.11 | 0.004 | 0.36 | |
| 10uM MG132 for 6 hrs followed by 2.5uM TSA for 24 hrs | 31.636 | 27.909 | 22.111 | 25.975 | 7.591 | 0.005 | 0.52 | |
| 1uM MG132 for 24 hrs followed by 1.25 TSA for 24hrs | 31.097 | 27.909 | 20.151 | 25.975 | 9.012 | 0.002 | 0.19 | 0.123 |
| 10uM MG132 for 24 hrs followed by 1.25 TSA for 24hrs | 31.191 | 27.909 | 20.096 | 25.975 | 9.161 | 0.002 | 0.17 | 0.125 |
| 1uM MG132 for 24 hrs followed by 2.5 TSA for 24hrs | 30.257 | 27.909 | 19.387 | 25.975 | 8.936 | 0.002 | 0.20 | 0.153 |
| 10uM MG132 for 24 hrs followed by 2.5 TSA for 24hrs | 30.81 | 27.909 | 20.03 | 25.975 | 8.846 | 0.002 | 0.22 | 0.165 |

| **H5F3P19 Cell Treatment: MG132 followed by TSA** | **HNF3-β Sample Ct** | **HNF3-β Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 26.893 | 19.927 | 25.975 | - | - | - | - |
| 1 uM MG132 for 6 hrs | - | 26.893 | 19.311 | 25.975 | - | - | - | - |
| 5uM MG132 for 6 hrs | - | 26.893 | 19.494 | 25.975 | - | - | - | - |
| 10uM MG132 for 6 hrs | - | 26.893 | 19.657 | 25.975 | - | - | - | - |
| 1uM MG132 for 24 hrs | 33.47 | 26.893 | 19.446 | 25.975 | 13.106 | 0.000 | 0.01 | 0.9 |
| 10uM MG132 for 24 hrs | 35.725 | 26.893 | 19.417 | 25.975 | 15.39 | 0.000 | 0.00 | - |
| 1.25uM TSA for 24 hrs | 32.75 | 26.893 | 19.373 | 25.975 | 12.459 | 0.000 | 0.02 | 0.598 |
| 2.5uM TSA for 24 hrs | - | 26,893 | 21.176 | 25.975 | - | - | - | - |
| 1 uM MG132 for 6 hrs followed by 2.5uM TSA for 24 hrs | - | 26.893 | 22.139 | 25.975 | - | - | - | - |
| 5uM MG132 for 6 hrs followed by 2.5uM TSA for 24 hrs | 32.191 | 26.893 | 21.967 | 25.975 | 9.306 | 0.002 | 0.16 | 0.066 |
| 10uM MG132 for 6 hrs followed by 2.5uM TSA for 24 hrs | 32.385 | 26.893 | 22.111 | 25.975 | 9.356 | 0.002 | 0.15 | 0.384 |
| 1 uM MG132 for 24 hrs followed by 1.25 TSA for 24 hrs | 31.771 | 26.893 | 20.151 | 25.975 | 10.702 | 0.001 | 0.06 | 0.384 |
| 10uM MG132 for 24 hrs followed by 1.25 TSA for 24hrs | 28.537 | 26.893 | 20.096 | 25.975 | 7.523 | 0.005 | 0.54 | 0.091 |
| 1 uM MG132 for 24 hrs followed by 2.5 TSA for 24hrs | 31.196 | 26.893 | 19.387 | 25.975 | 10.891 | 0.001 | 0.05 | 0.459 |
| 10uM MG132 for 24 hrs followed by 2.5 TSA for 24hrs | 28.39 | 26.893 | 20.03 | 25.975 | 7.442 | 0.006 | 0.58 | 0.088 |

| **H5F3P19 Cell Treatment: MG132 followed by TSA** | **Insulin Sample Ct** | **Insulin Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 15.946 | 19.927 | 25.975 | - | - | - | - |
| 1uMM G132 for 6 hrs | - | 15.946 | 19.311 | 25.975 | - | - | - | - |
| 5uM MG132 for 6 hrs | - | 15.946 | 19.494 | 25.975 | - | - | - | - |
| 10uM MG132 for 6 hrs | - | 15.946 | 19.657 | 25.975 | - | - | - | - |
| 1uM MG132 for 24 hrs | - | 15.946 | 19.446 | 25.975 | - | 0.000 | 0.00 | - |
| 10uM MG132 for 24 hrs | - | 15.946 | 19.417 | 25.975 | - | - | - | - |
| _ 1.25uM TSA for 24 hrs | - | 15.946 | 19.373 | 25.975 | - | | | |
| 2.5uM TSA for 24 hrs | - | 15.946 | 21.176 | 25.975 | - | - | - | - |
| 1uM MG132 for 6 hrs followed by 2.5uM TSA for 24 hrs | - | 15.946 | 22.139 | 25.975 | - | - | - | - |
| 5uM MG132 for 6 hrs followed by 2.5uM TSA for 24 hrs | - | 15.946 | 21.967 | 25.975 | - | - | - | - |
| 10uM MG132 for 6 hrs followed by 2.5uM TSA for 24 hrs | - | 15.946 | 22.111 | 25.975 | - | - | - | - |
| 1uM MG132 for 24 hrs followed by 1.25 TSA for 24hrs | - | 15.946 | 20.151 | 25.975 | - | - | - | - |
| 10uM MG132 for 24 hrs followed by 1.25 TSA for 24hrs | - | 15.946 | 20.096 | 25.975 | - | - | - | - |
| 1 uM MG132 for 24 hrs followed by 2.5 TSA for 24hrs | - | 15.946 | 19.387 | 25.975 | - | - | - | - |
| 10uM MG132 for 24 hrs followed by 2.5 TSA for 24hrs | - | 15.946 | 20.03 | 25.975 | - | - | - | - |

| **H5F3P19 Cell Treatment: MG132 followed by TSA** | **NKX6.1 Sample Ct** | **NKX6.1 Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 28.463 | 19.927 | 25.975 | - | - | - | - |
| 1 uM MG132 for 6 hrs | - | 28.463 | 19.311 | 25.975 | - | - | - | - |
| 5uM MG132 for 6 hrs | - | 28.463 | 19.494 | 25.975 | - | - | - | - |
| 10uM MG132 for 6 hrs | 35.307 | 28.463 | 19.657 | 25.975 | 14.732 | 0.000 | 0.00 | 0.092 |
| 1uM MG132 for 24 hrs | 33.673 | 28.463 | 19.446 | 25.975 | 24.256 | 0.000 | 5E-06 | 0.187 |
| 10uM MG132 for 24 hrs | - | 28.463 | 19.417 | 25.975 | - | - | - | - |
| 1.25uM TSA for 24 hrs | 29.345 | 28.463 | 19.373 | 25.975 | 9.054 | 0.002 | 0.19 | 0.169 |
| 2.5uM TSA for 24 hrs | 29.898 | 28.463 | 21.176 | 25.975 | 7.804 | 0.004 | 0.45 | 0.217 |
| 1uM MG132 for 6 hrs followed by 2.5uM TSA for 24 hrs | - | 28.463 | 22.139 | 25.975 | - | - | - | - |
| 5uM MG132 for 6 hrs followed by 2.5uM TSA for 24 hrs | 28.976 | 28.463 | 21.967 | 25.975 | 6.091 | 0.015 | 1.47 | 0.06 |
| 10uM MG132 for 6 hrs followed by 2.5uM TSA for 24 hrs | 28.874 | 28.463 | 22.111 | 25.975 | 5.845 | 0.017 | 1.74 | 0.108 |
| 1 uM MG 132 for 24 hrs followed by 1.25 TSA for 24hrs | 28.299 | 28.463 | 20.151 | 25.975 | 7.23 | 0.007 | 0.67 | 0.159 |
| 10uM MG132 for 24 hrs followed by 1.25 TSA for 24hrs . | 27.869 | 28.463 | 20.096 | 25.975 | 6.855 | 0.009 | 0.86 | 0.092 |
| 1uM MG132 for 24 hrs followed by 2.5 TSA for 24hrs | 27.306 | 28.463 | 19.387 | 25.975 | 7.001 | 0.008 | 0.78 | 0.158 |
| 10uM MG132 for 24 hrs followed by 2.5 TSA for 24hrs | 27.516 | 28.463 | 20.03 | 25.975 | 6.568 | 0.011 | 1.05 | 0.093 |

**TABLE XIX. EFFECTS OF A HDAC INHIBITOR AND MG-132 TREATMENT ON GENE EXPRESSION IN AN ADULT PANCREAS-DERIVED CELL LINE.**

| **H5F3P13 Cell Treatment** | **Pdx1 Sample Ct** | **Pdx1 Pancreas Ct** | **Sample GAPDH Ct** | **Pancreas GAPDH Ct** | **ΔΔCt** | **2^(-ΔΔCt)=RQ** | **% RQ** |
|---|---|---|---|---|---|---|---|
| untreated In 2% DMEM for 24 hrs | 38.337 | 31.818 | 25.543 | 31.010 | 11.988 | 0.000 | 0.025 |
| untreated in 2% DMEM for 24 hrs followed by 2% DMEM for 3 days | 37.797 | 31.818 | 25.785 | 31.010 | 11.224 | 0.000 | 0.042 |
| untreated in 2% DMEM for 24 hrs followed by 2% DMEM for 3 days followed by Diff Med #2 for 3 days | 37.892 | 31.818 | 24.877 | 31.010 | 12.208 | 0.000 | 0.021 |
| untreated in 2% DMEM for 24 hrs followed by 2% DMEM for 7 days | 37.045 | 31.618 | 24.869 | 31.010 | 11.368 | 0.000 | 0.038 |
| 1uM MG132 for 6hr in 2% DMEM | - | 31.818 | 24.532 | 31.010 | - | - | - |
| 1uM MG132 for 24hr in 2% DMEM | 39.237 | 31.818 | 25.014 | 31.010 | 13.415 | 0.000 | 0.009 |
| 2.5 uM TSA for 6hr in 2% DMEM | 38.428 | 31.818 | 24.333 | 31.010 | 13.285 | 0.000 | 0.010 |
| 2.5 uM TSA for 24hr in 2% DMEM | 34.854 | 31.819 | 24.767 | 31.010 | 9.279 | 0.002 | 0.161 |
| 2.5uM TSA for 6hr followed by 2% DMEM for 7days | 37.983 | 31.818 | 24.967 | 31.010 | 12.209 | 0.000 | 0.021 |
| 2.5uM TSA for 24hr followed by 2% DMEM for 7days | - | 31.818 | 25.206 | 31.010 | - | - | - |
| 2.5uM TSA for 6hr followed by Diff Med #2 for 7days | 37.075 | 31.818 | 24.013 | 31.010 | 12.255 | 0.000 | 0.020 |
| 2.5uM TSA for 24hr followed by Diff Med #2 for 7days | 39.025 | 31.818 | 24.479 | 31.010 | 13.738 | 0.000 | 0.007 |
| 1uM MG132 for 6hr followed by 2% DMEM for 3 days | 37.269 | 31.818 | 24.908 | 31.010 | 11.552 | 0.000 | 0.033 |
| 1uM MG132 for 24hr followed by 2% DMEM for 3 days | - | 31.818 | 24.508 | 31.010 | - | - | - |
| 1uM MG132 for 6hr followed by 2% DMEM for 7 days | 38.911 | 31.818 | 24.958 | 31.010 | 13.145 | 0.000 | 0.011 |
| 1uM MG132 for 24hr followed by 2% DMEM for 7 days | 38.234 | 31.818 | 25.278 | 31.010 | 12.150 | 0.000 | 0.022 |
| 2.5uM MG132 for 6hr followed by 2% DMEM for 3 days | 38.007 | 31.818 | 24.938 | 31.010 | 12.263 | 0.000 | 0.020 |
| 2.5uM MG132 for 24hr followed by 2% DMEM for 3 days | 37.901 | 31.818 | 25.516 | 31.010 | 11.578 | 0.000 | 0.033 |
| 1uM MG132 for 6hr followed by Diff Med #2 for 3 days | 39.010 | 31.818 | 24.845 | 31.010 | 13.357 | 0.000 | 0.010 |
| 1uM MG132 for 24hr followed by Diff Med #2 for 3 days | - | 31.818 | 24.995 | 31.010 | - | - | - |
| 1uM MG132 for 6hr followed by Diff Med #2 for 7 days | 38.046 | 31.818 | 24.548 | 31.010 | 12.890 | 0.000 | 0.015 |
| 1uM MG132 for 24hr followed by Diff Med #2 for 7 days | 38.621 | 31.818 | 24.658 | 31.010 | 13.157 | 0.000 | 0.011 |
| 2.5uM MG 132 for 6hr followed by Diff Med #2 for 3 days | 37.935 | 31.818 | 25.791 | 31.010 | 11.335 | 0.000 | 0.039 |
| 2.5uM MG132 for 24hr followed by Diff Med #2 for 3 days | 38.798 | 31.818 | 25.194 | 31.010 | 12.795 | 0.000 | 0.014 |
| 1uM MG132 for 6hr followed by 2.5uM TSA for 6hr | 38.120 | 31.818 | 23.972 | 31.010 | 11.340 | 0.000 | 0.039 |
| 1uM MG 132 for 6hr followed by 2.5uM TSA for 24hr | 34.479 | 31.818 | 24.511 | 31.010 | 9.081 | 0.002 | 0.187 |
| 1uM MG132 for 24hr followed by 2.5uM TSA for 6hr | 35.070 | 31.818 | 24.518 | 31.010 | 9.743 | 0.001 | 0.117 |
| 1uM MG132 for 24hr followed by 2.5uM TSA for 24hr | 34.241 | 31.818 | 24.438 | 31.010 | 8.995 | 0.002 | 0.198 |
| 1 uM MG 132 for 6hr followed by 2.5uM TSA for 6hr followed by 2% DMEM for 3 days | 38.802 | 31.818 | 24.801 | 31.010 | 13.183 | 0.000 | 0.011 |
| 1uM MG132 for 6hr followed by 2.5uM TSA for 24hr followed by 2% DMEM for 3 days | 38.746 | 31.818 | 24.676 | 31.010 | 13.282 | 0.000 | 0.010 |
| 1uM MG132 for 24hr followed by 2.5uM TSA for 6hr followed by 2% DMEM for 3 days | 38.425 | 31.818 | 24.705 | 31.010 | 12.912 | 0.000 | 0.013 |
| 1uM MG132 for 24hr followed by 2.5uM TSA for 24hr followed by 2% DMEM for 3 days | 37.637 | 31.818 | 23.958 | 31.010 | 12.871 | 0.000 | 0.013 |
| 1uM MG132 for 6hr followed by 2.5uM TSA for 6hr followed by 2% DMEM for 7 days | - | 31.818 | 23.831 | 31.010 | - | - | - |
| 1uM MG132 for 24hr followed by 2.5uM TSA for 6hr followed by 2% DMEM for 7 days | 38.685 | 31.818 | 24.571 | 31.010 | 13.306 | 0.000 | 0.010 |
| 1uM MG132 for 24hr followed by 2.5uM TSA for 24hr followed by 2% DMEM for 7 days | - | 31.818 | 24.951 | 31.010 | - | - | - |
| 1uM MG132 for 6hr followed by 2.5uM TSA for 6hr followed by Diff Med #2 for 3 days | 38.550 | 31.818 | 25.409 | 31.010 | 12.333 | 0.000 | 0.019 |
| 1uM MG132 for 6hr followed by 2.5uM TSA for 24hr followed by Diff Med #2 for 3 days | 37.667 | 31.818 | 24.583 | 31.010 | 12.298 | 0.000 | 0.020 |
| 1uM MG132 for 6hr followed by 2.5uM TSA for 6hr followed by Diff Med #2 for 7 days | 38.933 | 31.818 | 24.777 | 31.010 | 13.348 | 0.000 | 0.010 |
| 1uM MG132 for 8hr followed by 2.5uM TSA for 24hr followed by Diff Med #2 for 7 days | 37.276 | 31.818 | 24.170 | 31.010 | 12.298 | 0.000 | 0.020 |
| 1uM MG132 for 24hr followed by 2.5uM TSA for 6hr followed by Diff Med #2 for 3 days | 38.992 | 31.818 | 25.133 | 31.010 | 13.052 | 0.000 | 0.012 |
| 1uM MG132 for 24hr followed by 2.5uM TSA for 24hr followed by Diff Med #2 for 3 days | 38.771 | 31.818 | 28.999 | 31,010 | 10.965 | 0.001 | 0.050 |
| 1uM MG132 for 24hr followed by 2.5uM TSA for 6hr followed by Diff Med #2 for 7 days | 37.788 | 31.818 | 24.870 | 31.010 | 12.011 | 0.000 | 0.024 |
| 1uM MG132 for 24hr followed by 2.5uM TSA for 24hr followed by Diff Med #2 for 7 days | 38.805 | 31.818 | 24.862 | 31.010 | 13.135 | 0.000 | 0.011 |

**TABLE XX. EFFECTS OF A HDAC INHIBITOR AND MG-132 TREATMENT ON GENE EXPRESSION IN AN ADULT PANCREAS-DERIVED CELL LINE.**

| **H5F3P15 Cell Treatment: GSI followed by TSA** | **Pdx1 Sample Ct** | **Pdx1 Pancreas Ct** | **Sample GAPDH Ct** | **Pancreas GAPDH Ct** | **ΔΔCT** | **2^(ΔΔCt)=RQ** | **% RQ** | **Std err** |
|---|---|---|---|---|---|---|---|---|
| untreated control | - | 27.909 | 19.788 | 25.975 | - | - | - | - |
| 1uM GSI-IX for 24hrs | - | 27.909 | 20.2 | 25.975 | - | - | - | - |
| 10uM GSI-IX for 24hrs | - | 27.909 | 21.3 | 25.975 | - | - | - | - |
| 1uM GSI-X for 24hrs | - | 27.909 | 20.645 | 25.975 | - | - | - | - |
| 10uM GSI-X for 24hrs | - | 27.909 | 20.151 | 25.975 | - | - | - | - |
| 1.25uM TSA for 24hrs | - | 27.179 | 21.658 | 25.937 | - | - | - | - |
| 2.5uM TSA for 24hrs | 31.020 | 27.179 | 20.985 | 25.937 | 8.79 | 0.002 | 0.23 | 0.197 |
| 1 uM GSI-IX for 24hrs followed by 1.25uM TSA for 24hrs | 33.679 | 27.179 | 21.849 | 25.937 | 10.6 | 0.001 | 0.06 | 0.217 |
| 10uM GSI-IX for 24hrs followed by 1.25uM TSA for 24hrs | 37.125 | 27.179 | 22.004 | 25.937 | 13.9 | 0.000 | 0.01 | 1.372 |
| 1 uM GSI-IX for 24hrs followed by 2.5uM TSA for 24hrs | 31.457 | 27.179 | 21.573 | 25.937 | 8.64 | 0.003 | 0.25 | 0.094 |
| 10uM GSI-IX for 24hrs followed by 2.5uM TSA for 24hrs | 31.156 | 27.179 | 20.99 | 25.937 | 8.92 | 0.002 | 0.21 | 0.175 |
| 1 uM GSI-X for 24hrs followed by 1.25uM TSA for 24hrs | 36.200 | 27.179 | 21.388 | 25.937 | 13.6 | 0.000 | 0.01 | 1.84 |
| 10uM GSI-X for 24hrs followed 1.25uM TSA for 24hrs | 31.579 | 27.179 | 19.682 | 25.937 | 10.7 | 0.001 | 0.06 | 0.182 |
| by 1 uM GSI-X for 24hrs followed by 2.5uM TSA for 24hrs | 30.473 | 27.179 | 20.308 | 25.937 | 8.92 | 0.002 | 0.21 | 0.252 |
| 10uM GSI-X for 24hrs followed by 2.5uM TSA for 24hrs | 31.243 | 27.179 | 20.775 | 25.937 | 9.23 | 0.002 | 0.17 | 0.047 |

| **H5F3P15 Cell Treatment: GSI followed by TSA** | **HNF3-β Sample Ct** | **HNF3-β Pancreas Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| untreated control | - | 26.893 | 19.788 | 25.975 | - | - | - | - |
| 1 uM GSI-IX for 24hrs | - | 26.893 | 20.2 | 25.975 | - | - | - | - |
| 10uM GSI-IX for 24hrs | - | 26.893 | 21.3 | 25.975 | - | - | - | - |
| 1 uM GSI-X for 24hrs | - | 26.893 | 20.645 | 25.975 | - | - | - | - |
| 10uM GSI-X for 24hrs | - | 26.893 | 20.151 | 25.975 | - | - | - | - |
| 1.25uM TSA for 24hrs | 34.563 | 27.109 | 21.658 | 25.937 | 11.7 | 0.000 | 0.03 | 0.533 |
| 2.5uM TSA for 24hrs | 32.406 | 27.109 | 20,985 | 25.937 | 10.2 | 0.001 | 0.08 | 0.314 |
| 1 uM GSI-IX for 24hrs followed by 1.25uM TSA for 24hrs | 34.082 | 27.109 | 21.849 | 25.937 | 11.1 | 0.000 | 0.05 | 0.949 |
| 10uM GSI-IX for 24hrs followed by 1.25uM TSA for 24hrs | 34.849 | 27.109 | 22.004 | 25,937 | 11.7 | 0.000 | 0.03 | 0.428 |
| 1 uM GSI-IX for 24hrs followed by 2.5uM TSA for 24hrs | 32.355 | 27.109 | 21,573 | 25.937 | 9.61 | 0.001 | 0.13 | 0.086 |
| 10uM GSI-IX for 24hrs followed by 2.5uM TSA for 24hrs | 32.290 | 27.109 | 20.99 | 25.937 | 10.1 | 0.001 | 0.09 | 0.25 |
| 1uM GSI-X for 24hrs followed by 1.25uM TSA for 24hrs | 35.057 | 27.109 | 21.388 | 25.937 | 12.5 | 0.000 | 0.02 | |
| 10uM GSI-X for 24hrs followed by 1.25uM TSA for 24hrs | 31,610 | 27.109 | 19.682 | 25.937 | 10.8 | 0.001 | 0.06 | 0.05 |
| 1uM GSI-X for 24hrs followed by 2.5uM TSA for 24hrs | 31.889 | 27.109 | 20.308 | 25.937 | 10.4 | 0.001 | 0.07 | 0.35 |
| 10uM GSI-X for 24hrs followed by 2.5uM TSA for 24hrs | 31.866 | 27.109 | 20.775 | 25.937 | 9.92 | 0.001 | 0.10 | 0.032 |

| **H5F3P15 Cell Treatment: GSI followed by TSA** | **Insulin Sample Ct** | **Insulin Pancreas Ct** | **Sample GAPDH Ct** | **Pancreas GAPDH Ct** | **ΔΔCT** | **2^(-ΔΔCt)=RQ** | **% RQ** | **Std err** |
|---|---|---|---|---|---|---|---|---|
| untreated control | - | 15.946 | 19.788 | 25.975 | - | - | - | - |
| 1uM GSI-IX for 24hrs | - | 15.946 | 20.2 | 25.975 | - | - | - | - |
| 10uM GSI-IX for 24hrs | - | 15.946 | 21.3 | 25.975 | - | - | - | - |
| 1 uM GSI-X for 24hrs | - | 15.946 | 20.645 | 25.975 | - | - | - | - |
| 10uM GSI-X for 24hrs | - | 15.946 | 20.151 | 25.975 | - | - | - | - |
| 1.25uM TSA for 24hrs | - | 15.946 | 21.074 | 25.975 | - | - | - | - |
| 2.5uM TSA for 24hrs | - | 15.692 | 21.375 | 25.975 | - | - | - | - |
| 1 uM GSI-IX for 24hrs followed by 1.25uM TSA for 24hrs | - | 15.692 | 21.849 | 25.937 | - | - | - | - |
| 10uM GSI-IX for 24hrs followed by 1.25uM TSA for 24hrs | - | 15.692 | 22.004 | 25.937 | - | - | - | - |
| 1uM GSI-IX for 24hrs followed by 2.5uM TSA for 24hrs | - | 15.692 | 21.573 | 25.937 | - | - | - | - |
| 10uM GSI-IX for 24hrs followed by 2.5uM TSA for 24hrs | - | 15.692 | 20.99 | 25.937 | - | - | - | - |
| 1uM GSI-X for 24hrs followed by 1.25uM TSA for 24hrs | - | 15.692 | 21.388 | 25.937 | - | - | - | - |
| 10uM GSI-X for 24hrs followed by 1.25uM TSA for 24hrs | - | 15.692 | 19.682 | 25.937 | - | - | - | - |
| 1 uM GSI-X for 24hrs followed by 2.5uM TSA for 24hrs | - | 15.692 | 20.308 | 25.937 | - | - | - | - |
| 10uM GSI-X for 24hrs followed by 2.5uM TSA for 24hrs | - | 15.692 | 20.775 | 25.937 | - | - | - | - |

| **H5F3P15 Cell Treatment: GSI followed by TSA** | **NKX6.1 Sample Ct** | **NKX6.1 Pancreas Ct** | **Sample GAPDH Ct** | **Pancreas GAPDH Ct** | **ΔΔCt** | **2^(ΔΔCt)=RQ** | **% RQ** | **Std err** |
|---|---|---|---|---|---|---|---|---|
| untreated control | - | 28.463 | 19.788 | 25.975 | - | - | - | - |
| 1 uM GSI-IX for 24hrs | - | 28.463 | 20.2 | 25.975 | - | - | - | - |
| 10uM GSI-IX for 24hrs | - | 28.463 | 21.3 | 25.975 | - | - | - | - |
| 1 uM GSI-X for 24hrs | - | 28.463 | 20.645 | 25.975 | - | - | - | - |
| 10uM GSI-X for 24hrs | - | 28.463 | 20.151 | 25.975 | - | - | - | - |
| 1.25uM TSA for 24hrs | 30.915 | 29.081 | 21.658 | 25.937 | 6.11 | 0.014 4 | 1.44 | 0.128 |
| 2.5uM TSA for 24hrs | 31.398 | 29.081 | 20.985 | 25.937 | 7.27 | 0.006 | 0.65 | 2.977 |
| 1uM GSI-IX for 24hrs followed by 1,25uM TSA for 24hrs | 30.351 | 29.081 | 21.849 | 25.937 | 5.36 | 0.024 | 2.44 | 0.212 |
| 10uM GSI-IX for 24hrs followed by 1.25uM TSA for 24hrs | 31.283 | 29.081 | 22.004 | 25.937 | 6.14 | 0.014 | 1.42 | 0.144 |
| 1 uM GSI-IX for 24hrs followed by 2.5uM TSA for 24hrs | 29.346 | 29.081 | 21.573 | 25.937 | 4.63 | 0.040 | 4.04 | 0.108 |
| 10uM GSI-IX for 24hrs followed by 2.5uM TSA for 24hrs | 28.702 | 29.081 | 20.99 | 25.937 | 4.57 | 0.042 | 4.22 | 0.431 |
| 1uM GSI-X for 24hrs followed by 1.25uM TSA for 24hrs | 31.849 | 29.081 | 21.388 | 25.937 | 7.32 | 0.006 | 0.63 | 0.321 |
| 10uM GSI-X for 24hrs followed by 1.25uM TSA for 24hrs | 28.281 | 29.081 | 19.682 | 25.937 | 5.46 | 0.023 | 2.28 | 0.08 |
| 1 uM GSI-X for 24hrs followed by 2.5uM TSA for 24hrs | 28.241 | 29.081 | 20.308 | 25.937 | 4.79 | 0.036 | 3.62 | 0.132 |
| 10uM GSI-X for 24hrs followed by 2.5uM TSA for 24hrs | 28.456 | 29.081 | 20.775 | 25.937 | 4.54 | 0.043 | 4.31 | 0.1 |

**TABLE XXI. EFFECTS OF A HDAC INHIBITOR AND MG-132 TREATMENT ON GENE EXPRESSION IN AN ADULT PANCREAS-DERIVED CELL LINE.**

| **H5F3P16 Cell Treatment** | **Pdx1 Sample Ct** | **Pancreas Pdx1 Ct** | **Sample GAPDH Ct** | **Pancreas GAPDH Ct** | **ΔΔCt** | **2^(-ΔΔCt)=RQ** | **% RQ** | **Std err** |
|---|---|---|---|---|---|---|---|---|
| Untreated 1 day control | - | 27.825 | 20.774 | 24.365 | - | - | - | - |
| 1uM MG132 x 1 day | - | 27.825 | 20.564 | 24.365 | - | - | - | - |
| 5uM MG132 x 1 day | - | 27.825 | 20.693 | 24.365 | - | - | - | - |
| 10uM MG132 x 1 day | - | 27.825 | 20.783 | 24.365 | - | - | - | - |
| Untreated 2 day control | - | 27.825 | 21.227 | 24.365 | - | - | - | - |
| 5uM JNJ 27652677 x 1 day | 35.239 | 27.825 | 22.388 | 24.365 | 9.392 | 1.49E-03 | 0.149 | 0.105 |
| 1uM MG132 x 1 day + 1.25uM JNJ 27652677 x 1 day | 33.611 | 27.825 | 20.536 | 24.365 | 9.615 | 1.28E-03 | 0.128 | 0.245 |
| 1uM MG132 x 1 day + 2.5uM JNJ 27652677 x 1 day | 34.801 | 27.825 | 21.766 | 24.365 | 9.576 | 1.31E-03 | 0.131 | 0.012 |
| 1 uM MG132 x 1 day + 5uM JNJ 27652677 x 1 day | 33.995 | 27.825 | 21.092 | 24.365 | 9.443 | 1.44E-03 | 0.144 | 0.015 |
| 5uM MG132 x 1 day + 1.25uM JNJ 27652677 x 1 day | 35.160 | 27.825 | 24.274 | 24.365 | 7.426 | 5.81 E-03 | 0.581 | 0.063 |
| 5uM MG132 x 1 day + 2.5uM JNJ 27652677 x 1 day | 33.393 | 27.825 | 21.658 | 24.365 | 8.275 | 3.23E-03 | 0.323 | 0.185 |
| 5uM MG132 x 1 day + 5uM JNJ 27652677 x 1 day | 34.363 | 27.825 | 22.892 | 24.365 | 8.011 | 3.88E-03 | 0.388 | 0.387 |
| 10uM MG132 x 1 day + 1.25uM JNJ 27652677 x 1 day | 34.767 | 27.825 | 24.048 | 24.365 | 7.259 | 6.53E-03 | 0.653 | 0.023 |
| 10uM MG132 x 1 day + 2.5uM JNJ 27652677 x 1 day | 36.324 | 27.825 | 25.043 | 24.365 | 7.822 | 4.42E-03 | 0.442 | 0.346 |
| 10uM MG132 x 1 day + 5uM JNJ 27652677 x 1 day | 35.542 | 27.825 | 24.012 | 24.365 | 8.070 | 3.72E-03 | 0.372 | 0.070 |
| 5uM JNJ 28404844 x 1 day | 35.378 | 27.825 | 22.218 | 24.365 | 9.700 | 1.20E-03 | 0.120 | 0.175 |
| 1 uM MG132 x 1 day + 1.25uM JNJ 28404844 x 1 day | 33.580 | 27.825 | 20.354 | 24.365 | 9.766 | 1.15E-03 | 0.115 | 0.083 |
| 1uM MG1 32 x 1 day + 2.5uM JNJ 28404844 x 1 day | 33.186 | 27.825 | 20.163 | 24.365 | 9.563 | 1.32E-03 | 0.132 | 0.005 |
| 1 uM MG132 x 1 day + 5uM JNJ 28404844 x 1 day | 34.044 | 27.825 | 21.446 | 24.365 | 9.139 | 1.77E-03 | 0.177 | 0.073 |
| 5uM MG132 x 1 day + 1.25uM JNJ 28404844 x 1 day | 35.210 | 27.825 | 23.279 | 24.365 | 8.470 | 2.82E-03 | 0.282 | 0.406 |
| 5uM MG132 x 1 day + 2.5uM JNJ 28404844 x 1 day | 34.830 | 27.825 | 23.586 | 24.365 | 7.784 | 4.54E-03 | 0.454 | 0.032 |
| 5uM MG132 x 1 day + 5uM JNJ 28404844 x 1 day | 34.499 | 27.825 | 23.079 | 24.365 | 7.959 | 4.02E-03 | 0.402 | 0.222 |
| 10uM MG132 x 1 day + 1.25uM JNJ 28404844 x 1 day | 35.002 | 27.825 | 23.845 | 24.365 | 7.697 | 4.82E-03 | 0.482 | 0.254 |
| 10uM MG132 x 1 day + 2.5uM JNJ 28404844 x 1 day | 34.394 | 27.825 | 23.502 | 24.365 | 7.432 | 5.79E-03 | 0.579 | 0.258 |
| 10uM MG132 x 1 day + 5uM JNJ 28404844 x 1 day | 35.563 | 27.825 | 23.604 | 24.365 | 8.499 | 2.76E-03 | 0.276 | 0.128 |
| 5uM JNJ 28543944 x 1 day | 34.746 | 27.825 | 21.387 | 24.365 | 9.899 | 1.05E-03 | 0.105 | 0.394 |
| 1uM MG132 x 1 day 1.25uM JNJ 28543944 x 1 day | 35.545 | 27.825 | 23.158 | 24.365 | 8.927 | 2.06E-03 | 0.206 | 0.048 |
| 1 uM MG132 x 1 day + 2.5uM JNJ 28543944 x day | 35677 | 27.825 | 23.075 | 24.365 | 9.142 | 1.77E-03 | 0.177 | 0.315 5 |
| 1 uM MG132 x 1 day + 5uM JNJ 28543944 x 1 day | 36.178 | 27.825 | 23.144 | 24.365 | 9.574 | 1.31 E-03 | 0.131 | 0.379 |
| 5uM MG132 x 1 day + 1.25uM JNJ 28543944 x 1 day | 36.310 | 27.825 | 24.379 | 24.365 | 8.471 | 2.82E-03 | 0.282 | 0.375 |
| 5uM MG132 x 1 day + 2.5uM JNJ 28543944 x 1 day | 36.023 | 27.825 | 23.992 | 24.365 | 8.571 | 2.63E-03 | 0.263 | 0.421 |
| 5uM MG132 x 1 day + 5uM JNJ 28543944 x 1 day | 36.257 | 27.825 | 24.519 | 24.365 | 8.278 | 3.22E-03 | 0.322 | 0.234 |
| 10uM MG132 x 1 day + 1.25uM JNJ 28543944 x 1 day | 36.384 | 27.825 | 24.497 | 24.365 | 8.427 | 2.90E-03 | 0.290 | 0.276 |
| 10uM MG132 x 1 day + 2.5uM JNJ 28543944 x 1 day | 35.638 | 27.825 | 23.767 | 24.365 | 8.411 | 2.94E-03 | 0,294 | 0.383 |
| 10uM MG132 x 1 day + 5uM JNJ 28543944 x 1 day | 36.448 | 27.825 | 24.846 | 24.365 | 8.143 | 3.54E-03 | 0.354 | 0.260 |
| 5uM JNJ 28631590 x 1 day | 36.286 | 27.825 | 21.874 | 24.365 | 10.952 | 5.05E-04 | 0.050 | 0.357 |
| 1 uM MG132 x 1 day + 1.25uM JNJ 28631590 x 1 day | 35.219 | 27.825 | 22.349 | 24.365 | 9.409 | 1.47E-03 | 0.147 | 0.320 |
| 1uM MG132 x 1 day + 2.5uM JNJ28631590 x 1day | 35.248 | 27.825 | 22.024 | 24.365 | 9.764 | 1.15E-03 | 0.115 | 0.104 |
| 1uM MG132 x day + 5uM JNJ 28631590 x 1 day | 36.724 | 27.825 | 22.806 | 24.365 | 10.458 | 7.11E-04 | 0.071 | 0.011 |
| 5uM MG132 x 1 day + 1.25uM JNJ 28631590 x 1 day | 36.048 | 27.825 | 24.140 | 24.365 | 8.448 | 2.86E-03 | 0.286 | 0.438 |
| 5uM MG132 x 1 day + 2.5uM JNJ 28631590 x 1 day | 35.718 | 27.825 | 24.052 | 24.365 | 8.206 | 3.39E-03 | 0.339 | 0.273 |
| 5uM MG132 x 1 day + 5uM JNJ 28631590 x 1 day | 36.514 | 27.825 | 23.948 | 24.365 | 9.106 | 1.81 E-03 | 0.181 | 0.006 |
| 10uM MG132 x 1 day + 1.25uM JNJ 28631590 x 1 day | 34.665 | 27.825 | 22.838 | 24.365 | 8.367 | 3.03E-03 | 0.303 | 0.125 |
| 10uM MG132 x 1 day + 2.5uM JNJ 28631590 x 1 day | 36,250 | 27,825 | 23.627 | 24.365 | 9.163 | 1.74E-03 | 0.174 | 0.439 |
| 10uM MG132 x 1 day + 5uM JNJ 28631590 x 1 day | 37.978 | 27,825 | 26.296 | 24.365 | 8.222 | 3.35E-03 | 0.335 | 0.682 |
| 5uM JNJ 31040074 x 1 day | 34.586 | 27.825 | 20.266 | 24.365 | 10.860 | 5.38E-04 | 0.054 | 0.717 |
| 1uM MG132 x 1 day + 1.25uM JNJ 31040074 x 1 day | 35.977 | 27.825 | 23.329 | 24.365 | 9.188 | 1.71 E-03 | 0.171 | 0.399 |
| 1 uM MG132 x 1 day + 2.5uM JNJ 31040074 x 1 day | 35.865 | 27.825 | 22.948 | 24.365 | 9.457 | 1.42E-03 | 0.142 | 0.379 |
| 1 uM MG132 x 1 day + 5uM JNJ 31040074 x 1 day | 36.714 | 27.825 | 22.858 | 24.365 | 10.397 | 7.42E-04 | 0.074 | 0.076 |
| 5uM MG132 x 1 day + 1.25uM JNJ 31040074 x 1 day | 36.155 | 27.825 | 24.389 | 24.365 | 8.306 | 3.16E-03 | 0.316 | 0.200 |
| 5uM MG132 x 1 day + 2.5uM JNJ 31040074 x 1 day | 36.221 | 27.825 | 23.479 | 24.365 | 9.282 | 1.61 E-03 | 0.161 | 0.093 |
| 5uM MG132 x 1 day + 5uM JNJ 31040074 x 1 day | 36.536 | 27.825 | 23.897 | 24.365 | 9.179 | 1.72E-03 | 0.172 | 0.106 |
| 10uM MG132 x 1 day + 1.25uM JNJ 31040074 x 1 day | 35.878 | 27.825 | 23.845 | 24.365 | 8.573 | 2.63E-03 | 0.263 | 0.607 |
| 10uM MG132 x 1 day + 2.5uM JNJ 31040074 x 1 day | 36.008 | 27.825 | 23.322 | 24.365 | 9.226 | 1.67E-03 | 0.167 | 0.132 |
| 10uM MG132 x 1 day + 5uM JNJ 31040074 x 1 day | 36.468 | 27.825 | 23.552 | 24.365 | 9.457 | 1.42E-03 | 0.142 | 0.358 |
| 5uM JNJ 31053854 x 1 day | 33.632 | 27.825 | 19.947 | 24.365 | 10.225 | 8.36E-04 | 0.084 | 0.185 |
| 1uM MG132 x 1 day + 1.25uM JNJ 31053854 x 1 day | 38.281 | 27.825 | 23.493 | 24.365 | 11.329 | 3.89E-04 | 0.039 | 0.893 |
| 1uM MG132 x 1 day + 2.5uM JNJ 31053854 x 1 day | 36.741 | 27,825 | 23.468 | 24.365 | 9.813 | 1.11E-03 | 0.111 | 0.272 |
| 1uM MG132 x 1 day + 5uM JNJ 31053854 x 1 day | 36.485 | 27.825 | 22.937 | 24.365 | 10.088 | 9.19E-04 | 0.092 | 0.078 |
| 5uM MG132 x 1 day + 1.25uM JNJ 31053854 x 1 day | 36.628 | 27.825 | 24.771 | 24.365 | 8.397 | 2.97E-03 | 0.297 | 0.321 |
| 5uM MG132 x 1 day + 2.5uM JNJ 31053854 x 1 day | 35.302 | 27.825 | 23.764 | 24.365 | 8.078 | 3.70E-03 | 0.370 | 0.215 |
| 5uM MG132 x 1 day + 5uM JNJ 31053854 x 1 day | 35.769 | 27.825 | 23.740 | 24.365 | 8.569 | 2.63E-03 | 0.263 | 0.234 |
| 10uM MG132 x 1 day + 1.25uM JNJ 31053854 x 1 day | 36.324 | 27.825 | 25.037 | 24.365 | 7.826 | 4.41E-03 | 0.441 | 0.001 |
| 10uM MG132 x 1 day + 2.5uM JNJ 31053854 x 1 day | 34.539 | 27.825 | 23.207 | 24.365 | 7.872 | 4.27E-03 | 0.427 | 0.170 |
| 10uM MG132 x 1 day + 5uM JNJ 31053854 x 1 day | 35.660 | 27.825 | 23.908 | 24.365 | 8.292 | 3.19E-03 | 0.319 | 0.031 |
| 1 uM MG132x1day+1.25uM TSA x 1 day | - | 27.825 | 24.100 | 24.365 | - | - | - | - |
| 1uM MG132 x 1 day + 2.5uM TSA x 1 day | 36.059 | 27.825 | 23.226 | 24.365 | 9.373 | 1.51 E-03 | 0.151 | 0.028 |
| 5uM MG132 x 1 day + 1.25uM TSA x 1 day | 34.793 | 27.825 | 23.465 | 24.365 | 7.868 | 4.28E-03 | 0.428 | 0.386 |
| 5uM MG132 x 1 day + 2.5uM TSA x 1 day | 35.403 | 27.825 | 23.796 | 24.365 | 8.148 | 3.53E-03 | 0.353 | 0.551 |
| 10um MG132 x 1 day + 1.25uM TSA x 1 day | 33.002 | 27.825 | 22.010 | 24.365 | 7.532 | 5.40E-03 | 0.540 | 0.127 |
| 10um MG132 x 1 day + 2.5uM TSA x 1 day | 35.353 | 27.825 | 23.703 | 24.365 | 8.190 | 3.42E-03 | 0.342 | 0.214 |

**TABLE XXII. EFFECTS OF A HDAC INHIBITOR AND MG-132 TREATMENT ON GENE EXPRESSION IN AN ADULT PANCREAS-DERIVED CELL LINE.**

| **H5F3P16 Cell Treatment** | **Insulin Sample Ct** | **Pancreas Insulin Ct** | **Sample GAPDH Ct** | **Pancreas GAPDH Ct** | **ΔΔCt** | **2^(ΔΔdCt)=RQ** | **% RQ** | **Std** |
|---|---|---|---|---|---|---|---|---|
| Untreated 1 day control | - | 19.401 | 20.774 | 24.365 | - | - | - | - |
| 1uM MG132 x 1 day | - | 19.401 | 20.564 | 24.365 | - | - | - | - |
| 5uM MG132 x 1 day | - | 19.401 | 20.693 | 24.365 | - | - | - | - |
| 10uM MG132 x 1 day | - | 19.401 | 20.783 | 24.365 | - | - | - | - |
| Untreated 2 day control | - | 19,401 | 21.227 | 24.365 | - | - | - | - |
| 5uM JNJ 27652677 x 1 day | - | 19.401 | 22.388 | 24.365 | - | - | - | - |
| 1uM MG132 x 1 day + 1.25uM JNJ 27652677 x 1 day | - | 19.401 | 20.536 | 24.365 | - | - | - | - |
| 1 uM MG132 x 1 day + 2.5uM JNJ 27652677 x 1 day | - | 19.401 | 21.766 | 24.365 | - | - | - | - |
| 1 uM MG132 x 1 day + 5uM JNJ 27652677 x 1 day | - | 19.401 | 21.092 | 24.365 | - | - | - | - |
| 5uM MG132 x 1 day + 1.25uM JNJ 27652677 x 1 day | - | 19.401 | 24.274 | 24.365 | - | - | - | - |
| 5uM MG132 x 1 day + 2.5uM JNJ 27652677 x 1 day | - | 19.401 | 21.658 | 24.365 | - | - | - | - |
| 5uM MG132 x 1 day + 5uM JNJ 27652677 x 1 day | - | 19.401 | 22.892 | 24.365 | - | - | - | - |
| 10uM MG132 x 1 day + 1.25uM JNJ 27652677 x 1 day | - | 19.401 | 24.048 | 24.365 | - | - | - | - |
| 10uM MG132 x 1 day + 2.5uM JNJ 27652677 x 1 day | - | 19.401 | 25.043 | 24.365 | - | - | - | - |
| 10um MG132 x 1 day + 5uM JNJ 27652677 x 1 day | - | 19.401 | 24.012 | 24.365 | - | - | - | - |
| 5uM JNJ 28404844 x 1 day | - | 19.401 | 22.218 | 24.365 | - | - | - | - |
| 1uM MG132 x 1 day + 1.25uM JNJ 28404844 x 1 day | - | 19.401 | 20.354 | 24.365 | - | - | - | - |
| 1uM MG132 x 1 day + 2.5uM JNJ 28404844 x 1 day | - | 19.401 | 20.163 | 24.365 | - | - | - | - |
| 1uM MG132 x 1 day+ 5uM JNJ 28404844 x 1 day | - | 19.401 | 21.446 | 24.365 | - | - | - | - |
| 5uM MG132 x 1 day + 1.25uM JNJ 28404844 x 1 day | - | 19.401 | 23.279 | 24.365 | - | - | - | - |
| 5uM MG132 x 1 day + 2.5uM JNJ 28404844 x 1 day | - | 19.401 | 23.586 | 24.365 | - | - | - | - |
| 5uM MG132 x 1 day + 5uM JNJ 28404844 x 1 day | - | 19.401 | 23.079 | 24.365 | - | - | - | - |
| 10uMMG132x1day+1.25uM JNJ 28404844 x 1 day | - | 19.401 | 23.845 | 24.365 | - | - | - | - |
| 10uM MG132 x 1 day + 2.5uM JNJ 28404844 x 1 day | - | 19.401 | 23.502 | 24.365 | - | - | - | - |
| 10uM MG132 x 1 day + 5uM JNJ 28404844 x 1 day | - | 19.401 | 23.604 | 24.365 | - | - | - | - |
| 5uM JNJ 28543944 x 1 day | - | 19.401 | 21.387 | 24.365 | - | - | - | - |
| 1 uM MG132 x 1 day + 1.25uM JNJ 28543944 x 1 day | - | 19.401 | 23.158 | 24.365 | - | - | - | - |
| 1 uM MG132 x 1 day + 2.5uM JNJ 28543944 x 1 day | - | 19.401 | 23.075 | 24.365 | - | - | - | - |
| 1 uM MG132 x 1 day + 5uM JNJ 28543944 x 1 day | - | 19.401 | 23.144 | 24.365 | - | - | - | - |
| 5uM MG132 x 1 day + 1.25uM JNJ 28543944 x 1 day | - | 19.401 | 24.379 | 24.365 | - | - | - | - |
| 5uM MG132 x 1 day + 2.5uM JNJ 28543944 x 1 day | - | 19.401 | 23.992 | 24.365 | - | - | - | - |
| 5uM MG132 x 1 day + 5uM JNJ 28543944 x 1 day | - | 19.401 | 24.519 | 24.365 | - | - | - | - |
| 10uM MG132 x 1 day + 1.25uM JNJ 28543944 x 1 day | - | 19.401 | 24.497 | 24.365 | - | - | - | - |
| 10uM MG132 x 1 day + 2.5uM JNJ 28543944 x 1 day | - | 19.401 | 23.767 | 24.365 | - | - | - | - |
| 10uM MG132 x 1 day + 5uM JNJ 28543944 x 1 day | - | 19.401 | 24.846 | 24.365 | - | - | - | - |
| 5uM JNJ 28631590 x 1 day | - | 19.401 | 21.874 | 24.365 | - | - | - | - |
| 1 uM MG132 x 1 day + 1.25uM JNJ 28631590 x 1 day | - | 19.401 | 22.349 | 24.365 | - | - | - | - |
| 1 uM MG132 x 1 day + 2.5uM JNJ 28631590 x 1 day | - | 19.401 | 22.024 | 24.365 | - | - | - | - |
| 1 uM MG132 x 1 day + 5uM JNJ 28631590 x 1 day | - | 19.401 | 22.806 | 24,365 | - | - | - | - |
| 5uM MG132 x 1 day + 1.25uM JNJ 28631590 x 1 day | 38.603 | 19.401 | 24.140 | 24.365 | 19.427 | 1.42E-06 | 0.000 | 0.314 |
| 5uM MG132 x 1 day + 2.5uM JNJ 28631590 x 1 day | - | 19.401 | 24.052 | 24.365 | - | - | - | - |
| 5uM MG132 x 1 day + 5uM JNJ 28631590 x 1 day | - | 19.401 | 23.948 | 24.365 | - | - | - | - |
| 10uM MG132 x 1 day + 1.25uM JNJ 28631590 x 1 day | - | 19.401 | 22.838 | 24.365 | - | - | - | - |
| lout MG132 x 1 day + 2,5uM JNJ 28631590 x 1 day | - | 19.401 | 23.627 | 24.365 | - | - | - | - |
| 10uM MG132 x 1 day + 5uM JNJ 28631590 x 1 day | - | 19.401 | 26.296 | 24.365 | - | - | - | - |
| 5uM JNJ 31040074 x 1 day | - | 19.401 | 20.266 | 24.365 | - | - | - | - |
| 1uM MG132 x 1 day + 1.25uM JNJ 31040074 x 1 day | 38.645 | 19.401 | 23.329 | 24.365 | 20.280 | 7.85E-07 | 0.000 | 0.413 |
| 1 uM MG132 x 1 day + 2.5uM JNJ 31040074 x 1 day | - | 19.401 | 22.948 | 24.365 | - | - | - | - |
| 1uM MG132 x 1 day + 5uM JNJ 31040074 x 1 day | - | 19.401 | 22,858 | 24.365 | - | - | - | - |
| 5uM MG132 x 1 day + 1.25uM JNJ 31040074 x 1 day | - | 19.401 | 24.389 | 24.365 | - | - | - | - |
| 5uM MG132 x 1 day + 2.5uM JNJ 31040074 x 1 day | - | 19.401 | 23.479 | 24.365 | - | - | - | - |
| 5uM MG132 x 1 day + 5uM JNJ 31040074 x 1 day | - | 19.401 | 23.897 | 24.365 | - | - | - | - |
| 10uM MG132 x 1 day + 1.25uM JNJ 31040074 x 1 day | - | 19.401 | 23.845 | 24.365 | - | - | - | - |
| 10uM MG132 x 1 day + 2.5uM JNJ 31040074 x 1 day | - | 19.401 | 23.322 | 24.365 | - | - | - | - |
| 10uM MG132 x 1 day + 5uM JNJ 31040074 x 1 day | - | 19.401 | 23.552 | 24.365 | - | - | - | - |
| 5uM JNJ 31053854 x 1 day | - | 19.401 | 19.947 | 24.365 | - | - | - | - |
| 1 uM MG132 x 1 day + 1.25uM JNJ 31053854 x 1 day | - | 19.401 | 23.493 | 24.365 | - | - | - | - |
| 1 uM MG132 x 1 day + 2.5uM JNJ 31053854 x 1 day | - | 19.401 | 23.468 | 24.365 | - | - | - | - |
| 1uM MG132 x 1 day + 5uM JNJ 31053854 x 1 day | - | 19.401 | 22.937 | 24.365 | - | - | - | - |
| 5uM MG132 x 1 day + 1.25uM JNJ 31053854 x 1 day | - | 19.401 | 24.771 | 24.365 | - | - | - | - |
| 5uM MG132 x 1 day + 2.5uM JNJ 31053854 x 1 day | 38.070 | 19.401 | 23.764 | 24.365 | 19.270 | 1.58E-06 | 0.000 | 0.222 |
| 5uM MG132 x 1 day + 5uM JNJ 31053854 x 1 day | - | 19.401 | 23.740 | 24.365 | - | - | - | - |
| 10uM MG132 x 1 day + 1.25uM JNJ 31053854 x 1 day | - | 19.401 | 25.037 | 24.365 | - | - | - | - |
| 10uM MG132 x 1 day + 2.5uM JNJ 31053854 x 1 day | - | 19.401 | 23.207 | 24.365 | - | - | - | - |
| 10uM MG132 x 1 day + 5uM JNJ 31053854 x 1 day | - | 19.401 | 23.908 | 24.365 | - | - | - | - |
| 1uMMG132x1day+1.25uMTSAx1day | - | 19.401 | 24.100 | 24.365 | - | - | - | - |
| 1 uM MG132 x 1 day + 2.5uM TSA x 1 day | - | 19.401 | 23.226 | 24.365 | - | - | - | - |
| 5uM MG132 x 1 day + 1.25uM TSA x 1 day | - | 19.401 | 23.465 | 24.365 | - | - | - | - |
| 5uM MG132 x 1 day + 2.5uM TSA x 1 day | - | 19.401 | 23.796 | 24.365 | - | - | - | - |
| 10uM MG132 x 1 day + 1.25uM TSA x 1 day | - | 19.401 | 22.010 | 24.365 | - | - | - | - |
| 10uM MG132 x 1 day + 2.5uM TSA x 1 day | - | 19.401 | 23.703 | 24.365 | - | - | - | - |

**TABLE XXIII. EFFECTS OF A HDAC INHIBITOR AND A DNA-DEMETHYLATING AGENT TREATMENT ON GENE EXPRESSION IN AN AMNIOTIC FLUID-DERIVED CELL LINE.**

| **AFCA010-B-P8 Cells Treatment** | **Pdx1 Ct** | **Pdx1 RQ** | **HNF3-β Ct** | **HNF3-β RQ** |
|---|---|---|---|---|
| no treatment | nd | | nd | |
| 5-AZA 0.156uM | nd | | nd | |
| 5-AZA 0.3125uM | nd | | nd | |
| 5-AZA 0.625uM | nd | | nd | |
| 5-AZA 1.25uM | nd | | nd | |
| 5-AZA 2.5uM | nd | | nd | |
| TSA 1.25uM | 35.318 | 0.001 | 34.029 | 0.001 |
| Human pancreas control | 30.342 | 1.000 | 29.054 | 1.000 |

**TABLE XXIV. EFFECTS OF A HDAC INHIBITOR AND A DNA-DEMETHYLATING AGENT TREATMENT ON GENE EXPRESSION IN AN AMNIOTIC FLUID-DERIVED CELL LINE.**

| **AFCA010-B-P8 Cells Treatment** | **Pdx1 Ct** | **Pdx1 RQ** | **HNF3-β Ct** | **HNF3-β RQ** |
|---|---|---|---|---|
| no treatment | nd | | nd | |
| 5-AZA 0.156uM | nd | | nd | |
| 5-AZA 0.3125uM | nd | | nd | |
| 5-AZA 0.625uM | nd | | nd | |
| 5-AZA 1.25uM | nd | | nd | |
| 5-AZA 2.5uM | nd | | nd | |
| TSA 1.25uM | 35.318 | 0.001 | 34.029 | 0.001 |
| TSA 1.25uM with 5-AZA 0.156uM | 34.469 | 0.002 | 33.734 | 0.001 |
| TSA 1.25uM with 5-AZA 0.3125uM | 34.415 | 0.002 | 33.598 | 0.001 |
| TSA 1.25uM with 5-AZA 0.625uM | 34.117 | 0.003 | 33.600 | 0.002 |
| TSA 1.25uM with 5-AZA 1.25uM | 33.347 | 0.002 | 33.347 | 0.003 |
| TSA 1.25uM with 5-AZA 2.5uM | 33.231 | 0.002 | 33.061 | 0.001 |
| Human pancreas control | 30.342 | 1.000 | 29.054 | 1.000 |

**TABLE XXV. EFFECTS OF A HDAC INHIBITOR AND A DNA-DEMETHYLATING AGENT TREATMENT ON GENE EXPRESSION IN AN AMNIOTIC FLUID-DERIVED CELL LINE.**

| **AFCA010-B-P12 Cells Treatment** | | | **Pdx1 Ct** | **Pdx1 RQ** | **HNF3-β Ct** | **HNF3-β RQ** |
|---|---|---|---|---|---|---|
| **Part 1 (0-6 hrs)** | | **Part 2 (7-24 hrs)** | | | | |
| no treatment | | no treatment | nd | | nd | |
| TSA 1.25uM | | TSA 1.25uM | 34.427 | 9.94E-04 | 32.522 | 0.002 |
| TSA 2.5uM | | TSA 2.5uM | 31.145 | 0.011 | 31.144 | 0.006 |
| 5-AZA | 2.5uM | 5-AZA 2.5uM | nd | | nd | |
| 5-AZA | 5.0uM | 5-AZA 5.0uM | nd | | nd | |
| 5-AZA | 2.5uM | TSA 1.25uM | 32.52 | 0.004 | 31.242 | 0.005 |
| 5-AZA | 2.5uM | TSA 2.5uM | 30.515 | 0.013 | 30.348 | 0.008 |
| 5-AZA | 5.0uM | TSA 1.25uM | 35.241 | 0.001 | 32.451 | 0.002 |
| 5-AZA | 5.0uM | TSA 2.5uM | 30.543 | 0.013 | 29.853 | 0.012 |
| Human pancreas control | | | 28.802 | 1.000 | 27.962 | 1.000 |

Publications cited throughout this document are hereby incorporated by reference in their entirety. Although the various aspects of the invention have been illustrated above by reference to examples and preferred embodiments, it will be appreciated that the scope of the invention is defined not by the foregoing description, but by the following claims properly construed under principles of patent law.

## Claims

1. An in-vitro method for increasing the expression of PDX-1 in cells, comprising the steps of:
a. Providing the cells, and
b. Contacting the cells with at least one chromatin-remodeling agent, wherein the at least one chromatin-remodeling agent increases the expression of PDX-1 within the cells.

2. The method of claim 1, wherein the cells are selected from the group consisting of an undifferentiated cell, a partially differentiated cell, a fully differentiated cell, a stem cell and a progenitor cell.

3. The method of claim 1, wherein the cells do not express PDX-1 prior to the treatment with the at least one chromatin-remodeling agent.

4. The method of claim 1, wherein the treatment of at least one chromatin-remodeling agent restores the expression of PDX-1.

5. The method of claim 1, wherein the at least one chromatin-remodeling agent is an inhibitor ofhistone deacetylase activity.

6. The method of claim 5, wherein the inhibitor is selected from the group consisting of butyrates, hydroxamic acids, cyclic peptides and benzamides.

7. The method of claim 5, wherein the inhibitor is selected from the group consisting ofvalproic acid, 4-phenylbutyrate, sodium butyrate, trichostatin A, suberoyl anilide hydroxamic acid (SAHA), oxamflatin, trapoxin B, FR901228, apicidin, chlamydocin, depuecin, scriptaid, depsipeptide, and N-acetyldinaline.

8. An in-vitro method for promoting the differentiation of cells into a pancreatic progenitor cell, comprising the steps of:
a. Providing cells that do not express PDX-1, and
b. Contacting the cells with at least one chromatin-remodeling agent, wherein the at least one chromatin-remodeling agent increases the expression of PDX-1.

9. The method of claim 8, wherein the treatment of the cells with the at least one chromatin-remodeling agent causes increases in the expression of at least one of the genes HNF-3 beta or Sox-17.

10. The method of claim 8, wherein the cells are selected from the group consisting of an undifferentiated cell, a partially differentiated cell, a fully differentiated cell, a stem cell and a progenitor cell.

11. The method of claim 8, wherein the cells do not express PDX-1 prior to the treatment with the at least one chromatin-remodeling agent.

12. The method of claim 8, wherein the treatment of at least one chromatin-remodeling agent restores the expression of PDX-1.

13. The method of claim 8, wherein the at least one chromatin-remodeling agent is an inhibitor of histone deacetylase activity.

14. The method of claim 13, wherein the inhibitor is selected from the group consisting of butyrates, hydroxamic acids, cyclic peptides and benzamides.

15. The method of claim 13, wherein the inhibitor is selected from the group consisting of valproic acid, 4-phenylbutyrate, sodium butyrate, trichostatin A, suberoyl anilide hydroxamic acid (SAHA), oxamflatin, trapoxin B, FR901228, apicidin, chlamydocin, depuecin, scriptaid, depsipeptide, and N-acetyldinaline.

16. An in-vitro method for promoting the differentiation of cells into a pancreatic hormone-secreting cell, comprising the steps of:
a. Providing cells that do not express PDX-1, and
b. Contacting the cells with at least one chromatin-remodeling agent, wherein the chromatin-remodeling agent increases the expression of PDX-1.

17. The method of claim 16, wherein the treatment of the cells with the at least one chromatin-remodeling agent causes increases in the expression of at least one of the genes HNF-3 beta, Sox-17, insulin, glucagon, or somatostatin.

18. The method of claim 16, wherein the cells are selected from the group consisting of an undifferentiated cell, a partially differentiated cell, a fully differentiated cell, a stem cell and a progenitor cell.

19. The method of claim 16, wherein the cells do not express PDX-1 prior to the treatment with the at least one chromatin-remodeling agent.

20. The method of claim 16, wherein the treatment of at least one chromatin-remodeling agent restores the expression of PDX-1.

21. The method of claim 16, wherein the at least one chromatin-remodeling agent is an inhibitor of histone deacetylase activity.

22. The method of claim 21, wherein the inhibitor is selected from the group consisting of butyrates, hydroxamic acids, cyclic peptides and benzamides.

23. The method of claim 21, wherein the inhibitor is selected from the group consisting of valproic acid, 4-phenylbutyrate, sodium butyrate, trichostatin A, suberoyl anilide hydroxamic acid (SAHA), oxamflatin, trapoxin B, FR901228, apicidin, chlamydocin, depuecin, scriptaid, depsipeptide, and N-acetyldinaline.

## Patentansprüche

1. In-vitro-Verfahren zur Erhöhung der Expression von PDX-1 in Zellen, mit den folgenden Schritten:
a. Bereitstellen der Zellen und
b. Inkontaktbringen der Zellen mit wenigstens einem Chromatin-Remodellierungsagens, wobei das wenigstens eine Chromatin-Remodellierungsagens die Expression von PDX-1 innerhalb der Zellen erhöht.

2. Verfahren nach Anspruch 1, wobei die Zellen ausgewählt sind aus der Gruppe bestehend aus einer undifferenzierten Zelle, einer teilweise ausdifferenzierten Zelle, einer vollständig ausdifferenzierten Zelle, einer Stammzelle und einer Progenitorzelle.

3. Verfahren nach Anspruch 1, wobei die Zellen vor der Behandlung mit dem wenigstens einen Chromatin-Remodellierungsagens kein PDX-1 exprimieren.

4. Verfahren nach Anspruch 1, wobei die Expression von PDX-1 durch die Behandlung mit wenigstens einem Chromatin-Remodellierungsagens wiederhergestellt wird.

5. Verfahren nach Anspruch 1, wobei es sich bei dem wenigstens einen Chromatin-Remodellierungsagens um einen Inhibitor von Histon-Deacetylase-Aktivität handelt.

6. Verfahren nach Anspruch 5, wobei der Inhibitor ausgewählt ist aus der Gruppe bestehend aus Butyraten, Hydroxamsäuren, cyclischen Peptiden und Benzamiden.

7. Verfahren nach Anspruch 5, wobei der Inhibitor ausgewählt ist aus der Gruppe bestehend aus Valproinsäure, 4-Phenylbutyrat, Natriumbutyrat, Trichostatin A, Suberoylanilid-Hydroxamsäure (SAHA), Oxamflatin, Trapoxin B, FR901228, Apicidin, Chlamydocin, Depuecin, Scriptaid, Depsipeptid und N-Acetyldinalin.

8. In-vitro-Verfahren zur Förderung der Differenzierung von Zellen zu einer Pankreas-Progenitorzelle, mit den folgenden Schritten:
a. Bereitstellen von Zellen, die PDX-1 nicht exprimieren, und
b. Inkontaktbringen der Zellen mit wenigstens einem Chromatin-Remodellierungsagens, wobei das wenigstens eine Chromatin-Remodellierungsagens die Expression von PDX-1 erhöht.

9. Verfahren nach Anspruch 8, wobei die Behandlung der Zellen mit dem wenigstens einen Chromatin-Remodellierungsagens die Expression wenigstens eines der Gene HNF-3-beta oder Sox-17 erhöht.

10. Verfahren nach Anspruch 8, wobei die Zellen ausgewählt sind aus der Gruppe bestehend aus einer undifferenzierten Zelle, einer teilweise ausdifferenzierten Zelle, einer vollständig ausdifferenzierten Zelle, einer Stammzelle und einer Progenitorzelle.

11. herfahren nach Anspruch 8, wobei die Zellen vor der Behandlung mit dem wenigstens einen Chromatin-Remodellierungsagens kein PDX-1 exprimieren.

12. herfahren nach Anspruch 8, wobei die Expression von PDX-1 durch die Behandlung mit wenigstens einem Chromatin-Remodellierungsagens wiederhergestellt wird.

13. Verfahren nach Anspruch 8, wobei es sich bei dem wenigstens einen Chromatin-Remodellierungsagens um einen Inhibitor von Histon-Deacetylase-Aktivität handelt.

14. herfahren nach Anspruch 13, wobei der Inhibitor ausgewählt ist aus der Gruppe bestehend aus Butyraten, Hydroxamsäuren, cyclischen Peptiden und Benzamiden.

15. herfahren nach Anspruch 13, wobei der Inhibitor ausgewählt ist aus der Gruppe bestehend aus Valproinsäure, 4-Phenylbutyrat, Natriumbutyrat, Trichostatin A, Suberoylanilid-Hydroxamsäure (SAHA), Oxamflatin, Trapoxin B, FR901228, Apicidin, Chlamydocin, Depuecin, Scriptaid, Depsipeptid und N-Acetyldinalin.

16. In-vitro-Verfahren zur Förderung der Differenzierung von Zellen zu einer Pankreas-Hormonsekretionszelle, mit den folgenden Schritten:
a. Bereitstellen von Zellen, die PDX-1 nicht exprimieren, und
b. Inkontaktbringen der Zellen mit wenigstens einem Chromatin-Remodellierungsagens, wobei das Chromatin-Remodellierungsagens die Expression von PDX-1 erhöht.

17. herfahren nach Anspruch 16, wobei die Behandlung der Zellen mit dem wenigstens einen Chromatin-Remodellierungsagens die Expression wenigstens eines der Gene HNF-3-beta, Sox-17, Insulin, Glucagon oder Somatostatin erhöht.

18. Verfahren nach Anspruch 16, wobei die Zellen ausgewählt sind aus der Gruppe bestehend aus einer undifferenzierten Zelle, einer teilweise ausdifferenzierten Zelle, einer vollständig ausdifferenzierten Zelle, einer Stammzelle und einer Progenitorzelle.

19. herfahren nach Anspruch 16, wobei die Zellen vor der Behandlung mit dem wenigstens einen Chromatin-Remodellierungsagens kein PDX-1 exprimieren.

20. Verfahren nach Anspruch 16, wobei die Expression von PDX-1 durch die Behandlung mit wenigstens einem Chromatin-Remodellierungsagens wiederhergestellt wird.

21. herfahren nach Anspruch 16, wobei es sich bei dem wenigstens einen Chromatin-Remodellierungsagens um einen Inhibitor von Histon-Deacetylase-Aktivität handelt.

22. Verfahren nach Anspruch 21, wobei der Inhibitor ausgewählt ist aus der Gruppe bestehend aus Butyraten, Hydroxamsäuren, cyclischen Peptiden und Benzamiden.

23. Verfahren nach Anspruch 21, wobei der Inhibitor ausgewählt ist aus der Gruppe bestehend aus Valproinsäure, 4-Phenylbutyrat, Natriumbutyrat, Trichostatin A, Suberoylanilid-Hydroxamsäure (SAHA), Oxamflatin, Trapoxin B, FR901228, Apicidin, Chlamydocin, Depuecin, Scriptaid, Depsipeptid und N-Acetyldinalin.

## Revendications

1. Procédé *in vitro* pour augmenter l'expression du PDX-1 dans des cellules, comprenant les étapes de :
a. mise à disposition des cellules, et
b. mise en contact des cellules avec au moins un agent de remodelage de la chromatine, l'au moins un agent de remodelage de la chromatine augmentant l'expression du PDX-1 à l'intérieur des cellules.

2. Procédé de la revendication 1, dans lequel les cellules sont choisies dans le groupe consistant en une cellule non différenciée, une cellule partiellement différenciée, une cellule entièrement différenciée, une cellule souche et une cellule progénitrice.

3. Procédé de la revendication 1, dans lequel les cellules n'expriment pas le PDX-1 avant le traitement avec l'au moins un agent de remodelage de la chromatine.

4. Procédé de la revendication 1, dans lequel le traitement d'au moins un agent de remodelage de la chromatine rétablit l'expression du PDX-1.

5. Procédé de la revendication 1, dans lequel l'au moins un agent de remodelage de la chromatine est un inhibiteur de l'activité d'histone-désacétylase;

6. Procédé de la revendication 5, dans lequel l'inhibiteur est choisi dans le groupe consistant en les butyrates, les acides hydroxamiques, les peptides cycliques et les benzamides.

7. Procédé de la revendication 5, dans lequel l'inhibiteur est choisi dans le groupe consistant en l'acide valproïque, le 4-phénylbutyrate, le butyrate de sodium, la trichostatine A, l'acide subéroylanilide-hydroxamide (SAHA), l'oxamflatine, la trapoxine B, le FR901228, l'apicidine, la chlamydocine, la dépuécine, le scriptaïde, le depsipeptide et la N-acétyldinaline.

8. Procédé *in vitro* pour promouvoir la différenciation de cellules en une cellule progénitrice pancréatique, comprenant les étapes de :
a. mise à disposition de cellules qui n'expriment pas le PDX-1, et
b. mise en contact des cellules avec au moins un agent de remodelage de la chromatine, l'au moins un agent de remodelage de la chromatine augmentant l'expression du PDX-1.

9. Procédé de la revendication 8, dans lequel le traitement des cellules avec l'au moins un agent de remodelage de la chromatine provoque des augmentations de l'expression d'au moins l'un des gènes HNF-3 bêta ou Sox-17.

10. Procédé de la revendication 8, dans lequel les cellules sont choisies dans le groupe consistant en une cellule non différenciée, une cellule partiellement différenciée, une cellule entièrement différenciée, une cellule souche et une cellule progénitrice.

11. Procédé de la revendication 8, dans lequel les cellules n'expriment pas le PDX-1 avant le traitement avec l'au moins un agent de remodelage de la chromatine.

12. Procédé de la revendication 8, dans lequel le traitement d'au moins un agent de remodelage de la chromatine rétablit l'expression du PDX-1.

13. Procédé de la revendication 8, dans lequel l'au moins un agent de remodelage de la chromatine est un inhibiteur de l'activité d'histone-désacétylase.

14. Procédé de la revendication 13, dans lequel l'inhibiteur est choisi dans le groupe consistant en les butyrates, les acides hydroxamiques, les peptides cycliques et les benzamides.

15. Procédé de la revendication 13, dans lequel l'inhibiteur est choisi dans le groupe consistant en l'acide valproïque, le 4-phénylbutyrate, le butyrate de sodium, la trichostatine A, l'acide subéroylanilide-hydroxamide (SAHA), l'oxamflatine, la trapoxine B, le FR901228, l'apicidine, la chlamydocine, la dépuécine, le scriptaïde, le depsipeptide et la N-acétyldinaline.

16. Procédé *in vitro* pour promouvoir la différenciation de cellules en une cellule sécrétant des hormones pancréatiques, comprenant les étapes de :
a. mise à disposition de cellules qui n'expriment pas le PDX-1, et
b. mise en contact des cellules avec au moins un agent de remodelage de la chromatine, l'agent de remodelage de la chromatine augmentant l'expression du PDX-1.

17. Procédé de la revendication 16, dans lequel le traitement des cellules avec l'au moins un agent de remodelage de la chromatine provoque des augmentations de l'expression d'au moins l'un des gènes HNF-3 bêta, Sox-17, insuline, glucagon ou somatostatine.

18. Procédé de la revendication 16, dans lequel les cellules sont choisies dans le groupe consistant en une cellule non différenciée, une cellule partiellement différenciée, une cellule entièrement différenciée, une cellule souche et une cellule progénitrice.

19. Procédé de la revendication 16, dans lequel les cellules n'expriment pas le PDX-1 avant le traitement avec l'au moins un agent de remodelage de la chromatine.

20. Procédé de la revendication 16, dans lequel le traitement d'au moins un agent de remodelage de la chromatine rétablit l'expression du PDX-1.

21. Procédé de la revendication 16, dans lequel l'au moins un agent de remodelage de la chromatine est un inhibiteur de l'activité d'histone-désacétylase.

22. Procédé de la revendication 21, dans lequel l'inhibiteur est choisi dans le groupe consistant en les butyrates, les acides hydroxamiques, les peptides cycliques et les benzamides.

23. Procédé de la revendication 21, dans lequel l'inhibiteur est choisi dans le groupe consistant en l'acide valproïque, le 4-phénylbutyrate, le butyrate de sodium, la trichostatine A, l'acide subéroylanilide-hydroxamide (SAHA), l'oxamflatine, la trapoxine B, le FR901228, l'apicidine, la chlamydocine, la dépuécine, le scriptaïde, le depsipeptide et la N-acétyldinaline.
